(19) Europäisches Patentamt
European Patent Office
Office européen des brevets

(11) EP 3 992 290 A1

(12) EUROPEAN PATENT APPLICATION
published in accordance with Art. 153(4) EPC

(43) Date of publication:
**04.05.2022 Bulletin 2022/18**

(21) Application number: **20815633.1**

(22) Date of filing: **21.05.2020**

(51) International Patent Classification (IPC):
***C12N 15/113*** *(2010.01)* ***A61K 31/713*** *(2006.01)*
***A61K 47/28*** *(2006.01)* ***A61P 9/10*** *(2006.01)*

(52) Cooperative Patent Classification (CPC):
**A61K 31/713; A61K 47/28; A61P 9/10; C12N 15/113**

(86) International application number:
**PCT/CN2020/091606**

(87) International publication number:
**WO 2020/238758 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.05.2019 CN 201910441597**

(71) Applicant: **Suzhou Ribo Life Science Co., Ltd.**
**Kunshan, Jiangsu 215300 (CN)**

(72) Inventors:
- **ZHANG, Hongyan**
  **Suzhou, Jiangsu 215300 (CN)**
- **GAO, Shan**
  **Suzhou, Jiangsu 215300 (CN)**
- **KANG, Daiwu**
  **Suzhou, Jiangsu 215300 (CN)**

(74) Representative: **SSM Sandmair Patentanwälte Rechtsanwalt Partnerschaft mbB**
**Joseph-Wild-Straße 20**
**81829 München (DE)**

# (54) NUCLEIC ACID, PHARMACEUTICAL COMPOSITION AND CONJUGATE, PREPARATION METHOD THEREFOR AND USE THEREOF

(57) Provided are an siRNA for inhibiting gene expression of plasma prekallikrein (PKK), a pharmaceutical composition containing the siRNA and an siRNA conjugate. Also provided is the use of the siRNA or the pharmaceutical composition thereof or the siRNA conjugate in the preparation of drugs for treating and/or preventing inflammatory diseases or thromboembolic diseases.

Figure 3

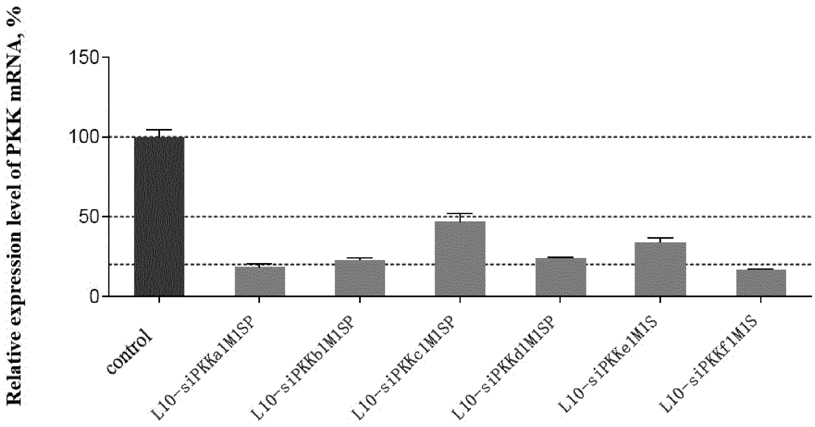


EP 3 992 290 A1

Sequence Listing

<110>  SUZHOU RIBO LIFE SCIENCE CO., LTD.

<120>  NUCLEIC ACID, PHARMACEUTICAL COMPOSITION AND CONJUGATE, PREPARATION METHOD THEREFOR AND USE THEREOF
<130>  DSP1P201009ZX

<150>  CN 201910441597.6
<151>  2019-05-24

<160>  405

<170>  SIPOSequenceListing 1.0

<210>  1
<211>  19
<212>  RNA
<213>  Artificial Sequence

<400>  1
gguguuugcu auucaguuu    19

<210>  2
<211>  19
<212>  RNA
<213>  Artificial Sequence

<400>  2
aaacugaaua gcaaacacc    19

<210>  3
<211>  19
<212>  RNA
<213>  Artificial Sequence

<220>
<221>  misc_feature
<222>  (19)
<223>  n=a, or g, or c, or u

<400>  3
gguguuugcu auucaguun    19

<210>  4
<211>  19
<212>  RNA
<213>  Artificial Sequence

<220>
<221>  misc_feature
<222>  (1)
<223>  n=a, or g, or c, or u

<400>  4
naacugaaua gcaaacacc    19

<210>  5
<211>  19
<212>  RNA
<213>  Artificial Sequence

<220>
<221> misc_feature
<222> (19)
<223> n=a, or g, or c, or u

<400> 5
gguguuugcu auucaguun 19

<210> 6
<211> 21
<212> RNA
<213> Artificial Sequence

<220>
<221> misc_feature
<222> (1)
<223> n=a, or g, or c, or u

<400> 6
naacugaaua gcaaacaccu u 21

<210> 7
<211> 21
<212> RNA
<213> Artificial Sequence

<220>
<221> misc_feature
<222> (21)
<223> n=a, or g, or c, or u

<400> 7
aagguguuug cuauucaguu n 21

<210> 8
<211> 23
<212> RNA
<213> Artificial Sequence

<220>
<221> misc_feature
<222> (1)
<223> n=a, or g, or c, or u

<400> 8
naacugaaua gcaaacaccu ugg 23

<210> 9
<211> 19
<212> RNA
<213> Artificial Sequence

<400> 9
gguguuugcu auucaguuu 19

<210> 10
<211> 21
<212> RNA

<213>  Artificial Sequence

<400>  10
aaacugaaua gcaaacaccu u    21

<210>  11
<211>  21
<212>  RNA
<213>  Artificial Sequence

<400>  11
aagguguuug cuauucaguu u    21

<210>  12
<211>  23
<212>  RNA
<213>  Artificial Sequence

<400>  12
aaacugaaua gcaaacaccu ugg    23

<210>  13
<211>  19
<212>  RNA
<213>  Artificial Sequence

<400>  13
gguguuugcu auucaguuu    19

<210>  14
<211>  21
<212>  RNA
<213>  Artificial Sequence

<400>  14
aaacugaaua gcaaacaccu u    21

<210>  15
<211>  19
<212>  RNA
<213>  Artificial Sequence

<400>  15
gguguuugcu auucaguuu    19

<210>  16
<211>  21
<212>  RNA
<213>  Artificial Sequence

<400>  16
aaacugaaua gcaaacaccu u    21

<210>  17
<211>  19
<212>  RNA
<213>  Artificial Sequence

<400>  17
gguguuugcu auucaguuu    19

<210>  18
<211>  21

<212> RNA
<213> Artificial Sequence

<400> 18
aaacugaaua gcaaacaccu u 21

<210> 19
<211> 21
<212> RNA
<213> Artificial Sequence

<400> 19
aagguguuug cuauucaguu u 21

<210> 20
<211> 23
<212> RNA
<213> Artificial Sequence

<400> 20
aaacugaaua gcaaacaccu ugg 23

<210> 21
<211> 21
<212> RNA
<213> Artificial Sequence

<400> 21
aagguguuug cuauucaguu u 21

<210> 22
<211> 23
<212> RNA
<213> Artificial Sequence

<400> 22
aaacugaaua gcaaacaccu ugg 23

<210> 23
<211> 21
<212> RNA
<213> Artificial Sequence

<400> 23
aagguguuug cuauucaguu u 21

<210> 24
<211> 23
<212> RNA
<213> Artificial Sequence

<400> 24
aaacugaaua gcaaacaccu ugg 23

<210> 25
<211> 19
<212> RNA
<213> Artificial Sequence

<400> 25
gguguuugcu auucaguuu 19

<210> 26

<211> 21
<212> RNA
<213> Artificial Sequence

<400> 26
aaacugaaua gcaaacaccu u 21

<210> 27
<211> 19
<212> RNA
<213> Artificial Sequence

<400> 27
gguguuugcu auucaguuu 19

<210> 28
<211> 21
<212> RNA
<213> Artificial Sequence

<400> 28
aaacugaaua gcaaacaccu u 21

<210> 29
<211> 19
<212> RNA
<213> Artificial Sequence

<400> 29
gguguuugcu auucaguuu 19

<210> 30
<211> 21
<212> RNA
<213> Artificial Sequence

<400> 30
aaacugaaua gcaaacaccu u 21

<210> 31
<211> 21
<212> RNA
<213> Artificial Sequence

<400> 31
aagguguuug cuauucaguu u 21

<210> 32
<211> 23
<212> RNA
<213> Artificial Sequence

<400> 32
aaacugaaua gcaaacaccu ugg 23

<210> 33
<211> 21
<212> RNA
<213> Artificial Sequence

<400> 33
aagguguuug cuauucaguu u 21

<210> 34
<211> 23
<212> RNA
<213> Artificial Sequence

<400> 34
aaacugaaua gcaaacaccu ugg 23

<210> 35
<211> 21
<212> RNA
<213> Artificial Sequence

<400> 35
aagguguuug cuauucaguu u 21

<210> 36
<211> 23
<212> RNA
<213> Artificial Sequence

<400> 36
aaacugaaua gcaaacaccu ugg 23

<210> 37
<211> 19
<212> RNA
<213> Artificial Sequence

<400> 37
gguguuugcu auucaguuu 19

<210> 38
<211> 21
<212> RNA
<213> Artificial Sequence

<400> 38
aaacugaaua gcaaacaccu u 21

<210> 39
<211> 19
<212> RNA
<213> Artificial Sequence

<400> 39
gguguuugcu auucaguuu 19

<210> 40
<211> 21
<212> RNA
<213> Artificial Sequence

<400> 40
aaacugaaua gcaaacaccu u 21

<210> 41
<211> 19
<212> RNA
<213> Artificial Sequence

<400> 41
gguguuugcu auucaguuu 19

<210> 42
<211> 21
<212> RNA
<213> Artificial Sequence

<400> 42
aaacugaaua gcaaacaccu u 21

<210> 43
<211> 21
<212> RNA
<213> Artificial Sequence

<400> 43
aagguguuug cuauucaguu u 21

<210> 44
<211> 23
<212> RNA
<213> Artificial Sequence

<400> 44
aaacugaaua gcaaacaccu ugg 23

<210> 45
<211> 21
<212> RNA
<213> Artificial Sequence

<400> 45
aagguguuug cuauucaguu u 21

<210> 46
<211> 23
<212> RNA
<213> Artificial Sequence

<400> 46
aaacugaaua gcaaacaccu ugg 23

<210> 47
<211> 21
<212> RNA
<213> Artificial Sequence

<400> 47
aagguguuug cuauucaguu u 21

<210> 48
<211> 23
<212> RNA
<213> Artificial Sequence

<400> 48
aaacugaaua gcaaacaccu ugg 23

<210> 49
<211> 19
<212> RNA
<213> Artificial Sequence

<400> 49

ggguguuugcu auucaguuu 19

<210> 50
<211> 21
<212> RNA
<213> Artificial Sequence

<400> 50
aaacugaaua gcaaacaccu u 21

<210> 51
<211> 19
<212> RNA
<213> Artificial Sequence

<400> 51
ggguguuugcu auucaguuu 19

<210> 52
<211> 21
<212> RNA
<213> Artificial Sequence

<400> 52
aaacugaaua gcaaacaccu u 21

<210> 53
<211> 19
<212> RNA
<213> Artificial Sequence

<400> 53
ggguguuugcu auucaguuu 19

<210> 54
<211> 21
<212> RNA
<213> Artificial Sequence

<400> 54
aaacugaaua gcaaacaccu u 21

<210> 55
<211> 21
<212> RNA
<213> Artificial Sequence

<400> 55
aagguguuug cuauucaguu u 21

<210> 56
<211> 23
<212> RNA
<213> Artificial Sequence

<400> 56
aaacugaaua gcaaacaccu ugg 23

<210> 57
<211> 21
<212> RNA
<213> Artificial Sequence

<400> 57
aagguguuug cuauucaguu u 21

<210> 58
<211> 23
<212> RNA
<213> Artificial Sequence

<400> 58
aaacugaaua gcaaacaccu ugg 23

<210> 59
<211> 21
<212> RNA
<213> Artificial Sequence

<400> 59
aagguguuug cuauucaguu u 21

<210> 60
<211> 23
<212> RNA
<213> Artificial Sequence

<400> 60
aaacugaaua gcaaacaccu ugg 23

<210> 61
<211> 19
<212> RNA
<213> Artificial Sequence

<400> 61
gcuucuugaa agauagugu 19

<210> 62
<211> 19
<212> RNA
<213> Artificial Sequence

<400> 62
acacuaucuu ucaagaagc 19

<210> 63
<211> 19
<212> RNA
<213> Artificial Sequence

<220>
<221> misc_feature
<222> (19)
<223> n=a, or g, or c, or u

<400> 63
gcuucuugaa agauagugn 19

<210> 64
<211> 19
<212> RNA
<213> Artificial Sequence

<220>

<221> misc_feature
<222> (1)
<223> n=a, or g, or c, or u

<400> 64
ncacuaucuu ucaagaagc 19

<210> 65
<211> 19
<212> RNA
<213> Artificial Sequence

<220>
<221> misc_feature
<222> (19)
<223> n=a, or g, or c, or u

<400> 65
gcuucuugaa agauagugn 19

<210> 66
<211> 21
<212> RNA
<213> Artificial Sequence

<220>
<221> misc_feature
<222> (1)
<223> n=a, or g, or c, or u

<400> 66
ncacuaucuu ucaagaagca a 21

<210> 67
<211> 21
<212> RNA
<213> Artificial Sequence

<220>
<221> misc_feature
<222> (21)
<223> n=a, or g, or c, or u

<400> 67
uugcuucuug aaagauagug n 21

<210> 68
<211> 23
<212> RNA
<213> Artificial Sequence

<220>
<221> misc_feature
<222> (1)
<223> n=a, or g, or c, or u

<400> 68
ncacuaucuu ucaagaagca acc 23

<210> 69
<211> 19
<212> RNA
<213> Artificial Sequence

<400> 69
gcuucuugaa agauagugu 19

<210> 70
<211> 21
<212> RNA
<213> Artificial Sequence

<400> 70
acacuaucuu ucaagaagca a 21

<210> 71
<211> 21
<212> RNA
<213> Artificial Sequence

<400> 71
uugcuucuug aaagauagug u 21

<210> 72
<211> 23
<212> RNA
<213> Artificial Sequence

<400> 72
acacuaucuu ucaagaagca acc 23

<210> 73
<211> 19
<212> RNA
<213> Artificial Sequence

<400> 73
gcuucuugaa agauagugu 19

<210> 74
<211> 21
<212> RNA
<213> Artificial Sequence

<400> 74
acacuaucuu ucaagaagca a 21

<210> 75
<211> 19
<212> RNA
<213> Artificial Sequence

<400> 75
gcuucuugaa agauagugu 19

<210> 76
<211> 21
<212> RNA
<213> Artificial Sequence

<400> 76

acacuaucuu ucaagaagca a 21

<210> 77
<211> 19
<212> RNA
<213> Artificial Sequence

<400> 77
gcuucuugaa agauagugu 19

<210> 78
<211> 21
<212> RNA
<213> Artificial Sequence

<400> 78
acacuaucuu ucaagaagca a 21

<210> 79
<211> 21
<212> RNA
<213> Artificial Sequence

<400> 79
uugcuucuug aaagauagug u 21

<210> 80
<211> 23
<212> RNA
<213> Artificial Sequence

<400> 80
acacuaucuu ucaagaagca acc 23

<210> 81
<211> 21
<212> RNA
<213> Artificial Sequence

<400> 81
uugcuucuug aaagauagug u 21

<210> 82
<211> 23
<212> RNA
<213> Artificial Sequence

<400> 82
acacuaucuu ucaagaagca acc 23

<210> 83
<211> 21
<212> RNA
<213> Artificial Sequence

<400> 83
uugcuucuug aaagauagug u 21

<210> 84
<211> 23
<212> RNA
<213> Artificial Sequence

<400> 84
acacuaucuu ucaagaagca acc 23

<210> 85
<211> 19
<212> RNA
<213> Artificial Sequence

<400> 85
gcuucuugaa agauagugu 19

<210> 86
<211> 21
<212> RNA
<213> Artificial Sequence

<400> 86
acacuaucuu ucaagaagca a 21

<210> 87
<211> 19
<212> RNA
<213> Artificial Sequence

<400> 87
gcuucuugaa agauagugu 19

<210> 88
<211> 21
<212> RNA
<213> Artificial Sequence

<400> 88
acacuaucuu ucaagaagca a 21

<210> 89
<211> 19
<212> RNA
<213> Artificial Sequence

<400> 89
gcuucuugaa agauagugu 19

<210> 90
<211> 21
<212> RNA
<213> Artificial Sequence

<400> 90
acacuaucuu ucaagaagca a 21

<210> 91
<211> 21
<212> RNA
<213> Artificial Sequence

<400> 91
uugcuucuug aaagauagug u 21

<210> 92
<211> 23
<212> RNA
<213> Artificial Sequence

<400> 92
acacuaucuu ucaagaagca acc 23

<210> 93
<211> 21
<212> RNA
<213> Artificial Sequence

<400> 93
uugcuucuug aaagauagug u 21

<210> 94
<211> 23
<212> RNA
<213> Artificial Sequence

<400> 94
acacuaucuu ucaagaagca acc 23

<210> 95
<211> 21
<212> RNA
<213> Artificial Sequence

<400> 95
uugcuucuug aaagauagug u 21

<210> 96
<211> 23
<212> RNA
<213> Artificial Sequence

<400> 96
acacuaucuu ucaagaagca acc 23

<210> 97
<211> 19
<212> RNA
<213> Artificial Sequence

<400> 97
gcuucuugaa agauagugu 19

<210> 98
<211> 21
<212> RNA
<213> Artificial Sequence

<400> 98
acacuaucuu ucaagaagca a 21

<210> 99
<211> 19
<212> RNA
<213> Artificial Sequence

<400> 99
gcuucuugaa agauagugu 19

<210> 100
<211> 21
<212> RNA

<213> Artificial Sequence

<400> 100
acacuaucuu ucaagaagca a 21

<210> 101
<211> 19
<212> RNA
<213> Artificial Sequence

<400> 101
gcuucuugaa agauagugu 19

<210> 102
<211> 21
<212> RNA
<213> Artificial Sequence

<400> 102
acacuaucuu ucaagaagca a 21

<210> 103
<211> 21
<212> RNA
<213> Artificial Sequence

<400> 103
uugcuucuug aaagauagug u 21

<210> 104
<211> 23
<212> RNA
<213> Artificial Sequence

<400> 104
acacuaucuu ucaagaagca acc 23

<210> 105
<211> 21
<212> RNA
<213> Artificial Sequence

<400> 105
uugcuucuug aaagauagug u 21

<210> 106
<211> 23
<212> RNA
<213> Artificial Sequence

<400> 106
acacuaucuu ucaagaagca acc 23

<210> 107
<211> 21
<212> RNA
<213> Artificial Sequence

<400> 107
uugcuucuug aaagauagug u 21

<210> 108
<211> 23

<212> RNA
<213> Artificial Sequence

<400> 108
acacuaucuu ucaagaagca acc 23

<210> 109
<211> 19
<212> RNA
<213> Artificial Sequence

<400> 109
gcuucuugaa agauagugu 19

<210> 110
<211> 21
<212> RNA
<213> Artificial Sequence

<400> 110
acacuaucuu ucaagaagca a 21

<210> 111
<211> 19
<212> RNA
<213> Artificial Sequence

<400> 111
gcuucuugaa agauagugu 19

<210> 112
<211> 21
<212> RNA
<213> Artificial Sequence

<400> 112
acacuaucuu ucaagaagca a 21

<210> 113
<211> 19
<212> RNA
<213> Artificial Sequence

<400> 113
gcuucuugaa agauagugu 19

<210> 114
<211> 21
<212> RNA
<213> Artificial Sequence

<400> 114
acacuaucuu ucaagaagca a 21

<210> 115
<211> 21
<212> RNA
<213> Artificial Sequence

<400> 115
uugcuucuug aaagauagug u 21

<210> 116

<211> 23
<212> RNA
<213> Artificial Sequence

<400> 116
acacuaucuu ucaagaagca acc 23

<210> 117
<211> 21
<212> RNA
<213> Artificial Sequence

<400> 117
uugcuucuug aaagauagug u 21

<210> 118
<211> 23
<212> RNA
<213> Artificial Sequence

<400> 118
acacuaucuu ucaagaagca acc 23

<210> 119
<211> 21
<212> RNA
<213> Artificial Sequence

<400> 119
uugcuucuug aaagauagug u 21

<210> 120
<211> 23
<212> RNA
<213> Artificial Sequence

<400> 120
acacuaucuu ucaagaagca acc 23

<210> 121
<211> 19
<212> RNA
<213> Artificial Sequence

<400> 121
gaagcaaugu ggucaucaa 19

<210> 122
<211> 19
<212> RNA
<213> Artificial Sequence

<400> 122
uugaugacca cauugcuuc 19

<210> 123
<211> 19
<212> RNA
<213> Artificial Sequence

<220>
<221> misc_feature
<222> (19)

<223> n=a, or g, or c, or u

<400> 123
gaagcaaugu ggucaucan 19

<210> 124
<211> 19
<212> RNA
<213> Artificial Sequence

<220>
<221> misc_feature
<222> (1)
<223> n=a, or g, or c, or u

<400> 124
nugaugacca cauugcuuc 19

<210> 125
<211> 19
<212> RNA
<213> Artificial Sequence

<220>
<221> misc_feature
<222> (19)
<223> n=a, or g, or c, or u

<400> 125
gaagcaaugu ggucaucan 19

<210> 126
<211> 21
<212> RNA
<213> Artificial Sequence

<220>
<221> misc_feature
<222> (1)
<223> n=a, or g, or c, or u

<400> 126
nugaugacca cauugcuuca a 21

<210> 127
<211> 21
<212> RNA
<213> Artificial Sequence

<220>
<221> misc_feature
<222> (21)
<223> n=a, or g, or c, or u

<400> 127
uugaagcaau guggucauca n 21

<210> 128

<211> 23
<212> RNA
<213> Artificial Sequence

<220>
<221> misc_feature
<222> (1)
<223> n=a, or g, or c, or u

<400> 128
nugaugacca cauugcuuca agg 23

<210> 129
<211> 19
<212> RNA
<213> Artificial Sequence

<400> 129
gaagcaaugu ggucaucaa 19

<210> 130
<211> 21
<212> RNA
<213> Artificial Sequence

<400> 130
uugaugacca cauugcuuca a 21

<210> 131
<211> 21
<212> RNA
<213> Artificial Sequence

<400> 131
uugaagcaau guggucauca a 21

<210> 132
<211> 23
<212> RNA
<213> Artificial Sequence

<400> 132
uugaugacca cauugcuuca agg 23

<210> 133
<211> 19
<212> RNA
<213> Artificial Sequence

<400> 133
gaagcaaugu ggucaucaa 19

<210> 134
<211> 21
<212> RNA
<213> Artificial Sequence

<400> 134
uugaugacca cauugcuuca a 21

<210> 135
<211> 19

<212> RNA
<213> Artificial Sequence

<400> 135
gaagcaaugu ggucaucaa 19

<210> 136
<211> 21
<212> RNA
<213> Artificial Sequence

<400> 136
uugaugacca cauugcuuca a 21

<210> 137
<211> 19
<212> RNA
<213> Artificial Sequence

<400> 137
gaagcaaugu ggucaucaa 19

<210> 138
<211> 21
<212> RNA
<213> Artificial Sequence

<400> 138
uugaugacca cauugcuuca a 21

<210> 139
<211> 21
<212> RNA
<213> Artificial Sequence

<400> 139
uugaagcaau guggucauca a 21

<210> 140
<211> 23
<212> RNA
<213> Artificial Sequence

<400> 140
uugaugacca cauugcuuca agg 23

<210> 141
<211> 21
<212> RNA
<213> Artificial Sequence

<400> 141
uugaagcaau guggucauca a 21

<210> 142
<211> 23
<212> RNA
<213> Artificial Sequence

<400> 142
uugaugacca cauugcuuca agg 23

<210> 143

<211>  21
<212>  RNA
<213>  Artificial Sequence

<400>  143
uugaagcaau guggucauca a                                                21

<210>  144
<211>  23
<212>  RNA
<213>  Artificial Sequence

<400>  144
uugaugacca cauugcuuca agg                                              23

<210>  145
<211>  19
<212>  RNA
<213>  Artificial Sequence

<400>  145
gaagcaaugu ggucaucaa                                                   19

<210>  146
<211>  21
<212>  RNA
<213>  Artificial Sequence

<400>  146
uugaugacca cauugcuuca a                                                21

<210>  147
<211>  19
<212>  RNA
<213>  Artificial Sequence

<400>  147
gaagcaaugu ggucaucaa                                                   19

<210>  148
<211>  21
<212>  RNA
<213>  Artificial Sequence

<400>  148
uugaugacca cauugcuuca a                                                21

<210>  149
<211>  19
<212>  RNA
<213>  Artificial Sequence

<400>  149
gaagcaaugu ggucaucaa                                                   19

<210>  150
<211>  21
<212>  RNA
<213>  Artificial Sequence

<400>  150
uugaugacca cauugcuuca a                                                21

<210> 151
<211> 21
<212> RNA
<213> Artificial Sequence

<400> 151
uugaagcaau gguggucauca a 21

<210> 152
<211> 23
<212> RNA
<213> Artificial Sequence

<400> 152
uugaugacca cauugcuuca agg 23

<210> 153
<211> 21
<212> RNA
<213> Artificial Sequence

<400> 153
uugaagcaau guggucauca a 21

<210> 154
<211> 23
<212> RNA
<213> Artificial Sequence

<400> 154
uugaugacca cauugcuuca agg 23

<210> 155
<211> 21
<212> RNA
<213> Artificial Sequence

<400> 155
uugaagcaau guggucauca a 21

<210> 156
<211> 23
<212> RNA
<213> Artificial Sequence

<400> 156
uugaugacca cauugcuuca agg 23

<210> 157
<211> 19
<212> RNA
<213> Artificial Sequence

<400> 157
gaagcaaugu ggucaucaa 19

<210> 158
<211> 21
<212> RNA
<213> Artificial Sequence

<400> 158
uugaugacca cauugcuuca a 21

<210> 159
<211> 19
<212> RNA
<213> Artificial Sequence

<400> 159
gaagcaaugu ggucaucaa 19

<210> 160
<211> 21
<212> RNA
<213> Artificial Sequence

<400> 160
uugaugacca cauugcuuca a 21

<210> 161
<211> 19
<212> RNA
<213> Artificial Sequence

<400> 161
gaagcaaugu ggucaucaa 19

<210> 162
<211> 21
<212> RNA
<213> Artificial Sequence

<400> 162
uugaugacca cauugcuuca a 21

<210> 163
<211> 21
<212> RNA
<213> Artificial Sequence

<400> 163
uugaagcaau guggucauca a 21

<210> 164
<211> 23
<212> RNA
<213> Artificial Sequence

<400> 164
uugaugacca cauugcuuca agg 23

<210> 165
<211> 21
<212> RNA
<213> Artificial Sequence

<400> 165
uugaagcaau guggucauca a 21

<210> 166
<211> 23
<212> RNA
<213> Artificial Sequence

<400> 166

uugaugacca cauugcuuca agg 23

<210> 167
<211> 21
<212> RNA
<213> Artificial Sequence

<400> 167
uugaagcaau guggucauca a 21

<210> 168
<211> 23
<212> RNA
<213> Artificial Sequence

<400> 168
uugaugacca cauugcuuca agg 23

<210> 169
<211> 19
<212> RNA
<213> Artificial Sequence

<400> 169
gaagcaaugu ggucaucaa 19

<210> 170
<211> 21
<212> RNA
<213> Artificial Sequence

<400> 170
uugaugacca cauugcuuca a 21

<210> 171
<211> 19
<212> RNA
<213> Artificial Sequence

<400> 171
gaagcaaugu ggucaucaa 19

<210> 172
<211> 21
<212> RNA
<213> Artificial Sequence

<400> 172
uugaugacca cauugcuuca a 21

<210> 173
<211> 19
<212> RNA
<213> Artificial Sequence

<400> 173
gaagcaaugu ggucaucaa 19

<210> 174
<211> 21
<212> RNA
<213> Artificial Sequence

<400> 174
uugaugacca cauugcuuca a 21

<210> 175
<211> 21
<212> RNA
<213> Artificial Sequence

<400> 175
uugaagcaau guggucauca a 21

<210> 176
<211> 23
<212> RNA
<213> Artificial Sequence

<400> 176
uugaugacca cauugcuuca agg 23

<210> 177
<211> 21
<212> RNA
<213> Artificial Sequence

<400> 177
uugaagcaau guggucauca a 21

<210> 178
<211> 23
<212> RNA
<213> Artificial Sequence

<400> 178
uugaugacca cauugcuuca agg 23

<210> 179
<211> 21
<212> RNA
<213> Artificial Sequence

<400> 179
uugaagcaau guggucauca a 21

<210> 180
<211> 23
<212> RNA
<213> Artificial Sequence

<400> 180
uugaugacca cauugcuuca agg 23

<210> 181
<211> 19
<212> RNA
<213> Artificial Sequence

<400> 181
cacaaagaaa uguuugucu 19

<210> 182
<211> 19
<212> RNA
<213> Artificial Sequence

<400> 182
agacaaacau uucuuugug 19

<210> 183
<211> 19
<212> RNA
<213> Artificial Sequence

<220>
<221> misc_feature
<222> (19)
<223> n=a, or g, or c, or u

<400> 183
cacaaagaaa uguuugucn 19

<210> 184
<211> 19
<212> RNA
<213> Artificial Sequence

<220>
<221> misc_feature
<222> (1)
<223> n=a, or g, or c, or u

<400> 184
ngacaaacau uucuuugug 19

<210> 185
<211> 19
<212> RNA
<213> Artificial Sequence

<220>
<221> misc_feature
<222> (19)
<223> n=a, or g, or c, or u

<400> 185
cacaaagaaa uguuugucn 19

<210> 186
<211> 21
<212> RNA
<213> Artificial Sequence

<220>
<221> misc_feature
<222> (1)
<223> n=a, or g, or c, or u

<400> 186
ngacaaacau uucuuuguga c 21

<210> 187
<211> 21
<212> RNA

<213> Artificial Sequence

<220>
<221> misc_feature
<222> (21)
<223> n=a, or g, or c, or u

<400> 187
gucacaaaga aauguuuguc n 21

<210> 188
<211> 23
<212> RNA
<213> Artificial Sequence

<220>
<221> misc_feature
<222> (1)
<223> n=a, or g, or c, or u

<400> 188
ngacaaacau uucuuuguga cuc 23

<210> 189
<211> 19
<212> RNA
<213> Artificial Sequence

<400> 189
cacaaagaaa uguuugucu 19

<210> 190
<211> 21
<212> RNA
<213> Artificial Sequence

<400> 190
agacaaacau uucuuuguga c 21

<210> 191
<211> 21
<212> RNA
<213> Artificial Sequence

<400> 191
gucacaaaga aauguuuguc u 21

<210> 192
<211> 23
<212> RNA
<213> Artificial Sequence

<400> 192
agacaaacau uucuuuguga cuc 23

<210> 193
<211> 19
<212> RNA
<213> Artificial Sequence

<400> 193
cacaaagaaa uguuugucu 19

<210> 194
<211> 21
<212> RNA
<213> Artificial Sequence

<400> 194
agacaaacau uucuuuguga c 21

<210> 195
<211> 19
<212> RNA
<213> Artificial Sequence

<400> 195
cacaaagaaa uguuugucu 19

<210> 196
<211> 21
<212> RNA
<213> Artificial Sequence

<400> 196
agacaaacau uucuuuguga c 21

<210> 197
<211> 19
<212> RNA
<213> Artificial Sequence

<400> 197
cacaaagaaa uguuugucu 19

<210> 198
<211> 21
<212> RNA
<213> Artificial Sequence

<400> 198
agacaaacau uucuuuguga c 21

<210> 199
<211> 21
<212> RNA
<213> Artificial Sequence

<400> 199
gucacaaaga aauguuuguc u 21

<210> 200
<211> 23
<212> RNA
<213> Artificial Sequence

<400> 200
agacaaacau uucuuuguga cuc 23

<210> 201
<211> 21
<212> RNA
<213> Artificial Sequence

<400> 201

gucacaaaga aauguuuguc u 21

<210> 202
<211> 23
<212> RNA
<213> Artificial Sequence

<400> 202
agacaaacau uucuuuguga cuc 23

<210> 203
<211> 21
<212> RNA
<213> Artificial Sequence

<400> 203
gucacaaaga aauguuuguc u 21

<210> 204
<211> 23
<212> RNA
<213> Artificial Sequence

<400> 204
agacaaacau uucuuuguga cuc 23

<210> 205
<211> 19
<212> RNA
<213> Artificial Sequence

<400> 205
cacaaagaaa uguuugucu 19

<210> 206
<211> 21
<212> RNA
<213> Artificial Sequence

<400> 206
agacaaacau uucuuuguga c 21

<210> 207
<211> 19
<212> RNA
<213> Artificial Sequence

<400> 207
cacaaagaaa uguuugucu 19

<210> 208
<211> 21
<212> RNA
<213> Artificial Sequence

<400> 208
agacaaacau uucuuuguga c 21

<210> 209
<211> 19
<212> RNA
<213> Artificial Sequence

<400> 209
cacaaagaaa uguuugucu 19

<210> 210
<211> 21
<212> RNA
<213> Artificial Sequence

<400> 210
agacaaacau uucuuuguga c 21

<210> 211
<211> 21
<212> RNA
<213> Artificial Sequence

<400> 211
gucacaaaga aauguuuguc u 21

<210> 212
<211> 23
<212> RNA
<213> Artificial Sequence

<400> 212
agacaaacau uucuuuguga cuc 23

<210> 213
<211> 21
<212> RNA
<213> Artificial Sequence

<400> 213
gucacaaaga aauguuuguc u 21

<210> 214
<211> 23
<212> RNA
<213> Artificial Sequence

<400> 214
agacaaacau uucuuuguga cuc 23

<210> 215
<211> 21
<212> RNA
<213> Artificial Sequence

<400> 215
gucacaaaga aauguuuguc u 21

<210> 216
<211> 23
<212> RNA
<213> Artificial Sequence

<400> 216
agacaaacau uucuuuguga cuc 23

<210> 217
<211> 19
<212> RNA
<213> Artificial Sequence

<400> 217
cacaaagaaa uguuugucu 19

<210> 218
<211> 21
<212> RNA
<213> Artificial Sequence

<400> 218
agacaaacau uucuuuguga c 21

<210> 219
<211> 19
<212> RNA
<213> Artificial Sequence

<400> 219
cacaaagaaa uguuugucu 19

<210> 220
<211> 21
<212> RNA
<213> Artificial Sequence

<400> 220
agacaaacau uucuuuguga c 21

<210> 221
<211> 19
<212> RNA
<213> Artificial Sequence

<400> 221
cacaaagaaa uguuugucu 19

<210> 222
<211> 21
<212> RNA
<213> Artificial Sequence

<400> 222
agacaaacau uucuuuguga c 21

<210> 223
<211> 21
<212> RNA
<213> Artificial Sequence

<400> 223
gucacaaaga aauguuuguc u 21

<210> 224
<211> 23
<212> RNA
<213> Artificial Sequence

<400> 224
agacaaacau uucuuuguga cuc 23

<210> 225
<211> 21
<212> RNA

<213> Artificial Sequence

<400> 225
gucacaaaga aauguuuguc u 21

<210> 226
<211> 23
<212> RNA
<213> Artificial Sequence

<400> 226
agacaaacau uucuuuguga cuc 23

<210> 227
<211> 21
<212> RNA
<213> Artificial Sequence

<400> 227
gucacaaaga aauguuuguc u 21

<210> 228
<211> 23
<212> RNA
<213> Artificial Sequence

<400> 228
agacaaacau uucuuuguga cuc 23

<210> 229
<211> 19
<212> RNA
<213> Artificial Sequence

<400> 229
cacaaagaaa uguuugucu 19

<210> 230
<211> 21
<212> RNA
<213> Artificial Sequence

<400> 230
agacaaacau uucuuuguga c 21

<210> 231
<211> 19
<212> RNA
<213> Artificial Sequence

<400> 231
cacaaagaaa uguuugucu 19

<210> 232
<211> 21
<212> RNA
<213> Artificial Sequence

<400> 232
agacaaacau uucuuuguga c 21

<210> 233
<211> 19

<212> RNA
<213> Artificial Sequence

<400> 233
cacaaagaaa uguuugucu 19

<210> 234
<211> 21
<212> RNA
<213> Artificial Sequence

<400> 234
agacaaacau uucuuuguga c 21

<210> 235
<211> 21
<212> RNA
<213> Artificial Sequence

<400> 235
gucacaaaga aauguuuguc u 21

<210> 236
<211> 23
<212> RNA
<213> Artificial Sequence

<400> 236
agacaaacau uucuuuguga cuc 23

<210> 237
<211> 21
<212> RNA
<213> Artificial Sequence

<400> 237
gucacaaaga aauguuuguc u 21

<210> 238
<211> 23
<212> RNA
<213> Artificial Sequence

<400> 238
agacaaacau uucuuuguga cuc 23

<210> 239
<211> 21
<212> RNA
<213> Artificial Sequence

<400> 239
gucacaaaga aauguuuguc u 21

<210> 240
<211> 23
<212> RNA
<213> Artificial Sequence

<400> 240
agacaaacau uucuuuguga cuc 23

<210> 241

<211> 19
<212> RNA
<213> Artificial Sequence

<400> 241
gaauuccaaa aaccaauau 19

<210> 242
<211> 19
<212> RNA
<213> Artificial Sequence

<400> 242
auauugguuu uuggaauuc 19

<210> 243
<211> 19
<212> RNA
<213> Artificial Sequence

<220>
<221> misc_feature
<222> (19)
<223> n=a, or g, or c, or u

<400> 243
gaauuccaaa aaccaauan 19

<210> 244
<211> 19
<212> RNA
<213> Artificial Sequence

<220>
<221> misc_feature
<222> (1)
<223> n=a, or g, or c, or u

<400> 244
nuauugguuu uuggaauuc 19

<210> 245
<211> 19
<212> RNA
<213> Artificial Sequence

<220>
<221> misc_feature
<222> (19)
<223> n=a, or g, or c, or u

<400> 245
gaauuccaaa aaccaauan 19

<210> 246
<211> 21
<212> RNA
<213> Artificial Sequence

<220>

<221> misc_feature
<222> (1)
<223> n=a, or g, or c, or u

<400> 246
nuauugguuu uuggaauuca g 21

<210> 247
<211> 21
<212> RNA
<213> Artificial Sequence

<220>
<221> misc_feature
<222> (21)
<223> n=a, or g, or c, or u

<400> 247
cugaauucca aaaaccaaua n 21

<210> 248
<211> 23
<212> RNA
<213> Artificial Sequence

<220>
<221> misc_feature
<222> (1)
<223> n=a, or g, or c, or u

<400> 248
nuauugguuu uuggaauuca gug 23

<210> 249
<211> 19
<212> RNA
<213> Artificial Sequence

<400> 249
gaauuccaaa aaccaauau 19

<210> 250
<211> 21
<212> RNA
<213> Artificial Sequence

<400> 250
auauugguuu uuggaauuca g 21

<210> 251
<211> 21
<212> RNA
<213> Artificial Sequence

<400> 251
cugaauucca aaaaccaaua u 21

<210> 252
<211> 23
<212> RNA

<213> Artificial Sequence

<400> 252
auauugguuu uuggaauuca gug 23

<210> 253
<211> 19
<212> RNA
<213> Artificial Sequence

<400> 253
gaauuccaaa aaccaauau 19

<210> 254
<211> 21
<212> RNA
<213> Artificial Sequence

<400> 254
auauugguuu uuggaauuca g 21

<210> 255
<211> 19
<212> RNA
<213> Artificial Sequence

<400> 255
gaauuccaaa aaccaauau 19

<210> 256
<211> 21
<212> RNA
<213> Artificial Sequence

<400> 256
auauugguuu uuggaauuca g 21

<210> 257
<211> 19
<212> RNA
<213> Artificial Sequence

<400> 257
gaauuccaaa aaccaauau 19

<210> 258
<211> 21
<212> RNA
<213> Artificial Sequence

<400> 258
auauugguuu uuggaauuca g 21

<210> 259
<211> 21
<212> RNA
<213> Artificial Sequence

<400> 259
cugaauucca aaaaccaaua u 21

<210> 260
<211> 23

<212> RNA
<213> Artificial Sequence

<400> 260
auauugguuu uuggaauuca gug 23

<210> 261
<211> 21
<212> RNA
<213> Artificial Sequence

<400> 261
cugaauucca aaaaccaaua u 21

<210> 262
<211> 23
<212> RNA
<213> Artificial Sequence

<400> 262
auauugguuu uuggaauuca gug 23

<210> 263
<211> 21
<212> RNA
<213> Artificial Sequence

<400> 263
cugaauucca aaaaccaaua u 21

<210> 264
<211> 23
<212> RNA
<213> Artificial Sequence

<400> 264
auauugguuu uuggaauuca gug 23

<210> 265
<211> 19
<212> RNA
<213> Artificial Sequence

<400> 265
gaauuccaaa aaccaauau 19

<210> 266
<211> 21
<212> RNA
<213> Artificial Sequence

<400> 266
auauugguuu uuggaauuca g 21

<210> 267
<211> 19
<212> RNA
<213> Artificial Sequence

<400> 267
gaauuccaaa aaccaauau 19

<210> 268

<211> 21
<212> RNA
<213> Artificial Sequence

<400> 268
auauugguuu uuggaauuca g 21

<210> 269
<211> 19
<212> RNA
<213> Artificial Sequence

<400> 269
gaauuccaaa aaccaauau 19

<210> 270
<211> 21
<212> RNA
<213> Artificial Sequence

<400> 270
auauugguuu uuggaauuca g 21

<210> 271
<211> 21
<212> RNA
<213> Artificial Sequence

<400> 271
cugaauucca aaaaccaaua u 21

<210> 272
<211> 23
<212> RNA
<213> Artificial Sequence

<400> 272
auauugguuu uuggaauuca gug 23

<210> 273
<211> 21
<212> RNA
<213> Artificial Sequence

<400> 273
cugaauucca aaaaccaaua u 21

<210> 274
<211> 23
<212> RNA
<213> Artificial Sequence

<400> 274
auauugguuu uuggaauuca gug 23

<210> 275
<211> 21
<212> RNA
<213> Artificial Sequence

<400> 275
cugaauucca aaaaccaaua u 21

<210> 276
<211> 23
<212> RNA
<213> Artificial Sequence

<400> 276
auauugguuu uuggaauuca gug 23

<210> 277
<211> 19
<212> RNA
<213> Artificial Sequence

<400> 277
gaauuccaaa aaccaauau 19

<210> 278
<211> 21
<212> RNA
<213> Artificial Sequence

<400> 278
auauugguuu uuggaauuca g 21

<210> 279
<211> 19
<212> RNA
<213> Artificial Sequence

<400> 279
gaauuccaaa aaccaauau 19

<210> 280
<211> 21
<212> RNA
<213> Artificial Sequence

<400> 280
auauugguuu uuggaauuca g 21

<210> 281
<211> 19
<212> RNA
<213> Artificial Sequence

<400> 281
gaauuccaaa aaccaauau 19

<210> 282
<211> 21
<212> RNA
<213> Artificial Sequence

<400> 282
auauugguuu uuggaauuca g 21

<210> 283
<211> 21
<212> RNA
<213> Artificial Sequence

<400> 283
cugaauucca aaaaccaaua u 21

<210> 284
<211> 23
<212> RNA
<213> Artificial Sequence

<400> 284
auauugguuu uuggaauuca gug 23

<210> 285
<211> 21
<212> RNA
<213> Artificial Sequence

<400> 285
cugaauucca aaaaccaaua u 21

<210> 286
<211> 23
<212> RNA
<213> Artificial Sequence

<400> 286
auauugguuu uuggaauuca gug 23

<210> 287
<211> 21
<212> RNA
<213> Artificial Sequence

<400> 287
cugaauucca aaaaccaaua u 21

<210> 288
<211> 23
<212> RNA
<213> Artificial Sequence

<400> 288
auauugguuu uuggaauuca gug 23

<210> 289
<211> 19
<212> RNA
<213> Artificial Sequence

<400> 289
gaauuccaaa aaccaauau 19

<210> 290
<211> 21
<212> RNA
<213> Artificial Sequence

<400> 290
auauugguuu uuggaauuca g 21

<210> 291
<211> 19
<212> RNA
<213> Artificial Sequence

<400> 291

gaauuccaaa aaccaauau 19

<210> 292
<211> 21
<212> RNA
<213> Artificial Sequence

<400> 292
auauugguuu uuggaauuca g 21

<210> 293
<211> 19
<212> RNA
<213> Artificial Sequence

<400> 293
gaauuccaaa aaccaauau 19

<210> 294
<211> 21
<212> RNA
<213> Artificial Sequence

<400> 294
auauugguuu uuggaauuca g 21

<210> 295
<211> 21
<212> RNA
<213> Artificial Sequence

<400> 295
cugaauucca aaaaccaaua u 21

<210> 296
<211> 23
<212> RNA
<213> Artificial Sequence

<400> 296
auauugguuu uuggaauuca gug 23

<210> 297
<211> 21
<212> RNA
<213> Artificial Sequence

<400> 297
cugaauucca aaaaccaaua u 21

<210> 298
<211> 23
<212> RNA
<213> Artificial Sequence

<400> 298
auauugguuu uuggaauuca gug 23

<210> 299
<211> 21
<212> RNA
<213> Artificial Sequence

<400> 299
cugaauucca aaaaccaaua u 21

<210> 300
<211> 23
<212> RNA
<213> Artificial Sequence

<400> 300
auauugguuu uuggaauuca gug 23

<210> 301
<211> 19
<212> RNA
<213> Artificial Sequence

<400> 301
ggucugugcu ggcuauaaa 19

<210> 302
<211> 19
<212> RNA
<213> Artificial Sequence

<400> 302
uuuauagcca gcacagacc 19

<210> 303
<211> 19
<212> RNA
<213> Artificial Sequence

<220>
<221> misc_feature
<222> (19)
<223> n=a, or g, or c, or u

<400> 303
ggucugugcu ggcuauaan 19

<210> 304
<211> 19
<212> RNA
<213> Artificial Sequence

<220>
<221> misc_feature
<222> (1)
<223> n=a, or g, or c, or u

<400> 304
nuuauagcca gcacagacc 19

<210> 305
<211> 19
<212> RNA
<213> Artificial Sequence

<220>
<221> misc_feature
<222> (19)

<223> n=a, or g, or c, or u

<400> 305
ggucugugcu ggcuauaan 19

<210> 306
<211> 21
<212> RNA
<213> Artificial Sequence

<220>
<221> misc_feature
<222> (1)
<223> n=a, or g, or c, or u

<400> 306
nuuauagcca gcacagacca u 21

<210> 307
<211> 21
<212> RNA
<213> Artificial Sequence

<220>
<221> misc_feature
<222> (21)
<223> n=a, or g, or c, or u

<400> 307
auggucugug cuggcuauaa n 21

<210> 308
<211> 23
<212> RNA
<213> Artificial Sequence

<220>
<221> misc_feature
<222> (1)
<223> n=a, or g, or c, or u

<400> 308
nuuauagcca gcacagacca ucc 23

<210> 309
<211> 19
<212> RNA
<213> Artificial Sequence

<400> 309
ggucugugcu ggcuauaaa 19

<210> 310
<211> 21
<212> RNA
<213> Artificial Sequence

<400> 310
uuuauagcca gcacagacca u 21

<210> 311
<211> 21
<212> RNA
<213> Artificial Sequence

<400> 311
auggucugug cuggcuauaa a 21

<210> 312
<211> 23
<212> RNA
<213> Artificial Sequence

<400> 312
uuuauagcca gcacagacca ucc 23

<210> 313
<211> 19
<212> RNA
<213> Artificial Sequence

<400> 313
ggucugugcu ggcuauaaa 19

<210> 314
<211> 21
<212> RNA
<213> Artificial Sequence

<400> 314
uuuauagcca gcacagacca u 21

<210> 315
<211> 19
<212> RNA
<213> Artificial Sequence

<400> 315
ggucugugcu ggcuauaaa 19

<210> 316
<211> 21
<212> RNA
<213> Artificial Sequence

<400> 316
uuuauagcca gcacagacca u 21

<210> 317
<211> 19
<212> RNA
<213> Artificial Sequence

<400> 317
ggucugugcu ggcuauaaa 19

<210> 318
<211> 21
<212> RNA
<213> Artificial Sequence

<400> 318

uuuauagcca gcacagacca u 21

<210> 319
<211> 21
<212> RNA
<213> Artificial Sequence

<400> 319
auggucugug cuggcuauaa a 21

<210> 320
<211> 23
<212> RNA
<213> Artificial Sequence

<400> 320
uuuauagcca gcacagacca ucc 23

<210> 321
<211> 21
<212> RNA
<213> Artificial Sequence

<400> 321
auggucugug cuggcuauaa a 21

<210> 322
<211> 23
<212> RNA
<213> Artificial Sequence

<400> 322
uuuauagcca gcacagacca ucc 23

<210> 323
<211> 21
<212> RNA
<213> Artificial Sequence

<400> 323
auggucugug cuggcuauaa a 21

<210> 324
<211> 23
<212> RNA
<213> Artificial Sequence

<400> 324
uuuauagcca gcacagacca ucc 23

<210> 325
<211> 19
<212> RNA
<213> Artificial Sequence

<400> 325
ggucugugcu ggcuauaaa 19

<210> 326
<211> 21
<212> RNA
<213> Artificial Sequence

<400> 326
uuuauagcca gcacagacca u 21

<210> 327
<211> 19
<212> RNA
<213> Artificial Sequence

<400> 327
ggucugugcu ggcuauaaa 19

<210> 328
<211> 21
<212> RNA
<213> Artificial Sequence

<400> 328
uuuauagcca gcacagacca u 21

<210> 329
<211> 19
<212> RNA
<213> Artificial Sequence

<400> 329
ggucugugcu ggcuauaaa 19

<210> 330
<211> 21
<212> RNA
<213> Artificial Sequence

<400> 330
uuuauagcca gcacagacca u 21

<210> 331
<211> 21
<212> RNA
<213> Artificial Sequence

<400> 331
auggucugug cuggcuauaa a 21

<210> 332
<211> 23
<212> RNA
<213> Artificial Sequence

<400> 332
uuuauagcca gcacagacca ucc 23

<210> 333
<211> 21
<212> RNA
<213> Artificial Sequence

<400> 333
auggucugug cuggcuauaa a 21

<210> 334
<211> 23
<212> RNA
<213> Artificial Sequence

<400> 334
uuuauagcca gcacagacca ucc 23

<210> 335
<211> 21
<212> RNA
<213> Artificial Sequence

<400> 335
auggucugug cuggcuauaa a 21

<210> 336
<211> 23
<212> RNA
<213> Artificial Sequence

<400> 336
uuuauagcca gcacagacca ucc 23

<210> 337
<211> 19
<212> RNA
<213> Artificial Sequence

<400> 337
ggucugugcu ggcuauaaa 19

<210> 338
<211> 21
<212> RNA
<213> Artificial Sequence

<400> 338
uuuauagcca gcacagacca u 21

<210> 339
<211> 19
<212> RNA
<213> Artificial Sequence

<400> 339
ggucugugcu ggcuauaaa 19

<210> 340
<211> 21
<212> RNA
<213> Artificial Sequence

<400> 340
uuuauagcca gcacagacca u 21

<210> 341
<211> 19
<212> RNA
<213> Artificial Sequence

<400> 341
ggucugugcu ggcuauaaa 19

<210> 342
<211> 21
<212> RNA

<213> Artificial Sequence

<400> 342
uuuauagcca gcacagacca u 21

<210> 343
<211> 21
<212> RNA
<213> Artificial Sequence

<400> 343
auggucugug cuggcuauaa a 21

<210> 344
<211> 23
<212> RNA
<213> Artificial Sequence

<400> 344
uuuauagcca gcacagacca ucc 23

<210> 345
<211> 21
<212> RNA
<213> Artificial Sequence

<400> 345
auggucugug cuggcuauaa a 21

<210> 346
<211> 23
<212> RNA
<213> Artificial Sequence

<400> 346
uuuauagcca gcacagacca ucc 23

<210> 347
<211> 21
<212> RNA
<213> Artificial Sequence

<400> 347
auggucugug cuggcuauaa a 21

<210> 348
<211> 23
<212> RNA
<213> Artificial Sequence

<400> 348
uuuauagcca gcacagacca ucc 23

<210> 350
<211> 19
<212> RNA
<213> Artificial Sequence

<400> 350
ggucugugcu ggcuauaaa 19

<210> 350
<211> 21

<212> RNA
<213> Artificial Sequence

<400> 350
uuuauagcca gcacagacca u 21

<210> 351
<211> 19
<212> RNA
<213> Artificial Sequence

<400> 351
ggucugugcu ggcuauaaa 19

<210> 352
<211> 21
<212> RNA
<213> Artificial Sequence

<400> 352
uuuauagcca gcacagacca u 21

<210> 353
<211> 19
<212> RNA
<213> Artificial Sequence

<400> 353
ggucugugcu ggcuauaaa 19

<210> 354
<211> 21
<212> RNA
<213> Artificial Sequence

<400> 354
uuuauagcca gcacagacca u 21

<210> 355
<211> 21
<212> RNA
<213> Artificial Sequence

<400> 355
auggucugug cuggcuauaa a 21

<210> 356
<211> 23
<212> RNA
<213> Artificial Sequence

<400> 356
uuuauagcca gcacagacca ucc 23

<210> 357
<211> 21
<212> RNA
<213> Artificial Sequence

<400> 357
auggucugug cuggcuauaa a 21

<210> 358

<211> 23
<212> RNA
<213> Artificial Sequence

<400> 358
uuuauagcca gcacagacca ucc 23

<210> 359
<211> 21
<212> RNA
<213> Artificial Sequence

<400> 359
auggucugug cuggcuauaa a 21

<210> 360
<211> 23
<212> RNA
<213> Artificial Sequence

<400> 360
uuuauagcca gcacagacca ucc 23

<210> 361
<211> 19
<212> RNA
<213> Artificial Sequence

<400> 361
gguguuugcu auucaguuu 19

<210> 362
<211> 21
<212> RNA
<213> Artificial Sequence

<400> 362
aaacugaaua gcaaacaccu u 21

<210> 363
<211> 19
<212> RNA
<213> Artificial Sequence

<400> 363
gcuucuugaa agauagugu 19

<210> 364
<211> 21
<212> RNA
<213> Artificial Sequence

<400> 364
acacuaucuu ucaagaagca a 21

<210> 365
<211> 19
<212> RNA
<213> Artificial Sequence

<400> 365
gaagcaaugu ggucaucaa 19

<210> 366
<211> 21
<212> RNA
<213> Artificial Sequence

<400> 366
uugaugacca cauugcuuca a 21

<210> 367
<211> 19
<212> RNA
<213> Artificial Sequence

<400> 367
cacaaagaaa uguuugucu 19

<210> 368
<211> 21
<212> RNA
<213> Artificial Sequence

<400> 368
agacaaacau uucuuuguga c 21

<210> 369
<211> 19
<212> RNA
<213> Artificial Sequence

<400> 369
gaauuccaaa aaccaauau 19

<210> 370
<211> 21
<212> RNA
<213> Artificial Sequence

<400> 370
auauugguuu uuggaauuca g 21

<210> 371
<211> 19
<212> RNA
<213> Artificial Sequence

<400> 371
ggucugugcu ggcuauaaa 19

<210> 372
<211> 21
<212> RNA
<213> Artificial Sequence

<400> 372
uuuauagcca gcacagacca u 21

<210> 373
<211> 19
<212> RNA
<213> Artificial Sequence

<400> 373
gguguuugcu auucaguuu 19

<210> 374
<211> 21
<212> RNA
<213> Artificial Sequence

<400> 374
aaacugaaua gcaaacaccu u 21

<210> 375
<211> 19
<212> RNA
<213> Artificial Sequence

<400> 375
gcuucuugaa agauagugu 19

<210> 376
<211> 21
<212> RNA
<213> Artificial Sequence

<400> 376
acacuaucuu ucaagaagca a 21

<210> 377
<211> 19
<212> RNA
<213> Artificial Sequence

<400> 377
gaagcaaugu ggucaucaa 19

<210> 378
<211> 21
<212> RNA
<213> Artificial Sequence

<400> 378
uugaugacca cauugcuuca a 21

<210> 379
<211> 19
<212> RNA
<213> Artificial Sequence

<400> 379
cacaaagaaa uguuugucu 19

<210> 380
<211> 21
<212> RNA
<213> Artificial Sequence

<400> 380
agacaaacau uucuuuguga c 21

<210> 381
<211> 19
<212> RNA
<213> Artificial Sequence

<400> 381

gaauuccaaa aaccaauau 19

<210> 382
<211> 21
<212> RNA
<213> Artificial Sequence

<400> 382
auauugguuu uuggaauuca g 21

<210> 383
<211> 19
<212> RNA
<213> Artificial Sequence

<400> 383
ggucugugcu ggcuauaaa 19

<210> 384
<211> 21
<212> RNA
<213> Artificial Sequence

<400> 384
uuuauagcca gcacagacca u 21

<210> 385
<211> 19
<212> RNA
<213> Artificial Sequence

<400> 385
uucuccgaac gugucacgu 19

<210> 386
<211> 19
<212> RNA
<213> Artificial Sequence

<400> 386
acgugacacg uucggagaa 19

<210> 387
<211> 19
<212> RNA
<213> Artificial Sequence

<400> 387
gguguuugcu auucaguuu 19

<210> 388
<211> 21
<212> RNA
<213> Artificial Sequence

<400> 388
aaacugaaua gcaaacaccu u 21

<210> 389
<211> 19
<212> RNA
<213> Artificial Sequence

<400> 389
ggucugugcu ggcuauaaa 19

<210> 390
<211> 21
<212> RNA
<213> Artificial Sequence

<400> 390
uuuauagcca gcacagacca u 21

<210> 391
<211> 19
<212> RNA
<213> Artificial Sequence

<400> 391
gguguuugcu auucaguuu 19

<210> 392
<211> 21
<212> RNA
<213> Artificial Sequence

<400> 392
aaacugaaua gcaaacaccu u 21

<210> 393
<211> 19
<212> RNA
<213> Artificial Sequence

<400> 393
ggucugugcu ggcuauaaa 19

<210> 394
<211> 21
<212> RNA
<213> Artificial Sequence

<400> 394
uuuauagcca gcacagacca u 21

<210> 395
<211> 19
<212> DNA
<213> Artificial Sequence

<400> 395
ggtgtttgct attcagttt 19

<210> 396
<211> 19
<212> DNA
<213> Artificial Sequence

<400> 396
gcttcttgaa agatagtgt 19

<210> 397
<211> 19
<212> DNA
<213> Artificial Sequence

<400> 397
gaagcaatgt ggtcatcaa 19

<210> 398
<211> 19
<212> DNA
<213> Artificial Sequence

<400> 398
cacaaagaaa tgtttgtct 19

<210> 399
<211> 19
<212> RNA
<213> Artificial Sequence

<400> 399
gaauuccaaa aaccaauau 19

<210> 400
<211> 19
<212> RNA
<213> Artificial Sequence

<400> 400
ggucugugcu ggcuauaaa 19

<210> 401
<211> 2176
<212> DNA
<213> Artificial Sequence

<400> 401
atgattttat tcaagcaagc aacttatttc atttccttgt ttgctacagt ttcctgtgga 60
tgtctgactc aactctatga aaacgccttc ttcagaggtg gggatgtagc ttccatgtac 120
accccaaatg cccaatactg ccagatgagg tgcacattcc acccaaggtg tttgctattc 180
agttttcttc cagcaagttc aatcaatgac atggagaaaa ggtttggttg cttcttgaaa 240
gatagtgtta caggaaccct gccaaaagta catcgaacag gtgcagtttc tggacattcc 300
ttgaagcaat gtggtcatca aataagtgct tgccatcgag acatttataa aggagttgat 360
atgagaggag tcaattttaa tgtgtctaag gttagcagtg ttgaagaatg ccaaaaaagg 420
tgcaccagta acattcgctg ccagtttttt tcatatgcca cgcaaacatt tcacaaggca 480
gagtaccgga acaattgcct attaaagtac agtcccggag gaacacctac cgctataaag 540
gtgctgagta acgtggaatc tggattctca ctgaagccct gtgccctttc agaaattggt 600
tgccacatga acatcttcca gcatcttgcg ttctcagatg tggatgttgc cagggttctc 660
actccagatg cttttgtgtg tcggaccatc tgcacctatc accccaactg cctcttcttt 720
acattctata caaatgtatg gaaaatcgag tcacaaagaa atgtttgtct tcttaaaaca 780
tctgaaagtg gcacaccaag ttcctctact cctcaagaaa acaccatatc tggatatagc 840
cttttaacct gcaaaagaac tttacctgaa ccctgccatt ctaaaattta cccgggagtt 900
gactttggag gagaagaatt gaatgtgact tttgttaaag gagtgaatgt ttgccaagag 960
acttgcacaa agatgattcg ctgtcagttt ttcacttatt ctttactccc agaagactgt 1020
aaggaagaga agtgtaagtg tttcttaaga ttatctatgg atggttctcc aactaggatt 1080
gcgtatggga cacaagggag ctctggttac tctttgagat tgtgtaacac tggggacaac 1140
tctgtctgca caacaaaaac aagcacacgc attgttggag gaacaaactc ttcttgggga 1200
gagtggccct ggcaggtgag cctgcaggtg aagctgacag ctcagaggca cctgtgtgga 1260
gggtcactca taggacacca gtgggtcctc actgctgccc actgctttga tgggcttccc 1320
ctgcaggatg tttggcgcat ctatagtggc attttaaatc tgtcagacat tacaaaagat 1380
acacctttct cacaaataaa agagattatt attcaccaaa actataaagt ctcagaaggg 1440
aatcatgata tcgccttgat aaaactccag gctcctttga attacactga attccaaaaa 1500
ccaatatgcc taccttccaa aggtgacaca agcacaattt ataccaactg ttgggtaacc 1560
ggatggggct tctcgaagga gaaaggtgaa atccaaaata ttctacaaaa ggtaaatatt 1620
cctttggtaa caaatgaaga atgccagaaa agatatcaag attataaaat aacccaacgg 1680
atggtctgtg ctggctataa agaaggggga aaagatgctt gtaagggaga ttcaggtggt 1740

```
ccccttagttt gcaaacacaa tggaatgtgg cgtttggtgg gcatcaccag ctggggtgaa      1800
ggctgtgccc gcagggagca acctggtgtc tacaccaaag tcgctgagta catggactgg      1860
attttagaga aaacacagag cagtgatgga aaagctcaga tgcagtcacc agcatgagaa      1920
gcagtccaga gtctaggcaa tttttacaac ctgagttcaa gtcaaattct gagcctgggg      1980
ggtcctcatc tgcaaagcat ggagagtggc atcttctttg catcctaagg acgaaaaaca      2040
cagtgcactc agagctgctg aggacaatgt ctggctgaag cccgctttca gcacgccgta      2100
accaggggct gacaatgcga ggtcgcaact gagatctcca tgactgtgtg ttgtgaaata      2160
aaatggtgaa agatca                                                      2176

<210>  402
<211>  19
<212>  DNA
<213>  Artificial Sequence

<400>  402
aactaggatt gcgtatggg                                                     19

<210>  403
<211>  18
<212>  DNA
<213>  Artificial Sequence

<400>  403
agtgggcagc agtgagga                                                      18

<210>  404
<211>  19
<212>  DNA
<213>  Artificial Sequence

<400>  404
ggtcggagtc aacggattt                                                     19

<210>  405
<211>  19
<212>  DNA
<213>  Artificial Sequence

<400>  405
ccagcatcgc cccacttga                                                     19
```

## Description

## TECHNICAL FIELD

The present disclosure relates to a nucleic acid capable of inhibiting expression of a plasma prekallikrein (PKK) gene, and a pharmaceutical composition and an siRNA conjugate containing the nucleic acid. The present disclosure also relates to a preparation method and use of the nucleic acid, the pharmaceutical composition and the siRNA conjugate.

## BACKGROUND

Bradykinin (BK) is the main regulator of enhancing vascular permeability. Too much BK will enhance the leakage of blood vessels, thus aggravating inflammation. Researches show that a hereditary defect of a C1-esterase inhibitor (C1-INH), which is the main natural inhibitor of BK, will lead to hereditary angioedema (HAE). Patients suffering from rare HAE disease often suffer from acute attack of painful edema caused by unknown inducement, while the attack in throat may be life-threatening.

Prekallikrein (PKK) is a precursor of plasma kallikrein (PK), PKK is transformed into PK under the activation of factor XIIIa (FXIIa), and PK cleaves high molecular weight kininogen to release bradykinin into blood vessels. Therefore, the excess of BK can be inhibited at a cellular level by inhibiting the expression of a PKK gene, thus preventing and treating diseases or symptoms such as inflammation caused by excessive BK, especially hereditary angioedema. Small interfering RNA (siRNA), based on the mechanism of RNA interference (RNAi), can inhibit or block the expression of any interested target genes in a sequence-specific way, thus achieving the purpose of treating diseases.

One of the keys to develop siRNA drugs for inhibiting the expression of the PKK gene and treating the hereditary angioedema lies in finding a suitable siRNA and modification thereof, as well as an effective delivery system.

## SUMMARY

The inventors of the present disclosure have surprisingly found that the following siRNA and modification sequence thereof provided by the present disclosure can specifically inhibit the expression of the PKK gene, and the pharmaceutical composition or the siRNA conjugate containing the siRNA can specifically target the liver, thereby inhibiting the expression of the PKK gene in the liver and realizing the treatment or prevention of inflammatory diseases, especially hereditary angioedema, thus completing the present disclosure.

In some embodiments, the present disclosure provides an siRNA capable of inhibiting expression of a PKK gene. The siRNA comprises a sense strand and an antisense strand, each nucleotide in the siRNA is independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region; and the nucleotide sequence I and the nucleotide sequence II are selected from a group of sequences shown in the following i)- vi):

i) the nucleotide sequence I has the same length as and no more than three nucleotide difference from the nucleotide sequence shown in SEQ ID NO: 1; and the nucleotide sequence II has the same length as and no more than three nucleotide difference from the nucleotide sequence shown in SEQ ID NO: 2:

5'-GGUGUUUGCUAUUCAGUUZ$_1$-3' (SEQ ID NO: 1);
5'-Z$_2$AACUGAAUAGCAAACACC-3' (SEQ ID NO: 2),
wherein, $Z_1$ is U, $Z_2$ is A, the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand;

ii) the nucleotide sequence I has the same length as and no more than three nucleotide differences from the nucleotide sequence shown in SEQ ID NO: 61; and the nucleotide sequence II has the same length as and no more than three nucleotide differences from the nucleotide sequence shown in SEQ ID NO: 62:

5'-GCUUCUUGAAAGAUAGUGZ$_5$-3' (SEQ ID NO: 61);
5'-Z$_6$CACUAUCUUUCAAGAAGC-3' (SEQ ID NO: 62),
wherein, $Z_5$ is U, $Z_6$ is A, the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand;

iii) the nucleotide sequence I has the same length as and no more than three nucleotide differences from the nucleotide sequence shown in SEQ ID NO: 121; and the nucleotide sequence II has the same length as and no more than three nucleotide differences from the nucleotide sequence shown in SEQ ID NO: 122:

5'-GAAGCAAUGUGGUCAUCA$Z_9$-3' (SEQ ID NO: 121);
5'-$Z_{10}$UGAUGACCACAUUGCUUC-3' (SEQ ID NO: 122),
wherein, $Z_9$ is A, $Z_{10}$ is U, the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand;

iv) the nucleotide sequence I has the same length as and no more than three nucleotide differences from the nucleotide sequence shown in SEQ ID NO: 181; and the nucleotide sequence II has the same length as and no more than three nucleotide differences from the nucleotide sequence shown in SEQ ID NO: 182:

5'-CACAAAGAAAUGUUUGUC$Z_{13}$-3' (SEQ ID NO: 181);
5'-$Z_{14}$GACAAACAUUUCUUUGUG-3' (SEQ ID NO: 182),
wherein, $Z_{13}$ is U, $Z_{14}$ is A, the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand;

v) the nucleotide sequence I has the same length as and no more than three nucleotide differences from the nucleotide sequence shown in SEQ ID NO: 241; and the nucleotide sequence II has the same length as and no more than three nucleotide differences from the nucleotide sequence shown in SEQ ID NO: 242:

5'-GAAUUCCAAAAACCAAUA$Z_{17}$-3' (SEQ ID NO: 241);
5'-$Z_{18}$UAUUGGUUUUUGGAAUUC-3' (SEQ ID NO: 242),
wherein, $Z_{17}$ is U, $Z_{18}$ is A, the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand; and

vi) the nucleotide sequence I has the same length as and no more than three nucleotide differences from the nucleotide sequence shown in SEQ ID NO: 301; and the nucleotide sequence II has the same length as and no more than three nucleotide differences from the nucleotide sequence shown in SEQ ID NO: 302:

5'-GGUCUGUGCUGGCUAUAA$Z_{21}$-3' (SEQ ID NO: 301);
5'-$Z_{22}$UUAUAGCCAGCACAGACC-3' (SEQ ID NO: 302),
wherein, $Z_{21}$ is A, $Z_{22}$ is U, the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand.

In some embodiments, the present disclosure provides a pharmaceutical composition, wherein the pharmaceutical composition comprises the siRNA of the present disclosure and a pharmaceutically acceptable carrier.

In some embodiments, the present disclosure provides an siRNA conjugate, wherein the siRNA conjugate comprises the siRNA provided by the present disclosure and a conjugating group conjugated to the siRNA.

In some embodiments, the present disclosure provides the use of the siRNA and/or the pharmaceutical composition and/or the siRNA conjugate of the present disclosure in the manufacture of a medicament for treating and/or preventing an inflammatory disease, especially hereditary angioedema.

In some embodiments, the present disclosure provides a method for treating and/or preventing an inflammatory disease, especially hereditary angioedema, wherein the method comprises administering an effective amount of the siRNA and/or the pharmaceutical composition and/or the siRNA conjugate of the present disclosure to a subject in need.

In some embodiments, the present disclosure provides a method for inhibiting the expression of a PKK gene in a cell, wherein the method comprises contacting an effective amount of the siRNA and/or the pharmaceutical composition and/or the siRNA conjugate of the present disclosure with the cell.

In some embodiments, the present disclosure provides a kit, wherein the kit comprises the siRNA and/or the pharmaceutical composition and/or the siRNA conjugate of the present disclosure.

## Incorporated by Reference

All publications, patents and patent applications mentioned in this specification are incorporated herein by reference to the same extent as each individual publication, patent or patent application is specifically and individually incorporated herein by reference.

## Advantageous Effects

The siRNA , the pharmaceutical composition and the siRNA conjugate containing the siRNA provided by the present disclosure have good stability, higher PKK mRNA inhibitory activity and very low off-target effect, and/or can significantly treat, prevent or alleviate a symptom of a PKK related disease.

In some embodiments, the siRNA, the pharmaceutical composition or the siRNA conjugate provided by the present disclosure exhibits excellent target mRNA inhibitory activity in cell experiments in vitro. In some embodiments, the siRNA, the pharmaceutical composition or the siRNA conjugate provided by the present disclosure exhibits an inhibition percentage on target mRNA expression in hepatocytes of at least 20%, 30%, 40%, 50%, 60%, 70%, 80%, 90%, or 95%. The siRNA provided by the present disclosure has higher inhibitory activity on PKK mRNA in an in vitro psiCHECK system, and has certain inhibitory effects on PKK target sequences at different siRNA concentrations, and in particular, the inhibition percentage of siPKKa1M1S, siPKKdIMIS and siPKKf1M1S on target sequences at 0.1 nM concentration can reach as high as about 90%. In some embodiments, the siRNA provided by the present disclosure has a good inhibitory effect on PKK mRNA in HEK293A cells stably transfected with human PKK gene fragments in vitro, especially the inhibition percentages of siPKKa1M1S and siPKKf1M1S on the PKK mRNA are both as high as about 80%. In some embodiments, the siRNA conjugate provided by the present disclosure has a good inhibitory effect on PKK mRNA in HEK293A cells stably transfected with human PKK gene fragments in vitro, and the inhibition percentage is at least 53.2%, even as high as 83.1%.

In some embodiments, the siRNA, the pharmaceutical composition or the siRNA conjugate provided by the present disclosure may has higher stability and/or higher activity in vivo. In some embodiments, the siRNA, the pharmaceutical composition or the siRNA conjugate provided by the present disclosure exhibits an inhibition percentage on target mRNA expression of at least 20%, 30%, 40%, 50%, 60%, 70%, 80%, 90%, or 95% in vivo. In some embodiments, the siRNA, the pharmaceutical composition or the siRNA conjugate provided by the present disclosure exhibits an inhibition percentage on PKK gene expression of at least 20%, 30%, 40%, 50%, 60%, 70%, 80%, 90%, or 95% in vivo. In some embodiments, the siRNA, the pharmaceutical composition or the siRNA conjugate provided by the present disclosure exhibits an inhibition percentage on PKK gene expression of at least 20%, 30%, 40%, 50%, 60%, 70%, 80%, 90%, or 95% in a liver in vivo. In some embodiments, the siRNA, the pharmaceutical composition or the siRNA conjugate provided by the present disclosure exhibits an inhibition percentage on PKK gene expression of at least 20%, 30%, 40%, 50%, 60%, 70%, 80%, 90%, or 95% in a liver of an animal model in vivo. In some embodiments, the siRNA, the pharmaceutical composition or the siRNA conjugate provided by the present disclosure exhibits an inhibition percentage on PKK gene expression of at least 20%, 30%, 40%, 50%, 60%, 70%, 80%, 90%, or 95% in a liver of a human subject in vivo.

In some embodiments, the siRNA, the pharmaceutical composition or the siRNA conjugate provided by the present disclosure does not exhibit significant off-target effect. An off-target effect may be, for example, inhibition on normal expression of a gene which is not the target mRNA. It is considered that the off-target effect is insignificant, if the binding/inhibition of off-target mRNA expression is at a level of lower than 50%, 40%, 30%, 20%, or 10% of the on-target mRNA effect.

In this way, it is indicated that the siRNA, the pharmaceutical composition and the siRNA conjugate provided by the present disclosure can inhibit the expression of PKK gene, effectively treat and/or prevent an inflammatory disease or an embolic disease or a physiological condition, especially hereditary angioedema and its related symptoms, and have good application prospects.

Other features and advantages of the present disclosure will be described in detail in the detailed description section that follows.

## BRIEF DESCRIPTION OF THE DRAWINGS

FIG. 1 is a histogram showing relative expression levels of target sequences in a psiCHECK system in vitro after transfection of a plurality of siRNAs with different concentrations respectively.

FIG. 2 is a histogram showing relative expression levels of PKK mRNA in hPKK-HEK293A cells in vitro after transfection of different siRNAs.

FIG. 3 is a histogram showing relative expression levels of PKK mRNA in hPKK-HEK293A cells in vitro after transfection of different siRNA conjugates.

FIG. 4A is a fluorescence imaging photograph of various organs in C57 mice after administration of 5 ml/kg 1 × PBS, 3 mg/kg Cy5-siPKKa1M1S or 3 mg/kg Cy5-L10-siPKKa1M1SP (based on siRNA) for 48 hours.

FIG. 4B is a fluorescence imaging photograph of various organs in C57 mice after administration of 5 ml/kg 1 × PBS, 3 mg/kg Cy5-siPKKf1M1S or 3 mg/kg Cy5-L10-siPKKf1M1S (based on siRNA) for 48 hours.

## DETAILED DESCRIPTION

The specific embodiments of the present disclosure are described in detail as below. It should be understood that the specific embodiments described herein are only for the purpose of illustration and explanation of the present disclosure and are not intended to limit the present disclosure.

In the present disclosure, PKK mRNA refers to the mRNA with the sequence shown by Genbank registration number NM_000892.4, NM_001318394.1 or NM_001318396.1. In some embodiments, PKK mRNA refers to the mRNA with the sequence shown by Genbank registration number NM_000892.4. Furthermore, unless otherwise stated, the term "target gene" used in the present disclosure refers to a gene capable of transcribing the above PKK mRNA, and the term "target mRNA" refers to the above PKK mRNA.

### Definitions

In the context of the present disclosure, unless otherwise specified, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by phosphorothioate group; PI represents that the nucleotide adjacent to the right side of PI is a 5'-phosphate nucleotide or a 5'-phosphate analogue modified nucleotide, the letter combination VP represents that the nucleotide adjacent to the right side of the letter combination VP is a vinyl phosphate modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide.

In the context of the present disclosure, the "fluoro modified nucleotide" refers to a nucleotide formed by substituting the hydroxy at the 2' position of the ribose group of the nucleotide with a fluoro, and the "non-fluoro modified nucleotide" refers to a nucleotide formed by substituting the hydroxy at the 2' position of the ribose group of the nucleotide with a non-fluoro group, or a nucleotide analogue. The "nucleotide analogue" refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide, such as an isonucleotide, a bridged nucleic acid (BNA) or an acyclic nucleotide. The "methoxy modified nucleotide" refers to a nucleotide formed by substituting the 2'-hydroxy of the ribose group with a methoxy group.

In the context of the present disclosure, expressions "complementary" and "reverse complementary" can be interchangeably used, and have a well-known meaning in the art, namely, in a double-stranded nucleic acid molecule, the bases in one strand are paired complementally with those in the other strand. In DNA, a purine base adenine (A) is always paired with a pyrimidine base thymine (T) (or uracil (U) in RNAs); and a purine base guanine (G) is always paired with a pyrimidine base cytosine (C). Each base pair comprises a purine and a pyrimidine. While adenines in one strand are always paired with thymines (or uracils) in another strand, and guanines are always paired with cytosines, these two strands are considered as being complementary to each other; and the sequence of a strand may be deduced from the sequence of its complementary strand. Correspondingly, in the art a "mispairing" means that in a double-stranded nucleic acid, the bases at corresponding sites are not presented in a manner of being paired complementally.

In the context of the present disclosure, unless otherwise specified, "basically reverse complementary" means that there are no more than 3 base mispairings between two nucleotide sequences. "Substantially reverse complementary" means that there is no more than 1 base mispairing between two nucleotide sequences. "Completely complementary" means that there is no base mispairing between two nucleotide sequences.

In the context of the present disclosure, when a nucleotide sequence has "nucleotide difference" from another nucleotide sequence, the base type of the nucleotide at the same position therebetween are changed. For example, if a nucleotide base in the second sequence is A and the nucleotide base at the same position in the first sequence is U, C, G or T, these two nucleotide sequences are considered as having a nucleotide difference at this position. In some embodiments, if a nucleotide at a position is replaced with an abasic nucleotide or a nucleotide analogue, it is also considered that there is a nucleotide difference at the position.

In the context of the present disclosure, particularly in the description of the method for preparing the siRNA,

the composition comprising the siRNA or the siRNA conjugate of the present disclosure, unless otherwise specified, the nucleoside monomer refers to, according to the type and sequence of the nucleotides in the siRNA or siRNA conjugate to be prepared, unmodified or modified RNA phosphoramidites used in a solid phase phosphoramidite synthesis (the RNA phosphoramidites are also called as Nucleoside phosphoramidites elsewhere). Solid phase phosphoramidite synthesis is a well-known method used in RNA synthesis to those skilled in the art. Nucleoside monomers used in the present disclosure are all commercially available.

In the context of the present disclosure, unless otherwise stated, "conjugating" refers to two or more chemical moieties each with specific function being linked to each other via a covalent linkage. Correspondingly, a "conjugate" refers to a compound formed by covalent linkage of the individual chemical moieties. Further, an "siRNA conjugate" represents a compound formed by covalently linking one or more chemical moieties with specific functions to siRNA. Hereinafter, the siRNA conjugate of the present disclosure is sometimes abbreviated as "conjugate". The siRNA conjugate should be understood according to the context as the generic term of a plurality of siRNA conjugates or siRNA conjugates shown in certain chemical formulae. In the context of the present disclosure, a "conjugating molecule" should be understood as a specific compound capable of being conjugated to an siRNA via reactions, thus finally forming the siRNA conjugate of the present disclosure.

As used herein, "optional" or "optionally" means that the subsequently described event or condition may or may not occur, and that the description includes instances wherein the event or condition may or may not occur. For example, "optionally substituted" "alkyl" encompasses both "alkyl" and "substituted alkyl" as defined below. Those skilled in the art would understand, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain having the specified number of carbon atoms, usually 1 to 20 carbon atoms, for example 1 to 10 carbon atoms, such as 1 to 8 or 1 to 6 carbon atoms. For example, $C_1$-$C_6$ alkyl encompasses both straight and branched chain alkyl of 1 to 6 carbon atoms. When naming an alkyl residue having a specific number of carbon atoms, all branched and straight chain forms having that number of carbon atoms are intended to be encompassed; thus, for example, "butyl" is meant to include n-butyl, sec-butyl, isobutyl and t-butyl; and "propyl" includes n-propyl and isopropyl. Alkylene is a subset of alkyl, referring to the same residues as alkyl, but having two attachment points.

As used herein, "alkenyl" refers to an unsaturated branched or linear alkyl having at least one carbon-carbon double bond which is obtained by removing one hydrogen molecule from two adjacent carbon atoms of the parent alkyl. The group may be in either cis or trans configuration of the double bond. Typical alkenyl groups include, but not limited to, ethenyl; propenyl such as prop-1-en-1-yl, prop-1-en-2-yl, prop-2-en-1-yl (allyl), prop-2-en-2-yl; and butenyl such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, and the like. In certain embodiments, an alkenyl group has 2 to 20 carbon atoms, and in other embodiments, 2 to 10, 2 to 8, or 2 to 6 carbon atoms. Alkenylene is a subset of alkenyl, referring to the same residues as alkenyl, but having two attachment points.

As used herein, "alkynyl" refers to an unsaturated branched or linear alkyl having at least one carbon-carbon triple bond which is obtained by removing two hydrogen molecules from two adjacent carbon atoms of the parent alkyl. Typical alkynyl groups include, but not limited to, ethynyl; propynyl such as prop-1-yn-1-yl, prop-2-yn-1-yl; and butynyl such as but-1-yn-1-yl, but-1-yn-3-yl, but-3-yn-1-yl, and the like. In certain embodiments, an alkynyl group has 2 to 20 carbon atoms, and in other embodiments, 2 to 10, 2 to 8, or 2 to 6 carbon atoms. Alkynylene is a subset of alkynyl, referring to the same residues as alkynyl, but having two attachment points.

As used herein, "alkoxy" refers to an alkyl group of the specified number of carbon atoms attached through an oxygen bridge, such as, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. An alkoxy usually has 1 to 10, 1 to 8, 1 to 6, or 1 to 4 carbon atoms attached through oxygen bridge.

As used herein, "aryl" refers to a group derived from an aromatic monocyclic or multicyclic hydrocarbon ring system by removing a hydrogen atom from a ring carbon atom. The aromatic monocyclic or multicyclic hydrocarbon ring system contains only hydrogen and carbon comprising 6 to 18 carbon carbon atoms, wherein at least one ring in the ring system is fully unsaturated, i.e., containing a cyclic, delocalized $(4n+2)\pi$-electron system in accordance with the Hückel theory. Aryl groups include, but not limited to, phenyl, fluorenyl, naphthyl and the like. Arylene is a subset of aryl, referring to the same residues as aryl, but having two attachment positions.

As used herein, "halo substituent" or " halogen" refers to fluoro, chloro, bromo, and iodo, and the term "halogen" includes fluorine, chlorine, bromine, or iodine.

As used herein, "haloalkyl" refers to the alkyl as defined above with the specified number of carbon atoms being substituted with one or more halogen atoms, up to the maximum allowable number of halogen atoms. Examples of haloalkyl include, but not limited to, trifluoromethyl, difluoromethyl, 2-fluoroethyl, and pentafluoroethyl.

"Heterocyclyl" refers to a stable 3- to 18-membered non-aromatic ring group that comprises 2-12 carbon atoms

and 1-6 heteroatoms selected from nitrogen, oxygen and sulfur. Unless stated otherwise in the description, heterocyclyl is a monocyclic, bicyclic, tricyclic, or tetracyclic ring system, which may include fused or bridged ring systems. The heteroatoms in the heterocyclyl may be optionally oxidized. One or more nitrogen atoms, if present, are optionally quaternized. The heterocyclyl is partially or fully saturated. The heterocyclyl may be linked to the rest of the molecule through any atom of the ring. Examples of such heterocyclyl include, but not limited to, dioxanyl, thienyl[1,3]disulfonyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heteroaryl" refers to a group derived from a 3- to 18-membered aromatic ring radical that comprises 2 to 17 carbon atoms and 1 to 6 heteroatoms selected from nitrogen, oxygen and sulfur. As used herein, the heteroaryl may be a monocyclic, bicyclic, tricyclic or tetracyclic ring system, wherein at least one ring in the ring system is fully unsaturated, i.e., containing a cyclic, delocalized $(4n+2)\pi$-electron system in accordance with the Hückel theory. The heteroaryl includes fused or bridged ring systems. The heteroatoms in the heteroaryl are optionally oxidized. One or more nitrogen atoms, if present, are optionally quaternized. The heteroaryl may be linked to the rest of the molecule through any atom of the ring. Examples of such heteroaryl include, but not limited to, azepinyl, acridinyl, benzimidazolyl, benzoindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[b]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[H]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl, and thiophenyl/thienyl.

Various hydroxy protecting groups may be used in the present disclosure. In general, protecting groups render chemical functionalities insensitive to specific reaction conditions, and may be attached to and removed from such functionalities in a molecule without substantially damaging the remainder of the molecule. Representative hydroxy protecting groups are disclosed in Beaucage, et al., Tetrahedron 1992, 48, 2223-2311, and also in Greene and Wuts, Protective Groups in Organic Synthesis, Chapter 2, 2d ed, John Wiley & Sons, New York, 1991, each of which is hereby incorporated by reference in their entirety. In some embodiments, the protecting group is stable under basic conditions but can be removed under acidic conditions. In some embodiments, non-exclusive examples of the hydroxy protecting groups used herein include dimethoxytrityl (DMT), monomethoxytrityl, 9-phenylxanthen-9-yl (Pixyl), and 9-(p-methoxyphenyl)xanthen-9-yl (Mox). In some embodiments, non-exclusive examples of the hydroxy protecting groups used herein include Tr (trityl), MMTr (4-methoxytrityl), DMTr (4,4'-dimethoxytrityl), and TMTr (4,4',4"-trimethoxytrityl).

The term "subject", as used herein, refers to any animal, e.g., mammal or marsupial. The subject of the present disclosure includes, but not limited to, human, non-human primate (e.g., rhesus or other kinds of macaque), mouse, pig, horse, donkey, cow, sheep, rat and any kind of poultry.

As used herein, "treatment" refers to a method for obtaining advantageous or desired result, including but not limited to, therapeutic benefit. "Therapeutic benefit" means eradication or improvement of potential disorder to be treated. Moreover, the therapeutic benefit is achieved by eradicating or ameliorating one or more of physiological symptoms associated with the potential disorder such that an improvement is observed in the subject, notwithstanding that the subject may still be afflicted with the potential disorder.

As used herein, "prevention" refers to a method for obtaining advantageous or desired result, including but not limited to, prophylactic benefit. For obtaining the "prophylactic benefit", the siRNA, the siRNA conjugate or the pharmaceutical composition may be administered to the subject at risk of developing a particular disease, or to the subject reporting one or more physiological symptoms of a disease, even though the diagnosis of this disease may not have been made.

In one aspect, the present disclosure provides first to sixth siRNAs capable of inhibiting the expression of a PKK gene. The siRNAs will be described in detail hereinafter in turn.

The siRNA of the present disclosure comprises nucleotides as basic structural units. It is well-known to those skilled in the art that the nucleotide comprises a phosphate group, a ribose group and a base. Detailed illustrations

relating to such groups are omitted herein.

The siRNA of the present disclosure comprises a sense strand and an antisense strand, wherein lengths of the sense strand and the antisense strand are the same or different, the length of the sense strand is 19-23 nucleotides, and the length of the antisense strand is 19-26 nucleotides. In this way, a length ratio of the sense strand to the antisense strand of the siRNA provided by the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the antisense strand of the siRNA is 19/21, 21/23 or 23/25.

## First siRNA

According to the present disclosure, the siRNA may be the first siRNA.

The first siRNA comprises a sense strand and an antisense strand. Each nucleotide in the first siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 1; and the nucleotide sequence II has the same length as and no more than three nucleotide differences from the nucleotide sequence shown in SEQ ID NO: 2:

5'-GGUGUUUGCUAUUCAGUU$Z_1$-3'(SEQ ID NO: 1);
5'-$Z_2$AACUGAAUAGCAAACACC-3'(SEQ ID NO: 2),
wherein, $Z_1$ is U, $Z_2$ is A, the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand.

In this context, the term "corresponding site" means being at the same site in the nucleotide sequence by counting from the same terminal of the nucleotide sequence. For example, the first nucleotide at the 3' terminal of the nucleotide sequence I is a nucleotide at the corresponding site to the first nucleotide at the 3' terminal of SEQ ID NO: 1.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 1, and/or the nucleotide sequence II has no more than one nucleotide difference from the nucleotide sequence shown in SEQ ID NO: 2.

In some embodiments, the nucleotide difference between the nucleotide sequence II and the nucleotide sequence shown in SEQ ID NO: 2 comprises a difference at the site of $Z_4$, and $Z_4$ is selected from U, C or G. In some embodiments, the nucleotide difference is a difference at the site of $Z_4$, and $Z_4$ is selected from U, C or G. In some embodiments, $Z_3$ is a nucleotide complementary to $Z_4$. The siRNAs having the above nucleotide difference have higher ability to inhibit the target mRNA, and these siRNAs comprising nucleotide differences are also within the protection scope of the present disclosure.

In some embodiments, the nucleotide sequence I is basically reverse complementary, substantially reverse complementary, or completely reverse complementary to the nucleotide sequence II. The basically reverse complementary refers to no more than three base mispairings between two nucleotide sequences; the substantially reverse complementary refers to no more than one base mispairing between two nucleotide sequences; and the completely reverse complementary refers to no base mispairing between two nucleotide sequences.

In some embodiments, the nucleotide sequence I is the nucleotide sequence shown in SEQ ID NO: 3, and the nucleotide sequence II is the nucleotide sequence shown in SEQ ID NO: 4:

5'-GGUGUUUGCUAUUCAGUU$Z_3$-3' (SEQ ID NO: 3);
5'-$Z_4$AACUGAAUAGCAAACACC-3' (SEQ ID NO: 4),
wherein, $Z_4$ is the first nucleotide from 5' terminal of the antisense strand; $Z_4$ is selected from A, U, G or C; and $Z_3$ is a nucleotide complementary to $Z_4$; and in some embodiments, $Z_3$ is U, and $Z_4$ is A.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide

sequence II. In some embodiments, the nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 1 in the target mRNA and having the same length as the nucleotide sequence IV.

In some embodiments, the nucleotide sequence III and the nucleotide sequence IV both have a length of one nucleotide, the base of the nucleotide sequence III is A, and the base of the nucleotide sequence IV is U; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is AA, and the base composition of the nucleotide sequence IV is UU; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is CAA, and the base composition of the nucleotide sequence IV is UUG; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is CCAA, and the base composition of the nucleotide sequence IV is UUGG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is AA, and the base composition of the nucleotide sequence IV is UU; in this case, the length ratio of the sense strand to the antisense strand is 21/21.

In some embodiments, the nucleotide sequence III is completely reverse complementary to the nucleotide sequence IV. Thus, if the bases of the nucleotide sequence III are provided, the bases of the nucleotide sequence IV are also determined.

Second siRNA

According to the present disclosure, the siRNA may be the second siRNA.

The second siRNA comprises a sense strand and an antisense strand. Each nucleotide in the second siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 61; and the nucleotide sequence II has the same length as and no more than three nucleotide differences from the nucleotide sequence shown in SEQ ID NO: 62:

5'-GCUUCUUGAAAGAUAGUG$Z_5$-3' (SEQ ID NO: 61);
5'-$Z_6$CACUAUCUUUCAAGAAGC-3' (SEQ ID NO: 62),
wherein, $Z_5$ is U, $Z_6$ is A, the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 61, and/or the nucleotide sequence II has no more than one nucleotide difference from the nucleotide sequence shown in SEQ ID NO: 62.

In some embodiments, the nucleotide difference between the nucleotide sequence II and the nucleotide sequence shown in SEQ ID NO: 62 comprises a difference at the site of $Z_8$, and $Z_8$ is selected from U, C or G. In some embodiments, the nucleotide difference is a difference at the site of $Z_8$, and $Z_8$ is selected from U, C or G. In some embodiments, $Z_7$ is a nucleotide complementary to $Z_8$. The siRNAs having the above nucleotide difference have higher ability to inhibit the target mRNA, and these siRNAs comprising the nucleotide differences are also within the protection scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 63, and the nucleotide sequence II is the nucleotide sequence shown in SEQ ID NO: 64:

5'-GCUUCUUGAAAGAUAGUG$Z_7$-3' (SEQ ID NO: 63);
5'-$Z_8$CACUAUCUUUCAAGAAGC-3' (SEQ ID NO: 64),

wherein, $Z_8$ is the first nucleotide from 5' terminal of the antisense strand; $Z_8$ is selected from A, U, G or C; and $Z_7$ is a nucleotide complementary to $Z_8$; and in some embodiments, $Z_7$ is U, and $Z_8$ is A.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 61 in the target mRNA and having the same length as the nucleotide sequence IV.

In some embodiments, the nucleotide sequence III and the nucleotide sequence IV both have a length of one nucleotide, the base of the nucleotide sequence III is U, and the base of the nucleotide sequence IV is A; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is UU, and the base composition of the nucleotide sequence IV is AA; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is GUU, and the base composition of the nucleotide sequence IV is AAC; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is GGUU, and the base composition of the nucleotide sequence IV is AACC; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is UU, and the base composition of the nucleotide sequence IV is AA; in this case, the length ratio of the sense strand to the antisense strand is 21/21.

In some embodiments, the nucleotide sequence III is completely reverse complementary to the nucleotide sequence IV. Thus, if the bases of the nucleotide sequence III are provided, the bases of the nucleotide sequence IV are also determined.

Third siRNA

According to the present disclosure, the siRNA may be the third siRNA.

The third siRNA comprises a sense strand and an antisense strand. Each nucleotide in the third siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 121; and the nucleotide sequence II has the same length as and no more than three nucleotide differences from the nucleotide sequence shown in SEQ ID NO: 122:

5'-GAAGCAAUGUGGUCAUCA$Z_9$-3' (SEQ ID NO: 121);
5'-$Z_{10}$UGAUGACCACAUUGCUUC-3' (SEQ ID NO: 122),
wherein, $Z_9$ is A, $Z_{10}$ is U, the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 121, and/or the nucleotide sequence II has no more than one nucleotide difference from the nucleotide sequence shown in SEQ ID NO: 122.

In some embodiments, the nucleotide difference between the nucleotide sequence II and the nucleotide sequence shown in SEQ ID NO: 122 comprises a difference at the site of $Z_{12}$, and $Z_{12}$ is selected from A, C or G. In some embodiments, the nucleotide difference is a difference at the site of $Z_{12}$, and $Z_{12}$ is selected from A, C or G. In some embodiments, $Z_{11}$ is a nucleotide complementary to $Z_{12}$. The siRNAs having the above nucleotide difference have higher ability to inhibit the target mRNA, and these siRNAs comprising the nucleotide differences are also within the protection

scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 123, and the nucleotide sequence II is the nucleotide sequence shown in SEQ ID NO: 124:

5'-GAAGCAAUGUGGUCAUCA$Z_{11}$-3' (SEQ ID NO: 123);
5'-$Z_{12}$UGAUGACCACAUUGCUUC-3' (SEQ ID NO: 124),
wherein, $Z_{12}$ is the first nucleotide from 5' terminal of the antisense strand; $Z_{12}$ is selected from A, U, G or C; and $Z_{11}$ is a nucleotide complementary to $Z_{12}$; and in some embodiments, $Z_{11}$ is A, and $Z_{12}$ is U.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 121 in the target mRNA and having the same length as the nucleotide sequence IV.

In some embodiments, the nucleotide sequence III and the nucleotide sequence IV both have a length of one nucleotide, the base of the nucleotide sequence III is U, and the base of the nucleotide sequence IV is A; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is UU, and the base composition of the nucleotide sequence IV is AA; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is CUU, and the base composition of the nucleotide sequence IV is AAG; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is CCUU, and the base composition of the nucleotide sequence IV is AAGG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is UU, and the base composition of the nucleotide sequence IV is AA; in this case, the length ratio of the sense strand to the antisense strand is 21/21.

In some embodiments, the nucleotide sequence III is completely reverse complementary to the nucleotide sequence IV. Thus, if the bases of the nucleotide sequence III are provided, the bases of the nucleotide sequence IV are also determined.

## Fourth siRNA

According to the present disclosure, the siRNA may be the fourth siRNA.

The fourth siRNA comprises a sense strand and an antisense strand. Each nucleotide in the fourth siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 181; and the nucleotide sequence II has the same length as and no more than three nucleotide differences from the nucleotide sequence shown in SEQ ID NO: 182:

5'-CACAAAGAAAUGUUUGUC$Z_{13}$-3' (SEQ ID NO: 181);
5'-$Z_{14}$GACAAACAUUUCUUUGUG-3' (SEQ ID NO: 182),
wherein, $Z_{13}$ is U, $Z_{14}$ is A, the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 181, and/or the nucleotide sequence II has no more than one nucleotide difference from the nucleotide sequence shown in SEQ ID NO: 182.

In some embodiments, the nucleotide difference between the nucleotide sequence II and the nucleotide sequence shown in SEQ ID NO: 182 comprises a difference at the site of $Z_{16}$, and $Z_{16}$ is selected from U, C or G. In some embodiments, the nucleotide difference is a difference at the site of $Z_{16}$, and $Z_{16}$ is selected from U, C or G. In some embodiments, $Z_{15}$ is a nucleotide complementary to $Z_{16}$. The siRNAs having the above nucleotide difference have higher ability to inhibit the target mRNA, and these siRNAs comprising the nucleotide differences are also within the protection scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 183, and the nucleotide sequence II is the nucleotide sequence shown in SEQ ID NO: 184:

5'-CACAAAGAAAUGUUUGUC$Z_{15}$-3' (SEQ ID NO: 183);
5'-$Z_{16}$GACAAACAUUUCUUUGUG-3' (SEQ ID NO: 184),
wherein, $Z_{16}$ is the first nucleotide from 5' terminal of the antisense strand; $Z_{16}$ is selected from A, U, G or C; and $Z_{15}$ is a nucleotide complementary to $Z_{16}$; and in some embodiments, $Z_{15}$ is U, and $Z_{16}$ is A.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 181 in the target mRNA and having the same length as the nucleotide sequence IV.

In some embodiments, the nucleotide sequence III and the nucleotide sequence IV both have a length of one nucleotide, the base of the nucleotide sequence III is U, and the base of the nucleotide sequence IV is A; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is GU, and the base composition of the nucleotide sequence IV is AC; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is AGU, and the base composition of the nucleotide sequence IV is ACU; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is GAGU, and the base composition of the nucleotide sequence IV is ACUC; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is GU, and the base composition of the nucleotide sequence IV is AC; in this case, the length ratio of the sense strand to the antisense strand is 21/21.

In some embodiments, the nucleotide sequence III is completely reverse complementary to the nucleotide sequence IV. Thus, if the bases of the nucleotide sequence III are provided, the bases of the nucleotide sequence IV are also determined.

<u>Fifth siRNA</u>

According to the present disclosure, the siRNA may be the fifth siRNA.

The fifth siRNA comprises a sense strand and an antisense strand. Each nucleotide in the fifth siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 241; and the nucleotide sequence II has the same length as and no more than three nucleotide differences from the nucleotide sequence shown in SEQ ID NO: 242:

5'-GAAUUCCAAAAACCAAUA$Z_{17}$-3' (SEQ ID NO: 241);

5'-$Z_{18}$UAUUGGUUUUUGGAAUUC-3' (SEQ ID NO: 242),

wherein, $Z_{17}$ is U, $Z_{18}$ is A, the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 241, and/or the nucleotide sequence II has no more than one nucleotide difference from the nucleotide sequence shown in SEQ ID NO: 242.

In some embodiments, the nucleotide difference between the nucleotide sequence II and the nucleotide sequence shown in SEQ ID NO: 242 comprises a difference at the site of $Z_{20}$, and $Z_{20}$ is selected from U, C or G. In some embodiments, the nucleotide difference is a difference at the site of $Z_{20}$, and $Z_{20}$ is selected from U, C or G. In some embodiments, $Z_{19}$ is a nucleotide complementary to $Z_{20}$. The siRNAs having the above nucleotide difference have higher ability to inhibit the target mRNA, and these siRNAs comprising the nucleotide differences are also within the protection scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 243, and the nucleotide sequence II is the nucleotide sequence shown in SEQ ID NO: 244:

5'-GAAUUCCAAAAACCAAUA$Z_{19}$-3' (SEQ ID NO: 243);

5'-$Z_{20}$UAUUGGUUUUUGGAAUUC-3' (SEQ ID NO: 244),

wherein, $Z_{20}$ is the first nucleotide from 5' terminal of the antisense strand; $Z_{19}$ is selected from A, U, G or C; and $Z_{20}$ is a nucleotide complementary to $Z_{19}$; and in some embodiments, $Z_{19}$ is U, and $Z_{20}$ is A.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 241 in the target mRNA and having the same length as the nucleotide sequence IV.

In some embodiments, the nucleotide sequence III and the nucleotide sequence IV both have a length of one nucleotide, the base of the nucleotide sequence III is U, and the base of the nucleotide sequence IV is A; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is CU, and the base composition of the nucleotide sequence IV is AG; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is ACU, and the base composition of the nucleotide sequence IV is AGU; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is CACU, and the base composition of the nucleotide sequence IV is AGUG; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is CU, and the base composition of the nucleotide sequence IV is AG; in this case, the length ratio of the sense strand to the antisense strand is 21/21.

In some embodiments, the nucleotide sequence III is completely reverse complementary to the nucleotide sequence IV. Thus, if the bases of the nucleotide sequence III are provided, the bases of the nucleotide sequence IV are also determined.

Sixth siRNA

According to the present disclosure, the siRNA may be the sixth siRNA.

The sixth siRNA comprises a sense strand and an antisense strand. Each nucleotide in the sixth siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 301; and the nucleotide sequence II has the same length as and no more than three nucleotide differences from the nucleotide sequence shown in SEQ ID NO: 302:

5'-GGUCUGUGCUGGCUAUAA$Z_{21}$-3' (SEQ ID NO: 301);
5'-$Z_{22}$UUAUAGCCAGCACAGACC-3' (SEQ ID NO: 302),
wherein, $Z_{21}$ is A, $Z_{22}$ is U, the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand.

In some embodiments, the sense strand exclusively comprises the nucleotide sequence I, and the antisense strand exclusively comprises the nucleotide sequence II.

In some embodiments, the nucleotide sequence I has no more than one nucleotide difference from the nucleotide sequence shown in SEQ ID NO: 301, and/or the nucleotide sequence II has no more than one nucleotide difference from the nucleotide sequence shown in SEQ ID NO: 302.

In some embodiments, the nucleotide difference between the nucleotide sequence II and the nucleotide sequence shown in SEQ ID NO: 302 comprises a difference at the site of $Z_{24}$, and $Z_{24}$ is selected from A, C or G. In some embodiments, the nucleotide difference is a difference at the site of $Z_{24}$, and $Z_{24}$ is selected from A, C or G. In some embodiments, $Z_{23}$ is a nucleotide complementary to $Z_{24}$. The siRNAs having the above nucleotide difference have higher ability to inhibit the target mRNA, and these siRNA conjuegates comprising the nucleotide differences are also within the protection scope of the present disclosure.

In some embodiments, the nucleotide sequence I is basically reverse complementary, substantially reverse complementary, or completely reverse complementary to the nucleotide sequence II.

In some embodiments, the nucleotide sequence I is the nucleotide sequence shown in SEQ ID NO: 303, and the nucleotide sequence II is the nucleotide sequence shown in SEQ ID NO: 304:

5'-GGUCUGUGCUGGCUAUAA$Z_{23}$-3' (SEQ ID NO: 303);
5'-$Z_{24}$UUAUAGCCAGCACAGACC-3' (SEQ ID NO: 304),
wherein, $Z_{24}$ is the first nucleotide from 5' terminal of the antisense strand; $Z_{24}$ is selected from A, U, G or C; and $Z_{23}$ is a nucleotide complementary to $Z_{24}$; and in some embodiments, $Z_{23}$ is A, and $Z_{24}$ is U.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 301 in the target mRNA and having the same length as the nucleotide sequence IV.

In some embodiments, the nucleotide sequence III and the nucleotide sequence IV both have a length of one nucleotide, the base of the nucleotide sequence III is U, and the base of the nucleotide sequence IV is A; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is AU, and the base composition of the nucleotide sequence IV is AU; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is GAU, and the base composition of the nucleotide sequence IV is AUC; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is GGAU, and the base composition of the nucleotide sequence IV is AUCC; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is AU, and the base composition of the nucleotide sequence IV is AU; in this case, the length

ratio of the sense strand to the antisense strand is 21/21.

The following description of the nucleotide sequence V, the nucleic acid sequence, the nucleotide modification in the siRNA and the modified sequence is applicable to any one of the first siRNA to the sixth siRNA. That is, unless otherwise specified, the following description of the siRNA should be regarded as describing the first siRNA, the second siRNA, the third siRNA, the fourth siRNA, the fifth siRNA, and the sixth siRNA one by one. For example, if no specific siRNA is specified, "the siRNA further comprises a nucleotide sequence V" means "the first siRNA, the second siRNA, the third siRNA, the fourth siRNA, the fifth siRNA, and the sixth siRNA further comprises a nucleotide sequence V".

In some embodiments, the antisense further comprises a nucleotide sequence V, the nucleotide sequence V has a length of 1-3 nucleotides and is linked to the 3' terminal of the antisense strand, thereby constituting a 3' overhang of the antisense strand. As such, the length ratio of the sense strand to the antisense strand in the siRNA of the present disclosure may be 19/20, 19/21, 19/22, 20/21, 20/22, 20/23, 21/22, 21/23, 21/24, 22/23, 22/24, 22/25, 23/24, 23/25, or 23/26. In some embodiments, the nucleotide sequence V has a length of 2 nucleotides. As such, the length ratio of the sense strand to the antisense strand in the siRNA of the present disclosure may be 19/21, 21/23 or 23/25.

Each nucleotide in the nucleotide sequence V may be any nucleotide. In order to facilitate synthesis and save synthesis cost, the nucleotide sequence V is 2 continuous thymidine deoxyribonucleotides (dTdT) or 2 continuous uracil ribonucleotides (UU); or, in order to improve the affinity of the antisense strand of the siRNA to the target mRNA, the nucleotide sequence V is complementary to the nucleotides at the corresponding site of the target mRNA. Therefore, in some embodiments, the length ratio of the sense strand to the antisense strand of the siRNA of the present disclosure is 19/21 or 21/23. In this case, the siRNA of the present disclosure has better silencing activity against target mRNA.

The nucleotide at the corresponding site of the target mRNA refers to the nucleotide or nucleotide sequence adjacent to a segment of nucleotide sequence of the target mRNA at the 5' terminal. This segment of nucleotide sequence of the target mRNA is substantially reverse complementary or completely reverse complementary to the nucleotide sequence II, or, is a segment of nucleotide sequence which is substantially reverse complementary or completely reverse complementary to the nucleotide sequence formed by the nucleotide sequence II and the nucleotide sequence IV.

In some embodiments, for the first siRNA, the sense strand of the siRNA comprises the nucleotide sequence shown in SEQ ID NO: 5, and the antisense strand of the siRNA comprises the nucleotide sequence shown in SEQ ID NO: 6:

5'-GGUGUUUGCUAUUCAGUU$Z_3$-3' (SEQ ID NO: 5);
5'-$Z_4$AACUGAAUAGCAAACACCUU-3' (SEQ ID NO: 6);
or, the sense strand of the siRNA comprises the nucleotide sequence shown in SEQ ID NO: 7, and the antisense strand of the siRNA comprises the nucleotide sequence shown in SEQ ID NO: 8;
5'-AAGGUGUUUGCUAUUCAGUU$Z_3$-3'(SEQ ID NO: 7);
5'-$Z_4$AACUGAAUAGCAAACACCUUGG-3'(SEQ ID NO: 8);
wherein, $Z_4$ is the first nucleotide from 5' terminal of the antisense strand; $Z_4$ is selected from A, U, G or C; and $Z_3$ is a nucleotide complementary to $Z_4$.

In some embodiments, for the second siRNA, the sense strand of the siRNA comprises the nucleotide sequence shown in SEQ ID NO: 65, and the antisense strand of the siRNA comprises the nucleotide sequence shown in SEQ ID NO: 66:

5'-GCUUCUUGAAAGAUAGUG$Z_7$-3' (SEQ ID NO: 65);
5'-$Z_8$CACUAUCUUUCAAGAAGCAA-3' (SEQ ID NO: 66),
or, the sense strand of the siRNA comprises the nucleotide sequence shown in SEQ ID NO: 67, and the antisense strand of the siRNA comprises the nucleotide sequence shown in SEQ ID NO: 68:

5'-UUGCUUCUUGAAAGAUAGUG$Z_7$-3' (SEQ ID NO: 67);
5'-$Z_8$CACUAUCUUUCAAGAAGCAACC-3' (SEQ ID NO: 68),
wherein, $Z_8$ is the first nucleotide from 5' terminal of the antisense strand; $Z_8$ is selected from A, U, G or C; and $Z_7$ is a nucleotide complementary to $Z_8$

In some embodiments, for the third siRNA, the sense strand of the siRNA comprises the nucleotide sequence shown in SEQ ID NO: 125, and the antisense strand of the siRNA comprises the nucleotide sequence shown in SEQ ID NO: 126:

5'-GAAGCAAUGUGGUCAUCA$Z_{11}$-3' (SEQ ID NO: 125);
5'-$Z_{12}$UGAUGACCACAUUGCUUCAA-3' (SEQ ID NO: 126),
or, the sense strand of the siRNA comprises the nucleotide sequence shown in SEQ ID NO: 127, and the antisense strand of the siRNA comprises the nucleotide sequence shown in SEQ ID NO: 128:

5'-UUGAAGCAAUGUGGUCAUCA$Z_{11}$-3' (SEQ ID NO: 127);
5'-$Z_{12}$UGAUGACCACAUUGCUUCAAGG-3' (SEQ ID NO: 128),
wherein, $Z_{12}$ is the first nucleotide from 5' terminal of the antisense strand; $Z_{12}$ is selected from A, U, G or C; and $Z_{11}$ is a nucleotide complementary to $Z_{12}$.

In some embodiments, for the fourth siRNA, the sense strand of the siRNA comprises the nucleotide sequence shown in SEQ ID NO: 185, and the antisense strand of the siRNA comprises the nucleotide sequence shown in SEQ ID NO: 186:

5'-CACAAAGAAAUGUUUGUC$Z_{15}$-3' (SEQ ID NO: 185);
5'-$Z_{16}$GACAAACAUUUCUUUGUGAC-3' (SEQ ID NO: 186),
or, the sense strand of the siRNA comprises the nucleotide sequence shown in SEQ ID NO: 187, and the antisense strand of the siRNA comprises the nucleotide sequence shown in SEQ ID NO: 188:

5'-GUCACAAAGAAAUGUUUGUC$Z_{15}$-3' (SEQ ID NO: 187);
5'-$Z_{16}$GACAAACAUUUCUUUGUGACUC-3' (SEQ ID NO: 188),
wherein, $Z_{16}$ is the first nucleotide from 5' terminal of the antisense strand; $Z_{16}$ is selected from A, U, G or C; and $Z_{15}$ is a nucleotide complementary to $Z_{16}$.

In some embodiments, for the fifth siRNA, the sense strand of the siRNA comprises the nucleotide sequence shown in SEQ ID NO: 245, and the antisense strand of the siRNA comprises the nucleotide sequence shown in SEQ ID NO: 246:

5'-GAAUUCCAAAAACCAAUA$Z_{19}$-3' (SEQ ID NO: 245);
5'-$Z_{20}$UAUUGGUUUUUGGAAUUCAG-3' (SEQ ID NO: 246),
or, the sense strand of the siRNA comprises the nucleotide sequence shown in SEQ ID NO: 247, and the antisense strand of the siRNA comprises the nucleotide sequence shown in SEQ ID NO: 248:

5'-CUGAAUUCCAAAAACCAAUA$Z_{19}$-3' (SEQ ID NO: 247);
5'-$Z_{20}$UAUUGGUUUUUGGAAUUCAGUG-3' (SEQ ID NO: 248),
wherein, $Z_{20}$ is the first nucleotide from 5' terminal of the antisense strand; $Z_{20}$ is selected from A, U, G or C; and $Z_{19}$ is a nucleotide complementary to $Z_{20}$.

In some embodiments, for the sixth siRNA, the sense strand of the siRNA comprises the nucleotide sequence shown in SEQ ID NO: 305, and the antisense strand of the siRNA comprises the nucleotide sequence shown in SEQ ID NO: 306:

5'-GGUCUGUGCUGGCUAUAA$Z_{23}$-3' (SEQ ID NO: 305);
5'-$Z_{24}$UUAUAGCCAGCACAGACCAU-3' (SEQ ID NO: 306),
or, the sense strand of the siRNA comprises the nucleotide sequence shown in SEQ ID NO: 307, and the antisense strand of the siRNA comprises the nucleotide sequence shown in SEQ ID NO: 308:

5'-AUGGUCUGUGCUGGCUAUAA$Z_{23}$-3' (SEQ ID NO: 307);
5'-$Z_{24}$UUAUAGCCAGCACAGACCAUCC-3' (SEQ ID NO: 308),
wherein, $Z_{24}$ is the first nucleotide from 5' terminal of the antisense strand; $Z_{24}$ is selected from A, U, G or C; and $Z_{23}$ is a nucleotide complementary to $Z_{24}$.

In some embodiments, the siRNA of the present disclosure is siPKKa1, siPKKa2, siPKKb1, siPKKb2, siPKKc1, siPKKc2, siPKKd1, siPKKd2, siPKKe1, siPKKe2, siPKKf1 and siPKKf2 listed in Tables 1a-1f.

As described above, the nucleotides in the siRNA of the present disclosure are each independently modified or unmodified nucleotides. In some embodiments, each nucleotide in the siRNA of the present disclosure is an unmodified nucleotide. In some embodiments, some or all nucleotides in the siRNA of the present disclosure are modified nucleotides. Such modifications on the nucleotides would not cause significant decrease or loss of the function of the siRNA of the present disclosure to inhibit the expression of PKK genes.

In some embodiments, the siRNA of the present disclosure comprises at least one modified nucleotide. In the context of the present disclosure, the term "modified nucleotide" employed herein refers to a nucleotide formed by substituting the hydroxy at the 2'-position of the ribose group of a nucleotide with other group, a nucleotide analogue, or a nucleotide with modified base. Such modified nucleotides would not cause significant decrease or loss of the function

of the siRNA to inhibit the expression of genes. For example, the modified nucleotides disclosed in J.K. Watts, G. F. Deleavey and M. J.Damha, Chemically Modified siRNA: tools and applications. Drug Discov Today, 2008.13(19-20): p. 842 -55 may be selected.

In some embodiments, at least one nucleotide in the sense strand or the antisense strand of the siRNA provided by the present disclosure is a modified nucleotide, and/or at least one phosphoester group is a phosphoester group with modified group. In other words, at least part of the phosphoester groups and/or ribose groups in phosphate-sugar backbone of at least one single strand in the sense strand and the antisense strand are phosphoester groups with modified group and/or ribose groups with modified group.

In some embodiments, all nucleotides in the sense strand and/or the antisense strand are modified nucleotides. In some embodiments, each nucleotide in the sense strand and the antisense strand of the siRNA provided by the present disclosure is independently a fluoro modified nucleotide or a non-fluoro modified nucleotide.

The inventors of the present disclosure have surprisingly found that the siRNA of the present disclosure has achieved a high degree of balance between the stability in plasma and the gene silencing efficiency in animal experiments.

In some embodiments, the fluoro modified nucleotides are located in the nucleotide sequence I and the nucleotide sequence II; and in the direction from 5' terminal to 3' terminal, at least the nucleotides at positions 7, 8 and 9 of the nucleotide sequence I are fluoro modified nucleotides; and in the direction from 5' terminal to 3' terminal, at least the nucleotides at positions 2, 6, 14 and 16 of the nucleotide sequence II are fluoro modified nucleotides.

In some embodiments, the fluoro modified nucleotides are located in the nucleotide sequence I and the nucleotide sequence II; no more than 5 fluoro modified nucleotides are present in the nucleotide sequence I, and in the direction from 5' terminal to 3' terminal, at least the nucleotides at positions 7, 8 and 9 in the nucleotide sequence I are fluoro modified nucleotides; no more than 7 fluoro modified nucleotides are present in the nucleotide sequence II, and at least the nucleotides at positions 2, 6, 14 and 16 in the nucleotide sequence II are fluoro modified nucleotides.

In some embodiments, in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the rest of positions in the sense strand are non-fluoro modified nucleotides; and in the direction from 5' terminal to 3' terminal, the nucleotides at positions 2, 6, 14 and 16 or at positions 2, 6, 8, 9, 14 and 16 of the nucleotide sequence II in the antisense strand are fluoro modified nucleotides, and the nucleotides at the rest of positions in the antisense strand are non-fluoro modified nucleotides.

In the context of the present disclosure, a "fluoro modified nucleotide" refers to a nucleotide which is formed by substituting the hydroxy at the 2'-position of the ribose group of a nucleotide with fluoro, which has a structure as shown by Formula (7). A "non-fluoro modified nucleotide", refers to a nucleotide which is formed by substituting the hydroxy at the 2'-position of the ribose group of a nucleotide with a non-fluoro group, or a nucleotide analogue. In some embodiments, each non-fluoro modified nucleotide is independently selected from a nucleotide formed by substituting the hydroxy at the 2'-position of the ribose group of the nucleotide with the non-fluoro group, or the nucleotide analogue.

These nucleotides formed by substituting the hydroxy at 2'-position of the ribose group with the non-fluoro group are well-known to those skilled in the art, and these nucleotides may be selected from one of a 2'-alkoxy modified nucleotide, a 2'-substituted alkoxy modified nucleotide, a 2'-alkyl modified nucleotide, a 2'-substituted alkyl modified nucleotide, a 2'-amino modified nucleotide, a 2'-substituted amino modified nucleotide and a 2'-deoxy nucleotide.

In some embodiments, the 2'-alkoxy modified nucleotide is a methoxy modified nucleotide (2'-OMe), as shown by Formula (8). In some embodiments, the 2'-substituted alkoxy modified nucleotide is, for example, a 2'-O-methoxyethoxy modified nucleotide (2'-MOE) as shown by Formula (9). In some embodiments, the 2'-amino modified nucleotide (2'-$NH_2$) is as shown by Formula (10). In some embodiments, the 2'-deoxy nucleotide (DNA) is as shown by Formula (11):

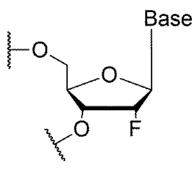 , 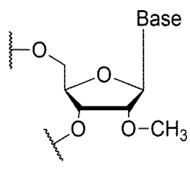 , 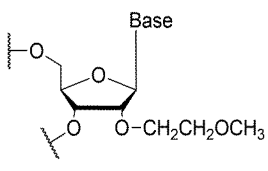 , 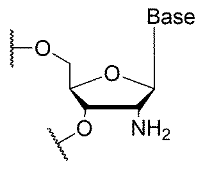 , 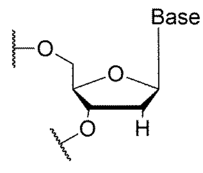 .

Formula (7) Formula (8) Formula (9) Formula (10) Formula (11).

The nucleotide analogue refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide. In some embodiments, the nucleotide analogue may be an isonucleotide, a bridged nucleic acid (referred to as BNA) or an acyclic nucleotide.

The BNA is a nucleotide that is constrained or is not accessible. The BNA may contain a 5-membered ring, 6-membered ring or 7-membered ring bridged structure with a "fixed" C3'-endo sugar puckering. The bridge is typically incorporated at the 2'- and 4'-position of the ribose to provide a 2',4'-BNA nucleotide. In some embodiments, the BNA may be an LNA, an ENA, a cET BNA, etc., wherein the LNA is as shown by Formula (12), the ENA is as shown by Formula (13) and the cET BNA is as shown by Formula (14):

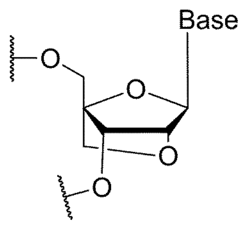
,
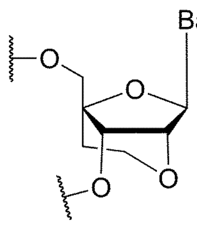
,
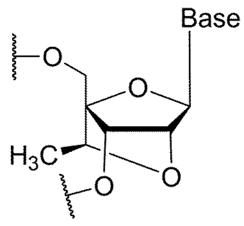
.

Formula (12) Formula (13) Formula (14).

An acyclic nucleotide is a nucleotide in which a sugar ring is opened. In some embodiments, the acyclic nucleotide may be an unlocked nucleic acid (UNA) or a glycerol nucleic acid (GNA), wherein the UNA is as shown by Formula (15), and the GNA is as shown by Formula (16):

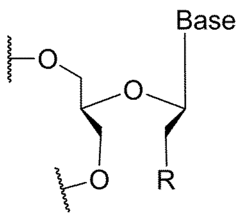
,
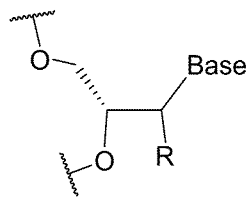
.

Formula (15) Formula (16).

In the Formula (15) and the Formula (16), R is selected from H, OH or alkoxy (O-alkyl).

An isonucleotide is a compound which is formed by altering the position of the base on the ribose ring in a nucleotide. In some embodiments, the isonucleotide may be a compound in which the base is transferred from 1'-position to 2'-position or 3'-position on the ribose ring, as shown by Formula (17) or (18).

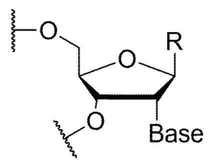

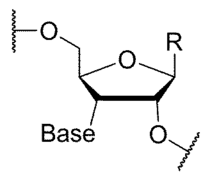

Formula (17) Formula (18).

In the compounds as shown by the Formula (17) and Formula (18) above, Base represents a nucleic acid base, such as A, U, G, C or T; and R is selected from H, OH, F or a non-fluoro group described above.

In some embodiments, the nucleotide analogue is selected from one of an isonucleotide, an LNA, an ENA, a cET, a UNA and a GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which the 2'-hydroxy of the ribose group is substituted with fluoro" and a "nucleotide with 2'-fluororibosyl" have the same meaning, referring to the compound formed by substituting the 2'-hydroxy of the nucleotide with fluoro, having a structure as shown by Formula (7). A "methoxy modified nucleotide", a "2'-methoxy modified nucleotide", a "nucleotide in which the 2'-hydroxy of the ribose group is substituted with methoxy" and a "nucleotide with 2'-methoxyribosyl" have the same meaning, referring to the compound formed by substituting the 2'-hydroxy of the ribose group of the nucleotide with methoxy, having a structure as shown by Formula (8).

In some embodiments, the siRNA of the present disclosure is an siRNA with the following modifications: in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the rest of positions in the sense strand are methoxy modified nucleotides; and the nucleotides at positions 2, 6, 14 and 16 or at positions 2, 6, 8, 9, 14 and 16 of the nucleotide sequence II in the antisense strand are fluoro modified nucleotides, and the nucleotides at the rest of positions in the antisense strand are methoxy modified nucleotides.

In some embodiments, the siRNA of the present disclosure is an siRNA with the following modifications: in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides.

In some embodiments, the siRNA provided by the present disclosure is any one of siPKKa1-M1, siPKKa1-M2, siPKKa1-M3, siPKKa2-M1, siPKKa2-M2, siPKKa2-M3, siPKKb1-M1, siPKKb1-M2, siPKKb1-M3, siPKKb2-M1, siPKKb2-M2, siPKKb2-M3, siPKKc1-M1, siPKKc1-M2, siPKKc1-M3, siPKKc2-M1, siPKKc2-M2, siPKKc2-M3, siPKKd1-M1, siPKKd1-M2, siPKKd1-M3, siPKKd2-M1, siPKKd2-M2, siPKKd2-M3, siPKKe1-M1, siPKKe1-M2, siPKKe1-M3, siPKKe2-M1, siPKKe2-M2, siPKKe2-M3, siPKKf1-M1, siPKKf1-M2, siPKKf1-M3, siPKKf2-M1, siPKKf2-M2 and siPKKf2-M3 listed in Tables 1a-1f.

The siRNAs with the above modifications is not only low-cost,, but also allow the ribonucleases in the blood to be less liable to cleaving the nucleic acid so as to increase the stability of the nucleic acid and enable the nucleic acid to have stronger resistance against nuclease hydrolysis. Meanwhile, the modified siRNA above has higher activity of inhibiting the target mRNA.

In some embodiments, at least part of the phosphoester groups in phosphate-sugar backbone of at least one single strand in the sense strand and the antisense strand of the siRNA provided by the present disclosure are phosphoester groups with modified group. In some embodiments, the phosphoester group with modified group is a phosphorothioate group formed by substituting at least one oxygen atom in a phosphodiester bond in the phosphoester group with a sulfur atom; and in some embodiments, the phosphoester group with modified group is a phosphorothioate group having a structure as shown by Formula (1):

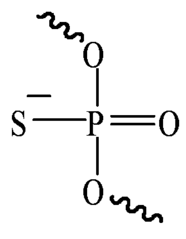
Formula (1).

This modification can stabilize the double-stranded structure of the siRNA, thereby maintaining high specificity and high affinity of base pairing.

In some embodiments, in the siRNA provided by the present disclosure, a phosphorothioate linkage exists in

at least one of the group consisiting of the following positions: the position between the first nucleotide and second nucleotides at either terminal of the sense strand or antisense strand; the position between the second and third nucleotides at either terminal of the sense strand or antisense strand; or any combination thereof. In some embodiments, a phosphorothioate linkage exists at all the above positions except for 5' terminal of the sense strand. In some embodiments, a phosphorothioate linkage exists at all the above positions except for 3' terminal of the sense strand. In some embodiments, a phosphorothioate linkage exists in at least one of the following positions:

the position between the first nucleotide and the second nucleotide at 5' terminal of the sense strand;

the position between the second nucleotide and the third nucleotide at 5' terminal of the sense strand;

the position between the first nucleotide and the second nucleotide at 3' terminal of the sense strand;

the position between the second nucleotide and the third nucleotide at 3' terminal of the sense strand;

the position between the first nucleotide and the second nucleotide at 5' terminal of the antisense strand;

the position between the second nucleotide and the third nucleotide at 5' terminal of the antisense strand;

the position between the first nucleotide and the second nucleotide at 3' terminal of the antisense strand; and

the position between the second nucleotide and the third nucleotide at 3' terminal of the antisense strand.

In some embodiments, the siRNA provided by the present disclosure is any one of siPKKa1-M1S, siPKKa1-M2S, siPKKa1-M3S, siPKKa2-M1S, siPKKa2-M2S, siPKKa2-M3S, siPKKb1-M1S, siPKKb1-M2S, siPKKb1-M3S, siPKKb2-M1S, siPKKb2-M2S, siPKKb2-M3S, siPKKc1-M1S, siPKKc1-M2S, siPKKc1-M3S, siPKKc2-M1S, siPKKc2-M2S, siPKKc2-M3S, siPKKd1-M1S, siPKKd1-M2S, siPKKd1-M3S, siPKKd2-M1S, siPKKd2-M2S, siPKKd2-M3S, siPKKe1-M1S, siPKKe1-M2S, siPKKe1-M3S, siPKKe2-M1S, siPKKe2-M2S, siPKKe2-M3S, siPKKf1-M1S, siPKKf1-M2S, siPKKf1-M3S, siPKKf2-M1S, siPKKf2-M2S and siPKKf2-M3S listed in Tables 1a-1f.

In some embodiments, the 5'-terminal nucleotide in the antisense strand of the siRNA is a 5'-phosphate nucleotide or a 5'-phosphate analogue modified nucleotide.

Common types of the 5'-phosphate nucleotides or 5'-phosphate analogue modified nucleotides are well known to those skilled in the art; for example, the 5'-phosphate nucleotides may have the following structure:

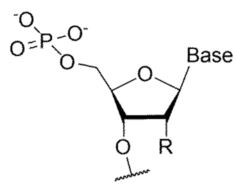

Formula (2).

For another example, Anastasia Khvorova and Jonathan K. Watts, The chemical evolution of oligonucleotide therapies of clinical utility. Nature Biotechnology, 2017, 35(3): 238-48, disclose the following four 5'-phosphate analogue modified nucleotides:

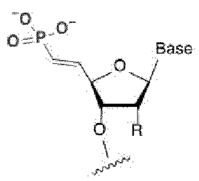

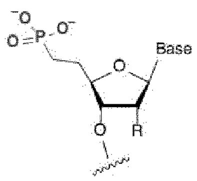

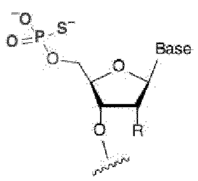

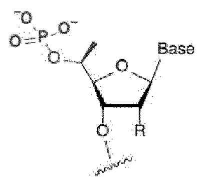

Formula (3) Formula (4) Formula (5) Formula (6)

wherein, R is selected from H, OH, methoxy or F; and Base represents a nucleic acid base selected from A, U, C, G, or T.

In some embodiments, the 5'-phosphate nucleotide is a nucleotide with 5'-phosphate modification as shown

by Formula (2); the 5'-phosphate analogue modified nucleotide is a nucleotide with 5'-(E)-vinylphosphonate (E-VP) modification as shown by Formula (3) or a phosphorothioate modified nucleotide as shown by Formula (5).

In some embodiments, the siRNA provided by the present disclosure is any one of siPKKa1-M1P1, siPKKa1-M2P1, siPKKa1-M3P1, siPKKa2-M1P1, siPKKa2-M2P1, siPKKa2-M3P1, siPKKa1-M1SP1, siPKKa1-M2SP1, siPKKa1-M3SP1, siPKKa2-M1SP1, siPKKa2-M2SP1, siPKKa2-M3SP1, siPKKb1-M1P1, siPKKb1-M2P1, siPKKb1-M3P1, siPKKb2-M1P1, siPKKb2-M2P1, siPKKb2-M3P1, siPKKb1-M1SP1, siPKKb1-M2SP1, siPKKb1-M3SP1, siPKKb2-M1SP1, siPKKb2-M2SP1, siPKKb2-M3SP1, siPKKc1-M1P1, siPKKc1-M2P1, siPKKc1-M3P1, siPKKc2-M1P1, siPKKc2-M2P1, siPKKc2-M3P1, siPKKc1-M1SP1, siPKKc1-M2SP1, siPKKc1-M3SP1, siPKKc2-M1SP1, siPKKc2-M2SP1, siPKKc2-M3SP1, siPKKd1-M1P1, siPKKd1-M2P1, siPKKd1-M3P1, siPKKd2-M1P1, siPKKd2-M2P1, siPKKd2-M3P1, siPKKd1-M1SP1, siPKKd1-M2SP1, siPKKd1-M3SP1, siPKKd2-M1SP1, siPKKd2-M2SP1, siPKKd2-M3SP1, siPKKe1-M1P1, siPKKe1-M2P1, siPKKe1-M3P1, siPKKe2-M1P1, siPKKe2-M2P1, siPKKe2-M3P1, siPKKe1-M1SP1, siPKKe1-M2SP1, siPKKe1-M3SP1, siPKKe2-M1SP1, siPKKe2-M2SP1, siPKKe2-M3SP1, siPKKf1-M1P1, siPKKf1-M2P1, siPKKf1-M3P1, siPKKf2-M1P1, siPKKf2-M2P1, siPKKf2-M3P1, siPKKf1-M1SP1, siPKKf1-M2SP1, siPKKf1-M3SP1, siPKKf2-M1SP1, siPKKf2-M2SP1 and siPKKf2-M3SP1 listed in Tables 1a-1f.

The inventors of the present disclosure have surprisingly found that the siRNA provided by the present disclosure not only has significantly enhanced plasma and lysosomal stability, but alos has higher inhibitory activity of target mRNA.

The siRNA provided by the present disclosure can be obtained by conventional methods for preparing siRNAs in the art (e.g., solid phase synthesis and liquid phase synthesis methods). Wherein, commercial customization services have already been available for solid phase synthesis. Modified nucleotide groups may be introduced into the siRNA of the present disclosure by using a nucleotide monomer having a corresponding modification, wherein the methods for preparing the nucleotide monomer having the corresponding modification and the methods for introducing the modified nucleotide group into the siRNA are also well-known to those skilled in the art.

Pharmaceutical composition

The present disclosure provides a pharmaceutical composition, wherein the pharmaceutical composition comprises the siRNA described above as an active ingredient, and a pharmaceutically acceptable carrier.

The pharmaceutically acceptable carrier may be a carrier conventionally used in the field of siRNA administration, for example, but not limited to, one or more of magnetic nanoparticles (such as $Fe_3O_4$ or $Fe_2O_3$-based nanoparticle), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, in the pharmaceutical composition, there are no special requirements for the contents of the siRNA and the pharmaceutically acceptable carrier. In some embodiments, the weight ratio of the siRNA to the pharmaceutically acceptable carrier is 1:(1-500), and in some embodiments, the weight ratio above is 1:(1-50).

In some embodiments, the pharmaceutical composition may also comprise other pharmaceutically acceptable excipients, which may be one or more of various conventional formulations or compounds in the art. For example, other pharmaceutically acceptable excipients may comprise at least one of a pH buffer solution, a protective agent and an osmotic pressure regulator.

The pH buffer solution may be a tris(hydroxymethyl) aminomethane hydrochloride buffer solution with a pH of 7.5-8.5, and/or a phosphate buffer solution with a pH of 5.5-8.5, for example a phosphate buffer solution with a pH of 5.5-8.5.

The protective agent may be at least one of inositol, sorbitol, sucrose, trehalose, mannose, maltose, lactose, and glucose. The content of the protective agent may be from 0.01 wt% to 30 wt% on the basis of the total weight of the pharmaceutical composition.

The osmotic pressure regulator may be sodium chloride and/or potassium chloride. The content of the osmotic pressure regulator allows an osmotic pressure of the pharmaceutical composition to be 200-700 milliosmol/kg (mOsm/kg). Depending on the desired osmotic pressure, those skilled in the art can readily determine the content of the osmotic pressure regulator.

In some embodiments, the pharmaceutical composition may be a liquid formulation, for example, an injection solution; or a lyophilized powder for injection, which is mixed with a liquid excipient to form a liquid formulation upon administration. The liquid formulation may be administered by, but not limited to, subcutaneous, intramuscular or intravenous injection routes, and also may be administered to, but not limited to, lung by spray, or other organs (such as liver) via lung by spray. In some embodiments, the pharmaceutical composition is administered by intravenous injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid and/or a pegylated lipid. The

organic amine, the helper lipid and the pegylated lipid may be respectively selected from one or more of the amine-containing transfection compounds or the pharmaceutically acceptable salts or derivatives thereof, the helper lipids and the pegylated lipids as described in Chinese patent application CN103380113A (which is incorporated herein by reference in its entirety).

In some embodiments, the organic amine may be a compound as shown by Formula (201) as described in CN103380113A or a pharmaceutically acceptable salt thereof:

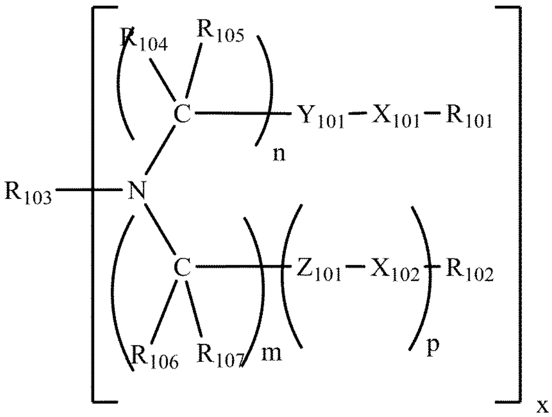

Formula (201),

wherein:

each $X_{101}$ or $X_{102}$ is independently selected from O, S, N-A or C-A, wherein A is hydrogen or a C1-C20 hydrocarbon chain;
each $Y_{101}$ or $Z_{101}$ is independently selected from C=O, C=S, S=O, CH-OH or $SO_2$;
each $R_{101}$, $R_{102}$, $R_{103}$, $R_{104}$, $R_{105}$, $R_{106}$ or $R_{107}$ is independently hydrogen; a cyclic or acyclic, substituted or unsubstituted, branched or linear aliphatic group; a cyclic or acyclic, substituted or unsubstituted, branched or linear heteroaliphatic group; a substituted or unsubstituted, branched or linear acyl group; a substituted or unsubstituted, branched or linear aryl, or a substituted or unsubstituted, branched or linear heteroaryl;
x is an integer of 1-10;
n is an integer of 1-3, m is an integer of 0-20, and p is 0 or 1, wherein if m=p=0, then $R_{102}$ is hydrogen, and
if at least one of n or m has is 2, then $R_{103}$ and the nitrogen in Formula (201) form a structure as shown by Formula (202) or (203):

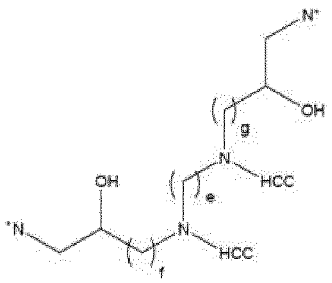

Formula (202),

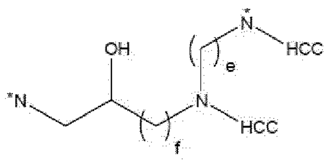

Formula (203);

wherein g, e and f are each independently an integer of 1-6, "HCC" represents a hydrocarbon chain, and each *N represents a nitrogen atom shown in Formula (201).

In some embodiments, $R_{103}$ is a polyamine. In other embodiments, $R_{103}$ is a ketal. In some embodiments, each of $R_{101}$ and $R_{102}$ in the Formula (201) is independently any substituted or unsubstituted, branched or linear alkyl or alkenyl, wherein the alkyl or alkenyl has 3 to about 20 carbon atoms (such as 8 to about 18 carbon atoms) and 0 to 4 double bonds (such as 0 to 2 double bonds).

In some embodiments, if each of n and m is independently 1 or 3, $R_{103}$ may be any of the following Formulae (204)-(213):

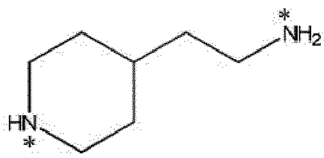 Formula (204), 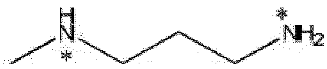 Formula (205),

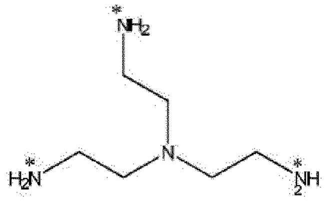 Formula (206), 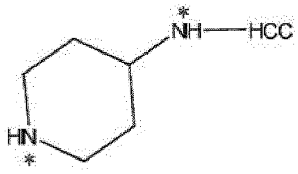 Formula (207),

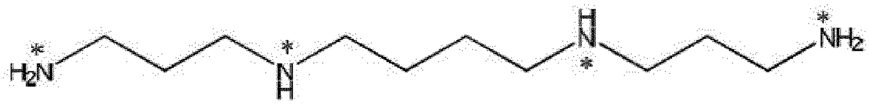 Formula (208),

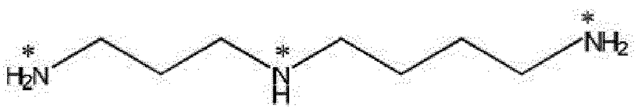 Formula (209),

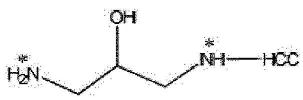 Formula (210), 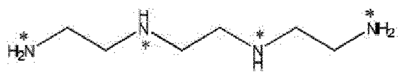 Formula (211),

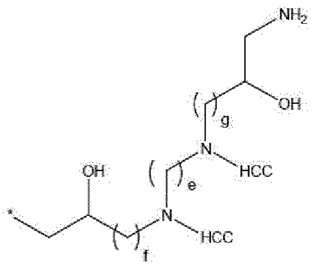 Formula (212) and 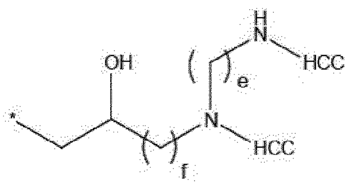 Formula (213);

wherein, in Formula (204) to Formula (213), each of g, e and f is independently an integer of 1-6; each "HCC" represents a hydrocarbon chain, and each * represents a potential attachment point of $R_{103}$ to the nitrogen atom in Formula (201), wherein each H at any * position may be replaced to realize the attachment to the nitrogen atom in Formula (201).

Wherein, the compound as shown by (201) may be prepared as described in CN103380113A.

In some embodiments, the organic amine may be an organic amine as shown by Formula (214) and/or an organic amine as shown by Formula (215):

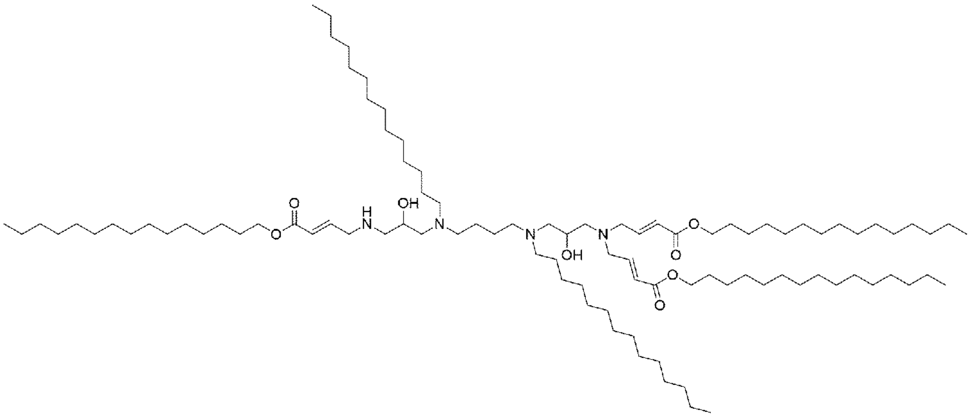

Formula (214),

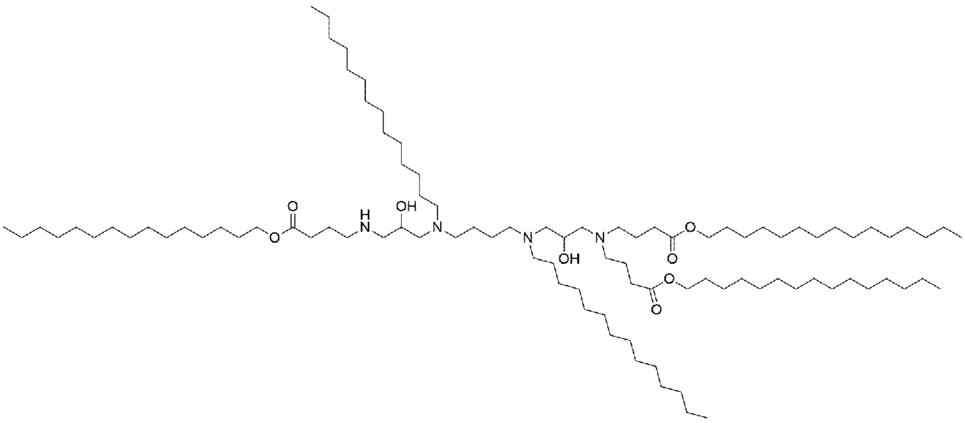

Formula (215);

The helper lipid is a cholesterol, a cholesterol analogue and/or a cholesterol derivative.

The pegylated lipid is 1,2-dipalmitoyl-sn-glycero-3-phosphatidylethanolamine-N-[methoxy(polyethylene glycol)]-2000.

In some embodiments, the molar ratio among the organic amine, the helper lipid, and the pegylated lipid in the pharmaceutical composition is (19.7-80):(19.7-80):(0.3-50); for example, the molar ratio may be (50-70):(20-40):(3-20).

In some embodiments, the pharmaceutical composition particles formed by the siRNA of the present disclosure and the above amine-containing transfection agent have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30, 40, 50, 60, 70, 75, 80, 85, 90, 100, 110, 120, 130, 140, 150 or 160 nm.

In some embodiments, in the pharmaceutical composition formed by the siRNA of the present disclosure and the above amine-containing transfection agent, the weight ratio (weight/weight ratio) of the siRNA to total lipids (e.g., the organic amine, the helper lipid and/or the pegylated lipid), ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the siRNA of the present disclosure to the total lipids is about 1:5, 1:6, 1:7, 1:8, 1:9, 1:10, 1:11, 1:12, 1:13, 1:14, 1:15, 1:16, 1:17 or 1:18.

In some embodiments, the pharmaceutical composition may be marketed with each component being separate, and used in the form of a liquid formulation. In some embodiments, the pharmaceutical composition formed by the siRNA of the present disclosure and the above pharmaceutically acceptable carrier may be prepared by various known processes, except replacing the existing siRNA with the siRNA of the present disclosure. In some embodiments, the pharmaceutical composition may be prepared according to the following process.

The organic amine, the helper lipid and the pegylated lipid are suspended in alcohol at a molar ratio as described above and mixed homogeneously to yield a lipid solution; and the alcohol is used in an amount such that the resultant lipid solution is present at a total mass concentration of 2 to 25 mg/mL, e.g., 8 to 18 mg/mL. The alcohol is selected from pharmaceutically acceptable alcohols, such as an alcohol that is in liquid form near room temperature, for example, one or more of ethanol, propylene glycol, benzyl alcohol, glycerol, PEG 200, PEG 300, PEG 400, and for example, may be

ethanol.

The siRNA provided by the present disclosure is dissolved in a buffered salt solution to obtain an aqueous solution of the siRNA. The buffered salt solution has a concentration of 0.05-0.5 M, e.g., may be 0.1-0.2 M. The pH of the buffered salt solution is adjusted to 4.0-5.5, e.g., may be 5.0-5.2. The buffered salt solution is used in an amount such that the siRNA is present at a concentration of no more than 0.6 mg/ml, e.g., may be 0.2-0.4 mg/mL. The buffered salt may be one or more selected from the group consisting of soluble acetate and soluble citrate, e.g., may be sodium acetate and/or potassium acetate.

The lipid solution and the aqueous solution of the siRNA are mixed. The product obtained after mixing is incubated at a temperature of 40-60°C for at least 2 minutes (e.g., may be 5-30 minutes) to produce an incubated liposome formulation. The volume ratio of the lipid solution to the aqueous solution of the siRNA is 1:(2-5), for example, may be 1:4.

The incubated liposome formulation is concentrated or diluted, purified to remove impurities, and then sterilized to obtain the pharmaceutical composition provided by the present disclosure, which has physicochemical parameters as follows: a pH of 6.5-8, an encapsulation percentage of not less than 80%, a particle size of 40-200 nm, a polydispersity index of not higher than 0.30, and an osmotic pressure of 250-400 mOsm/kg; for example, the physicochemical parameters may be as follows: a pH of 7.2-7.6, an encapsulation percentage of not less than 90%, a particle size of 60-100 nm, a polydispersity index of not higher than 0.20, and an osmotic pressure of 300-400 mOsm/kg.

The concentration or dilution step may be performed before, after or simultaneously with the step of impurity removal. The method for removing impurities may be any of various existing methods, for example, ultrafiltration using tangential flow system viahollow fiber column at 100 KDa and with a phosphate buffer solution (PBS) at pH 7.4 as an ultrafiltration exchange solution. The method for sterilization may be any of various existing methods, such as filtration sterilization on a 0.22 μm filter.

siRNA conjugate

The present disclosure provides an siRNA conjugate, wherein the siRNA conjugate comprises the above-mentioned siRNA and a conjugating group conjugated to the siRNA.

Generally, the conjugating group comprises at least one pharmaceutically acceptable targeting group and an optional linker. Moreover, the siRNA, the linker and the targeting group are linked in succession. In some embodiments, there are 1-6 targeting groups. In some embodiments, there are 2-4 targeting groups. The siRNA molecule may be non-covalently or covalently conjugated to the conjugating group, for example, the siRNA molecule may be covalently conjugated to the conjugating group. The conjugating site between the siRNA and the conjugating group may be at 3'-terminal or 5'-terminal of the sense strand of the siRNA, or at 5'-terminal of the antisense strand, or may be within the internal sequence of the siRNA. In some embodiments, the conjugating site between the siRNA and the conjugating group is at 3' terminal of the sense strand of the siRNA.

In some embodiments, the conjugating group may be linked to a phosphate group, the 2'-hydroxy or the base of a nucleotide. In some embodiments, the conjugating group may be linked to a 3'-hydroxy when the nucleotides are linked to each other via a 2'-5'-phosphodiester bond. When the conjugating group is linked to a terminal of the siRNA, the conjugating group is typically linked to the phosphate group of a nucleotide; when the conjugating group is linked to the internal sequence of the siRNA, the conjugating group is typically linked to a ribose ring or a base. For various linking modes, reference may be made to: Muthiah Manoharan et.al, siRNA conjugates carrying sequentially assembled trivalent N-acetylgalactosamine linked through nucleosides elicit robust gene silencing in vivo in hepatocytes. ACS Chemical biology, 2015,10(5):1181-7.

In some embodiments, the siRNA and the conjugating group may be linked by an acid labile or reducible chemical bond, and these chemical bonds may be degraded under the acidic environment of cell endosomes, thereby rendering the siRNA to be in free state. For non-degradable conjugating modes, the conjugating group may be linked to the sense strand of the siRNA, thereby minimizing the effect of conjugation on the activity of the siRNA.

In some embodiments, the pharmaceutically acceptable targeting group may be a conventionally used ligand in the field of siRNA administration, for example, the various ligands as described in WO2009082607A2, which is incorporated herein by reference in its entirety.

In some embodiments, the pharmaceutically acceptable targeting group may be selected from one or more of the ligands formed by the following targeting molecules or derivatives thereof: lipophilic molecules, such as cholesterol, bile acids, vitamins (such as vitamin E), lipid molecules of different chain lengths; polymers, such as polyethylene glycol; polypeptides, such as cell-penetrating peptide; aptamers; antibodies; quantum dots; saccharides, such as lactose, polylactose, mannose, galactose, and N-acetylgalactosamine (GalNAc); folate; and receptor ligands expressed in hepatic parenchymal cells, such as asialoglycoprotein, asialo-sugar residue, lipoproteins (such as high density lipoprotein, low density lipoprotein), glucagon, neurotransmitters (such as adrenaline), growth factors, transferrin and the like.

In some embodiments, each ligand is independently selected from a ligand capable of binding to a cell surface

receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human cell surface receptor. In some embodiments, at least one ligand is a ligand capable of binding to a hepatic surface asialoglycoprotein receptor (ASGP-R). The types of these ligands are well-known to those skilled in the art and they typically serve the function of binding to specific receptors on the surface of the target cell, thereby mediating delivery of the siRNA linked to the ligand into the target cell.

In some embodiments, the pharmaceutically acceptable targeting group may be any ligand that binds to asialoglycoprotein receptors (ASGP-R) on the surface of mammalian hepatocytes. In one embodiment, each ligand is independently an asialoglycoprotein, such as asialoorosomucoid (ASOR) or asialofetuin (ASF). In some embodiments, the ligand is a saccharide or a saccharide derivative.

In some embodiments, at least one ligand is a saccharide. In some embodiments, each ligand is a saccharide. In some embodiments, at least one ligand is a monosaccharide, polysaccharide, modified monosaccharide, modified polysaccharide, or saccharide derivative. In some embodiments, at least one ligand may be a monosaccharide, disaccharide or trisaccharide. In some embodiments, at least one ligand is a modified saccharide. In some embodiments, each ligand is a modified saccharide. In some embodiments, each ligand is independently selected from the group consisting of polysaccharides, modified polysaccharides, monosaccharides, modified monosaccharides, polysaccharide derivatives or monosaccharide derivatives. In some embodiments, each ligand or at least one ligand is selected from the group consisting of the following saccharides: glucose and derivative thereof, mannose and derivative thereof, galactose and derivative thereof, xylose and derivative thereof, ribose and derivative thereof, fucose and derivative thereof, lactose and derivative thereof, maltose and derivative thereof, arabinose and derivative thereof, fructose and derivative thereof, and sialic acid.

In some embodiments, each ligand may be independently selected from D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucofuranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-a-D-glucoheptopyranoside, 2,5-anhydro-D-allononitrile, ribose, D-ribose, D-4-thioribose, L-ribose, or L-4-thioribose. Other selections for the ligand may be found, for example, in the description of CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecules may be monovalent, bivalent, trivalent and tetravalent. It should be understood that the terms monovalent, bivalent, trivalent and tetravalent described herein respectively mean that the molar ratio of the siRNA molecule to the galactose or N-acetylgalactosamine molecule in the siRNA conjugate is 1:1, 1:2, 1:3 or 1:4, when the siRNA conjugate is formed by the siRNA molecule and the conjugating group containing galactose or N-acetylgalactosamine as the targeting group. In some embodiments, the pharmaceutically acceptable targeting group is N-acetylgalactosamine. In some embodiments, when the siRNA of the present disclosure is conjugated to a conjugating group comprising N-acetylgalactosamine, the N-acetylgalactosamine molecule is trivalent or tetravalent. In some embodiments, when the siRNA of the present disclosure is conjugated to a conjugating group containing N-acetylgalactosamine, the N-acetylgalactosamine molecule is trivalent.

The targeting group may be linked to the siRNA molecule via an appropriate linker, and the appropriate linker may be selected by those skilled in the art according to the specific type of the targeting group. The types of these linkers and targeting groups and the linking modes to the siRNA may be found in the disclosure of W2015006740A2, which is incorporated herein by reference in its entirety.

In some embodiments, when the targeting group is N-acetylgalactosamine, an appropriate linker may be a structure as shown by Formula (301):

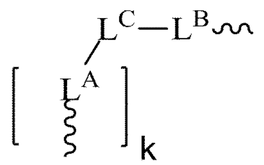

Formula (301)

wherein,

k is an integer of 1-3; and

$L^A$ is an amide bond-comprising chain moiety that has a structure as shown by Formula (302), each $L^A$ being respectively linked to the targeting group and the $L^C$ moiety through an ether bond at two terminals thereof:

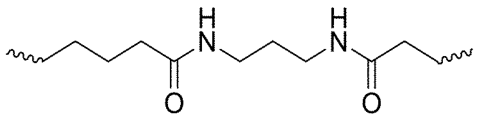
Formula (302);

$L^B$ is an N-acylpyrrolidine-comprising chain moiety that has a structure as shown by Formula (303), the chain moiety having a carbonyl at one terminal thereof and being linked to the $L^C$ moiety through an amide bond, and having an oxy-group at the other terminal thereof and being linked to the siRNA via a phosphoester bond:

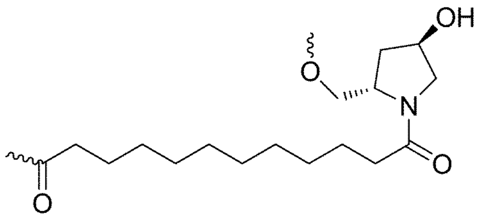
Formula (303);

$L^C$ is a bivalent to tetravalent linking group based on hydroxymethyl aminomethane, dihydroxymethyl aminomethane or trihydroxymethyl aminomethane, the $L^C$ being linked to each of the $L^A$ moieties through an ether bond via an oxygen atom, and being linked to the $L^B$ moiety through an amide bond via a nitrogen atom.

In some embodiments, when n=3 and $L^C$ is a tetravalent linking group based on trihydroxymethyl aminomethane, the siRNA conjugate formed by linking an N-acetylgalactosamine molecule with an siRNA molecule via -$(L^A)_3$-trihydroxymethyl aminomethane-$L^B$- as a linker has a structure as shown by Formula (304):

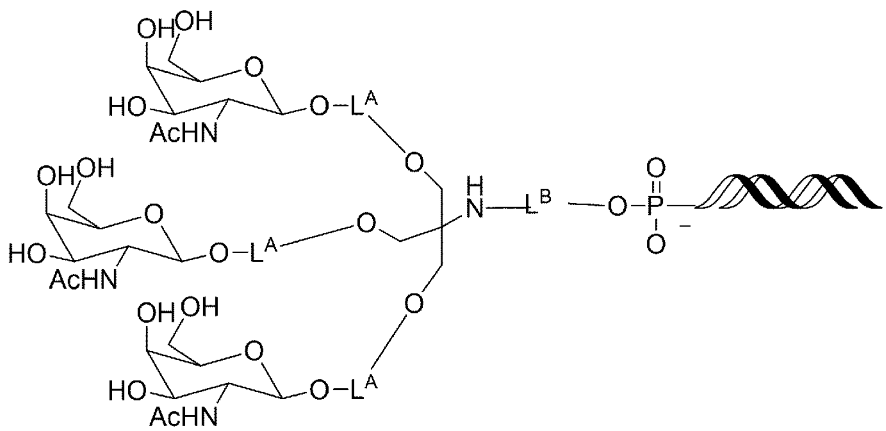
Formula (304),

wherein the double helix structure represents an siRNA.

Likewise, the conjugating site between the siRNA and the conjugating group may be at the 3'-terminal or 5'-terminal of the sense strand of the siRNA, or may be at the 5'-terminal of the antisense strand, or may be within the internal sequence of the siRNA.

In some embodiments, the 3'-terminal of the sense strand of the siRNA of the present disclosure is covalently conjugated to three N-acetylgalactosamine (GalNAc) molecules via a linker -$(L^A)_3$-trihydroxymethyl aminomethane-$L^B$- to obtain an siRNA conjugate in which the molar ratio of the siRNA molecule to the GalNAc molecule is 1:3, which may also be hereinafter referred to as $(GalNAc)_3$-siRNA, and the siRNA conjugate has a structure as shown by Formula (305):

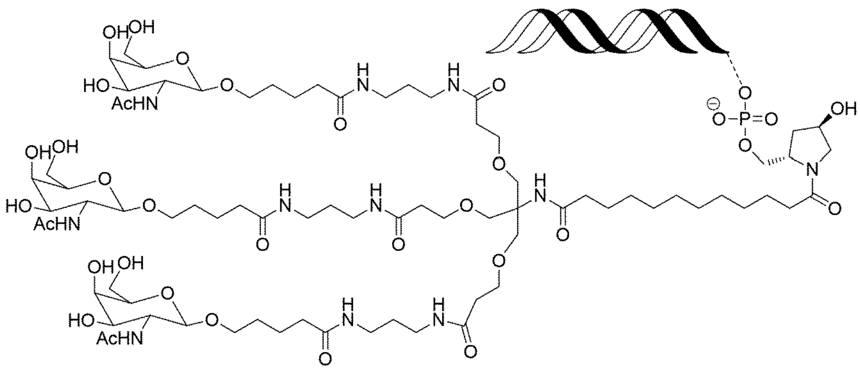

Formula (305),

wherein the double helix structure represents an siRNA; and the linker is linked to the 3' terminal of the sense strand of the siRNA.

In some embodiments, when the targeting group is N-acetylgalactosamine, an appropriate linker may have a structure as shown by Formula (306):

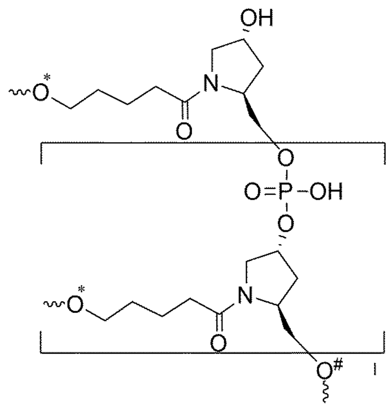

, Formula (306)

wherein,

1 is an integer of 0-3;

* represents a site linked to the targeting group via an ether bond on the linker; and

# represents a site linked to the siRNA via a phosphoester bond on the linker.

In some embodiments, when 1=2, the siRNA conjugate has a structure as shown by Formula (307):

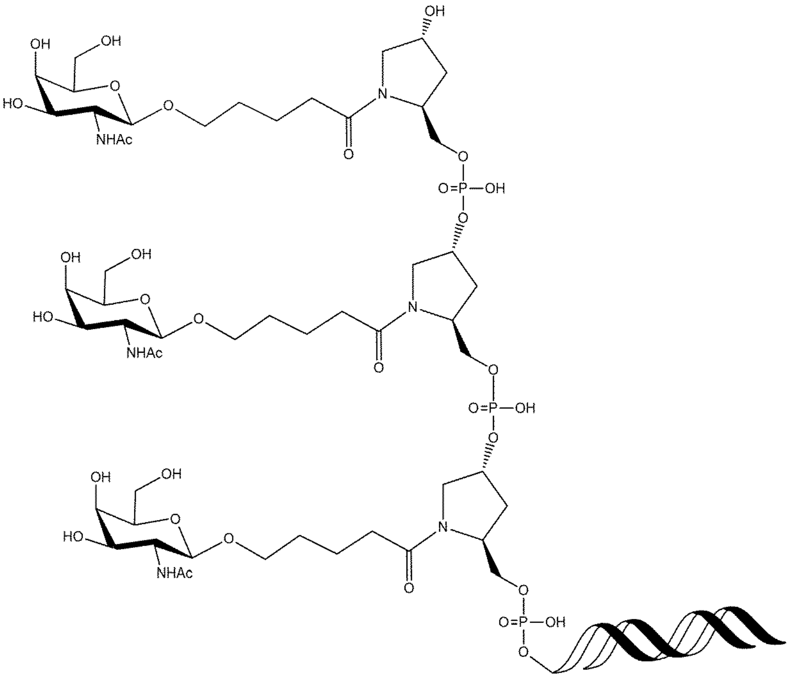

Formula (307),

wherein the double helix structure represents an siRNA; and the linker is linked to the 3' terminal of the sense strand of the siRNA.

The above siRNA conjugates may be synthesized according to the methods described in detail in the prior art. For example, WO2015006740A2 describes the methods of preparing various conjugates in detail. The siRNA conjugate of the present disclosure may be obtained by methods well known to those skilled in the art. For example, WO2014025805A1 describes the preparation method of the structure as shown by Formula (305). Rajeev et al., describes the preparation method of the structure as shown by Formula (307) in ChemBioChem 2015, 16, 903-908.

In some embodiments, the siRNA conjugate has a structure as shown by Formula (308):

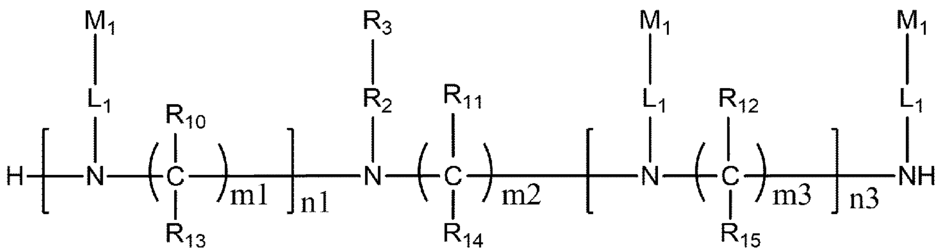

Formula (308),

wherein:

n1 is an integer selected from 1-3, and n3 is an integer selected from 0-4;
m1, m2, and m3 is independently an integer selected from 2-10;
$R_{10}$, $R_{11}$, $R_{12}$, $R_{13}$, $R_{14}$ or $R_{15}$ is each independently H or selected from the group consisting of $C_1$-$C_{10}$ alkyl, $C_1$-$C_{10}$ haloalkyl and $C_1$-$C_{10}$ alkoxy; and
$R_3$ is a group having a structure as shown by Formula A59:

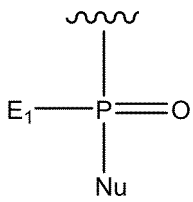

(A59)

wherein, $E_1$ is OH, SH or $BH_2$, and Nu is the siRNA of the present disclosure;

$R_2$ is a linear alkylene of 1-20 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ may optionally have any one or more substituents in the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -CO2H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl); and

each $L_1$ is a linear alkylene of 1-70 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ may optionally have any one or more substituents in the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2H$, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl).

In some embodiments, $L_1$ may be selected from the group consisting of groups A1-A26 and any combination thereof, wherein the structures and definitions of A1-A26 are as follows:

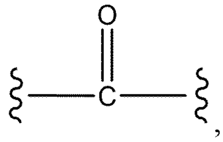 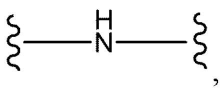 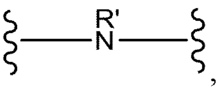 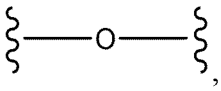

(A1) (A2) (A3) (A4)

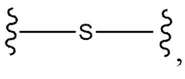 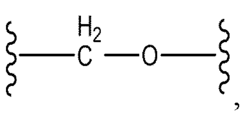 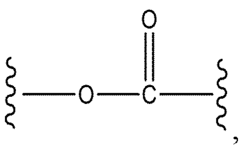 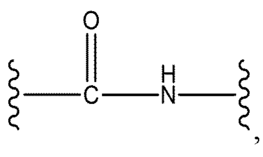

(A5) (A6) (A7) (A8)

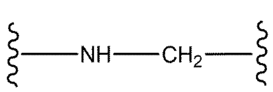, 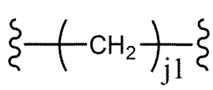, 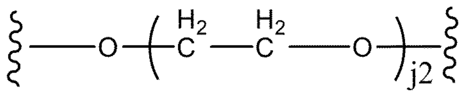,

(A9) (A10) (A11)

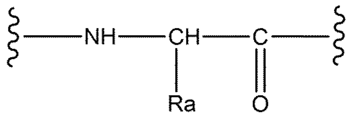, 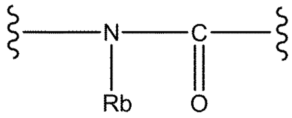, 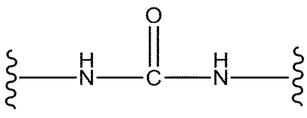,

(A12) (A13) (A14)

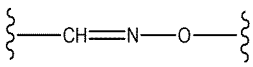, 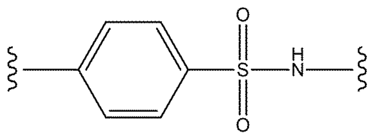, 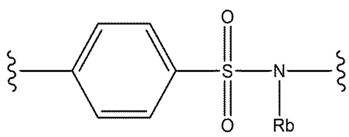,

(A15) (A16) (A17)

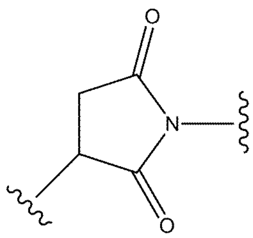, 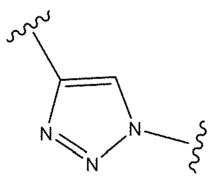, 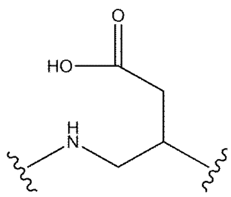, 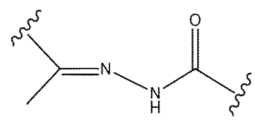,

(A18) (A19) (A20) (A21)

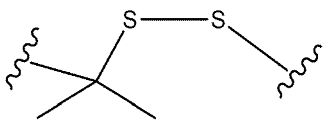, 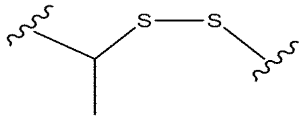, 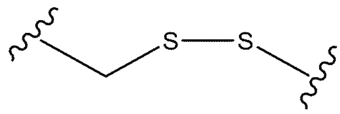,

(A22) (A23) (A24)

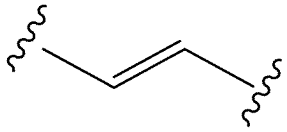, 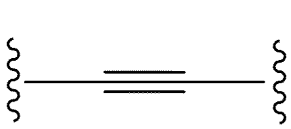;

(A25) (A26)

wherein, j 1 is an integer of 1-20; and j2 is an integer of 1-20;

R' is a $C_1$-$C_{10}$ alkyl; and

Ra is selected from the group consisting of groups A27-A45 and any combinations thereof:

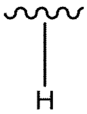 , 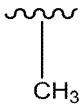 , 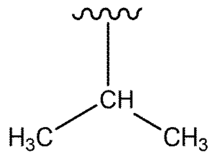 , 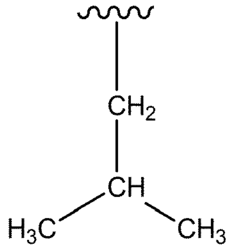 , 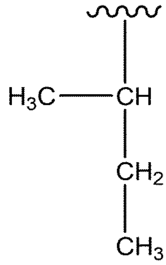 , 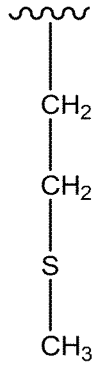 ,

(A27) (A28) (A29) (A30) (A31) (A32)

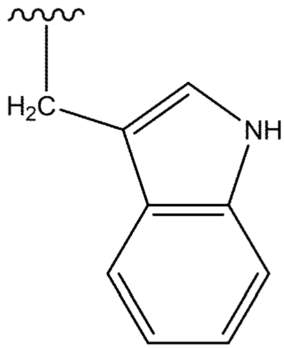 , 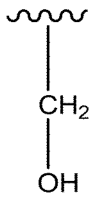 , 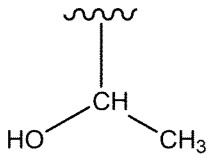 , 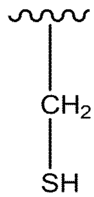 , 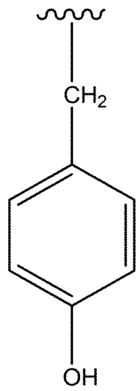 ,

(A33) (A34) (A35) (A36) (A37)

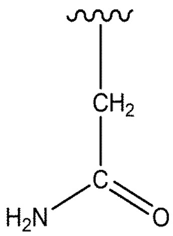 , 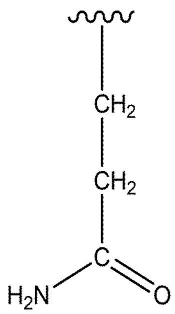 , 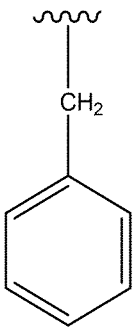 , 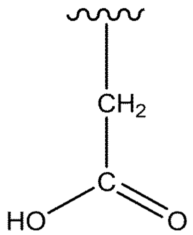 , 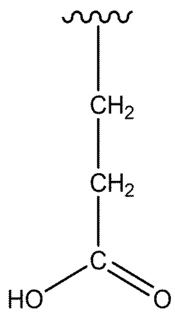 ,

(A38) (A39) (A40) (A41) (A42)

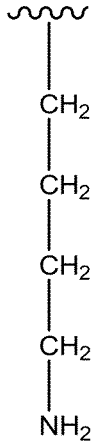, 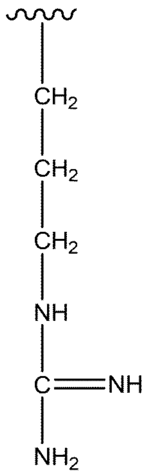 or 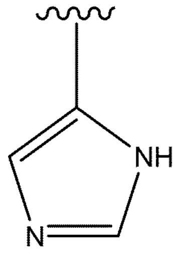 ;

(A43) (A44) (A45)

Rb is a $C_1$-$C_{10}$ alkyl; and  represents a site where the group is covalently linked.

Those skilled in the art would understand that, though $L_1$ is defined as a linear alkylene for convenience, but it may not be a linear group or be named differently, such as an amine or alkenyl produced by the above replacement and/or substitution. For the purpose of the present disclosure, the length of $L_1$ is the number of the atoms in the chain connecting the two attachment points. For this purpose, a ring obtained by replacement of a carbon atom of the linear alkylene, such as a heterocyclylene or heteroarylene, is counted as one atom.

$M_1$ represents a targeting group, of which the definitions and options are the same as those described above. In some embodiments, each $M_1$ is independently selected from one of the ligands that have affinity to the asialoglycoprotein receptor on the surface of mammalian hepatocytes.

When $M_1$ is a ligand that has affinity to the asialoglycoprotein receptor on the surface of mammalian hepatocytes, in some embodiments, nl may be an integer of 1-3, and n3 may be an integer of 0-4 to ensure that the number of the $M_1$ targeting group in the siRNA conjugate may be at least 2. In some embodiments, n1+n3≥2, such that the number of the $M_1$ targeting group in the conjugate may be at least 3, thereby allowing the $M_1$ targeting group to more easily bind to the asialoglycoprotein receptor on the surface of hepatocytes, which may facilitate the endocytosis of the siRNA conjugate into cells. Experiments have shown that when the number of the $M_1$ targeting group is greater than 3, the ease of binding the $M_1$ targeting group to the asialoglycoprotein receptor on the surface of hepatocytes is not significantly increased. Therefore, in view of various aspects such as synthesis convenience, structure/process costs and delivery efficiency, in some embodiments, n1 is an integer of 1-2, n3 is an integer of 0-1, and n+n3=2-3.

In some embodiments, when ml, m2, or m3 is independently selected from an integer of 2-10, the steric mutual positions among a plurality of $M_1$ targeting groups may be fit for the binding of the $M_1$ targeting groups to the asialoglycoprotein receptor on the surface of hepatocytes. In order to make the siRNA conjugate provided by the present disclosure simpler, easier to synthesis and/or reduce cost, in some embodiments, ml, m2 and m3 are each independently an integer of 2-5, and in some embodiments, ml=m2=m3.

Those skilled in the art would understand that when $R_{10}$, $R_{11}$, $R_{12}$, $R_{13}$, $R_{14}$, or $R_{15}$ is each independently selected from one of H, $C_1$-$C_{10}$ alkyl, $C_1$-$C_{10}$ haloalkyl, and $C_1$-$C_{10}$ alkoxy, they would not change the properties of the siRNA conjugate of the present disclosure and all could achieve the purpose of the present disclosure. In some embodiments, $R_{10}$, $R_{11}$, $R_{12}$, $R_{13}$, $R_{14}$, or $R_{15}$ is each independently selected from selected from H, methyl or ethyl. In some embodiments, $R_{10}$, $R_{11}$, $R_{12}$, $R_{13}$, $R_{14}$, and $R_{15}$ are all H.

$R_3$ is a group having the structure as shown by Formula A59, wherein $E_1$ is OH, SH or $BH_2$, and considering the availability of starting materials, in some embodiments, $E_1$ is OH or SH.

$R_2$ is selected to achieve the linkage between the group as shown by Formula A59 and the N atom on a nitrogenous backbone. In the context of the present disclosure, the "nitrogenous backbone" refers to a chain structure in which the carbon atoms attached to $R_{10}$, $R_{11}$, $R_{12}$, $R_{13}$, $R_{14}$, and $R_{15}$ and the N atoms are linked to each other. Therefore, $R_2$ may be any linking group capable of attaching the group A59 to the N atom on a nitrogenous backbone by suitable means. In some embodiments, in the case where the siRNA conjugate as shown by Formula (308) is prepared by a solid phase synthesis process, $R_2$ group needs to have both a site linking to the N atom on the nitrogenous backbone and a site linking to the P atom in $R_3$. In some embodiments, in $R_2$, the site linking to the N atom on the nitrogenous

backbone forms an amide bond with the N atom, and the site linking to the P atom in $R_3$ forms a phosphoester bond with the P atom. In some embodiments, $R_2$ may be B5, B6, B5' or B6':

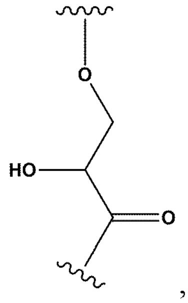
,
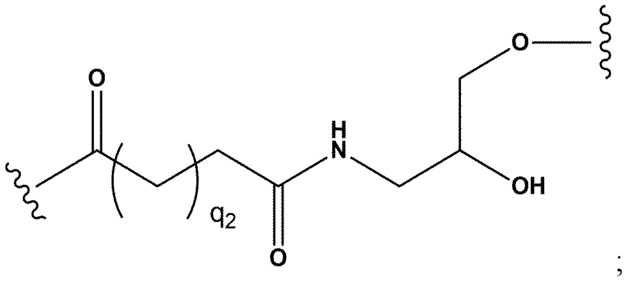
;

(B5) (B6),

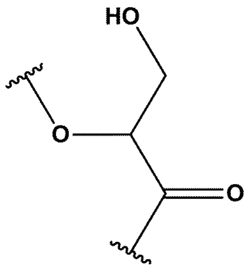
,
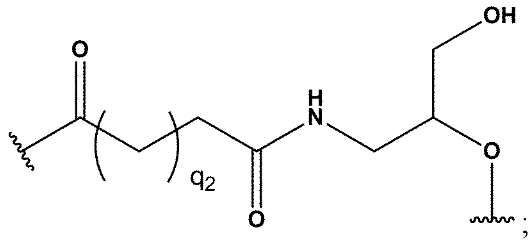
;

(B5') (B6'),

wherein,  represents a site where the group is covalently linked.

A value range of $q_2$ may be an integer of 1-10; and in some embodiments, $q_2$ is an integer of 1-5.

The role of $L_1$ is to link the $M_1$ targeting group to the N atom on the nitrogenous backbone, thereby providing liver targeting function for the siRNA conjugate as shown by Formula (308). In some embodiments, $L_1$ is selected from the connection combinations of one or more of groups Al-A26. In some embodiments, $L_1$ is selected from the connection combinations of one or more of A1, A4, A5, A6, A8, A10, A11, and A13. In some embodiments, $L_1$ is selected from the connection combinations of at least two of A1, A4, A8, A10, and A11. In some embodiments, $L_1$ is selected from the connection combinations of at least two of A1, A8, and A10.

In some embodiments, the length of $L_1$ may be 3-25 atoms, 3-20 atoms, 4-15 atoms or 5-12 atoms. In some embodiments, the length of $L_1$ is 3, 4, 5, 6, 7, 8, 9, 10, 11, 12, 13, 14, 15, 16, 17, 18, 19, 20, 21, 22, 23, 24, 25, 30, 35, 40, 45, 50, 55, 60 atoms.

In some embodiments, j1 is an integer of 2-10, and in some embodiments, j1 is an integer of 3-5. In some embodiments, j2 is an integer of 2-10, and in some embodiments, j2 is an integer of 3-5. R' is a $C_1$-$C_4$ alkyl, and in some embodiments, R' is one of methyl, ethyl, and isopropyl. Ra is one of A27, A28, A29, A30, and A31, and in some embodiments, Ra is A27 or A28. Rb is a $C_1$-$C_5$ alkyl, and in some embodiments, Rb is one of methyl, ethyl, isopropyl, and butyl. In some embodiments, jl, j2, R', Ra, and Rb are respectively selected by Formulae Al-A26 to achieve the linkage between the $M_1$ targeting group and the N atom on the nitrogenous backbone, and to make the steric mutual position among the $M_1$ targeting group more suitable for the binding of the $M_1$ targeting group to the asialoglycoprotein receptor on the surface of hepatocytes.

In some embodiments, the siRNA conjugate has a structure as shown by Formula (403), (404), (405), (406), (407), (408), (409), (410), (411), (412), (413), (414), (415), (416), (417), (418), (419), (420), (421) or (422):

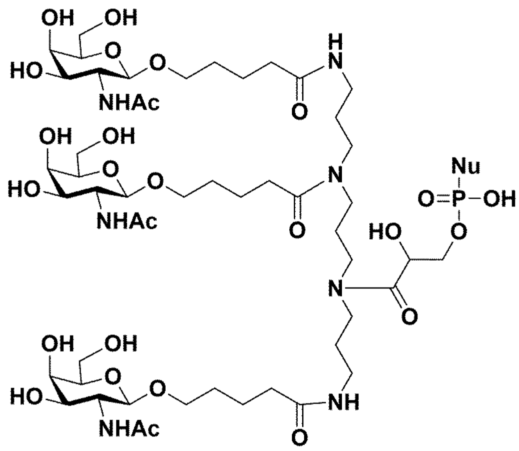

Formula (403)

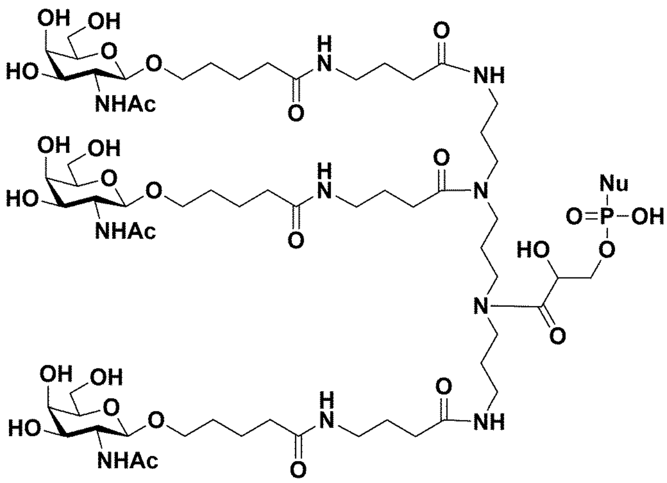

Formula (404)

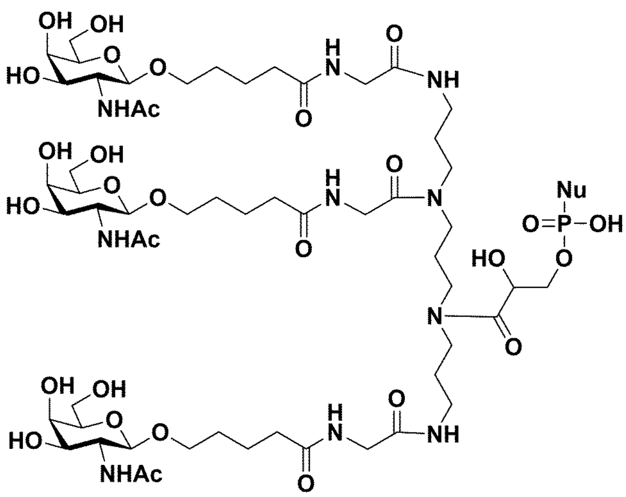

Formula (405)

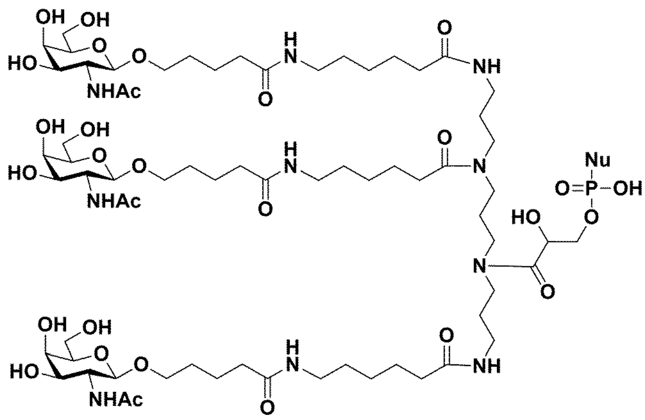

Formula (406)

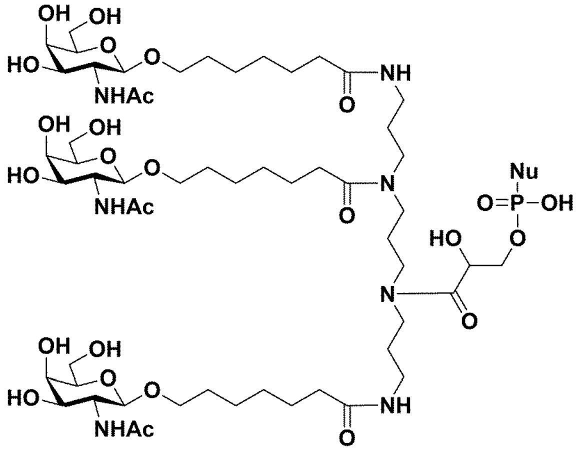

Formula (407)

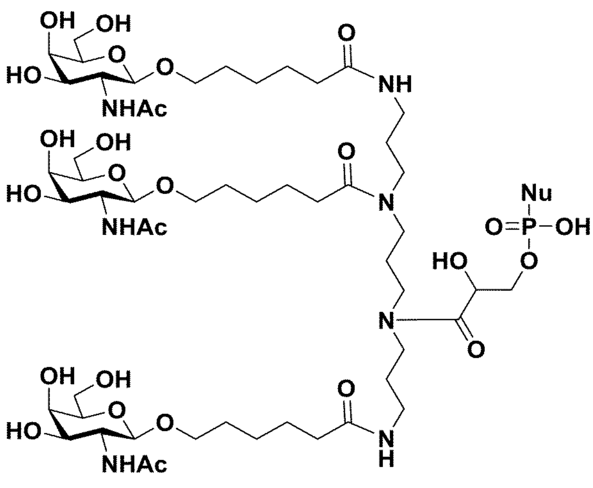

Formula (408)

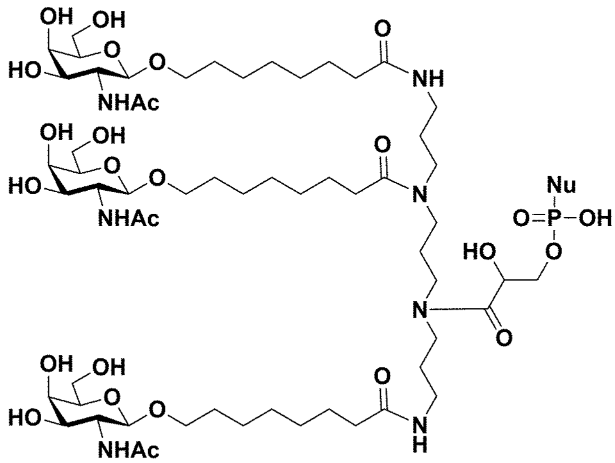

Formula (409)

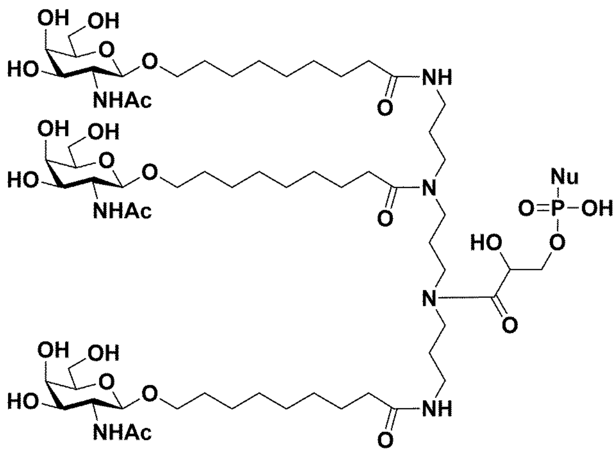

Formula (410)

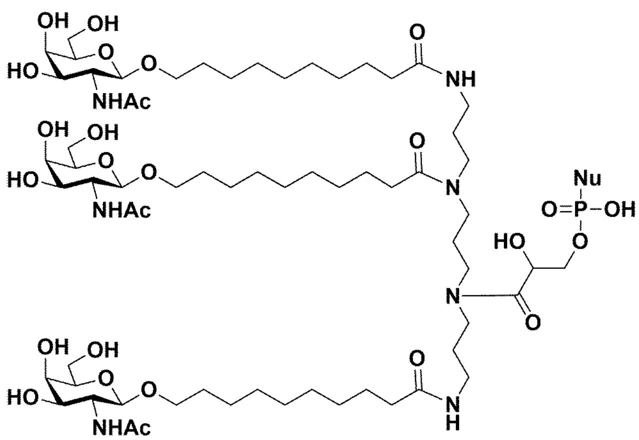

Formula (411)

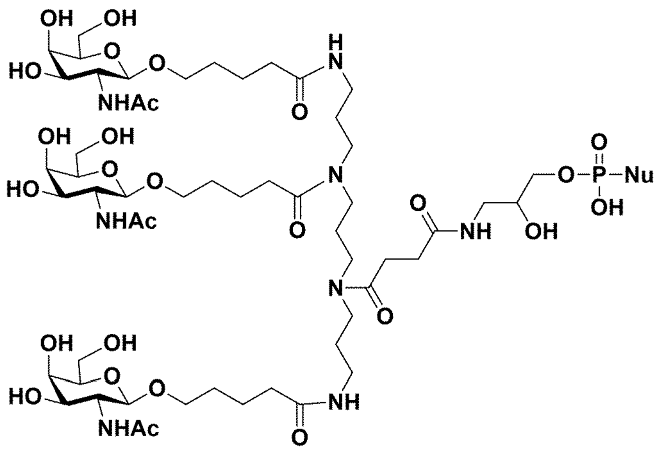

Formula (412)

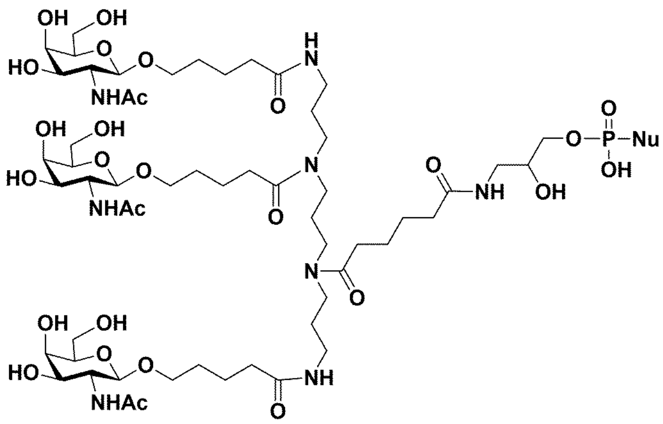

Formula (413)

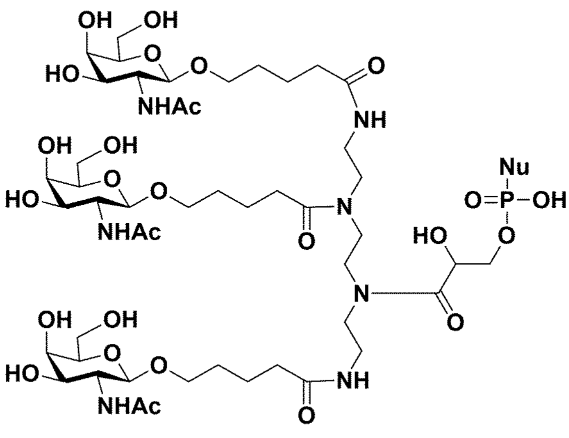

Formula (414)

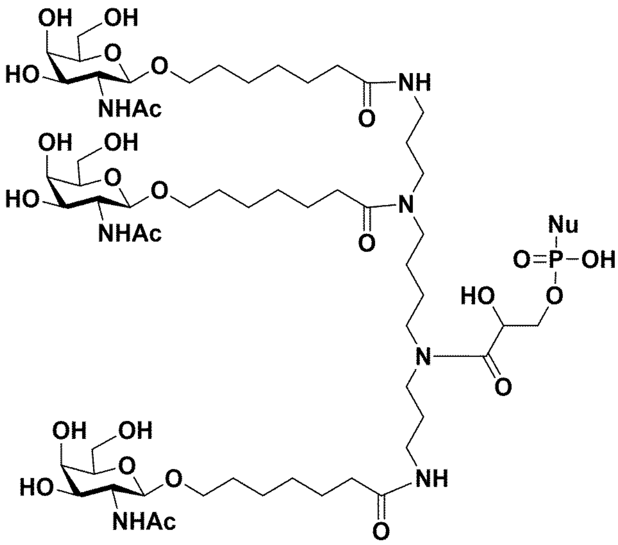

Formula (415)

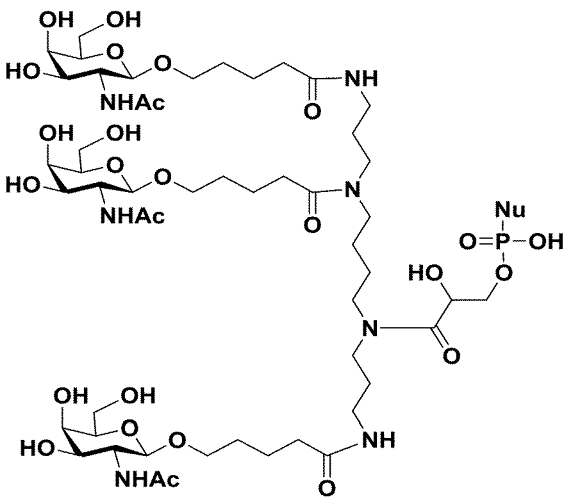

Formula (416)

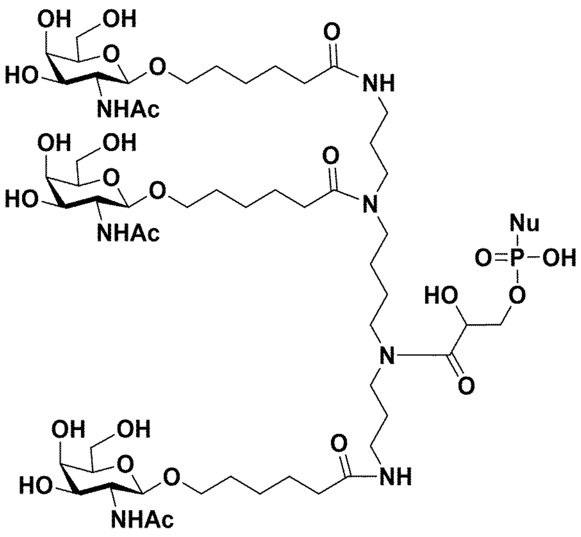

Formula (417)

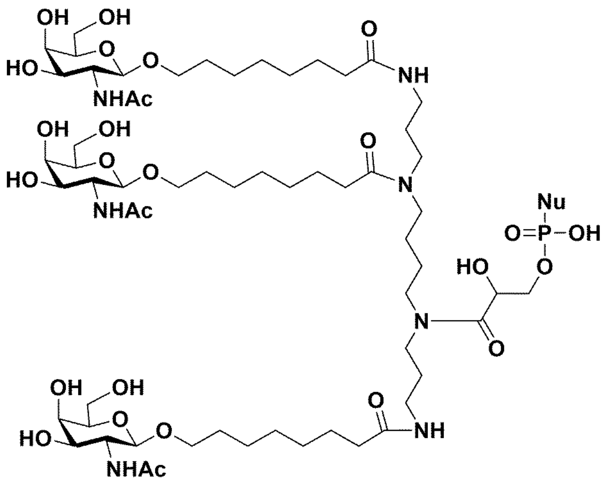

Formula (418)

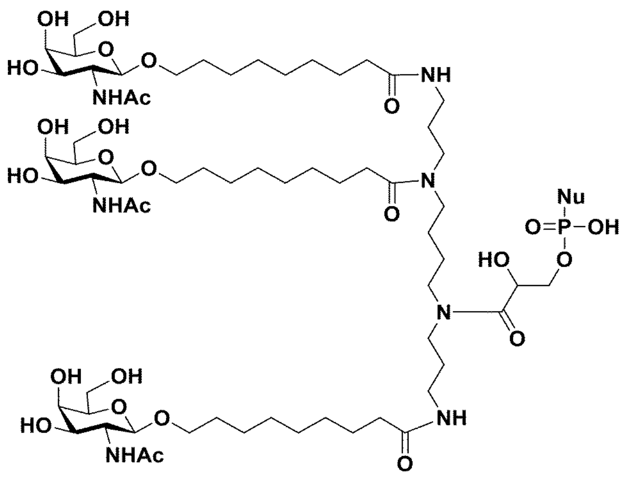

Formula (419)

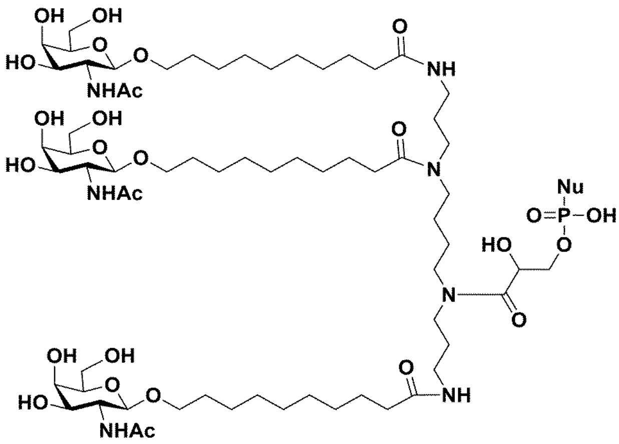

Formula (420)

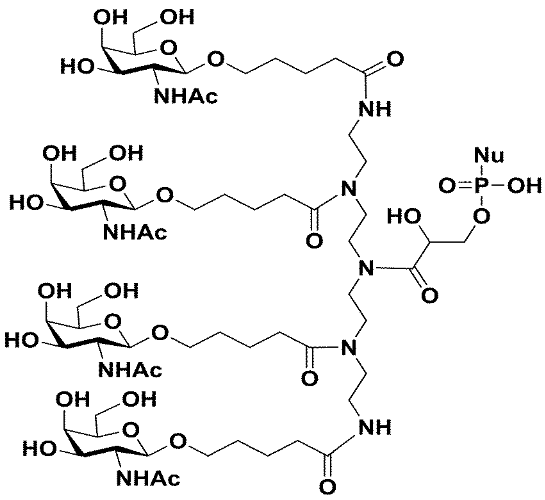

Formula (421)

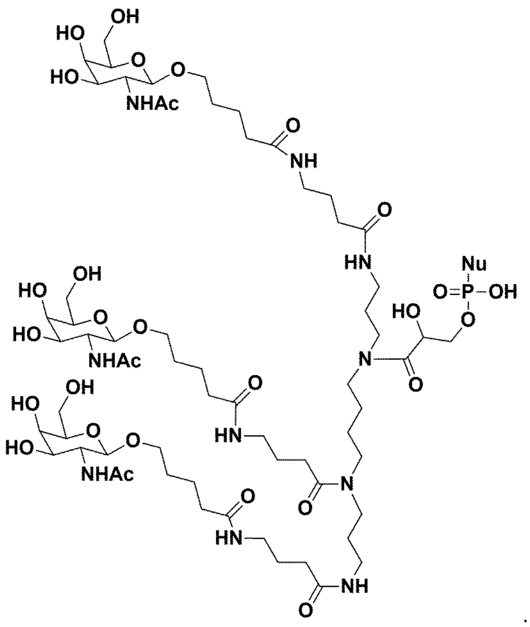

Formula (422)

In some embodiments, the P atom in Formula A59 may be linked to any possible position in the siRNA sequence, for example, the P atom in Formula A59 may be linked to any nucleotide in the sense strand or the antisense strand of the siRNA. In some embodiments, the P atom in Formula A59 is linked to any nucleotide in the sense strand of the siRNA. In some embodiments, the P atom in Formula A59 is linked to an end of the sense strand or the antisense strand of the siRNA. In some embodiments, the P atom in Formula A59 is linked to an end of the sense strand of the siRNA. The end refers to the first 4 nucleotides counted from one terminal of the sense strand or antisense strand. In some embodiments, the P atom in Formula A59 is linked to the terminal of the sense strand or the antisense strand of the siRNA. In some embodiments, the P atom in Formula A59 is linked to 3' terminal of the sense strand of the siRNA. In the case where the P atom in Formula A59 is linked to the above position in the sense strand of the siRNA, after entering into cells, the siRNA conjugate as shown by Formula (308) can release a separate antisense strand of the siRNA during unwinding, thereby blocking the translation of the PKK mRNA into protein and inhibiting the expression of PKK gene.

In some embodiments, the P atom in Formula A59 may be linked to any possible position of a nucleotide in the siRNA, for example, to position 5', 2' or 3', or to the base of the nucleotide. In some embodiments, the P atom in Formula A59 may be linked to position 2', 3', or 5' of a nucleotide in the siRNA by forming a phosphodiester bond. In some embodiments, the P atom in Formula A59 is linked to an oxygen atom formed by deprotonation of 3'-hydroxy of the nucleotide at 3' terminal of the sense strand of the siRNA (in this time, the P atom in Formula A59 may also be regarded as a P atom in a phosphate group contained in the siRNA), or the P atom in Formula A59 is linked to a nucleotide of the sense strand of the siRNA by substituting the hydrogen in the 2'-hydroxy of the nucleotide, or the P atom in Formula A59 is linked to a nucleotide at 5' terminal of the sense strand of the siRNA by substituting the hydrogen in the 5'-hydroxy of the nucleotide.

The inventors of the present disclosure have surprisingly found that the siRNA conjugate of the present disclosure has significantly improved stability in plasma and low off-target effect, and also shows higher silencing activity against PKK mRNA. In some embodiments, the siRNA of the present disclosure may be one of the siRNAs shown in Tables 1a-1f. The siRNA conjugates containing these siRNAs show higher silencing activity against PKK mRNA.

Table 1a First siRNA sequence of the present disclosure

| siRNA No. | SEQ ID NO: | Sequence direction 5' - 3' |
|---|---|---|
| siPKKa 1 | 9 | GGUGUUUGCUAUUCAGUUU |
| | 10 | AAACUGAAUAGCAAACACCUU |
| siPKKa2 | 11 | AAGGUGUUUGCUAUUCAGUUU |
| | 12 | AAACUGAAUAGCAAACACCUUGG |
| siPKKa1-M1 | 13 | GmGmUmGmUmUmUfGfCfUmAmUmUmCmAmGmUmUmUm |
| | 14 | AmAfAmCmUmGfAmAmUmAmGmCmAmAfAmCfAmCmCmUmUm |
| siPKKa1-M2 | 15 | GmGmUmGmUfUmUfGfCfUmAmUmUmCmAmGmUmUmUm |
| | 16 | AmAfAmCmUmGfAmAfUfAmGmCmAmAfAmCfAmCmCmUmUm |
| siPKKa1-M3 | 17 | GmGmUmGmUfUmUfGfCfUmAmUmUmCmAmGmUmUmUm |
| | 18 | AmAfAmCmUmGfAmAmUmAmGmCmAmAfAmCfAmCmCmUmUm |
| siPKKa2-M1 | 19 | AmAmGmGmUmGmUmUmUfGfCfUmAmUmUmCmAmGmUmUmUm |
| | 20 | AmAfAmCmUmGfAmAmUmAmGmCmAmAfAmCfAmCmCmUmUmGmGm |
| siPKKa2-M2 | 21 | AmAmGmGmUmGmUfUmUfGfCfUmAmUmUmCmAmGmUmUmUm |
| | 22 | AmAfAmCmUmGfAmAfUfAmGmCmAmAfAmCfAmCmCmUmUmGmGm |
| siPKKa2-M3 | 23 | AmAmGmGmUmGmUfUmUfGfCfUmAmUmUmCmAmGmUmUmUm |
| | 24 | AmAfAmCmUmGfAmAmUmAmGmCmAmAfAmCfAmCmCmUmUmGmGm |

(continued)

| siRNA No. | SEQ ID NO: | Sequence direction 5' - 3' |
|---|---|---|
| siPKKa1-M1S | 25 | GmsGmsUmGmUmUmUfGfCfUmAmUmUmCmAmGmUmUmUm |
| | 26 | AmsAfsAmCmUmGfAmAmUmAmGmCmAmAfAmCfAmCmCmsUmsUm |
| siPKKa1-M2S | 27 | GmsGmsUmGmUfUmUfGfCfUmAmUmUmCmAmGmUmUmUm |
| | 28 | AmsAfsAmCmUmGfAmAfUfAmGmCmAmAfAmCfAmCmCmsUmsUm |
| siPKKa1-M3S | 29 | GmsGmsUmGmUfUmUfGfCfUmAmUmUmCmAmGmUmUmUm |
| | 30 | AmsAfsAmCmUmGfAmAmUmAmGmCmAmAfAmCfAmCmCmsUmsUm |
| siPKKa2-M1S | 31 | AmsAmsGmGmUmGmUmUmUfGfCfUmAmUmUmCmAmGmUmUmUm |
| | 32 | AmsAfsAmCmUmGfAmAmUmAmGmCmAmAfAmCfAmCmCmUmUmsGmsGm |
| siPKKa2-M2S | 33 | AmsAmsGmGmUmGmUfUmUfGfCfUmAmUmUmCmAmGmUmUmUm |
| | 34 | AmsAfsAmCmUmGfAmAfUfAmGmCmAmAfAmCfAmCmCmUmUmsGmsGm |
| siPKKa2-M3S | 35 | AmsAmsGmGmUmGmUfUmUfGfCfUmAmUmUmCmAmGmUmUmUm |
| | 36 | AmsAfsAmCmUmGfAmAmUmAmGmCmAmAfAmCfAmCmCmUmUmsGmsGm |

(continued)

| siRNA No. | SEQ ID NO: | Sequence direction 5' - 3' |
|---|---|---|
| siPKKa1-M1P1 | 37 | GmGmUmGmUmUmUfGfCfUmAmUmUmCmAmGmUmUmUm |
| | 38 | P1AmAfAmCmUmGfAmAmUmAmGmCmAmAfAmCfAmCmCmUmUm |
| siPKKa1 M2P1 | 39 | GmGmUmGmUfUmUfGfCfUmAmUmUmCmAmGmUmUmUm |
| | 40 | P1AmAfAmCmUmGfAmAfUfAmGmCmAmAfAmCfAmCmCmUmUm |
| siPKKa1-M3P1 | 41 | GmGmUmGmUfUmUfGfCfUmAmUmUmCmAmGmUmUmUm |
| | 42 | P1AmAfAmCmUmGfAmAmUmAmGmCmAmAfAmCfAmCmCmUmUm |
| siPKKa2-M1P1 | 43 | AmAmGmGmUmGmUmUmUfGfCfUmAmUmUmCmAmGmUmUmUm |
| | 44 | P1AmAfAmCmUmGfAmAmUmAmGmCmAmAfAmCfAmCmCmUmUmGmGm |
| siPKKa2-M2P1 | 45 | AmAmGmGmUmGmUfUmUfGfCfUmAmUmUmCmAmGmUmUmUm |
| | 46 | P1AmAfAmCmUmGfAmAfUfAmGmCmAmAfAmCfAmCmCmUmUmGmGm |
| siPKKa2-M3P1 | 47 | AmAmGmGmUmGmUfUmUfGfCfUmAmUmUmCmAmGmUmUmUm |
| | 48 | P1AmAfAmCmUmGfAmAmUmAmGmCmAmAfAmCfAmCmCmUmUmGmGm |

(continued)

| siRNA No. | SEQ ID NO: | Sequence direction 5' - 3' |
|---|---|---|
| siPKKa1-M1SP1 | 49 | GmsGmsUmGmUmUmUfGfCfUmAmUmUmCmAmGmUmUmUm |
| | 50 | P1AmsAfsAmCmUmGfAmAmUmAmGmCmAmAfAmCfAmCmCmsUmsUm |
| siPKKa1-M2SP1 | 51 | GmsGmsUmGmUfUmUfGfCfUmAmUmUmCmAmGmUmUmUm |
| | 52 | P1AmsAfsAmCmUmGfAmAfUfAmGmCmAmAfAmCfAmCmCmsUmsUm |
| siPKKa1-M3SP1 | 53 | GmsGmsUmGmUfUmUfGfCfUmAmUmUmCmAmGmUmUmUm |
| | 54 | P1AmsAfsAmCmUmGfAmAmUmAmGmCmAmAfAmCfAmCmCmsUmsUm |
| siPKKa2-M1SP1 | 55 | AmsAmsGmGmUmGmUmUmUfGfCfUmAmUmUmCmAmGmUmUmUm |
| | 56 | P1AmsAfsAmCmUmGfAmAmUmAmGmCmAmAfAmCfAmCmCmUmUmsGmsGm |
| siPKKa2-M2SP1 | 57 | AmsAmsGmGmUmGmUfUmUfGfCfUmAmUmUmCmAmGmUmUmUm |
| | 58 | P1AmsAfsAmCmUmGfAmAfUfAmGmCmAmAfAmCfAmCmCmUmUmsGmsGm |
| siPKKa2-M3SP1 | 59 | AmsAmsGmGmUmGmUfUmUfGfCfUmAmUmUmCmAmGmUmUmUm |
| | 60 | P1AmsAfsAmCmUmGfAmAmUmAmGmCmAmAfAmCfAmCmCmUmUmsGmsGm |

Table 1b Second siRNA sequence of the present disclosure

| siRNA No. | SEQ ID NO: | Sequence direction 5' - 3' |
|---|---|---|
| siPKKb1 | 69 | GCUUCUUGAAAGAUAGUGU |
| | 70 | ACACUAUCUUUCAAGAAGCAA |
| siPKKb2 | 71 | UUGCUUCUUGAAAGAUAGUGU |
| | 72 | ACACUAUCUUUCAAGAAGCAACC |
| siPKKb1-M1 | 73 | GmCmUmUmCmUmUfGfAfAmAmGmAmUmAmGmUmGmUm |
| | 74 | AmCfAmCmUmAfUmCmUmUmUmCmAmAfGmAfAmGmCmAmAm |
| siPKKb1-M2 | 75 | GmCmUmUmCfUmUfGfAfAmAmGmAmUmAmGmUmGmUm |
| | 76 | AmCfAmCmUmAfUmCfUfUmUmCmAmAfGmAfAmGmCmAmAm |
| siPKKb 1-M3 | 77 | GmCmUmUmCfUmUfGfAfAmAmGmAmUmAmGmUmGmUm |
| | 78 | AmCfAmCmUmAfUmCmUmUmUmCmAmAfGmAfAmGmCmAmAm |
| siPKKb2-M1 | 79 | UmUmGmCmUmUmCmUmUfGfAfAmAmGmAmUmAmGmUmGmUm |
| | 80 | AmCfAmCmUmAfUmCmUmUmUmCmAmAfGmAfAmGmCmAmAmCmCm |
| siPKKb2-M2 | 81 | UmUmGmCmUmUmCfUmUfGfAfAmAmGmAmUmAmGmUmGmUm |
| | 82 | AmCfAmCmUmAfUmCfUfUmUmCmAmAfGmAfAmGmCmAmAmCmCm |
| siPKKb2-M3 | 83 | UmUmGmCmUmUmCfUmUfGfAfAmAmGmAmUmAmGmUmGmUm |
| | 84 | AmCfAmCmUmAfUmCmUmUmUmCmAmAfGmAfAmGmCmAmAmCmCm |

(continued)

| siRNA No. | SEQ ID NO: | Sequence direction 5' - 3' |
|---|---|---|
| siPKKb1-M1S | 85 | GmsCmsUmUmCmUmUfGfAfAmAmGmAmUmAmGmUmGmUm |
| | 86 | AmsCfsAmCmUmAfUmCmUmUmUmCmAmAfGmAfAmGmCmsAmsAm |
| siPKKb1-M2S | 87 | GmsCmsUmUmCfUmUfGfAfAmAmGmAmUmAmGmUmGmUm |
| | 88 | AmsCfsAmCmUmAfUmCfUfUmUmCmAmAfGmAfAmGmCmsAmsAm |
| siPKKb1-M3S | 89 | GmsCmsUmUmCfUmUfGfAfAmAmGmAmUmAmGmUmGmUm |
| | 90 | AmsCfsAmCmUmAfUmCmUmUmUmCmAmAfGmAfAmGmCmsAmsAm |
| siPKKb2-M1S | 91 | UmsUmsGmCmUmUmCmUmUfGfAfAmAmGmAmUmAmGmUmGmUm |
| | 92 | AmsCfsAmCmUmAfUmCmUmUmUmCmAmAfGmAfAmGmCmAmAmsCmsCm |
| siPKKb2-M2S | 93 | UmsUmsGmCmUmUmCfUmUfGfAfAmAmGmAmUmAmGmUmGmUm |
| | 94 | AmsCfsAmCmUmAfUmCfUfUmUmCmAmAfGmAfAmGmCmAmAmsCmsCm |
| siPKKb2-M3S | 95 | UmsUmsGmCmUmUmCfUmUfGfAfAmAmGmAmUmAmGmUmGmUm |
| | 96 | AmsCfsAmCmUmAfUmCmUmUmUmCmAmAfGmAfAmGmCmAmAmsCmsCm |

(continued)

| siRNA No. | SEQ ID NO: | Sequence direction 5' - 3' |
|---|---|---|
| siPKKb1-M1P1 | 97 | GmCmUmUmCmUmUfGfAfAmAmGmAmUmAmGmUmGmUm |
| | 98 | P1AmCfAmCmUmAfUmCmUmUmUmCmAmAfGmAfAmGmCmAmAm |
| siPKKb1-M2P1 | 99 | GmCmUmUmCfUmUfGfAfAmAmGmAmUmAmGmUmGmUm |
| | 100 | P1AmCfAmCmUmAfUmCfUfUmUmCmAmAfGmAfAmGmCmAmAm |
| siPKKb1-M3P1 | 101 | GmCmUmUmCfUmUfGfAfAmAmGmAmUmAmGmUmGmUm |
| | 102 | P1AmCfAmCmUmAfUmCmUmUmUmCmAmAfGmAfAmGmCmAmAm |
| siPKKb2-M1P1 | 103 | UmUmGmCmUmUmCmUmUfGfAfAmAmGmAmUmAmGmUmGmUm |
| | 104 | P1AmCfAmCmUmAfUmCmUmUmUmCmAmAfGmAfAmGmCmAmAmCmCm |
| siPKKb2-M2P1 - | 105 | UmUmGmCmUmUmCfUmUfGfAfAmAmGmAmUmAmGmUmGmUm |
| | 106 | P1AmCfAmCmUmAfUmCfUfUmUmCmAmAfGmAfAmGmCmAmAmCmCm |
| siPKKb2-M3P1 | 107 | UmUmGmCmUmUmCfUmUfGfAfAmAmGmAmUmAmGmUmGmUm |
| | 108 | P1AmCfAmCmUmAfUmCmUmUmUmCmAmAfGmAfAmGmCmAmAmCmCm |

(continued)

| siRNA No. | SEQ ID NO: | Sequence direction 5' - 3' |
|---|---|---|
| siPKKb1-siPKKb1-M1SP1 | 109 | GmsCmsUmUmCmUmUfGfAfAmAmGmAmUmAmGmUmGmUm |
| | 110 | P1AmsCfsAmCmUmAfUmCmUmUmUmCmAmAfGmAfAmGmCmsAmsAm |
| siPKKb1-M2SP1 | 111 | GmsCmsUmUmCfUmUfGfAfAmAmGmAmUmAmGmUmGmUm |
| | 112 | P1AmsCfsAmCmUmAfUmCfUfUmUmCmAmAfGmAfAmGmCmsAmsAm |
| siPKKb1-M3SP1 | 113 | GmsCmsUmUmCfUmUfGfAfAmAmGmAmUmAmGmUmGmUm |
| | 114 | P1AmsCfsAmCmUmAfUmCmUmUmUmCmAmAfGmAfAmGmCmsAmsAm |
| siPKKb2-M1SP1 | 115 | UmsUmsGmCmUmUmCmUmUfGfAfAmAmGmAmUmAmGmUmGmUm |
| | 116 | P1AmsCfsAmCmUmAfUmCmUmUmUmCmAmAfGmAfAmGmCmAmAmsCmsCm |
| siPKKb2-M2SP1 | 117 | UmsUmsGmCmUmUmCfUmUfGfAfAmAmGmAmUmAmGmUmGmUm |
| | 118 | P1AmsCfsAmCmUmAfUmCfUfUmUmCmAmAfGmAfAmGmCmAmAmsCmsCm |
| siPKKb2-M3SP1 | 119 | UmsUmsGmCmUmUmCfUmUfGfAfAmAmGmAmUmAmGmUmGmUm |
| | 120 | P1AmsCfsAmCmUmAfUmCmUmUmUmCmAmAfGmAfAmGmCmAmAmsCmsCm |

Table 1c Third siRNA sequence of the present disclosure

| siRNA No. | SEQ ID NO: | Sequence direction 5'- 3' |
|---|---|---|
| siPKKc1 | 129 | GAAGCAAUGUGGUCAUCAA |
|  | 130 | UUGAUGACCACAUUGCUUCAA |
| siPKKc2 | 131 | UUGAAGCAAUGUGGUCAUCAA |
|  | 132 | UUGAUGACCACAUUGCUUCAAGG |
| siPKKc1- | 133 | GmAmAmGmCmAmAfUfGfUmGmGmUmCmAmUmCmAmAm |
| M1 | 134 | UmUfGmAmUmGfAmCmCmAmCmAmUmUfGmCfUmUmCmAmAm |
| siPKKc1-M2 | 135 | GmAmAmGmCfAmAfUfGfUmGmGmUmCmAmUmCmAmAm |
|  | 136 | UmUfGmAmUmGfAmCfCfAmCmAmUmUfGmCfUmUmCmAmAm |
| siPKKc1-siPLLc1-M3 | 137 | GmAmAmGmCfAmAfUfGfUmGmGmUmCmAmUmCmAmAm |
|  | 138 | UmUfGmAmUmGfAmCmCmAmCmAmUmUfGmCfUmUmCmAmAm |
| siPKKc2-M1 | 139 | UmUmGmAmAmGmCmAmAfUfGfUmGmGmUmCmAmUmCmAmAm |
|  | 140 | UmUfGmAmUmGfAmCmCmAmCmAmUmUfGmCfUmUmCmAmAmGmGm |
| siPKKc2-M2 | 141 | UmUmGmAmAmGmCfAmAfUfGfUmGmGmUmCmAmUmCmAmAm |
|  | 142 | UmUfGmAmUmGfAmCfCfAmCmAmUmUfGmCfUmUmCmAmAmGmGm |
| siPKKc2-M3 | 143 | UmUmGmAmAmGmCfAmAfUfGfUmGmGmUmCmAmUmCmAmAm |
|  | 144 | UmUfGmAmUmGfAmCmCmAmCmAmUmUfGmCfUmUmCmAmAmGmGm |

(continued)

| siRNA No. | SEQ ID NO: | Sequence direction 5'- 3' |
|---|---|---|
| siPKKc1-M1S | 145 | GmsAmsAmGmCmAmAfUfGfUmGmGmUmCmAmUmCmAmAm |
| | 146 | UmsUfsGmAmUmGfAmCmCmAmCmAmUmUfGmCfUmUmCmsAmsAm |
| siPKKc1-M2S | 147 | GmsAmsAmGmCfAmAfUfGfUmGmGmUmCmAmUmCmAmAm |
| | 148 | UmsUfsGmAmUmGfAmCfCfAmCmAmUmUfGmCfUmUmCmsAmsAm |
| siPKKc 1-M3S | 149 | GmsAmsAmGmCfAmAfUfGfUmGmGmUmCmAmUmCmAmAm |
| | 150 | UmsUfsGmAmUmGfAmCmCmAmCmAmUmUfGmCfUmUmCmsAmsAm |
| siPKKc2-M1S | 151 | UmsUmsGmAmAmGmCmAmAfUfGfUmGmGmUmCmAmUmCmAmAm |
| | 152 | UmsUfsGmAmUmGfAmCmCmAmCmAmUmUfGmCfUmUmCmAmAmsGmsGm |
| siPKKc2-M2S | 153 | UmsUmsGmAmAmGmCfAmAfUfGfUmGmGmUmCmAmUmCmAmAm |
| | 154 | UmsUfsGmAmUmGfAmCfCfAmCmAmUmUfGmCfUmUmCmAmAmsGmsGm |
| siPKKc2-M3S | 155 | UmsUmsGmAmAmGmCfAmAfUfGfUmGmGmUmCmAmUmCmAmAm |
| | 156 | UmsUfsGmAmUmGfAmCmCmAmCmAmUmUfGmCfUmUmCmAmAmsGmsGm |

(continued)

| siRNA No. | SEQ ID NO: | Sequence direction 5'- 3' |
| --- | --- | --- |
| siPKKc1-M1P1 | 157 | GmAmAmGmCmAmAfUfGfUmGmGmUmCmAmUmCmAmAm |
| | 158 | P1UmUfGmAmUmGfAmCmCmAmCmAmUmUfGmCfUmUmCmAmAm |
| siPKKc 1-M2P1 | 159 | GmAmAmGmCfAmAfUfGfUmGmGmUmCmAmUmCmAmAm |
| | 160 | P1UmUfGmAmUmGfAmCfCfAmCmAmUmUfGmCfUmUmCmAmAm |
| siPKKc1-M3P1 | 161 | GmAmAmGmCfAmAfUfGfUmGmGmUmCmAmUmCmAmAm |
| | 162 | P1UmUfGmAmUmGfAmCmCmAmCmAmUmUfGmCfUmUmCmAmAm |
| siPKKc2-M1P1 | 163 | UmUmGmAmAmGmCmAmAfUfGfUmGmGmUmCmAmUmCmAmAm |
| | 164 | P1UmUfGmAmUmGfAmCmCmAmCmAmUmUfGmCfUmUmCmAmAmGmGm |
| siPKKc2-M2P1 | 165 | UmUmGmAmAmGmCfAmAfUfGfUmGmGmUmCmAmUmCmAmAm |
| | 166 | P1UmUfGmAmUmGfAmCfCfAmCmAmUmUfGmCfUmUmCmAmAmGmGm |
| siPKKc2-M3P1 | 167 | UmUmGmAmAmGmCfAmAfUfGfUmGmGmUmCmAmUmCmAmAm |
| | 168 | P1UmUfGmAmUmGfAmCmCmAmCmAmUmUfGmCfUmUmCmAmAmGmGm |

(continued)

| siRNA No. | SEQ ID NO: | Sequence direction 5'- 3' |
|---|---|---|
| siPKKc1-M1SP1 | 169 | GmsAmsAmGmCmAmAfUfGfUmGmGmUmCmAmUmCmAmAm |
| | 170 | P1UmsUfsGmAmUmGfAmCmCmAmCmAmUmUfGmCfUmUmCmsAmsAm |
| siPKKc1-M2SP1 | 171 | GmsAmsAmGmCfAmAfUfGfUmGmGmUmCmAmUmCmAmAm |
| | 172 | P1UmsUfsGmAmUmGfAmCfCfAmCmAmUmUfGmCfUmUmCmsAmsAm |
| siPKKc1-M3SP1 | 173 | GmsAmsAmGmCfAmAfUfGfUmGmGmUmCmAmUmCmAmAm |
| | 174 | P1UmsUfsGmAmUmGfAmCmCmAmCmAmUmUfGmCfUmUmCmsAmsAm |
| siPKKc2-M1SP1 | 175 | UmsUmsGmAmAmGmCmAmAfUfGfUmGmGmUmCmAmUmCmAmAm |
| | 176 | P1UmsUfsGmAmUmGfAmCmCmAmCmAmUmUfGmCfUmUmCmAmAmsGmsGm |
| siPKKc2-M2SP1 | 177 | UmsUmsGmAmAmGmCfAmAfUfGfUmGmGmUmCmAmUmCmAmAm |
| | 178 | P1UmsUfsGmAmUmGfAmCfCfAmCmAmUmUfGmCfUmUmCmAmAmsGmsGm |
| siPKKc2-M3SP1 | 179 | UmsUmsGmAmAmGmCfAmAfUfGfUmGmGmUmCmAmUmCmAmAm |
| | 180 | P1UmsUfsGmAmUmGfAmCmCmAmCmAmUmUfGmCfUmUmCmAmAmsGmsGm |

Table 1d Fourth siRNA sequence of the present disclosure

| siRNA No. | SEQ ID NO: | Sequence direction 5'- 3' |
|---|---|---|
| siPKKd 1 | 189 | CACAAAGAAAUGUUUGUCU |
| | 190 | AGACAAACAUUUCUUUGUGAC |
| siPKKd2 | 191 | GUCACAAAGAAAUGUUUGUCU |
| | 192 | AGACAAACAUUUCUUUGUGACUC |
| siPKKd1-M1 | 193 | CmAmCmAmAmAmGfAfAfAmUmGmUmUmUmGmUmCmUm |
| | 194 | AmGfAmCmAmAfAmCmAmUmUmUmCmUfUmUfGmUmGmAmCm |
| siPKKd 1-M2 | 195 | CmAmCmAmAfAmGfAfAfAmUmGmUmUmUmGmUmCmUm |
| | 196 | AmGfAmCmAmAfAmCfAfUmUmUmCmUfUmUfGmUmGmAmCm |
| siPKKd 1-M3 | 197 | CmAmCmAmAfAmGfAfAfAmUmGmUmUmUmGmUmCmUm |
| | 198 | AmGfAmCmAmAfAmCmAmUmUmUmCmUfUmUfGmUmGmAmCm |
| siPKKd2-M1 | 199 | GmUmCmAmCmAmAmAmGfAfAfAmUmGmUmUmUmGmUmCmUm |
| | 200 | AmGfAmCmAmAfAmCmAmUmUmUmCmUfUmUfGmUmGmAmCmUmCm |
| siPKKd2-M2 | 201 | GmUmCmAmCmAmAfAmGfAfAfAmUmGmUmUmUmGmUmCmUm |
| | 202 | AmGfAmCmAmAfAmCfAfUmUmUmCmUfUmUfGmUmGmAmCmUmCm |
| siPKKd2-M3 | 203 | GmUmCmAmCmAmAfAmGfAfAfAmUmGmUmUmUmGmUmCmUm |
| | 204 | AmGfAmCmAmAfAmCmAmUmUmUmCmUfUmUfGmUmGmAmCmUmCm |
| siPKKd1-M1S | 205 | CmsAmsCmAmAmAmGfAfAfAmUmGmUmUmUmGmUmCmUm |
| | 206 | AmsGfsAmCmAmAfAmCmAmUmUmUmCmUfUmUfGmUmGmsAmsCm |
| siPKKd1- | 207 | CmsAmsCmAmAfAmGfAfAfAmUmGmUmUmUmGmUmCmUm |
| M2S | 208 | AmsGfsAmCmAmAfAmCfAfUmUmUmCmUfUmUfGmUmGmsAmsCm |

(continued)

| siRNA No. | SEQ ID NO: | Sequence direction 5'- 3' |
|---|---|---|
| siPKKd 1-M3S | 209 | CmsAmsCmAmAfAmGfAfAfAmUmGmUmUmUmGmUmCmUm |
| | 210 | AmsGfsAmCmAmAfAmCmAmUmUmUmCmUfUmUfGmUmGmsAmsCm |
| siPKKd2-M1S | 211 | GmsUmsCmAmCmAmAmAmGfAfAfAmUmGmUmUmUmGmUmCmUm |
| | 212 | AmsGfsAmCmAmAfAmCmAmUmUmUmCmUfUmUfGmUmGmAmCmsUmsCm |
| siPKKd2-M2S | 213 | GmsUmsCmAmCmAmAfAmGfAfAfAmUmGmUmUmUmGmUmCmUm |
| | 214 | AmsGfsAmCmAmAfAmCfAfUmUmUmCmUfUmUfGmUmGmAmCmsUmsCm |
| siPKKd2-M3S | 215 | GmsUmsCmAmCmAmAfAmGfAfAfAmUmGmUmUmUmGmUmCmUm |
| | 216 | AmsGfsAmCmAmAfAmCmAmUmUmUmCmUfUmUfGmUmGmAmCmsUmsCm |
| siPKKd1-M1P1 | 217 | CmAmCmAmAmAmGfAfAfAmUmGmUmUmUmGmUmCmUm |
| | 218 | P1AmGfAmCmAmAfAmCmAmUmUmUmCmUfUmUfGmUmGmAmCm |
| siPKKd1-M2P1 | 219 | CmAmCmAmAfAmGfAfAfAmUmGmUmUmUmGmUmCmUm |
| | 220 | P1AmGfAmCmAmAfAmCfAfUmUmUmCmUfUmUfGmUmGmAmCm |
| siPKKd1-M3P1 | 221 | CmAmCmAmAfAmGfAfAfAmUmGmUmUmUmGmUmCmUm |
| | 222 | P1AmGfAmCmAmAfAmCmAmUmUmUmCmUfUmUfGmUmGmAmCm |

(continued)

| siRNA No. | SEQ ID NO: | Sequence direction 5'- 3' |
|---|---|---|
| siPKKd2-M1P1 | 223 | GmUmCmAmCmAmAmAmGfAfAfAmUmGmUmUmUmGmUmCmUm |
| | 224 | P1AmGfAmCmAmAfAmCmAmUmUmUmCmUfUmUfGmUmGmAmCmUmCm |
| siPKKd2-M2P1 | 225 | GmUmCmAmCmAmAfAmGfAfAfAmUmGmUmUmUmGmUmCmUm |
| | 226 | P1AmGfAmCmAmAfAmCfAfUmUmUmCmUfUmUfGmUmGmAmCmUmCm |
| siPKKd2-M3P1 | 227 | GmUmCmAmCmAmAfAmGfAfAfAmUmGmUmUmUmGmUmCmUm |
| | 228 | P1AmGfAmCmAmAfAmCmAmUmUmUmCmUfUmUfGmUmGmAmCmUmCm |
| siPKKd1-M1SP1 | 229 | CmsAmsCmAmAmAmGfAfAfAmUmGmUmUmUmGmUmCmUm |
| | 230 | P1AmsGfsAmCmAmAfAmCmAmUmUmUmCmUfUmUfGmUmGmsAmsCm |
| siPKKd1-M2SP1 | 231 | CmsAmsCmAmAfAmGfAfAfAmUmGmUmUmUmGmUmCmUm |
| | 232 | P1AmsGfsAmCmAmAfAmCfAfUmUmUmCmUfUmUfGmUmGmsAmsC |
| | | m |
| siPKKd1-M3SP1 | 233 | CmsAmsCmAmAfAmGfAfAfAmUmGmUmUmUmGmUmCmUm |
| | 234 | P1AmsGfsAmCmAmAfAmCmAmUmUmUmCmUfUmUfGmUmGmsAmsCm |
| siPKKd2-M1SP1 | 235 | GmsUmsCmAmCmAmAmAmGfAfAfAmUmGmUmUmUmGmUmCmUm |
| | 236 | P1AmsGfsAmCmAmAfAmCmAmUmUmUmCmUfUmUfGmUmGmAmCmsUmsCm |

(continued)

| siRNA No. | SEQ ID NO: | Sequence direction 5'- 3' |
|---|---|---|
| siPKKd2-M2SP1 | 237 | GmsUmsCmAmCmAmAfAmGfAfAfAmUmGmUmUmUmGmUmCmUm |
| | 238 | P1AmsGfsAmCmAmAfAmCfAfUmUmUmCmUfUmUfGmUmGmAmCmsUmsCm |
| siPKKd2-M3SP1 | 239 | GmsUmsCmAmCmAmAfAmGfAfAfAmUmGmUmUmUmGmUmCmUm |
| | 240 | P1AmsGfsAmCmAmAfAmCmAmUmUmUmCmUfUmUfGmUmGmAmCmsUmsCm |

Table 1e Fifth siRNA sequence of the present disclosure

| siRNA No. | SEQ ID NO: | Sequence direction 5'- 3' |
|---|---|---|
| siPKKe1 | 249 | GAAUUCCAAAAACCAAUAU |
| | 250 | AUAUUGGUUUUUGGAAUUCAG |
| siPKKe2 | 251 | CUGAAUUCCAAAAACCAAUAU |
| | 252 | AUAUUGGUUUUUGGAAUUCAGUG |
| siPKKe1-M1 | 253 | GmAmAmUmUmCmCfAfAfAmAmAmCmCmAmAmUmAmUm |
| | 254 | AmUfAmUmUmGfGmUmUmUmUmUmGmGfAmAfUmUmCmAmGm |
| siPKKe1-M2 | 255 | GmAmAmUmUfCmCfAfAfAmAmAmCmCmAmAmUmAmUm |
| | 256 | AmUfAmUmUmGfGmUfUfUmUmUmGmGfAmAfUmUmCmAmGm |
| siPKKe1-M3 | 257 | GmAmAmUmUfCmCfAfAfAmAmAmCmCmAmAmUmAmUm |
| | 258 | AmUfAmUmUmGfGmUmUmUmUmUmGmGfAmAfUmUmCmAmGm |
| siPKKe2-M1 | 259 | CmUmGmAmAmUmUmCmCfAfAfAmAmAmCmCmAmAmUmAmUm |
| | 260 | AmUfAmUmUmGfGmUmUmUmUmUmGmGfAmAfUmUmCmAmGmUmGm |
| siPKKe2-M2 | 261 | CmUmGmAmAmUmUfCmCfAfAfAmAmAmCmCmAmAmUmAmUm |
| | 262 | AmUfAmUmUmGfGmUfUfUmUmUmGmGfAmAfUmUmCmAmGmUmGm |
| siPKKe2- | 263 | CmUmGmAmAmUmUfCmCfAfAfAmAmAmCmCmAmAmUmAmUm |
| M3 | 264 | AmUfAmUmUmGfGmUmUmUmUmUmGmGfAmAfUmUmCmAmGmUmGm |
| siPKKe1-M1S | 265 | GmsAmsAmUmUmCmCfAfAfAmAmAmCmCmAmAmUmAmUm |
| | 266 | AmsUfsAmUmUmGfGmUmUmUmUmUmGmGfAmAfUmUmCmsAmsGm |
| siPKKe1-M2S | 267 | GmsAmsAmUmUfCmCfAfAfAmAmAmCmCmAmAmUmAmUm |
| | 268 | AmsUfsAmUmUmGfGmUfUfUmUmUmGmGfAmAfUmUmCmsAmsGm |

(continued)

| siRNA No. | SEQ ID NO: | Sequence direction 5'- 3' |
|---|---|---|
| siPKKe1-M3S | 269 | GmsAmsAmUmUfCmCfAfAfAmAmAmCmCmAmAmUmAmUm |
| | 270 | AmsUfsAmUmUmGfGmUmUmUmUmUmGmGfAmAfUmUmCmsAmsGm |
| siPKKe2-M1S | 271 | CmsUmsGmAmAmUmUmCmCfAfAfAmAmAmCmCmAmAmUmAmUm |
| | 272 | AmsUfsAmUmUmGfGmUmUmUmUmUmGmGfAmAfUmUmCmAmGmsUmsGm |
| siPKKe2-M2S | 273 | CmsUmsGmAmAmUmUfCmCfAfAfAmAmAmCmCmAmAmUmAmUm |
| | 274 | AmsUfsAmUmUmGfGmUfUfUmUmUmGmGfAmAfUmUmCmAmGmsUmsGm |
| siPKKe2-M3S | 275 | CmsUmsGmAmAmUmUfCmCfAfAfAmAmAmCmCmAmAmUmAmUm |
| | 276 | AmsUfsAmUmUmGfGmUmUmUmUmUmGmGfAmAfUmUmCmAmGmsUmsGm |
| siPKKe1-M1P1 | 277 | GmAmAmUmUmCmCfAfAfAmAmAmCmCmAmAmUmAmUm |
| | 278 | P1AmUfAmUmUmGfGmUmUmUmUmUmGmGfAmAfUmUmCmAmGm |
| siPKKe1-M2P1 | 279 | GmAmAmUmUfCmCfAfAfAmAmAmCmCmAmAmUmAmUm |
| | 280 | P1AmUfAmUmUmGfGmUfUfUmUmUmGmGfAmAfUmUmCmAmGm |
| siPKKe1-M3P1 | 281 | GmAmAmUmUfCmCfAfAfAmAmAmCmCmAmAmUmAmUm |
| | 282 | P1AmUfAmUmUmGfGmUmUmUmUmUmGmGfAmAfUmUmCmAmGm |

(continued)

| siRNA No. | SEQ ID NO: | Sequence direction 5'- 3' |
|---|---|---|
| siPKKe2-M1P1 | 283 | CmUmGmAmAmUmUmCmCfAfAfAmAmAmCmCmAmAmUmAmUm |
| | 284 | P1AmUfAmUmUmGfGmUmUmUmUmUmGmGfAmAfUmUmCmAmGmUmGm |
| siPKKe2-M2P1 | 285 | CmUmGmAmAmUmUfCmCfAfAfAmAmAmCmCmAmAmUmAmUm |
| | 286 | P1AmUfAmUmUmGfGmUfUfUmUmUmGmGfAmAfUmUmCmAmGmUmGm |
| siPKKe2-M3P1 | 287 | CmUmGmAmAmUmUfCmCfAfAfAmAmAmCmCmAmAmUmAmUm |
| | 288 | P1AmUfAmUmUmGfGmUmUmUmUmUmGmGfAmAfUmUmCmAmGmUmGm |
| siPKKe1-M1SP1 | 289 | GmsAmsAmUmUmCmCfAfAfAmAmAmCmCmAmAmUmAmUm |
| | 290 | P1AmsUfsAmUmUmGfGmUmUmUmUmUmGmGfAmAfUmUmCmsAmsGm |
| siPKKe1-M2SP1 | 291 | GmsAmsAmUmUfCmCfAfAfAmAmAmCmCmAmAmUmAmUm |
| | 292 | P1AmsUfsAmUmUmGfGmUfUfUmUmUmGmGfAmAfUmUmCmsAmsGm |
| siPKKe1-M3SP1 | 293 | GmsAmsAmUmUfCmCfAfAfAmAmAmCmCmAmAmUmAmUm |
| | 294 | P1AmsUfsAmUmUmGfGmUmUmUmUmUmGmGfAmAfUmUmCmsAmsGm |
| siPKKe2-M1SP1 | 295 | CmsUmsGmAmAmUmUmCmCfAfAfAmAmAmCmCmAmAmUmAmUm |
| | 296 | P1AmsUfsAmUmUmGfGmUmUmUmUmUmGmGfAmAfUmUmCmAmGmsUmsGm |

(continued)

| siRNA No. | SEQ ID NO: | Sequence direction 5'- 3' |
|---|---|---|
| siPKKe2-M2SP1 | 297 | CmsUmsGmAmAmUmUfCmCfAfAfAmAmAmCmCmAmAmUmAmUm |
| | 298 | P1AmsUfsAmUmUmGfGmUfUfUmUmUmGmGfAmAfUmUmCmAmGmsUmsGm |
| siPKKe2-M3SP1 | 299 | CmsUmsGmAmAmUmUfCmCfAfAfAmAmAmCmCmAmAmUmAmUm |
| | 300 | P1AmsUfsAmUmUmGfGmUmUmUmUmUmGmGfAmAfUmUmCmAmGmsUmsGm |

Table If Sixth siRNA sequence of the present disclosure

| siRNA No. | SEQ ID NO: | Sequence direction 5'- 3' |
|---|---|---|
| siPKKf1 | 309 | GGUCUGUGCUGGCUAUAAA |
| | 310 | UUUAUAGCCAGCACAGACCAU |
| siPKKf2 | 311 | AUGGUCUGUGCUGGCUAUAAA |
| | 312 | UUUAUAGCCAGCACAGACCAUCC |
| siPKKf1-M1 | 313 | GmGmUmCmUmGmUfGfCfUmGmGmCmUmAmUmAmAmAm |
| | 314 | UmUfUmAmUmAfGmCmCmAmGmCmAmCfAmGfAmCmCmAmUm |
| siPKKf1-M2 | 315 | GmGmUmCmUfGmUfGfCfUmGmGmCmUmAmUmAmAmAm |
| | 316 | UmUfUmAmUmAfGmCfCfAmGmCmAmCfAmGfAmCmCmAmUm |
| siPKKf1-M3 | 317 | GmGmUmCmUfGmUfGfCfUmGmGmCmUmAmUmAmAmAm |
| | 318 | UmUfUmAmUmAfGmCmCmAmGmCmAmCfAmGfAmCmCmAmUm |
| siPKKf2- | 319 | AmUmGmGmUmCmUmGmUfGfCfUmGmGmCmUmAmUmAmAmAm |
| M1 | 320 | UmUfUmAmUmAfGmCmCmAmGmCmAmCfAmGfAmCmCmAmUmCmCm |
| siPKKf2-M2 | 321 | AmUmGmGmUmCmUfGmUfGfCfUmGmGmCmUmAmUmAmAmAm |
| | 322 | UmUfUmAmUmAfGmCfCfAmGmCmAmCfAmGfAmCmCmAmUmCmCm |
| siPKKf2-M3 | 323 | AmUmGmGmUmCmUfGmUfGfCfUmGmGmCmUmAmUmAmAmAm |
| | 324 | UmUfUmAmUmAfGmCmCmAmGmCmAmCfAmGfAmCmCmAmUmCmCm |
| siPKKf1-M1S | 325 | GmsGmsUmCmUmGmUfGfCfUmGmGmCmUmAmUmAmAmAm |
| | 326 | UmsUfsUmAmUmAfGmCmCmAmGmCmAmCfAmGfAmCmCmsAmsUm |
| siPKKf1-M2S | 327 | GmsGmsUmCmUfGmUfGfCfUmGmGmCmUmAmUmAmAmAm |
| | 328 | UmsUfsUmAmUmAfGmCfCfAmGmCmAmCfAmGfAmCmCmsAmsUm |

(continued)

| siRNA No. | SEQ ID NO: | Sequence direction 5'- 3' |
|---|---|---|
| siPKKf1-M3S | 329 | GmsGmsUmCmUfGmUfGfCfUmGmGmCmUmAmUmAmAmAm |
| | 330 | UmsUfsUmAmUmAfGmCmCmAmGmCmAmCfAmGfAmCmCmsAmsUm |
| siPKKf2-M1S | 331 | AmsUmsGmGmUmCmUmGmUfGfCfUmGmGmCmUmAmUmAmAmAm |
| | 332 | UmsUfsUmAmUmAfGmCmCmAmGmCmAmCfAmGfAmCmCmAmUmsCmsCm |
| siPKKf2-M2S | 333 | AmsUmsGmGmUmCmUfGmUfGfCfUmGmGmCmUmAmUmAmAmAm |
| | 334 | UmsUfsUmAmUmAfGmCfCfAmGmCmAmCfAmGfAmCmCmAmUmsCmsCm |
| siPKKf2-M3S | 335 | AmsUmsGmGmUmCmUfGmUfGfCfUmGmGmCmUmAmUmAmAmAm |
| | 336 | UmsUfsUmAmUmAfGmCmCmAmGmCmAmCfAmGfAmCmCmAmUmsCmsCm |
| siPKKf1-M1P1 | 337 | GmGmUmCmUmGmUfGfCfUmGmGmCmUmAmUmAmAmAm |
| | 338 | P1UmUfUmAmUmAfGmCmCmAmGmCmAmCfAmGfAmCmCmAmUm |
| siPKKf1-M2P1 | 339 | GmGmUmCmUfGmUfGfCfUmGmGmCmUmAmUmAmAmAm |
| | 340 | P1UmUfUmAmUmAfGmCfCfAmGmCmAmCfAmGfAmCmCmAmUm |
| siPKKf1-M3P1 | 341 | GmGmUmCmUfGmUfGfCfUmGmGmCmUmAmUmAmAmAm |
| | 342 | P1UmUfUmAmUmAfGmCmCmAmGmCmAmCfAmGfAmCmCmAmUm |
| siPKKf2-M1P1 | 343 | AmUmGmGmUmCmUmGmUfGfCfUmGmGmCmUmAmUmAmAmAm |
| | 344 | P1UmUfUmAmUmAfGmCmCmAmGmCmAmCfAmGfAmCmCmAmUmCmCm |
| siPKKf2- | 345 | AmUmGmGmUmCmUfGmUfGfCfUmGmGmCmUmAmUmAmAmAm |

(continued)

| siRNA No. | SEQ ID NO: | Sequence direction 5'- 3' |
|---|---|---|
| M2P1 | 346 | P1UmUfUmAmUmAfGmCfCfAmGmCmAmCfAmGfAmCmCmAmUmCmCm |
| siPKKf2-M3P1 | 347 | AmUmGmGmUmCmUfGmUfGfCfUmGmGmCmUmAmUmAmAmAm |
| | 348 | P1UmUfUmAmUmAfGmCmCmAmGmCmAmCfAmGfAmCmCmAmUmCmCm |
| siPKKf1-M1SP1 | 349 | GmsGmsUmCmUmGmUfGfCfUmGmGmCmUmAmUmAmAmAm |
| | 350 | P1UmsUfsUmAmUmAfGmCmCmAmGmCmAmCfAmGfAmCmCmsAmsUm |
| siPKKf1-M2SP1 | 351 | GmsGmsUmCmUfGmUfGfCfUmGmGmCmUmAmUmAmAmAm |
| | 352 | P1UmsUfsUmAmUmAfGmCfCfAmGmCmAmCfAmGfAmCmCmsAmsUm |
| siPKKf1-M3SP1 | 353 | GmsGmsUmCmUfGmUfGfCfUmGmGmCmUmAmUmAmAmAm |
| | 354 | P1UmsUfsUmAmUmAfGmCmCmAmGmCmAmCfAmGfAmCmCmsAmsUm |
| siPKKf2-M1SP1 | 355 | AmsUmsGmGmUmCmUmGmUfGfCfUmGmGmCmUmAmUmAmAmAm |
| | 356 | P1UmsUfsUmAmUmAfGmCmCmAmGmCmAmCfAmGfAmCmCmAmUmsCmsCm |
| siPKKf2-M2SP1 | 357 | AmsUmsGmGmUmCmUfGmUfGfCfUmGmGmCmUmAmUmAmAmAm |
| | 358 | P1UmsUfsUmAmUmAfGmCfCfAmGmCmAmCfAmGfAmCmCmAmUmsCmsCm |

(continued)

| siRNA No. | SEQ ID NO: | Sequence direction 5'- 3' |
|---|---|---|
| siPKKf2-M3SP1 | 359 | AmsUmsGmGmUmCmUfGmUfGfCfUmGmGmCmUmAmUmAmAmAm |
| | 360 | P1UmsUfsUmAmUmAfGmCmCmAmGmCmAmCfAmGfAmCmCmAmUmsCmsCm |

Wherein, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by phosphorothioate group; and Pl represents that the nucleotide adjacent to the right side of Pl is a 5'-phosphate nucleotide or a 5'-phosphate analogue modified nucleotide. In some embodiments, Pl represents specifically modified VP, Ps or P, wherein the letter combination VP represents that the nucleotide adjacent to the right side of the letter combination VP is a 5'-(E)-vinylphosphonate (E-VP) modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide.

In the siRNA or the siRNA conjugate of the present disclosure, each pair of adjacent nucleotides is linked via a phosphodiester bond or phosphorothioate diester bond. The non-bridging oxygen atom or sulfur atom in the phosphodiester bond or phosphorothioate diester bond is negatively charged, and may be present in the form of hydroxy or sulfhydryl. Moreover, the hydrogen ion in the hydroxy or sulfhydryl may be partially or completely substituted with a cation. The cation may be any cation, such as one of a metal cation, an ammonium ion $NH4^+$ or an organic ammonium cation. In order to increase solubility, in some embodiments, the cation is selected from one or more of an alkali metal ion, an ammonium cation formed by a tertiary amine and a quaternary ammonium cation. The alkali metal ion may be $K^+$ and/or $Na^+$, and the cation formed by the tertiary amine may be an ammonium ion formed by triethylamine and/or an ammonium ion formed by N,N-diisopropylethylamine. Thus, the siRNA or siRNA conjugate of the present disclosure may be at least partially present in the form of salt. In some embodiment, the non-bridging oxygen atom or sulfur atom in the phosphodiester bond or phosphorothioate diester bond at least partly binds to a sodium ion, and thus the siRNA or the siRNA conjugate of the present disclosure is present or partially present in the form of sodium salt.

Those skilled in the art clearly know that a modified nucleotide group may be introduced into the siRNA of the present disclosure by using a nucleoside monomer having a corresponding modification. The methods for preparing the nucleoside monomer having the corresponding modification and the methods for introducing the modified nucleotide group into the siRNA are also well-known to those skilled in the art. All the modified nucleoside monomers may be either commercially available or prepared by known methods.

Preparation of the siRNA conjugate as shown by Formula (308)

The siRNA conjugate as shown by Formula (308) may be prepared by any appropriate synthetic routes.

In some embodiments, the siRNA conjugate as shown by Formula (308) may be prepared by the following method. The method comprises: successively linking nucleoside monomers in the direction from 3' to 5' according to the nucleotide types and sequences in the sense strand and antisense strand of the siRNA respectively under the condition of solid phase phosphoramidite synthesis, wherein the step of linking each nucleoside monomer comprises a four-step reaction of deprotection, coupling, capping, and oxidation or sulfurization; isolating the sense strand and the antisense strand of the siRNA; and annealing; wherein the siRNA is the siRNA of the present disclosure mentioned above.

Moreover, the method further comprises: contacting the compound as shown by Formula (321) with a nucleoside monomer or a nucleotide sequence linked to a solid phase support under coupling reaction condition and in the presence of a coupling agent, thereby linking the compound as shown by Formula (321) to the nucleotide sequence through a coupling reaction. Hereinafter, the compound as shown by Formula (321) is also called a conjugating molecule.

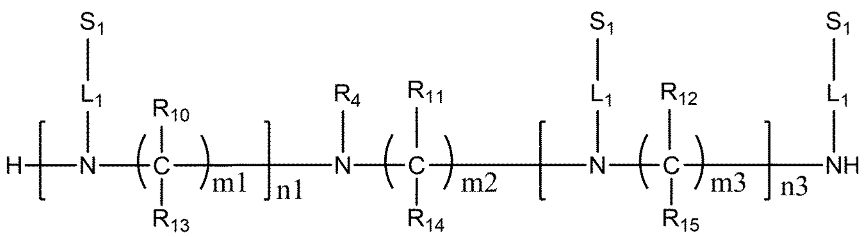

Formula (321)

wherein:

$R_4$ is a group capable of binding to the siRNA represented by Nu in the compound as shown by Formula (308). In some embodiments, $R_4$ is a group capable of binding to the siRNA represented by Nu via a covalent bond. In some embodiments, $R_4$ is a group capable of being conjugated to any functional group of the siRNA represented by Nu

via a phosphodiester bond by reaction;

Each $S_1$ is independently a group formed by substituting all active hydroxyl in M1 with YCOO- groups, wherein each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. In some embodiments, Y is a methyl.

Definitions and optional ranges of n1, n3, m1, m2, m3, $R_{10}$, $R_{11}$, $R_{12}$, $R_{13}$, $R_{14}$, $R_{15}$, $L_1$, and $M_1$ are respectively as described above.

$R_4$ is selected to achieve the linkage to the N atom on a nitrogenous backbone and to provide a suitable reaction site for synthesizing the siRNA conjugate as shown by Formula (308). In some embodiments, $R_4$ comprises a $R_2$ linking group or a protected $R_2$ linking group, and can form a functional group as shown by Formula (A59) with an siRNA via reaction.

In some embodiments, R4 comprises a first functional group that can form a phosphite esterwith a group on an siRNA or a nucleoside monomer represented by Nu, and a second functional group that can react with a hydroxy or an amino to form a covalent bond, or comprises a solid phase support linked via the covalent bond. In some embodiments, the first functional group is a phosphoramidite, a hydroxy or a protected hydroxy. In some embodiments, the second functional group is a phosphoramidite, a carboxyl or a carboxylate. In some embodiments, the second functional group is a solid phase support linked to the rest of the molecule via a covalent bond which is formed by a hydroxy or an amino. In some embodiments, the solid phase support is linked via a phosphoester bond, a carboxyl ester bond, or an amide bond. In some embodiments, the solid phase support is a resin.

In some embodiments, the first functional group comprises a hydroxy, $-OR_k$ or a group as shown by Formula (C3); and the second functional group has a structure as shown by Formula (Cl), (C2), (C3), (C1'), or (C3'):

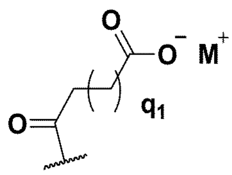,
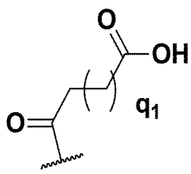,
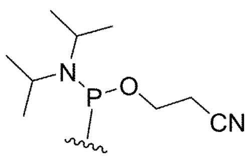,

(C1) (C2) (C3)

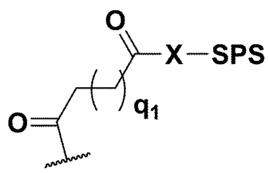,
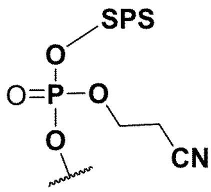,

(C1') (C3')

wherein $q_1$ is an integer of 1-4, X is O or NH, $M^+$ is a cation, $R_k$ is a hydroxy protecting group, SPS represents a solid phase support, and  represents the site where a group is covalently linked.

In some embodiments, the first functional group comprises a phosphoramidite group as shown by Formula (C3). The phosphoramidite group can form a phosphite ester with a hydroxy at any position on a nucleotide such as a 2'- or 3'- hydroxy by a coupling reaction, and the phosphite ester can form a phosphodiester bond or phosphorothioate ester bond as shown by Formula (A59) via oxidation or sulfurization, so as to conjugate the conjugating molecule to the siRNA. In this case, even if the second functional group does not exist, the compound as shown by Formula (321) will still be able to be conjugated to the nucleotide, without affecting the acquisition of the siRNA conjugate as shown by Formula (308). Under such circumstances, after obtaining a sense strand or an antisense strand of the siRNA by a method such as solid phase phosphoramidite synthesis, the compound as shown by Formula (321) is reacted with a hydroxy on the terminal nucleotide of the nucleotide sequence, and phosphodiester bond or phosphorothioate bond is formed by a subsequent oxidation or sulfurization process, thereby conjugating the compound as shown by Formula (321) to the siRNA.

In some embodiments, the first functional group comprises a protected hydroxy. In some embodiments, the

second functional group comprises a group that can react with a solid phase support to provide a conjugating molecule comprising the solid phase support. In some embodiments, the second functional group comprises a carboxyl, a carboxylate or a phosphoramidite as shown by Formula (C1), (C2) or (C3). When the second functional group comprises a carboxyl or a carboxylate, the compound as shown by Formula (321) reacts with a hydroxy or an amino on a solid phase support such as a resin via an esterification or an amidation reaction, to form a conjugating molecule comprising the solid phase support linked via a carboxyl ester bond. When the second functional group comprises a phosphoramidite functional group, the compound as shown by Formula (321) may be coupled with a hydroxy on a universal solid phase support, such as a resin, and to form, by oxidation, a conjugating molecule comprising the solid phase support linked via a phosphodiester bond. Subsequently, starting from the above product linked to the solid phase support, the nucleoside monomers are linked sequentially by a solid phase phosphoramidite synthesis method, thereby obtaining a sense strand or an antisense strand of the siRNA linked to the conjugation group. During the solid phase phosphoramidite synthesis, the first functional group is deprotected, and then coupled with a phosphoramidite group on a nucleoside monomer under coupling reaction condition.

In some embodiments, the first functional group comprises a hydroxy or a protected hydroxy; and the second functional group comprises a solid phase support linked via a carboxyl ester bond, a solid phase support linked via an amide bond or a solid phase support linked via a phosphoester bond, as shown by Formula (C1') or (C3'). In this case, starting from the compound as shown by Formula (321) in place of the solid phase support, the nucleoside monomers are linked sequentially by a solid phase phosphoramidite synthesis, thereby obtaining a sense strand or an antisense strand of the siRNA linked to a conjugating group.

In some embodiments, the carboxylate may be expressed as $-COO^-M^+$, wherein $M^+$ is a cation, such as one selected from a metal cation, an ammonium cation $NH4^+$ and an organic ammonium cation. In one embodiment, the metal ion may be one selected from an alkali metal ion, such as $K^+$ or $Na^+$. In order to increase solubility and facilitate the reaction, in some embodiments, the organic ammonium ion is an ammonium cation formed by a tertiary amine, or a quaternary ammonium cation, such as an ammonium ion formed by triethylamine or an ammonium ion formed by N,N-diisopropylethylamine. In some embodiments, the carboxylate is a triethylamine carboxylate or an N,N-diisopropylethylamine carboxylate.

In some embodiments, $R_4$ comprises a structure as shown by Formula (B9), (B10), (B9'), (B10'), (B11), (B12), (Bl1') or (B12'):

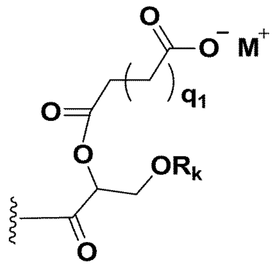
,
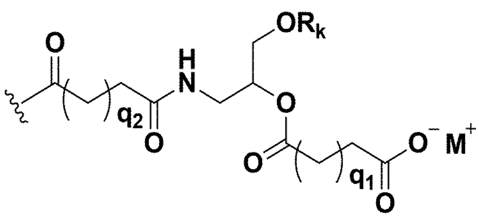
,

(B9) (B10)

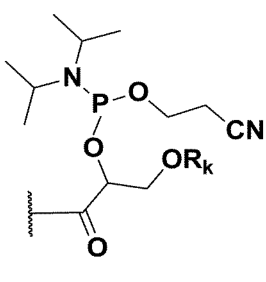
,
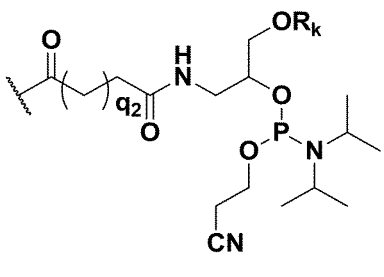
,

(B9') (B10')

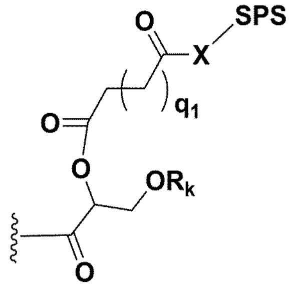,

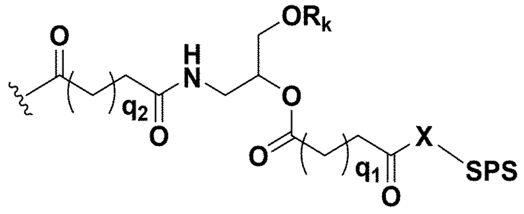,

(B11) (B12)

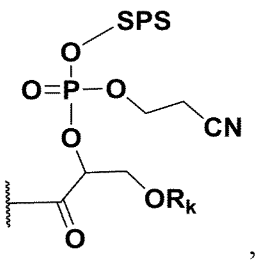,

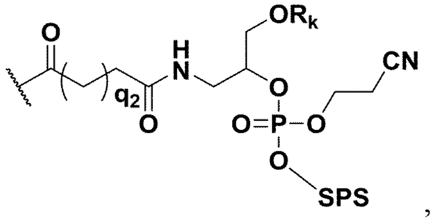,

(B11') (B12')

wherein $q_1$ is an integer of 1-4, $q_2$ is an integer of 1-10, X is O or NH, $M^+$ is a cation, $R_k$ is a hydroxy protecting group, SPS represents a solid phase support, and  represents a site where a group is covalently linked. In some embodiments, $q_1$ is 1 or 2. In some embodiments, $q_2$ is an integer of 1-5. In some embodiments, $R_4$ comprises a structure as shown by Formula (B9) or (B10). In some embodiments, $R_4$ comprises a structure as shown by Formula (B11) or (B12).

In some embodiments, $R_k$ is one or more of Tr (trityl), MMTr (4-methoxytrityl), DMTr (4,4'-dimethoxytrityl), and TMTr (4,4',4"-trimethoxytrityl). In some embodiments, $R_k$ may be DMTr, i.e., 4,4'-dimethoxytrityl.

The definition of $L_1$ is as described above.

In some embodiments, $L_1$ is used to link the $M_1$ targeting group to the N atom on the nitrogenous backbone, thereby providing liver targeting function for the siRNA conjugate as shown by Formula (308). In some embodiments, $L_1$ comprises any one of A1-A26, or the combination thereof.

According to the description above, those skilled in the art would easily understand that as compared with the well-known solid phase phosphoramidite synthesis methods in the art, an siRNA conjugate as shown by Formula (308) in which a conjugating molecule is linked to any possible position of the nucleotide sequence can be obtained through the above first functional group and an optional second functional group. For example, the conjugating molecule is linked to an end of the nucleotide sequence or to a terminal of the nucleotide sequence. Correspondingly, unless otherwise specified, in the following description regarding siRNA conjugate and/or conjugating molecule preparation, when referring to the reactions such as "deprotection", "coupling", "capping", "oxidation", "sulfurization", it will be understood that the reaction conditions and agents involved in the well-known phosphoramidite nucleic acid solid phase synthesis methods in the art would also apply to these reactions. Exemplary reaction conditions and agents will be described in detail hereinafter.

In some embodiments, each $S_1$ is independently an $M_1$. In some embodiments, each $S_1$ is independently a group formed by protecting at least one active hydroxy in $M_1$ with a hydroxy protecting group. In some embodiments, each $S_1$ is independently a group formed by protecting all active hydroxys in $M_1$ with hydroxy protecting groups. In some embodiments, any hydroxy protecting groups known to those skilled in the art may be used to protect the active hydroxy in $M_1$. In some embodiments, the protected hydroxy may be represented by the formula YCOO-, wherein each Y is independently selected from the group consisting of $C_1$-$C_{10}$ alkyl and $C_6$-$C_{10}$ aryl, wherein the $C_1$-$C_{10}$ alkyl and $C_6$-$C_{10}$ aryl are optionally substituted with one or more substituents selected from the group consisting of halo and $C_1$-C6 alkyl. In some embodiments, each Y is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and $C_1$-$C_6$ alkylphenyl.

In some embodiments, each $S_1$ is independently selected from the group consisting of Formulae A46-A54:

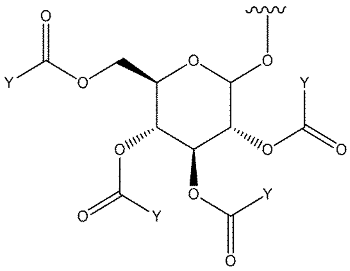, 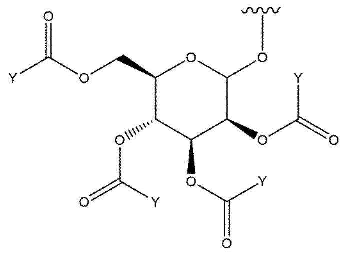, 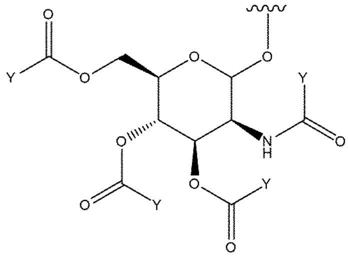,

(A46) (A47) (A48)

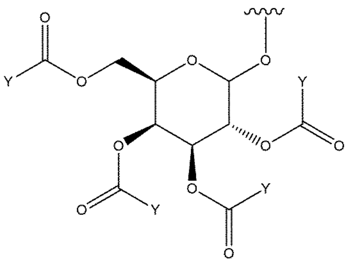, 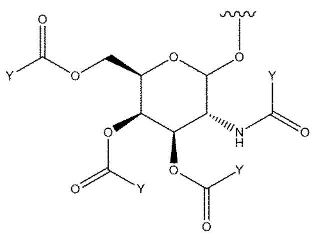, 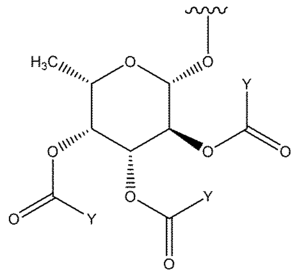,

(A49) (A50) (A51)

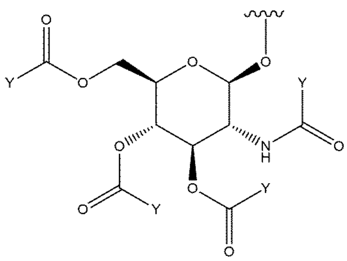, 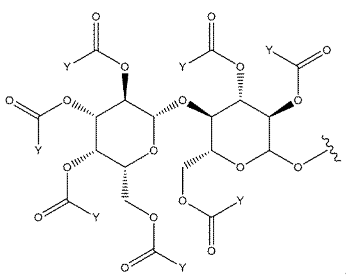, 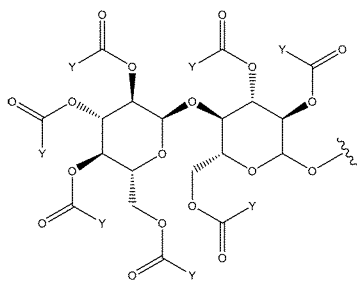.

(A52) (A53) (A54)

In some embodiments, $S_1$ is Formula A49 or A50.

In some embodiments, each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. In some embodiments, Y is a methyl.

As mentioned previously, the method for preparing the siRNA conjugate as shown by Formula (308) further comprises the following steps of: synthesizing the other strand of the siRNA (for example, when the sense strand of the siRNA linked to the conjugating molecule is synthesized in the above steps, the method further comprises synthesizing the antisense strand of the siRNA by the solid phase synthesis method, and vice versa); isolating the sense strand and the antisense strand; and annealing. In particular, in the isolating step, the solid phase support linked to the nucleotide sequence and/or the conjugating molecule is cleaved and at the same time the necessary protecting group is removed (in this case, each $S_1$ group in the compound as shown by Formula (321) is converted to a corresponding $M_1$ targeting group), thereby obtaining the sense strand (or antisense strand) of the siRNA linked to the conjugating molecule and the corresponding antisense strand (or sense strand). The sense strand and the antisense strand are annealed to form a double-stranded RNA structure, thereby obtaining the siRNA conjugate as shown by Formula (308).

In some embodiments, the method for preparing the siRNA conjugate as shown by Formula (308) comprises the following steps of: contacting the compound as shown by Formula (321) with the first nucleoside monomer at

3'terminal of the sense strand or antisense strand under coupling reaction condition in the presence of a coupling agent, thereby linking the compound as shown by Formula (321) to the first nucleotide in the sequence; successively linking nucleoside monomers in the direction from 3' to 5' to synthesize the sense strand or the antisense strand of the siRNA according to the desired nucleotide type and sequence of the sense strand or antisense strand, under the condition of solid phase phosphoramidite synthesis; wherein the compound as shown by Formula (321) is a compound in which R4 comprises a first functional group and a second functional group, the first functional group comprises a protected hydroxy and the second functional group comprises a structure as shown by Formula (C1') or (C3'), and the compound as shown by Formula (321) is deprotected before linked to the first nucleoside monomer; and the linking of each nucleoside monomer comprises a four-step reaction of deprotection, coupling, capping, and oxidation or sulfurization, thus obtaining a sense strand or an antisense strand of a nucleic acid linked to the conjugating molecule; successively linking the nucleoside monomers in the direction from 3' to 5' to synthesize the sense strand or antisense strand of the nucleic acid according to the nucleotide type and sequence of the sense strand or the antisense strand, under the condition of solid phase phosphoramidite synthesis; wherein the linking of each nucleoside monomer comprises a four-step reaction of deprotection, coupling, capping, and oxidation or sulfurization; removing the protecting groups and cleaving the solid phase support; isolating and purifying to obtain the sense strand and the antisense strand; and annealing.

In some embodiments, the method for preparing the siRNA conjugate as shown by Formula (308) further comprises the following steps of: successively linking nucleoside monomers in the direction from 3' to 5' to synthesize the sense strand or the antisense strand according to the nucleotide type and sequence of the sense strand or antisense strand in the double-stranded siRNA; wherein the linking of each nucleoside monomer comprises a four-step reaction of deprotection, coupling, capping, and oxidation or sulfurization, thus obtaining a sense strand linked to the solid phase support and an antisense strand linked to the solid phase support; contacting the compound as shown by Formula (321) with the sense strand linked to the solid phase support or the antisense strand linked to the solid phase support under coupling reaction condition in the presence of a coupling agent, thereby linking the compound as shown by Formula (321) to the sense strand or the antisense strand; wherein the compound as shown by Formula (321) is a compound in which $R_4$ comprises a phosphoramidite group as the first functional group; removing the protecting groups and cleaving the solid phase support; respectively isolating and purifying to obtain the sense strand or the antisense strand of the siRNA; and annealing; wherein the sense strand or the antisense strand of the siRNA is linked to a conjugating molecule.

In some embodiments, the P atom in Formula A59 is linked to the 3' terminal of the sense strand of the siRNA, and the method for preparing the siRNA conjugate as shown by Formula (308) comprises:

(1) removing the hydroxy protecting group $R_k$ in the compound as shown by Formula (321) (wherein the compound as shown by Formula (321) is a compound in which $R_4$ comprises a first functional group and a second function group, the first functional group comprises a protected hydroxy $OR_k$, and the second function group has a structure as shown by Formula (C1') or (C3')); and contacting the deprotected product with a nucleoside monomer to obtain a nucleoside monomer linked to a solid phase support via the conjugating molecule under a coupling reaction condition in the presence of a coupling agent;

(2) starting from the nucleoside monomer linked to the solid phase support via the conjugating molecule, synthesizing the sense strand of the siRNA in the direction from 3' to 5' by a solid phase phosphoramidite synthesis;

(3) synthesizing the antisense strand of the siRNA by a solid phase phosphoramidite synthesis method; and

(4) isolating the sense strand and the antisense strand of the siRNA, and annealing the same to obtain the siRNA conjugate as shown by Formula (308).

Wherein, in step (1), the method for removing the protecting group $R_k$ in the above compound as shown by Formula (321) comprises contacting the compound as shown by Formula (321) with a deprotection agent under a deprotection condition. The deprotection condition comprises a temperature of 0-50°C, and in some embodiments, 15-35°C, and a reaction time of 30-300 seconds, and in some embodiments, 50-150 seconds. The deprotection agent may be selected from one or more of trifluoroacetic acid, trichloroacetic acid, dichloroacetic acid, and monochloroacetic acid, and in some embodiments, the deprotection agent is dichloroacetic acid. The molar ratio of the deprotection agent to the compound as shown by Formula (321) may be 10:1 to 1000:1, and in some embodiments, 50:1 to 500:1.

The coupling reaction condition and the coupling agent may be any conditions and agents suitable for the above coupling reaction. In some embodiments, the same condition and agent as those of the coupling reaction in the solid phase synthesis method may be used.

In some embodiments, the coupling reaction condition comprises a reaction temperature of 0-50°C, and in some embodiments, 15-35°C. The molar ratio of the compound as shown by Formula (321) to the nucleoside monomer may be 1:1 to 1:50, and in some embodiments, 1:2 to 1:5. The molar ratio of the compound as shown by Formula (321) to the coupling agent may be 1:1 to 1:50, and in some embodiments, 1:3 to 1:10. The reaction time may be 200-3000 seconds, and in some embodiments, 500-1500 seconds. The coupling agent may be selected from one or more of 1H-tetrazole, 5-ethylthio-1H-tetrazole and 5-benzylthio-1H-tetrazole, and in some embodiments, is 5-ethylthio-1H-tetrazole.

The coupling reaction may be carried out in an organic solvent, and the organic solvent may be selected from one or more of anhydrous acetonitrile, anhydrous DMF and anhydrous dichloromethane, and in some embodiments, is anhydrous acetonitrile. The amount of the organic solvent may be 3-50 L/mol, and in some embodiments, 5-20 L/mol, with respect to the compound as shown by Formula (321).

In step (2), a sense strand SS of the second siRNA conjugate is synthesized in the direction from 3' to 5' by the phosphoramidite nucleic acid solid phase synthesis method, starting from the nucleoside monomer linked to the solid phase support via the conjugating molecule prepared in the above steps. In this case, the conjugating molecule is linked to 3' terminal of the resultant sense strand.

Other conditions for the solid phase synthesis in steps (2) and (3), comprising the deprotection condition for the nucleoside monomer, the type and amount of the deprotection agent, the coupling reaction condition, the type and amount of the coupling agent, the capping reaction condition, the type and amount of the capping agent, the oxidation reaction condition, the type and amount of the oxidation agent, the sulfurization reaction condition, and the type and amount of the sulfurization agent, adopt various conventional agents, amounts, and conditions in the art.

For instance, in some embodiments, the solid phase synthesis in steps (2) and (3) may use the following conditions:

The deprotection condition for the nucleoside monomer comprises a temperature of 0-50°C, and in some embodiments, 15-35°C, and a reaction time of 30-300 seconds, and in some embodiments, 50-150 seconds. The deprotection agent may be selected from one or more of trifluoroacetic acid, trichloroacetic acid, dichloroacetic acid, and monochloroacetic acid, and in some embodiments, the deprotection agent is dichloroacetic acid. The molar ratio of the deprotection agent to the protecting group 4,4'-dimethoxytrityl on the solid phase support is 2:1 to 100:1, and in some embodiments, is 3:1 to 50:1.

The coupling reaction condition comprises a reaction temperature of 0-50°C, and in some embodiments, 15-35°C. The molar ratio of the nucleic acid sequence linked to the solid phase support to the nucleoside monomer is 1:1 to 1:50, and in some embodiments, is 1:5 to 1:15. The molar ratio of the nucleic acid sequence linked to the solid phase support to the coupling agent is 1:1 to 1:100, and in some embodiments, is 1:50 to 1:80. The selection of the reaction time and the coupling agent can be same as above.

The capping reaction condition comprises a reaction temperature of 0-50°C, and in some embodiments, 15-35°C, and a reaction time of 5-500 seconds, and in some embodiments, 10-100 seconds. The selection of the capping agent can be same as above. The molar ratio of the total amount of the capping agent to the nucleic acid sequence linked to the solid phase support may be 1:100 to 100:1, and in some embodiments, is 1:10 to 10:1. In the case where the capping agent uses equimolar acetic anhydride and N-methylimidazole, the molar ratio of the acetic anhydride to the N-methylimidazole and the nucleic acid sequence linked to the solid phase support may be 1:1:10 to 10:10:1, and in some embodiments, is 1:1:2 to 2:2:1.

The oxidation reaction condition comprises a reaction temperature of 0-50°C, and in some embodiments, 15-35°C, and a reaction time of 1-100 seconds, and in some embodiments, 5-50 seconds. In some embodiments, the oxidation agent is iodine (in some embodiments, provided in the form of iodine water). The molar ratio of the oxidation agent to the nucleic acid sequence linked to the solid phase support in the coupling step may be 1:1 to 100:1, and in some embodiments, is 5: to 50:1. In some embodiments, the oxidation reaction is performed in a mixed solvent in which the ratio of tetrahydrofuran:water:pyridine is 3:1:1 to 1:1:3. The sulfurization reaction condition comprises a reaction temperature of 0-50°C, and in some embodiments, 15-35°C, and a reaction time of 50-2000 seconds, and in some embodiments, 100-1000 seconds. In some embodiments, the sulfurization agent is xanthane hydride. The molar ratio of the sulfurization agent to the nucleic acid sequence linked to the solid phase support in the coupling step is 10:1 to 1000:1, and in some embodiments, is 10:1 to 500:1. In some embodiments, the sulfurization reaction is performed in a mixed solvent in which the ratio of acetonitrile:pyridine is 1:3 to 3:1.

The method further comprises isolating the sense strand and the antisense strand of the siRNA after linking all nucleoside monomers and before the annealing. Methods for isolation are well-known to those skilled in the art and generally comprise cleaving the synthesized nucleotide sequence from the solid phase support, removing protecting groups on the bases, phosphate groups and ligands, purifying and desalting.

The conventional cleavage and deprotection methods in the synthesis of siRNAs can be used to cleave the synthesized nucleotide sequence from the solid phase support, and remove the protecting groups on the bases, phosphate groups and ligands. For example, contacting the resultant nucleotide sequence linked to the solid phase support with strong aqua; during deprotection, the protecting group YCOO- in groups A46-A54 is converted to a hydroxy, and thus the $S_1$ groups is converted to a corresponding $M_1$ group, providing the siRNA conjugate as shown by Formula (308). Wherein the strong aqua may be aqueous ammonia of a concentration of 25-30% by weight. The amount of the strong aqua may be 0.2 ml/$\mu$mol-0.8 ml/$\mu$mol with respect to the target siRNA sequence.

When there is at least one 2'-TBDMS protection on the synthesized nucleotide sequence, the method further comprises contacting the nucleotide sequence removed from the solid phase support with triethylamine trihydrofluoride to remove the 2'-TBDMS protection. In this case, the resultant target siRNA sequence comprises the corresponding

nucleoside having free 2'-hydroxy. The amount of pure triethylamine trihydrofluoride is 0.4 ml/μmol-1.0 ml/μmol with respect to the target siRNA sequence. As such, the siRNA conjugate as shown by Formula (308) may be obtained.

Methods for purification and desalination are well-known to those skilled in the art. For example, nucleic acid purification may be performed using a preparative ion chromatography purification column with a gradient elution of NaBr or NaCl; after collection and combination of the product, the desalination may be performed using a reverse phase chromatography purification column.

The non-bridging oxygen atom or sulfur atom in the phosphodiester bond or phosphorothioate diester bond between the nucleotides in the resultant siRNA conjugate as shown by Formula (308) substantially binds to a sodium ion, and the siRNA conjugate as shown by Formula (308) is substantially present in the form of a sodium salt. The well-known ion-exchange methods may be used, and the sodium ion may be replaced with hydrogen ion and/or other cations, thereby providing other forms of siRNA conjugates as shown by Formula (308). The cations are as described above.

During synthesis, the purity and molecular weight of the nucleic acid sequence may be determined at any time in order to better control the synthesis quality, and such detection methods are well-known to those skilled in the art. For example, the purity of the nucleic acid may be detected by ion exchange chromatography, and the molecular weight may be determined by liquid chromatography-mass spectrometry (LC-MS).

Methods for annealing are also well-known to those skilled in the art. For example, the synthesized sense strand (S strand) and antisense strand (AS strand) may be simply mixed in water for injection at an equimolar ratio, heated to 70-95°C, and then cooled at room temperature to form a double-stranded structure via hydrogen bond. As such, the siRNA conjugate as shown by Formula (308) may be obtained.

After obtaining the siRNA conjugate, in some embodiments, the synthesized siRNA conjugate as shown by Formula (308) can also be characterized by the means such as molecular weight detection using the methods such as liquid chromatography-mass spectrometry, to confirm that the synthesized siRNA conjugate is the designed siRNA conjugate as shown by Formula (308) of interest, and the sequence of the synthesized siRNA is the sequence of the siRNA sequence desired to be synthesized, for example, is one of the sequences listed in Tables 1.

The compound as shown by Formula (321) may be prepared by the following method comprising: contacting a compound as shown by Formula (313) with a cyclic anhydride in an organic solvent under esterification reaction condition in the presence of a base and an esterification catalyst; and isolating the compound as shown by Formula (321) by ion exchange:

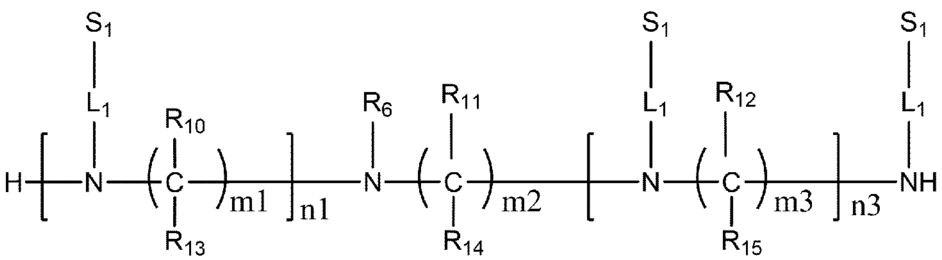

Formula (313)

wherein the definitions and optional ranges of n1, n3, m1, m2, m3, $R_{10}$, $R_{11}$, $R_{12}$, $R_{13}$, $R_{14}$, $R_{15}$, $L_1$, and $S_1$are respectively as described above;

$R_6$ is a group for providing $R_4$ of Formula (321). In some embodiments, $R_6$ comprises a structure as shown by Formula (A61):

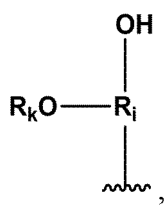

(A61)

wherein, $R_i$ is any group capable of linking to the N atom on the nitrogenous backbone, linking to $R_kO$ and linking to a

free hydroxy; and $R_k$ is a hydroxy protecting group. In this case, the compound as shown by Formula (321) is obtained, wherein $R_4$ comprises a first functional group as a hydroxy protecting group and a second functional group comprising a group as shown by Formula (C1) or (C2).

The esterification reaction condition comprises a reaction temperature of 0-100°C and a reaction time of 8-48 hours. In some embodiments, the esterification reaction condition comprises a reaction temperature of 10-40°C and a reaction time of 20-30 hours.

In some embodiments, the organic solvent comprises one or more of an epoxy solvent, an ether solvent, a haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tertbutyl ether, and the haloalkane solvent is one or more of dichloromethane, trichloromethane and 1,2-dichloroethane. In some embodiments, the organic solvent is dichloromethane. The amount of the organic solvent is 3-50 L/mol, and in some embodiments, 5-20 L/mol, with respect to the compound as shown by Formula (313).

In some embodiments, the cyclic anhydride is one of succinic anhydride, glutaric anhydride, adipic anhydride or pimelic anhydride, and in some embodiments, the cyclic anhydride is succinic anhydride. The molar ratio of the cyclic anhydride to the compound as shown by Formula (313) is 1:1 to 10:1, and in some embodiments, 2:1 to 5:1.

The esterification catalyst may be any catalyst capable of catalyzing the esterification, for example, the catalyst may be 4-dimethylaminopyridine. The molar ratio of the catalyst to the compound as shown by Formula (313) is 1:1 to 10:1, and in some embodiments, 2:1 to 5:1.

In some embodiments, the base may be any inorganic base, organic base or a combination thereof. Considering solubility and product stability, the base may be, for example, tertiary amine. In some embodiments, the tertiary amine is triethylamine or N,N-diisopropylethylamine. The molar ratio of the tertiary amine to the compound as shown by Formula (313) is 1:1 to 20:1, and in some embodiments, is 3:1 to 10:1.

The ion exchange serves the function of converting the compound as shown by Formula (321) into a desired form of carboxylic acid or carboxylic salt and the methods of ion exchange are well-known to those skilled in the art. The conjugating molecule having the cation $M^+$ may be obtained by using suitable ion exchange solution and ion exchange condition, which is not described here in detail. In some embodiments, a triethylamine phosphate solution is used in the ion exchange reaction, and the concentration of the triethylamine phosphate solution is 0.2-0.8 M. In some embodiments, the concentration of the triethylamine phosphate solution is 0.4-0.6 M. The amount of the triethylamine phosphate solution is 3-6 L/mol, and in further embodiment, 4-5 L/mol, with respect to the compound as shown by Formula (313).

The compound as shown by Formula (321) may be isolated from the reaction mixture using any suitable isolation methods. In some embodiments, the compound as shown by Formula (321) may be isolated by removal of solvent via evaporation and followed by chromatography, for example, using the following two chromatographic conditions for the isolation: (1) normal phase purification silica gel: 200-300 mesh silica gel filler, and gradient elution of 1wt‰ triethylamine in dichloromethane: methanol = 100:18 to 100:20; or (2) reverse phase purification: C18, C8 reverse phase fillers, and gradient elution of methanol: acetonitrile = 0.1:1 to 1:0.1. In some embodiments, the solvent may be directly removed to obtain a crude product of the compound as shown by Formula (321), which may be directly used in subsequent reactions.

In some embodiments, the method for preparing the compound as shown by Formula (321) further comprises: contacting the product obtained from the above ion exchanging reaction with a solid phase support containing amino or hydroxy in an organic solvent under condensation reaction condition in the presence of a condensing agent, a condensation catalyst and tertiary amine. In this case, the compound as shown by Formula (321) is obtained, wherein $R_4$ comprises a first functional group comprising a hydroxy protecting group and a second functional group having a structure as shown by Formula (C1').

The solid phase support is one of the carriers used in solid phase synthesis of siRNA, some of which are well-known to those skilled in the art. For example, the solid phase support may be selected from the solid phase supports containing an active hydroxy or amino functional group. In some embodiments, the solid phase support is an amino resin or hydroxy resin. In some embodiments, the amino or hydroxy resin has the following parameters: particle size of 100-400 mesh, and surface amino or hydroxy loading of 0.2-0.5 mmol/g. The dosage ratio of the compound as shown by Formula (321) to the solid phase support is 10-400 μmol compound per gram of solid phase support (μmol/g). In some embodiments, the dosage ratio of the compound of Formula (321) to the solid phase support is 50-200 μmol/g.

The organic solvent may be any suitable solvent or mixed solvent known to those skilled in the art. In some embodiments, the organic solvent is one or more of acetonitrile, an epoxy solvent, an ether solvent, a haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tertbutyl ether, and the haloalkane solvent is one or more of dichloromethane, trichloromethane and 1,2-dichloroethane. In some embodiments, the organic solvent is acetonitrile. The amount of the organic solvent may be 20-200 L/mol, and in some embodiments, 50-100 L/mol, with respect to the compound as shown by Formula (321).

In some embodiments, the condensing agent may be benzotriazol-1-yl-oxytripyrrolidino phosphonium hexafluorophosphate (PyBop), 3-(Diethoxyphosphoryloxy)-1,2,3-benzotriazin-4(3H)-one (DEPBT) and/or O-benzotriazol-1-yl-tetramethyluronium hexafluorophosphate. In some embodiments, the condensing agent is O-benzotriazol-1-yl-tetramethyluronium hexafluorophosphate. The molar ratio of the condensing agent to the compound as shown by Formula (321) is 1:1 to 20:1, and in some embodiments, 1:1 to 5:1.

In some embodiments, the tertiary amine is triethylamine and/or N,N-diisopropylethylamine, and in some embodiments, N,N-diisopropylethylamine. The molar ratio of the tertiary amine to the compound as shown by Formula (321) is 1:1 to 20:1, and in some embodiments, 1:1 to 5:1.

In some embodiments, the method for preparing the compound as shown by Formula (321) further comprises: contacting the resultant condensation product with a capping agent and an acylation catalyst in an organic solvent under capping reaction condition, and isolating the compound as shown by Formula (321). The capping reaction is used to remove any active functional group that does not completely react, so as to avoid producing unnecessary by products in subsequent reactions. The capping reaction condition comprises a reaction temperature of 0-50°C, and in some embodiments, 15-35°C, and a reaction time of 1-10 hours, and in some embodiments, 3-6 hours. The capping agent may be a capping agent used in solid phase synthesis of siRNA, and the capping agent used in solid phase synthesis of siRNA is well known to those skilled in the art.

In some embodiments, the capping agent is composed of a capping agent 1 (cap1) and a capping agent 2 (cap2). The cap1 is N-methylimidazole, and in some embodiments, provided in the form of a mixed solution of N-methylimidazole in pyridine/acetonitrile, wherein the volume ratio of the pyridine to the acetonitrile is 1:10 to 1:1, and in some embodiments, 1:3 to 1:1. In some embodiments, the ratio of the total volume of the pyridine and acetonitrile to the volume of the N-methylimidazole is 1:1 to 10:1, and in some embodiments, 3:1 to 7:1. The capping agent 2 is acetic anhydride. In some embodiments, the capping agent 2 is provided in the form of a solution of acetic anhydride in acetonitrile, wherein the volume ratio of the acetic anhydride to the acetonitrile is 1:1 to 1:10, and in some embodiments, 1:2 to 1:6.

In some embodiments, the ratio of the volume of the mixed solution of N-methylimidazole in pyridine/acetonitrile to the mass of the compound as shown by Formula (321) is 5 ml/g to 50 ml/g, and in some embodiments, 15 ml/g to 30 ml/g. The ratio of the volume of the solution of acetic anhydride in acetonitrile to the mass of the compound as shown by Formula (321) is 0.5 ml/g to 10 ml/g, and in some embodiments, 1 ml/g to 5 ml/g.

In some embodiments, the capping agent uses equimolar acetic anhydride and N-methylimidazole. In some embodiments, the organic solvent is one or more of acetonitrile, an epoxy solvent, an ether solvent, a haloalkane solvent, dimethyl sulfoxide, N,N-dimethylformamide, and N,N-diisopropylethylamine. In some embodiments, the organic solvent is acetonitrile. The amount of the organic solvent may be 10-50 L/mol, and in some embodiments, 5-30 L/mol, with respect to the compound as shown by Formula (321).

In some embodiments, the acylation catalyst may be selected from any catalyst that may be used for esterification condensation or amidation condensation, such as alkaline heterocyclic compounds. In some embodiments, the acylation catalyst is 4-dimethylaminopyridine. The mass ratio of the catalyst to the compound as shown by Formula (321) may be 0.001:1 to 1:1, and in some embodiments, 0.01:1 to 0.1:1.

In some embodiments, the compound as shown by Formula (321) may be isolated from the reaction mixture using any suitable isolation methods. In some embodiments, the compound as shown by Formula (321) may be obtained by thoroughly washing with an organic solvent and filtering to remove unreacted reactants, excess capping agent and other impurities, wherein the organic solvent is selected from acetonitrile, dichloromethane, or methanol. In some embodiments, the organic solvent is acetonitrile.

In some embodiments, the preparation of the conjugating molecule as shown by Formula (321) comprises contacting a compound as shown by Formula (313) with a phosphorodiamidite in an organic solvent under coupling reaction condition in the presence of a coupling agent, and isolating the compound as shown by Formula (321). In this case, the compound as shown by Formula (321) is obtained, where $R_4$ comprises a first functional group comprising a hydroxy protecting group and a second functional group having a structure as shown by Formula (C3).

In some embodiments, the coupling reaction condition comprises that thetemperature may be 0-50°C, such as 15-35°C; the molar ratio of the compound as shown by Formula (313) to the phosphorodiamidite may be 1:1 to 1:50, such as 1:5 to 1:15; the molar ratio of the compound as shown by Formula (313) to the coupling agent may be 1:1 to 1:100, such as 1:50 to 1:80; the reaction time may be 200-3000 seconds, such as 500-1500 seconds. The phosphorodiamidite may be, for example, bis(diisopropylamino)(2-cyanoethoxy)phosphine, which may be commercially available or synthesized according to well-known methods in the art. The coupling agent is selected from one or more of 1H-tetrazole, 5-ethylthio-1H-tetrazole and 5-benzylthio-1H tetrazole, such as 5-ethylthio-1H-tetrazole. The coupling reaction may be performed in an organic solvent, and the organic solvent is selected from one or more of anhydrous acetonitrile, anhydrous DMF and anhydrous dichloromethane, such as anhydrous acetonitrile. In some embodiments, the amount of the organic solvent may be 3-50 L/mol, such as 5-20 L/mol, with respect to the compound as shown by Formula (313). By performing the coupling reaction, the hydroxy in the compound as shown by Formula (313) reacts with the phosphorodiamidite to form a phosphoramidite group. In some embodiments, the solvent may be directly removed to obtain a

crude product of the compound as shown by Formula (321), which may be directly used in subsequent reactions.

In some embodiments, the method for preparing the compound as shown by Formula (321) further comprises: contacting the isolated product with a solid phase support containing hydroxy in an organic solvent under coupling reaction condition in the presence of a coupling agent, followed by capping, oxidation, and isolation, to obtain the compound as shown by Formula (321). In this case, the compound as shown by Formula (321) is obtained, where $R_4$ comprises a first functional group comprising a hydroxy protecting group and a second functional group having a structure as shown by Formula (C3').

In some embodiments, the solid phase support is a well-known solid phase support in the art for solid phase synthesis of a nucleic acid, such as a deprotected commercially available universal solid phase support (NittoPhase®HL UnyLinker™ 300 Oligonucleotide Synthesis Support, Kinovate Life Sciences, as shown by Formula B80):

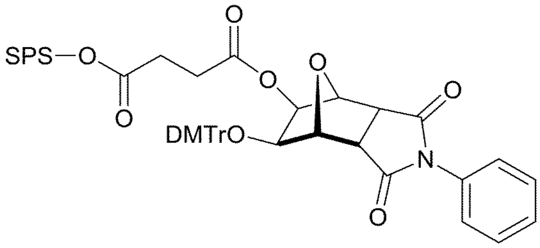

(B80).

A deprotection reaction is well-known in the art. In some embodiments, the deprotection condition comprises a temperature of 0-50°C, such as 15-35°C; and a reaction time of 30-300 seconds, such as 50-150 seconds. The deprotection agent may be selected from one or more of trifluoroacetic acid, trichloroacetic acid, dichloroacetic acid, and monochloroacetic acid. In some embodiments, the deprotection agent is dichloroacetic acid. The molar ratio of the deprotection agent to the protecting group -DMTr (4,4'-dimethoxytrityl) on the solid phase may be 2:1 to 100:1, such as 3:1 to 50:1. By such deprotection, reactive free hydroxyl groups are obtained on the surface of the solid phase support, for facilitating the subsequent coupling reaction.

The coupling reaction condition and the coupling agent may be selected as above. By performing the coupling reaction, the free hydroxy groups formed in the deprotection reaction reacts with the phosphoramidite groups, so as to form a phosphite ester linkage.

In some embodiments, the capping reaction condition comprises a reaction temperature of 0-50°C, such as 15-35°C, and a reaction time of 5-500 seconds, such as 10-100 seconds. The capping reaction is performed in the presence of a capping agent. The selection and amount of the capping agent may be as described above.

The oxidation reaction condition may comprise a temperature of 0-50°C, such as 15 35°C, and a reaction time of 1-100 seconds, such as 5-50 seconds. The oxidation agent may be, for example, iodine (in some embodiments, provided in the form of iodine water). In some embodiments, the molar ratio of the oxidation agent to the nucleic acid sequence linked to the solid phase support is 1:1 to 100:1, such as 5:1 to 50:1. In some embodiments, the oxidation reaction is performed in a mixed solvent in which the ratio of tetrahydrofuran: water: pyridine is 3:1:1 to 1:1:3.

In some embodiments, $R_6$ is one of the group as shown by Formula B7 or B8:

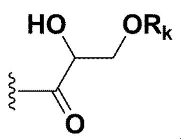

,

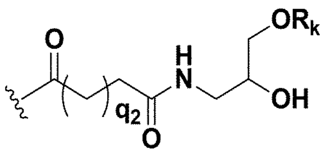

,

(B7) (B8)

wherein the definition of $q_2$ is as described above.

In this case, the compound as shown by Formula (313) may be prepared by the following method: contacting a compound as shown by Formula (314) with a compound as shown by Formula (A-1) or a compound as shown by

Formula (A-2) in an organic solvent under amidation reaction condition in the presence of an agent for amidation condensation and tertiary amine, and then isolating:

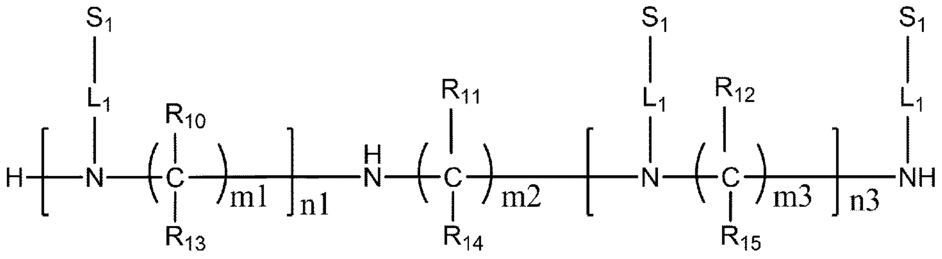

Formula (314)

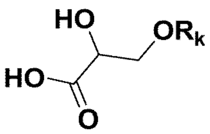

,

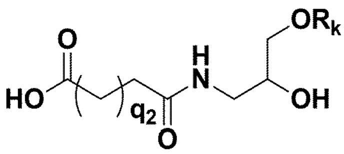

,

(A-1) (A-2)

wherein the definitions and optional ranges of n1, n3, m1, m2, m3, $R_{10}$, $R_{11}$, $R_{12}$, $R_{13}$, $R_{14}$, $R_{15}$, $L_1$, $S_1$, $q_2$ and $R_k$ are respectively as described above.

The amidation reaction condition may comprise a reaction temperature of 0-100°C and a reaction time of 1-48 hours. In some embodiments, the amidation reaction condition comprises a reaction temperature of 10-40°C and a reaction time of 2-16 hours.

In some embodiments, the organic solvent is one or more of an alcohol solvent, an epoxy solvent, an ether solvent, a haloalkane solvent, dimethyl sulfoxide, N,N-dimethylformamide, and N,N-diisopropylethylamine. In some embodiments, the alcohol solvent is one or more of methanol, ethanol and propanol, and in some embodiments, is ethanol. In some embodiments, the epoxy solvent is dioxane and/or tetrahydrofuran. In some embodiments, the ether solvent is diethyl ether and/or methyl tertbutyl ether. In some embodiments, the haloalkane solvent is one or more of dichloromethane, trichloromethane and 1,2-dichloroethane. In some embodiments, the organic solvent is dichloromethane. The amount of the organic solvent is 3-50 L/mol, and in further embodiments, 3-20 L/mol, with respect to the compound as shown by Formula (314).

In some embodiments, the agent for amidation condensation is benzotriazol-1-yl-oxytripyrrolidinophosphonium hexafluorophosphate, 3-(Diethoxyphosphoryloxy)-1,2,3-benzotriazin-4(3H)-one, 4-(4,6-dimethoxytriazin-2-yl)-4-methylmorpholine hydrochloride, 2 ethoxy-1-ethoxycarbonyl-1,2-dihydroquinoline (EEDQ) or O-benzotriazol-1-yl-tetramethyluronium hexafluorophosphate, and in further embodiments, 3-(Diethoxyphosphoryloxy)-1,2,3-benzotriazin-4(3H)-one. The molar ratio of the agent for amidation condensation to the compound as shown by Formula (314) may be 1:1 to 10:1, and in some embodiments, 2.5:1 to 5:1.

In some embodiments, the tertiary amine is triethylamine and/or N,N-diisopropylethylamine, and in further embodiments, N,N-diisopropylethylamine. The molar ratio of the tertiary to the compound as shown by Formula (314) is 3:1 to 20:1, and in some embodiments, is 5:1 to 10:1.

In some embodiments, the compounds as shown by Formula (A-1) and Formula (A-2) may be prepared by any suitable methods. For example, when $R_k$ is a DMTr group, the compound as shown by Formula (A-1) may be prepared by reacting calcium glycerate with DMTrCl. Similarly, the compound as shown by Formula (A-2) may be prepared by contacting 3-amino-1,2-propanediol with a cyclic anhydride and then reacting with DMTrCl, wherein the cyclic anhydride may be a cyclic anhydride having 4-13 carbon atoms, and in some embodiments, having 4-8 carbon atoms. Those skilled in the art would readily understand that the selections of the cyclic anhydride correspond to different values for $q_2$ in the compound as shown by Formula (A-2). For example, when the cyclic anhydride is succinic anhydride, $q_2$=1; when the cyclic anhydride is glutaric anhydride, $q_2$=2, and so on.

In some variants, the compound as shown by Formula (313) can also be prepared by successively reacting the compound as shown by Formula (314) with the cyclic anhydride, 3-amino-1,2-propanediol, and DMTrCl. Those skilled in the art would readily understand that these variants would not affect the structure and function of the compound as shown by Formula (313), and these variants can be readily achieved by those skilled in the art on the basis of the

above methods.

Similarly, the compound as shown by Formula (313) may be isolated from the reaction mixture by any suitable isolation methods. In some embodiments, the compound as shown by Formula (313) may be isolated by removal of solvent via evaporation and followed by chromatography, for example, using the following two chromatographic conditions for isolation: (1) normal phase purification silica gel: 200-300 mesh silica gel filler, and gradient elution of petroleum ether: ethyl acetate: dichloromethane: N,N-dimethylformamide = 1:1:1:0.5 to 1:1:1:0.6; and (2) reverse phase purification: C18, C8 reverse phase fillers, and gradient elution of methanol: acetonitrile = 0.1:1 to 1:0.1. In some embodiments, the solvent may be directly removed to obtain a crude product of the compound as shown by Formula (313), which may be directly used in subsequent reactions.

In some embodiments, the compound as shown by Formula (314) may be prepared by the following method comprising: contacting a compound as shown by Formula (320) with a compound as shown by Formula (316) in an organic solvent under condensation reaction condition in the presence of an agent for amidation condensation and tertiary amine, and then isolating:

$$S_1—L_1—OH$$

Formula (316)

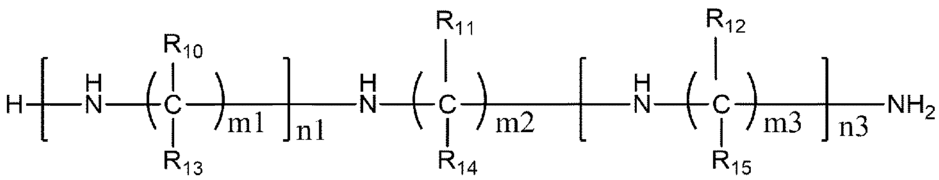

Formula (320)

wherein the definitions and optional ranges of n1, n3, m1, m2, m3, $R_{10}$, $R_{11}$, $R_{12}$, $R_{13}$, $R_{14}$, and $R_{15}$ are respectively as described above.

The compound as shown by Formula (316) can be, such as, those disclosed in J. Am. Chem. Soc. 2014, 136, 16958-16961, or the compound as shown by Formula (316) may be prepared by those skilled in the art via various methods. For example, some compound as shown by Formula (316) may be prepared according to the methods as disclosed in Example 1 of US patent 8,106,022 B2, which is incorporated herein by reference in its entirety.

In some embodiments, the condensation reaction condition comprises a reaction temperature of 0-100°C and a reaction time of 0.1-24 hours. In some embodiments, the condensation reaction condition comprises a reaction temperature is 10-40°C and a reaction time is 0.5-16 hours.

Considering the structure of the desired compound as shown by Formula (314), the molar ratio of the compound as shown by Formula (316) to the compound as shown by Formula (320) should be determined based on the sum of n1 and n3 in Formula (320). In some embodiments, for example, when n1+n3=3, in order to ensure that the reaction is complete and not excessive, the molar ratio of the compound as shown by Formula (316) to the compound as shown by Formula (320) may be 3:1 to 3.5:1, and in some embodiments, is 3.01:1 to 3.15:1.

In some embodiments, the organic solvent is one or more of acetonitrile, an epoxy solvent, an ether solvent, a haloalkane solvent, dimethyl sulfoxide, N,N-dimethylformamide, and N,N-diisopropylethylamine. In some embodiments, the epoxy solvent is dioxane and/or tetrahydrofuran. In some embodiments, the ether solvent is diethyl ether and/or methyl tertbutyl ether. In some embodiments, the haloalkane solvent is one or more of dichloromethane, trichloromethane and 1,2-dichloroethane. In some embodiments, the organic solvent is dichloromethane. The amount of the organic solvent is 3-50 L/mol, and in some embodiments, 5-20 L/mol, with respect to the compound as shown by Formula (320).

In some embodiments, the agent for amidation condensation is benzotriazol-1-yl-oxytripyrrolidinophosphonium hexafluorophosphate, 3-(Diethoxyphosphoryloxy)-1,2,3-benzotriazin-4(3H)-one (DEPBT), O-benzotriazol-1-yl-tetramethyluronium hexafluorophosphate, 4-(4,6-dimethoxytriazin-2-yl)-4-methylmorpholine hydrochloride or 1-hydroxybenzotriazole, and in further embodiments, is a mixture of the benzotriazol-1-yl-oxytripyrrolidinophosphonium hexafluorophosphate and the 1-hydroxybenzotriazole, wherein the benzotriazol-1-yl-oxytripyrrolidino phosphonium hexafluorophosphate and the 1-hydroxybenzotriazole are equimolar. The molar ratio of the total agents for amidation condensation to the compound as shown by Formula (316) may be 1:1 to 3:1, and in some embodiments, is 1.05:1 to 1.5:1.

The tertiary amine may be N-methylmorpholine, triethylamine or N,N-diisopropylethylamine, and in some embodiments, N-methylmorpholine. The molar ratio of the tertiary amine to the compound as shown by Formula (316) may be 2:1 to 10:1, and in some embodiments, is 2:1 to 5:1.

Similarly, the compound as shown by Formula (314) may be isolated from the reaction mixture by any suitable isolation methods. In some embodiments, the compound as shown by Formula (314) is isolated by removal of solvent via evaporation and followed by chromatography, for example, using the following two chromatographic conditions for isolation: (1) normal phase purification silica gel: 200-300 mesh silica gel filler, and gradient elution of dichloromethane: methanol = 100: 5 to 100: 7; and (2) reverse phase purification: C18, C8 reverse phase fillers, and gradient elution of methanol: acetonitrile = 0.1:1 to 1:0.1. In some embodiments, the solvent is directly removed to obtain a crude product of the compound as shown by Formula (314), and the crude product can be directly used in subsequent reactions.

The compound as shown by Formula (320) may be commercially available, or obtained by those skilled in the art via the known methods. For example, in the case that ml=m2=m3=3, nl=1, n3=2, and $R_{10}$, $R_{11}$, $R_{12}$, $R_{13}$, $R_{14}$, and $R_{15}$ are all H, the compound as shown by Formula (320) is commercially available from Alfa Aesar Inc.

The siRNA conjugate of the present disclosure may also be used in combination with other pharmaceutically acceptable excipients, which may be one or more of the various conventional formulations or compounds in the art. For details, please refer to the above description of the pharmaceutical compositions of the present disclosure.

<u>Use of the siRNA, the pharmaceutical composition and the siRNA conjugate of the present disclosure</u>

In some embodiments, the present disclosure provides the use of the siRNA and/or the pharmaceutical composition and/or the siRNA conjugate of the present disclosure in the manufacture of a medicament for treating and/or preventing a PKK related disease.

In some embodiments, the present disclosure provides a method for preventing and/or treating a PKK related disease, wherein the method comprises administering an effective amount of the siRNA and/or the pharmaceutical composition and/or the siRNA conjugate of the present disclosure to a subject in need.

It is possible to achieve the purpose of preventing and/or treating the PKK related disease through the mechanism of RNA interference by administering the active ingredients of the siRNA of the present disclosure to the subject in need. Thus, the siRNA and/or the pharmaceutical composition and/or the siRNA conjugate of the present disclosure may be used for preventing and/or treating PKK related diseases, or for the manufacture of a medicament for preventing and/or treating or alleviating the PKK related disease.

In some embodiments, the PKK related disease is an inflammatory disease or a thrombembolia disease. In some embodiments, the inflammatory disease may be a chronic inflammatory disease or an acute inflammatory disease. In some embodiments, the inflammatory disease includes but is not limited to hereditary angioedema (HAE), edema, angioedema, swelling, angioedema of eyelid, eye edema, macular edema or cerebral edema. In some embodiments, the thrombembolia disease includes but is not limited to thrombus, thrombembolia, deep vein thrombosis, pulmonary embolism, myocardial infarction, apoplexy or infarction.

The diseases may have one or more risk factors, causes or consequences in common.

Some risk factors and causes for the development of the inflammatory diseases comprise genetic predisposition and environmental factors to the inflammatory disease. In some embodiments, the subject has a mutated complement 1 esterase inhibitor (C1-INH) gene or a mutated factor 12 (FXII) gene. In some embodiments, the subject carries or has an angiotensin-converting enzyme inhibitor (ACE inhibitor) or an angiotensin II receptor blocker (ARB). In some embodiments, the subject already has an allergic reaction leading to angioedema. In some embodiments, the subject suffers from HAE type I. In some embodiments, the subject suffers from HAE type II. In some embodiments, the subject suffers from HAE type III. Some results related to the development of inflammatory diseases comprise edema/swelling of various body parts comprising extremities (i.e., hands, feet, arms, legs), intestines (abdominal cavity), face, genitalia, throat (i.e., larynx); blood vessel permeability; blood vessel leakage; systemic inflammation; abdominal pain; flatulence; vomiting; diarrhea; itchy skin; respiratory (asthmatic) reaction; rhinitis; allergic reaction; bronchoconstriction; hypotension; coma and death.

Some risk factors and causes for the development of the thrombembolia diseases comprise genetic predisposition to the thrombembolia, surgery (specifically orthopedic surgery), malignant tumor, pregnancy, old age, use of oral contraceptives, atrial fibrillation, previous thromboembolic symptoms, chronic inflammatory diseases and hereditary or acquired prevention of thrombotic coagulation diseases. Some results related to the development of the thromboembolic symptoms comprise decreased blood flow through affected blood vessels, tissue death and death.

As used herein, the term "administration/administer" refers to the placement of the siRNA, the pharmaceutical composition, and/or the siRNA conjugate of the present disclosure into a body of a subject by a method or a route that at least partly locates the siRNA, the pharmaceutical composition, and/or the siRNA conjugate of the present disclosure at a desired site to produce a desired effect. Suitable administration routes for the methods of the present disclosure comprise topical administration and systemic administration. In general, the topical administration results in the delivery of more siRNA conjugate to a particular site compared with the systemic circulation of the subject; whereas the systemic

administration results in the delivery of the siRNA, the pharmaceutical composition, and/or the siRNA conjugate of the present disclosure to the substantial systemic circulation of the subject. Considering that the present disclosure aims at providing a means for preventing and/or treating the inflammatory disease and/or thrombembolia disease, in some embodiments, an administration mode capable of delivering drugs to liver is used.

The administration to a subject may be achieved by any suitable routes known in the art, including but not limited to, oral or parenteral route, such as intravenous administration, intramuscular administration, subcutaneous administration, transdermal administration, airway administration (aerosol), pulmonary administration, nasal administration, rectal administration and topical administration (including buccal administration and sublingual administration). The administration frequency may be once or more times daily, weekly, biweekly, triweekly, monthly, bimonthly, quarterly, semiannually or annually.

The dose of the siRNA, the pharmaceutical composition, or the siRNA conjugate of the present disclosure may be a conventional dose in the art, and the dose may be determined according to various parameters, especially age, weight and gender of a subject. Toxicity and efficacy may be measured in cell cultures or experimental animals by standard pharmaceutical procedures, for example, by determining $LD_{50}$ (the lethal dose that causes 50% population death), and $ED_{50}$ (the dose that can cause 50% of the maximum response intensity in a quantitative response, and that causes 50% of the experimental subjects to have a positive response in a qualitative response). The dose range for human may be derived based on the data obtained from cell culture analysis and animal studies.

When administrating the siRNA, the pharmaceutical composition or the siRNA conjugate of the present disclosure, for example, to male or female C57BL/6J mice of 6-12 weeks old and 18-25 g body weight or ob/ob mice of 30-45 g, and calculating based on the amount of the siRNA: (i) for the siRNA conjugate, the dosage of the siRNA thereof may be 0.001-100 mg/kg body weight, and in further embodiments, is 0.01-50 mg/kg body weight, and in some embodiments, is 0.05-20 mg/kg body weight, in some other embodiments is 0.1-15 mg/kg body weight, and in some other embodiments, is 0.1-10 mg/kg body weight; and (ii) for a pharmaceutical composition formed by an siRNA and a pharmaceutically acceptable carrier, the dosage of the siRNA thereof may be 0.001-50 mg/kg body weight, in some embodiments, is 0.01-10 mg/kg body weight, in some embodiments, is 0.05-5 mg/kg body weight, and in some embodiments, is 0.1-3 mg/kg body weight.

In some embodiments, the present disclosure provides a method for inhibiting expression of a PKK gene. The method comprises contacting an effective amount of the siRNA and/or the pharmaceutical composition and/or the siRNA conjugate of the present disclosure with the cell with high PKK expression, introducing the siRNA and/or the pharmaceutical composition and/or the siRNA conjugate of the present disclosure into the cell, and achieving the purpose of inhibiting the expression of the PKK gene in the cell through a mechanism of RNA interference. The cell may be selected from SMMC-7721, HepG2, Huh7 and other hepatoma cell lines or isolated primary hepatocytes, and may also be selected from IMR-32 human neuroblastoma cells. In some embodiments, the cell is a cell transfected with a plasmid expressing PKK mRNA or a sequence fragment thereof. In some embodiments, the cell is a HEK293A cell transfected with a plasmid expressing PKK mRNA or a sequence fragment thereof.

In the case where the expression of the PKK gene in the cell is inhibited by using the method provided by the present disclosure, the amount of the siRNA in the modified siRNA, the pharmaceutical composition, and/or the siRNA conjugate provided is typically: in an amount sufficient to reduce the expression of the target mRNA and result in an extracellular concentration of 1 pM to 1 μM, or 0.01 nM to 100 nM, or 0.05 nM to 50 nM or 0.05 nM to about 5 nM on the surface of the target cell. The amount required to achieve this local concentration will vary with various factors, including the delivery method, the delivery site, the number of cell layers between the delivery site and the target cells or tissues, the delivery route (topical or systemic), etc. The concentration at the delivery site may be significantly higher than that on the surface of the target cells or tissues.

Kit

The present disclosure provides a kit, wherein the kit comprises an effective amount of at least one of the modified siRNA, the pharmaceutical composition, and the siRNA conjugate of the present disclosure.

In some embodiments, the kit disclosed herein may provide the modified siRNA in a container. In some embodiments, the kit of the present disclosure may comprise a container providing pharmaceutically acceptable excipients. In some embodiments, the kit may further comprise additional ingredients, such as stabilizers or preservatives. In some embodiments, the kit herein may comprise at least one additional therapeutic agent in a container other than the container providing the modified siRNA herein. In some embodiments, the kit may comprise an instruction for mixing the modified siRNA with the pharmaceutically acceptable carrier and/or excipients or other ingredients (if present).

In the kit of the present disclosure, the modified siRNA and the pharmaceutically acceptable carrier and/or the excipients as well as the modified siRNA, the pharmaceutical composition, and/or the siRNA conjugate and/or the pharmaceutically acceptable excipients may be provided in any form, e.g., in a liquid form, a dry form, or a lyophilized form. In some embodiments, the modified siRNA and the pharmaceutically acceptable carrier and/or the excipients as

well as the pharmaceutical composition and/or the siRNA conjugate and optional pharmaceutically acceptable excipients are substantially pure and/or sterile. In some embodiments, sterile water may be provided in the kit of the present disclosure.

Hereinafter, the present disclosure will be further described by examples, but is not limited thereto in any respect.

## Examples

Unless otherwise specified, the agents and culture media used in following examples are all commercially available, and the procedures used such as nucleic acid electrophoresis, real-time PCR and plasmid construction are all performed according to methods described in Molecular Cloning (Cold Spring Harbor Laboratory Press (1989)).

The Lipofectamine™2000(Invitrogen) is used as the transfection reagent when cells are transfected with the siRNA and the siRNA conjugate for PKK gene synthesized in the present disclosure or the siRNA and the siRNA conjugate as negative controls, the specific operation refers to the instructions provided by the manufacturer.

Unless otherwise specified, ratios of reagents provided below are all calculated by volume ratio (v/v).

Unless otherwise specified, the following experimental data of the in vivo/ in vitro are all expressed as $\overline{X}\pm$SEM, and the data analysis is carried out by using Graphpad prism 5.0 statistical analysis software.

Preparation Example 1

Preparation of siRNA conjugate L10-siPKKa1M1SP

In this preparation example, the siRNA conjugate L10-siPKKa1M1SP was synthesized. An siRNA conjugated in the siRNA conjugate has a sense strand and an antisense strand sequences corresponding to the siRNA conjugate L10-siPKKa1M1SP in Table 3.

(1-1) Synthesis of compound L-10

The compound L-10 was synthesized according to the following method:

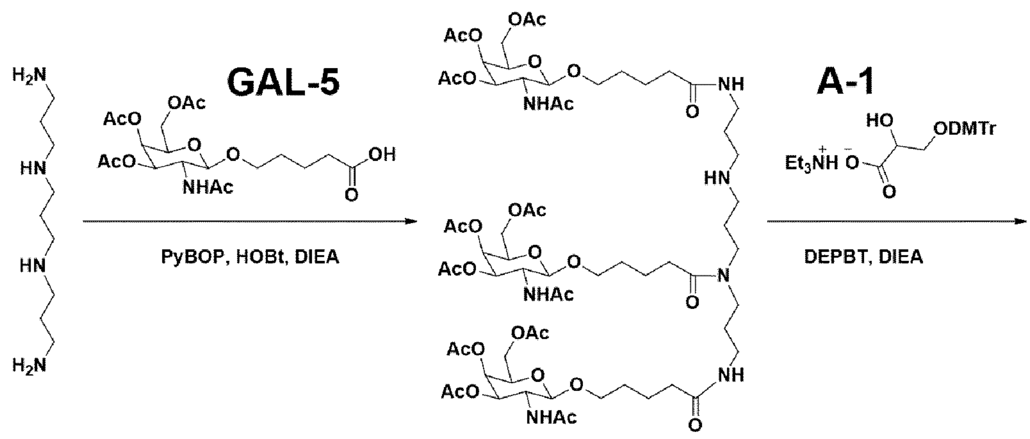


J-0 L-8

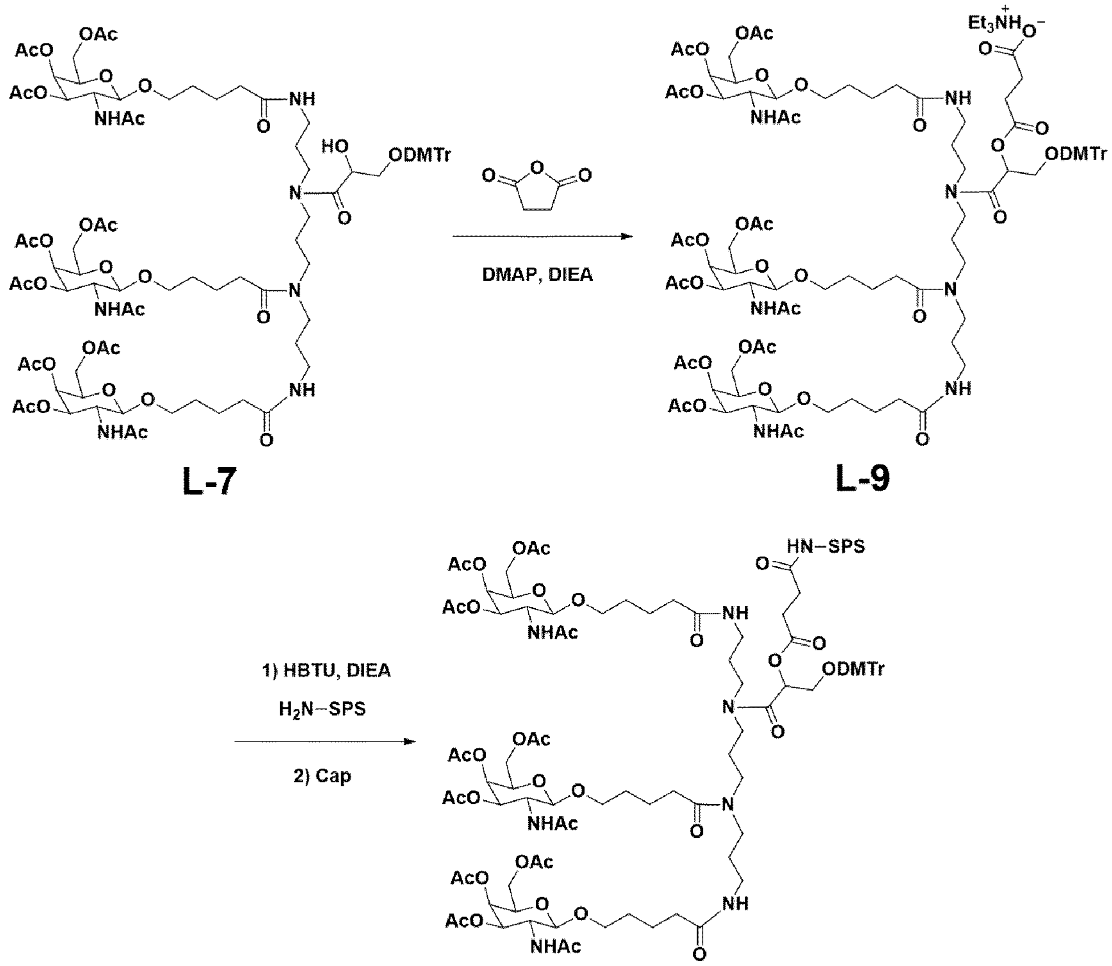


L-7 L-9

L-10

(1-1-1) Synthesis of GAL-5 (a terminal segment of the conjugate)

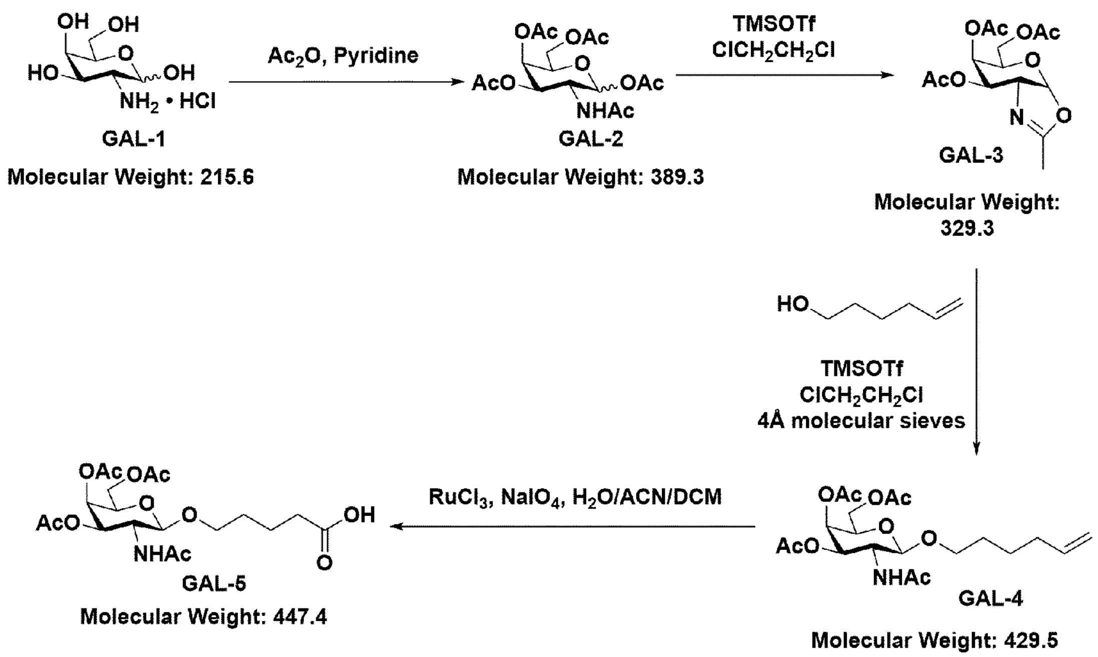


GAL-1
Molecular Weight: 215.6

GAL-2
Molecular Weight: 389.3

GAL-3
Molecular Weight: 329.3

GAL-4
Molecular Weight: 429.5

GAL-5
Molecular Weight: 447.4

(1-1-1a) Synthesis of GAL-2

100.0 g of **GAL-1** (N-acetyl-D-galactosamine hydrochloride, CAS No.: 1772-03-8, purchased from Ningbo Hongxiang Bio-Chem Co., Ltd., 463.8 mmol) was dissolved in 1000 ml of anhydrous pyridine, to which 540 ml of acetic anhydride (purchased from Enox Inc., 5565.6 mmol) was added in an ice water bath to react under stirring at room temperature for 1.5 hours. The resultant reaction solution was poured into 10 L of ice water and subjected to suction filtration under reduced pressure. The residue was washed with 2 L of ice water, and then added with a mixed solvent of acetonitrile/toluene (v/v ratio = 1:1) until completely dissolved. The solvent was removed by evaporation to give 130.0 g of product **GAL-2** as a white solid.

(1-1-1b) Synthesis of GAL-3

**GAL-2** (35.1 g, 90.0 mmol) obtained in step (1-1-la) was dissolved in 213 ml of anhydrous 1,2-dichloroethane, to which 24.0 g of TMSOTf (CAS No.: 27607-77-8, purchased from Macklin Inc., 108.0 mmol) was added under an ice water bath and nitrogen protection to react at room temperature overnight.

400 ml of dichloromethane was added to the reaction solution for dilution, filtered with diatomite, and then added with 1 L of saturated aqueous sodium bicarbonate solution and stirred evenly. An organic phase was isolated. An aqueous phase remained was extracted twice, each time with 300 ml of dichloroethane, and all organic phases were combined and washed with 300 ml of saturated aqueous sodium bicarbonate solution and 300 ml of saturated brine, respectively. The resultant organic phase was isolated and dried with anhydrous sodium sulfate. The solvent was removed by evaporation under reduced pressure to give 26.9 g of product **GAL-3** as a light yellow viscous syrup.

(1-1-1c) Synthesis of GAL-4

**GAL-3** (26.9 g, 81.7 mmol) obtained in step (1-1-1b) was dissolved in 136 ml of anhydrous 1,2-dichloroethane, added with 30 g of dry 4Å molecular sieve powder anf followed by 9.0 g of 5-hexen-1-ol (CAS No.: 821-41-0, purchased from Adamas-beta Inc., 89.9 mmol), and stirred at room temperature for 30 minutes. 9.08 ml of TMSOTf (40.9 mmol) was added in an ice bath and nitrogen protection to react under stirring at room temperature overnight. The 4Å molecular sieve powder was removed by filtration. The filtrate was added with 300 ml of dichloroethane for dilution, filtered with diatomite, and then added with 500 ml of saturated aqueous sodium bicarbonate solution and stirred for 10 minutes for washing. An organic phase was isolated. An aqueous phase was extracted once with 300 ml of dichloroethane. All organic phases were combined and washed with 300 ml of saturated aqueous sodium bicarbonate solution and 300 ml of saturated brine respectively. The resultant organic phase was isolated and dried with anhydrous sodium sulfate. The solvent was removed by evaporation under reduced pressure to give 41.3 g of product **GAL-4** as a yellow syrup, which was directly used in the next oxidation reaction without purification.

(1-1-1d) Synthesis of GAL-5

**GAL-4** (14.9 g, 34.7 mmol) obtained according to the method described in step (1-1 1c) was dissolved in a mixed solvent of 77 ml of dichloromethane and 77 ml of acetonitrile, added with 103 ml of deionized water and 29.7 g of sodium periodate (CAS No.: 7790-28-5, purchased from Aladdin Inc., 138.8 mmol) respectively, and stirred in an ice bath for 10 minutes. Ruthenium trichloride (CAS No.: 14898-67-0, purchased from Energy Chemical, 238 mg, 1.145 mmol) was added to react at room temperature overnight. The resultant reaction solution was diluted by adding 300 ml of water under stirring, and adjusted to a pH of about 7.5 by adding saturated sodium bicarbonate. An organic phase was isolated and discarded. An aqueous phase was extracted three times, each time with 200 ml of dichloromethane, and the organic phase resulted from the extraction was discarded. The aqueous phase resulted from the extraction was adjusted to a pH of about 3 with citric acid solids and extracted three times, each time with 200 ml of dichloromethane, and the resultant organic phases were combined and dried with anhydrous sodium sulfate. The solvent is removed by evaporation under reduced pressure to give 6.85 g of product GAL-5 as a white foamy solid.$^1$H NMR (400 MHz, DMSO) δ 12.01 (br, 1H), 7.83 (d, *J*= 9.2 Hz, 1H), 5.21 (d, *J*= 3.2 Hz, 1H), 4.96 (dd, *J*= 11.2, 3.2 Hz, 1H), 4.49 (d, *J*= 8.4 Hz, 1H), 4.07 - 3.95 (m, 3H), 3.92 - 3.85 (m, 1H), 3.74 - 3.67 (m, 1H), 3.48 - 3.39 (m, 1H), 2.20 (t, *J* = 6.8 Hz, 2H), 2.11 (s, 3H), 2.00 (s, 3H), 1.90 (s, 3H), 1.77 (s, 3H), 1.55 - 1.45 (m, 4H).

(1-1-2) Synthesis of L-8

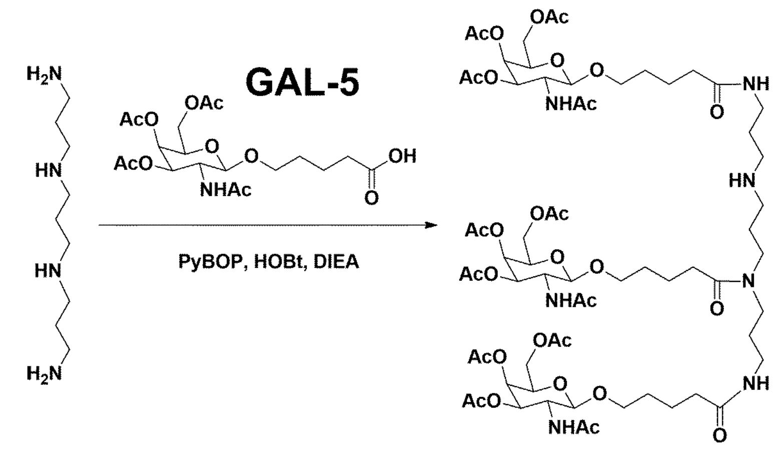


J-0 L-8

J-0 (9.886 g, 52.5 mmol, purchased from AlfaAesar) and GAL-5 (72.819 g, 162.75 mmol, obtained by combining the products of multiple batches) obtained in step (1-1-1) were dissolved in 525 ml of dichloromethane, added with diisopropylethylamine (DIEA, 44.782 g, 346.50 mmol), benzotriazol-1-yl-oxytripyrrolidino phosphonium hexafluorophosphate (PyBop, 90.158 g, 173.25 mmol) and hydroxybenzotriazole (HOBt, 23.410 g, 173.25 mmol) to react at room temperature for 4 hours, and then added with 20 ml of saturated sodium bicarbonate and 200 ml of saturated brine for washing. An aqueous phase was extracted twice, each time with 100 ml of dichloromethane, and the resultant organic phases were combined and dried with anhydrous sodium sulfate. After filtration, the solvent was removed by evaporation under reduced pressure to give a crude product. The crude product was purified by using a normal phase silica gel column (200-300 mesh). The column was added with 10wt% triethylamine for neutralizing the acidity of silica gel and equilibrated with 1wt‰ triethylamine, and eluted with a gradient elution of dichloromethane: methanol = 100:25 to 100:40. The eluate was collected, and the solvent was removed by evaporation under reduced pressure to give 38.8 g of pure product L-8. $^1$H NMR (400 MHz, DMSO) δ 7.84 (d, *J* = 9.0 Hz, 3H), 7.27 - 7.23 (m, 1H), 7.13 - 7.18 (m, 1H), 5.22 (d, *J* = 3.1 Hz, 3H), 4.97 (dd, *J* = 11.3, 3.1 Hz, 3H), 4.48 (d, *J* = 8.4 Hz, 3H), 4.09 - 3.98 (m, 9H), 3.88 (dd, *J* = 19.3, 9.3 Hz, 3H), 3.75 - 3.66 (m, 3H), 3.44 - 3.38 (m, 3H), 3.17 - 3.30 (m, 4H), 3.10 - 2.97 (m, 4H), 2.35 - 2.20 (m, 6H), 2.15 - 2.08 (m, 9H), 2.07 - 1.98 (m, 13H), 1.94 - 1.87 (m, 9H), 1.81 - 1.74 (m, 9H), 1.65 - 1.42 (m, 18H). MS m/z: $C_{85}H_{119}N_7O_{30}$, $[M+H]^+$, theoretical: 1477.59, measured: 1477.23.

(1-1-3a) Synthesis of A-1

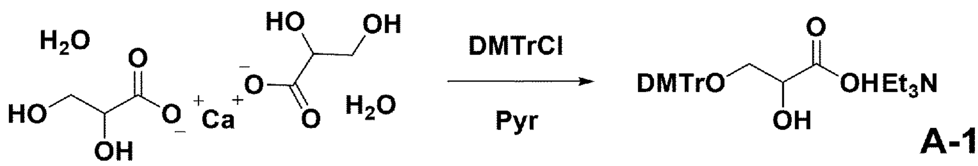


A-1

Molecular Weight: 286.25 Molecular Weight: 509.64

DMTrCl (4,4'-dimethoxytrityl chloride, 101.65 g, 300 mmol) was dissolved in 1000 ml of anhydrous pyridine, and added with calcium DL-glycerate hydrate (28.63 g, 100 mmol) to react at 45°C for 20 hours. The reaction solution was filtered. The residue was rinsed with 200 ml of DCM, and the filtrate was concentrated to dryness under reduced pressure. The residue was redissolved in 500 ml of dichloromethane and washed twice, each with 200 ml of 0.5 M triethylamine phosphate (pH = 7-8). An aqueous phase isolated was extracted twice, each time with 200 ml of dichloromethane. All organic phases were combined, dried with anhydrous sodium sulfate, and filtered. The solvent was removed by evaporation under reduced pressure, and the residue was purified by using a normal phase silica gel column (200-300 mesh) which was eluted with a gradient elution of petroleum ether: ethyl acetate: dichloromethane: methanol =1:1:1:0.35 to 1:1:1:0.55. The eluate was collected, and the solvent was removed by evaporation under reduced pressure. The residue was redissolved in 600 ml of dichloromethane, and washed once with 200 ml of 0.5 M triethylamine phosphate. The aqueous phase isolated was extracted once with 200 ml of dichloromethane. All organic phases were combined, dried with anhydrous sodium sulfate, and filtered. The solvent was removed by evaporation under reduced pressure

and overnight under reduced pressure in a vacuum oil pump to give 50.7 g of product A-1 as a white solid. $^1$H NMR (400 MHz, DMSO-d6) δ 7.46 (ddd, J = 6.5, 2.3, 1.1 Hz, 1H), 7.40 - 7.28 (m, 7H), 6.89 - 6.81 (m, 4H), 4.84 (d, J = 5.0 Hz, 1H), 4.36 - 4.24 (m, 1H), 4.29 (s, 6H), 3.92 (dd, J = 12.4, 7.0 Hz, 1H), 3.67 (dd, J = 12.3, 7.0 Hz, 1H), 2.52 (q, J = 6.3 Hz, 6H), 1.03 (t, J = 6.3 Hz, 9H). MS m/z: $C_{24}H_{23}O_6$, $[M-H]^-$, theoretical: 407.15, measured: 406.92.

(1-1-3b) Synthesis of L-7

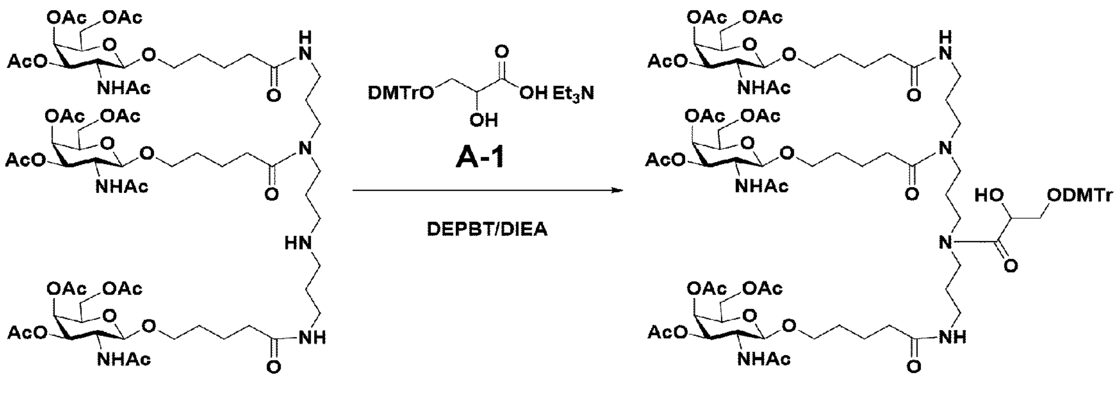


L-8 L-7

L-8 (40 g, 27.09 mmol, obtained by combining the products of multiple batches) obtained in step (1-1-2) and A-1 (41.418 g, 81.27 mmol) obtained in step (1-1-3a) were mixed and dissolved in 271 ml of dichloromethane, added with 3-(Diethoxyphosphoryloxy)-1,2,3-benzotriazin-4(3H)-one (DEPBT, 24.318 g, 81.37 mmol), and further added with diisopropylethylamine (21.007 g, 162.54 mmol) to react under stirring at 25°C for 1.5 hours. An organic phase was washed with 800 ml of saturated sodium bicarbonate. An aqueous phase was extracted three times, each time with 50 ml of dichloromethane, the organic phase was washed with 150 ml of saturated brine, and the aqueous phase was extracted once with 50 ml of dichloromethane. The resultant organic phases were combined and dried with anhydrous sodium sulfate. The solvent was removed by evaporation under reduced pressure and the residue was foam-dried in a vacuum oil pump overnight to give a crude product. The crude product was subjected to a column purification. The column was filled with 2 kg of normal phase silica gel (200-300 mesh), added with 200 ml of triethylamine for neutralizing the acidity of the silica gel, equilibrated with petroleum ether containing 1wt% triethylamine, and eluted with a gradient elution of petroleum ether: ethyl acetate: dichloromethane: N,N-dimethylformamide=1:1:1:0.5 to 1:1:1:0.6. The eluate was collected, and the solvent was removed by evaporation under reduced pressure to give 40.4 g of pure product L-7. $^1$H NMR (400 MHz, DMSO) δ7.90 - 7.78 (m, 4H), 7.75 - 7.64 (m, 1H), 7.38 - 7.18 (m, 9H), 6.91 - 6.83 (m, 4H), 5.25 - 5.10 (m, 4H), 4.97 (dd, *J* = 11.2, 3.2 Hz, 3H), 4.48 - 4.30 (m, 4H), 4.02 (s, 9H), 3.93 - 3.84 (m, 3H), 3.76 - 3.66 (m, 9H), 3.45 - 3.35 (m, 3H), 3.24 - 2.98 (m, 10H), 2.30 - 2.20 (m, 2H), 2.11 - 1.88 (m, 31H), 1.80 - 1.40 (m, 28H). MS m/z: $C_{90}H_{128}N_7O_{35}$, $[M-DMTr]^+$, theoretical: 1564.65, measured: 1564.88.

(1-1-4) Synthesis of L-9

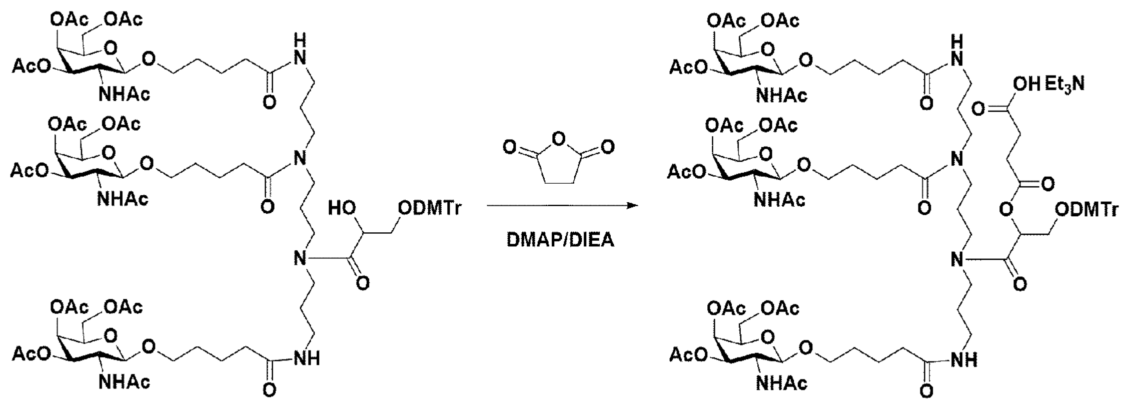


L-7 L-9

L-7 (40 g, 21.4247 mmol) obtained in step (1-1-3b), succinic anhydride (4.288 g, 42.8494 mmol) and 4-dimethylaminopyridine (DMAP, 5.235 g, 42.8494 mmol) were mixed and dissolved in 215 ml of dichloromethane, further added with diisopropylethylamine (DIPEA, 13.845 g, 107.1235 mmol), and stirred at 25°C for 24 hours. The reaction solution was washed with 800 ml of 0.5 M triethylamine phosphate. An aqueous phase was extracted three times, each time with 5 ml of dichloromethane. All organic phases were combined, and the solvent was evaporated under reduced pressure to give a crude product. The crude product was subjected to a column purification. The column was filled with 1 kg normal phase silica gel (200-300 mesh), added with 1 wt% triethylamine for neutralizing the acidity of the silica gel, equilibrated with dichloromethane and eluted with a gradient elution of 1wt‰ triethylamine-containing dichloromethane: methanol=100:18 to 100:20. The eluate was collected, and the solvent was evaporated under reduced pressure to give 31.0 g of pure product of L-9 conjugating molecule. $^1$H NMR (400 MHz, DMSO) δ 8.58 (d, *J* = 4.2 Hz, 1H), 7.94 - 7.82 (m, 3H), 7.41 - 7.29 (m, 5H), 7.22 (d, *J* = 8.1 Hz, 5H), 6.89 (d, *J* = 8.3 Hz, 4H), 5.49 - 5.37 (m, 1H), 5.21 (d, *J* = 3.0 Hz, 3H), 4.97 (d, *J* = 11.1 Hz, 3H), 4.49 (d, *J* = 8.2 Hz, 3H), 4.02 (s, 9H), 3.88 (dd, *J* = 19.4, 9.4 Hz, 3H), 3.77 - 3.65 (m, 9H), 3.50 - 3.39 (m, 6H), 3.11 - 2.90 (m, 5H), 2.61 - 2.54 (m, 4H), 2.47 - 2.41 (m, 2H), 2.26 - 2.17 (m, 2H), 2.15 - 1.95 (m, 22H), 1.92 - 1.84 (m, 9H), 1.80 - 1.70 (m, 10H), 1.65 - 1.35 (m, 17H), 1.31 - 1.19 (m, 4H), 0.96 (t, *J* = 7.1 Hz, 9H). MS m/z: $C_{94}H_{132}N_7O_{38}$, $[M-DMTr]^+$, theoretical: 1664.72, measured: 1665.03.

(1-1-5) Synthesis of Compound L-10

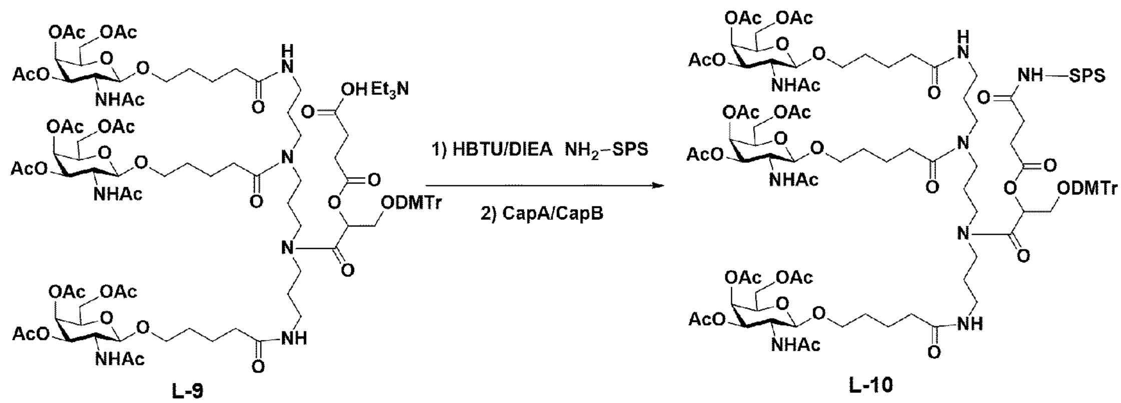


L-9 L-10

In this step, the compound L-10 was prepared by linking the L-9 conjugating molecule to a solid phase support.

The L-9 conjugating molecule (22.751 g, 0.1126 mmol) obtained in step (1-1-4), O-benzotriazol-1-yl-tetramethyluronium hexafluorophosphate (HBTU, 6.257 g, 16.5 mmol) and diisopropylethylamine (DIEA, 2.843 g, 22 mmol) were mixed and dissolved in 900 ml of acetonitrile, and stirred at room temperature for 5 minutes. Aminomethyl resin (88 g, 100-200 mesh, amino loading: 400 μmol/g, purchased from Tianjin Nankai HECHENG S&T Co., Ltd.) was added into the reaction liquid. A reaction was performed on a shaker at 25°C and 150 rpm/min for 18 hours, followed by filtration. The residue was rinsed twice, each time with 300 ml of DCM, and rinsed three times, each time with 300 ml of acetonitrile, and dried for 18 hours with a vacuum oil pump. Then a capping reaction was performed by adding starting materials

(CapA, CapB, 4-dimethylaminopyridine (DMAP) and acetonitrile) according to the charge ratio shown in Table 2. A reaction was performed on a shaker at 25°C and 150 rpm/min for 5 hours. The reaction liquid was filtrated. The residue was rinsed three times, each time with 300 ml of acetonitrile, the solvent was evaporated to dryness, and the mixture was dried overnight under a reduced pressure with a vacuum oil pump to give 102 g of compound L-10 (i.e., the L-9 conjugating molecule linked to the solid phase support), with a loading of 90.8 μmol/g.

Table 2 The charge ratio of capping reaction

| Starting materials | Amount | Grade | Lot No. | Manufacturer |
|---|---|---|---|---|
| CapA | 1980 ml | - | - | - |
| CapB | 220 ml | - | - | - |
| DMAP | 1.100 g | Analytical pure | 11422139 | Aladdin |
| Acetonitrile | 220 ml | Spectroscopic pure | 015161001 | CINC (Shanghai) Co., Ltd |

In the above table, CapA and CapB are solutions of capping agents. CapA is a mixed solution of 20% by volume of N-methylimidazole in pyridine/acetonitrile, wherein the volume ratio of the pyridine to the acetonitrile is 3:5. CapB is a solution of 20% by volume of acetic anhydride in acetonitrile.

(1-2) Synthesis of the sense strand of siRNA conjugate L10-siPKKa1M1SP

Nucleoside monomers were linked one by one in the direction from 3' to 5' according to the arrangement sequence of nucleotides in the sense strand by the solid phase phosphoramidite method, starting the cycles from the Compound L-10 prepared in the above step. The linking of each nucleoside monomer comprised a four-step reaction of deprotection, coupling, capping, and oxidation or sulfurization. Wherein, when two nucleotides are linked via a phosphoester, a four-step reaction of deprotection, coupling, capping, and oxidation was comprised during linking of the later nucleoside monomer. When two nucleotides are linked via a phosphorothioate, a four-step reaction of protection, coupling, capping, and sulfurization was comprised during linking of the later nucleoside monomer. The synthesis condition was given as follows.

The nucleoside monomers were provided in a 0.1 M acetonitrile solution. The condition for deprotection reaction in each step was identical, i.e., a temperature of 25°C, a reaction time of 70 seconds, a solution of dichloroacetic acid in dichloromethane (3% v/v) as a deprotection agent, and a molar ratio of the dichloroacetic acid to the protecting group 4,4'-dimethoxytrityl on the solid phase support of 5:1.

The condition for coupling reaction in each step was identical, comprising a temperature of 25°C, a molar ratio of the nucleic acid sequence linked to the solid phase support to the nucleoside monomers of 1:10, a molar ratio of the nucleic acid sequence linked to the solid phase support to a coupling agent of 1:65, a reaction time of 600 seconds, and 0.5 M solution of 5-ethylthio-1H-tetrazole (ETT) in acetonitrile as a coupling agent.

The condition for capping reaction in each step was identical, comprising a temperature of 25°C and a reaction time of 15 seconds. A capping agent was a mixed solution of Cap A and Cap B in a molar ratio of 1: 1, and a molar ratio of the capping agent to the nucleic acid sequence linked to the solid phase support (acetic anhydride: N-methylimidazole: the nucleic acid sequence linked to the solid phase support) was 1:1:1.

The condition for oxidation reaction in each step was identical, comprising a temperature of 25°C, a reaction time of 15 seconds, and 0.05 M iodine water as an oxidation agent. A molar ratio of iodine to the nucleic acid sequence linked to the solid phase support in the coupling step was 30:1. The reaction was carried out in a mixed solvent in which the ratio of tetrahydrofuran: water: pyridine was 3:1:1.

The condition for sulfurization reaction in each step was identical, comprising a temperature of 25°C, a reaction time of 300 seconds, and xanthane hydride as a sulfurization agent. A molar ratio of the sulfurization agent to the nucleic acid sequence linked to the solid phase support in the coupling step was 120:1. The reaction was carried out in a mixed solvent in which the ratio of acetonitrile: pyridine was 1:1.

After the last nucleoside monomer was linked, the nucleic acid sequence linked to the solid phase support was cleaved, deprotected, purified and desalted in turn, and then freeze-dried to obtain the sense strand, wherein,
the conditions for cleavage and deprotection were as follows: adding the synthesized nucleotide sequence linked to the support into 25 wt% aqueous ammonia to react for 16 hours at 55°C, wherein the aqueous ammonia was in an amount of 0.5 ml/μmol; filtering to remove the remaining support, and concentrating the supernatant in vacuum to dryness.

The conditions for purification and desalination were as follows: purifying the nucleic acid by using a preparative ion chromatography column (Source 15Q) with a gradient elution of NaCl. Specifically, eluent A: 20 mM sodium phosphate (pH 8.1), solvent: water/acetonitrile = 9:1 (v/v); eluent B: 1.5 M sodium chloride, 20 mM sodium phosphate (pH 8.1),

solvent: water/acetonitrile = 9:1 (v/v); elution gradient: eluent A: eluent B = 100:0 to 50:50. The eluate was collected, combined and desalted by using a reverse phase chromatography purification column. The specific conditions comprised using a Sephadex column (filler: Sephadex-G25) for desalination and deionized water for elution.

The detection method was as follows: determining the purity of the sense strand above by ion exchange chromatography (IEX-HPLC); and analyzing the molecular weight by Liquid Chromatography-Mass Spectrometry (LC-MS). The measured value was in conformity with the theoretical value, indicating that a sense strand SS conjugated with L-9 conjugating molecule at 3' terminal was synthesized.

(1-3) Synthesis of antisense strand of siRNA conjugate L10-siPKKa1M1SP

The antisense strand of the siRNA conjugate L10-siPKKa1M1SP was synthesized by starting the cycles using a universal solid phase support (UnyLinker™ loaded NittoPhase®HL Solid Supports, Kinovate Life Sciences Inc.) according to the solid phase phosphoramidite method. The deprotection, coupling, capping, oxidation or sulfurization reaction conditions in the solid phase synthesis method were conducted under the same conditions as those in the synthesis of the sense strand. A CPR-I monomer (Suzhou GenePharma, article number Cat#13-2601-XX) was linked to the 5' terminal of the antisense strand to form 5'-phosphate nucleotide by four steps of deprotection, coupling, capping and oxidation after the last nucleoside monomer of the antisense strand was linked.

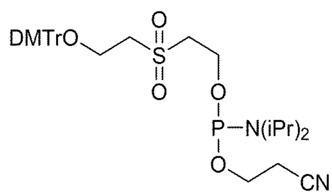

(CPR-I)

In this linkage, the deprotection, coupling, capping and oxidation reaction conditions used were the same as those in the synthesis of the sense strand. After the linkage was completed, the steps of cleaving and deprotection, purification and desalination were performed, wherein the conditions for the cleaving and deprotection, purification and desalination were the same as those in the synthesis of the sense strand. The residue was freeze-dried to obtain the antisense strand finally.

The purity of the antisense strand was detected by ion exchange chromatography (IEX-HPLC), and the molecular weight was analyzed by liquid chromatography-mass spectrometry (LC-MS). The measured value was in conformity with the theoretical value, indicating that an antisense strand AS having a target sequence was synthesized.

(1-4) Synthesis of siRNA conjugate L10-siPKKa1M1SP

For the siRNA conjugate L10-siPKKa1M1SP, the sense strand and the antisense strand obtained in steps (1-2) and (1-3) were respectively dissolved in water for injection to give a solution of 40 mg/mL, mixed at an equimolar ratio, heated at 50°C for 15 minutes, and then cooled at room temperature, such that a double-stranded structure was formed by the sense strand and the antisense strand through hydrogen bonding. The siRNA conjugate was diluted to a concentration of 0.2 mg/mL with ultra-pure water (prepared by Milli-Q ultra-pure water instrument, with resistivity of 18.2MΩ*cm(25°C)). The molecular weight was measured by Liquid Chromatography-Mass Spectrometry (LC-MS, purchased from Waters Corp., model: LCT Premier). Since the measured value was in conformity with the theoretical value, it was confirmed that the synthesized siRNA conjugate was the designed double stranded nucleic acid sequence of interest with the L-9 conjugating molecule. The structure thereof was as shown by Formula (403). The siRNA conjugate has the sense strand and the antisense strand sequences corresponding to the siRNA conjugate L10-siPKKa1M1SP in Table 3.

Table 3 siRNA conjugates

| Preparation Example No. | siRNA conjugate No. | Sequence direction 5'-3' | | SEQ ID NO |
|---|---|---|---|---|
| Preparation Example 1 | L10-siPKKa 1M1SP | Sense strand | GmsGmsUmGmUmUmUfGfCfUmAmUmUmCmAmGmUmUmUm | 361 |
| | | Antisense strand | P-AmsAfsAmCmUmGfAmAmUmAmGmCmAmAfAmCfAmCmCmsUmsUm | 362 |
| Preparation Example 2 | L10-siPKKb 1M1SP | Sense strand | GmsCmsUmUmCmUmUfGfAfAmAmGmAmUmAmGmUmGmUm | 363 |
| | | Antisense strand | P-AmsCfsAmCmUmAfUmCmUmUmUmCmAmAfGmAfAmGmCmsAmsAm | 364 |
| Preparation Example 3 | L10-siPKKc 1M1SP | Sense strand | GmsAmsAmGmCmAmAfUfGfUmGmGmUmCmAmUmCmAmAm | 365 |
| | | Antisense strand | P-UmsUfsGmAmUmGfAmCmCmAmCmAmUmUfGmCfUmUmCmsAmsAm | 366 |
| Preparation Example 4 | L10-siPKKd 1M1SP | Sense strand | CmsAmsCmAmAmAmGfAfAfAmUmGmUmUmUmGmUmCmUm | 367 |
| | | Antisense strand | P-AmsGfsAmCmAmAfAmCmAmUmUmUmCmUfUmUfGmUmGmsAmsCm | 368 |
| Preparation Example 5 | L10-siPKKe 1M1S | Sense strand | GmsAmsAmUmUmCmCfAfAfAmAmAmCmCmAmAmUmAmUm | 369 |
| | | Antisense strand | AmsUfsAmUmUmGfGmUmUmUmUmUmGmGfAmAfUmUmCmsAmsGm | 370 |
| Preparation Example 6 | L10-siPKKf 1M1S | Sense strand | GmsGmsUmCmUmGmUfGfCfUmGmGmCmUmAmUmAmAmAm | 371 |
| | | Antisense strand | UmsUfsUmAmUmAfGmCmCmAmGmCmAmCfAmGfAmCmCmsAmsUm | 372 |

Wherein, capital letters C, G, U, and A indicated the base composition of the nucleotides; the lowercase m indicated that the nucleotide adjacent to the left side of the letter m was a methoxy modified nucleotide; the lowercase f indicated that the nucleotide adjacent to the left side of the letter f was a fluoro modified nucleotide; the lowercase letter s indicated that the two nucleotides adjacent to the left and right of the letter s were linked by phosphorothioate; and and the capital letter P indicated that the nucleotide adjacent to the right side of the letter P was a 5'-phosphate nucleotide modified nucleotide.

Preparation Examples 2-6

Synthesis of the siRNA conjugates of the present disclosure

According to the same method as Preparation Example 1, the following siRNA conjugates of the present disclosure were further synthesized respectively: L10-siPKKb1M1SP, L10-siPKKc1M1SP, L10-siPKKd1M1SP, L10-siPKKe1M1S and L10-siPKKf1M1S. The siRNAs contained in these siRNA conjugates had the sense strand and antisense strand sequences corresponding to the siRNA conjugates numbered L10-siPKKb1M1SP, L10-siPKKc1M1SP, L10-siPKKd1M1SP, L10-siPKKe1M1S and L10-siPKKf1M1S listed in Table 3. The only difference between the preparation methods was that (i) sense strands and antisense strands were respectively synthesized according to the sense strand and antisense strand sequences of the siRNAs corresponding to each siRNA conjugate in Table 3; and (ii) for the siRNA conjugates L10-siPKKe1M1S and L10-siPKKf1M1S, the 5'- terminals of the antisense strands thereof did not contain 5'-phosphoric acid, and accordingly, it was not necessary to perform the aforementioned step of linking the CPR-I monomer.

After preparation, the molecular weights of the prepared siRNA conjugates were respectively detected according to the method of the Preparation Example 1, and the measured values were consistent with the theoretical values, indicating that the synthesized siRNA conjugate was a target designed double-stranded nucleic acid sequence with the L-9 conjugating molecule. The structures thereof were all as shown by Formula (403). The siRNAs contained in these siRNA conjugates were respectively the sequences corresponding to the siRNA conjugates L10-siPKKb1M1SP, L10-siPKKc1M1SP, L10-siPKKd1M1SP, L10-siPKKe1M1S and L10-siPKKf1M1S in Table 3.

Preparation Examples 7-12

Synthesis of the siRNA provided by the present disclosure

The sense strands or the antisense strands of the siRNA sequences listed in Table 4 were respectively synthesized by a solid phase synthesis method, and DEPC water was used to dissolve the mutually complementary sense strands and antisense strands in Table 4 in equimolar, and then followed by annealing to obtain the following siRNAs provided by the present disclosure comprising siPKKa1M1S, siPKKb1M1S, siPKKc1M1S, siPKKdIMIS, siPKKe1M1S and siPKKf1M1S. The sequences of the above siRNAs were shown in Table 4.

Comparative Preparation Example 1

Synthesis of reference siRNA

The sense strand and the antisense strand corresponding to the siRNA numbered NC in Table 4 were respectively synthesized by solid phase synthesis. The obtained equimolar sense strand and antisense strand were dissolved respectively with DEPC water, and then annealed to obtain the reference siRNA numbered NC.

Table 4 siRNA sequences

| Preparati on Example No. | No. | Sequence direction 5'-3' | | SEQ ID NO |
|---|---|---|---|---|
| Preparati on Example 7 | siPKKa1 MIS | Sense strand | GmsGmsUmGmUmUmUfGfCfUmAmUm UmCmAmGmUmUmUm | 373 |
| | | Antise nse strand | AmsAfsAmCmUmGfAmAmUmAmGmCm AmAfAmCfAmCmCmsUmsUm | 374 |
| Preparati on Example 8 | siPKKb 1 MIS | Sense strand | GmsCmsUmUmCmUmUfGfAfAmAmGm AmUmAmGmUmGmUm | 375 |
| | | Antise nse strand | AmsCfsAmCmUmAfUmCmUmUmUmCm AmAfGmAfAmGmCmsAmsAm | 376 |

(continued)

| Preparati on Example No. | No. | | Sequence direction 5'-3' | SEQ ID NO |
|---|---|---|---|---|
| Preparati on Example 9 | siPKKc1 MIS | Sense strand | GmsAmsAmGmCmAmAfUfGfUmGmGm UmCmAmUmCmAmAm | 377 |
| | | Antisense strand | UmsUfsGmAmUmGfAmCmCmAmCmAm UmUfGmCfUmUmCmsAmsAm | 378 |
| Preparati on Example 10 | siPKKd1 MIS | Sense strand | CmsAmsCmAmAmAmGfAfAfAmUmGm UmUmUmGmUmCmUm | 379 |
| | | Antisense strand | AmsGfsAmCmAmAfAmCmAmUmUmUm CmUfUmUfGmUmGmsAmsCm | 380 |
| Preparati on Example 11 | siPKKe1 MIS | Sense strand | GmsAmsAmUmUmCmCfAfAfAmAmAm CmCmAmAmUmAmUm | 381 |
| | | Antisense strand | AmsUfsAmUmUmGfGmUmUmUmUmUm GmGfAmAfUmUmCmsAmsGm | 382 |
| Preparati on Example 12 | siPKKf1 MIS | Sense strand | GmsGmsUmCmUmGmUfGfCfUmGmGm CmUmAmUmAmAmAm | 383 |
| | | Antisense strand | UmsUfsUmAmUmAfGmCmCmAmGmCm AmCfAmGfAmCmCmsAmsUm | 384 |
| NC | siNC | Sense strand | UUCUCCGAACGUGUCACGUdTdT | 385 |
| | | Antisense strand | ACGUGACACGUUCGGAGAAdTdT | 386 |

Wherein, capital letters C, G, U, and A indicated the base composition of the nucleotides; the lowercase m indicated that the nucleotide adjacent to the left side of the letter m was a methoxy modified nucleotide; the lowercase f indicated that the nucleotide adjacent to the left side of the letter f was a fluoro modified nucleotide; the lowercase letter s indicated that the two nucleotides adjacent to the left and right of the letter s are linked by phosphorothioate; and the capital letter P indicated that the nucleotide adjacent to the right side of the letter P was a 5'-phosphate nucleotide.

In the preparation process of the sequences above, when the target sequence contained an unmodified nucleotide, under the conditions of cleavage and deprotection, after aqueous ammonia treatment, 0.4 ml/$\mu$mol N-methyl pyrrolidone was used to dissolve the product, and then 0.3 ml/$\mu$mol triethylamine and 0.6 ml/$\mu$mol triethylamine trihydrofluoride were added to remove 2'-TBDMS protection on ribose, with respect to the amount of the single-stranded nucleic acid. The molecular weights of the above siRNAs were respectively detected according to the method of Preparation Example 1, and the measured values were consistent with the theoretical values, confirming that the obtained siRNAs had sequences corresponding to each siRNA shown in Table 4.

Preparation Examples 13-16

Synthesis of Cy5-labeled siRNA conjugates (Cy5-L10-siPKKa1M1SP and Cy5-L10-siPKKf1M1S) and Cy5-labeled siRNAs (Cy5-siPKKa1M1S and Cy5-siPKKf1M1S)

(1) Synthesis of siRNA conjugates Cy5-L10-siPKKa1M1SP and Cy5-L10-siPKKf1M1S

Cy5-L10-siPKKa1M1SP was prepared by the same method as preparing L10-siPKKa1M1SP, except that (i) after linking the last nucleoside monomer of the sense strand, the Cy5 fluorescent group was additionally linked to the sense strand; and (ii) the conditions for cleavage and deprotection of the sense strands were different.

Specifically, in (i), in the process of preparing the sense strand according to the solid phase phosphoramidite method described in Preparation Examples (1-2), after linking the last nucleoside monomer of the sense chain, the Cy5 phosphoramidite monomer (purchased from Shanghai HonGene Biotech, article number OP-057) was linked to the 5' terminal of the sense chain through four steps of deprotection, coupling, capping and oxidation. Wherein, the deprotection, coupling, capping, oxidation or sulfurization reaction conditions used were the same as those in the synthesis of the sense strand in step (1-2), except that: 1) the deprotection reaction time was extended to 300 seconds; and 2) the Cy5 coupling reaction time was extended to 900 seconds.

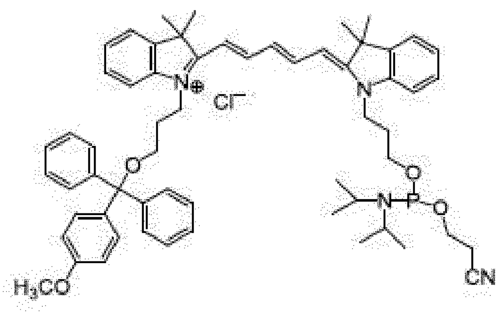

Cy5 phosphoramidite monomer

In (ii), the conditions for cleavage and deprotection were as follows: adding the synthesized nucleotide sequence linked to a support into an AMA solution (mixed solution of 40wt% methylamine aqueous solution and 25wt% ammonia water at a volume ratio of 1:1), wherein the amount of the AMA solution was 0.5 ml/μmol, reacting for 2 hours in a water bath at 25°C, removing the remaining support by filtration, and concentrating the supernatant to dryness in vacuum.

The conditions for purification and desalination of the sense strand were the same as those of the synthesis of the sense strand in step (1-2) in the Preparation Example. Then the residue was freeze-dried to obtain the sense strand of the Cy5-L10-siPKKa1M1SP.

Thereby, the siRNA conjugate Cy5-L10-siPKKa1M1SP was obtained, and a Cy5 fluorescent group was covalently linked to the 5' terminal of the sense strand of the siRNA of the siRNA conjugate, which had the sense strand and antisense strand sequences shown in Table 5 corresponding to the siRNA conjugate Cy5-L10-siPKKa1M1SP.

Cy5-L10-siPKKf1M1S was prepared by the same method as preparing Cy5-L10-siPKKa1M1SP, except that the nucleic acid sequences of the sense strand and the antisense strand of the siRNA based on the synthesis were the sense strand and antisense strand sequences of the siRNA corresponding to Cy5-L10-siPKKf1M1S shown in Table 5 .Therefore, the siRNA conjugate Cy5-L10-siPKKf1M1S was obtained, and the 5' terminal of the sense strand of the siRNA of the siRNA conjugate was covalently linked to a Cy5 fluorescent group.

(4-2) Synthesis of Cy5-siPKKa1M1S and Cy5-siPKKf1M1S

Cy5-siPKKa1M1S was prepared by the same method as preparing Cy5-L10-siPKKa1M1SP, except that the sense strand of Cy5-siPKKa1M1S was synthesized by starting the cycles using a universal solid phase support (UnyLinker™ loaded NittoPhase®HL Solid Supports, Kinovate Life Sciences Inc.). The deprotection, coupling, capping, oxidation or sulfurization reaction conditions, cleavage and deprotection, purification and desalting conditions in the solid phase synthesis method were conducted under the same conditions as those in the synthesis of the sense strand of Cy5-L10-siPKKa1M1S.

Cy5-siPKKf1M1S was prepared by the same method as preparing Cy5-siPKKa1M1S, except that the siRNA sequence based on the synthesis was the siRNA sequence corresponding to Cy5-siPKKf1M1S shown in Table 5.

The siRNA sequences in Cy5-L10-siPKKa1M1SP, Cy5-L10-siPKKf1M1S, Cy5-siPKKa1M1S and Cy5-siPKKf1M1S were shown in Table 5.

Table 5 siRNA sequences in Cy5-labeled siRNA conjugates and Cy5-labeled siRNAs

| Preparation Example No. | siRNA or siRNA conjugate No. | Sequence direction 5'-3' | | SEQ ID NO |
|---|---|---|---|---|
| Preparation Example 13 | Cy5-siP $KK_a$1M1S | Sense strand | GmsGmsUmGmUmUmUfGfCfUmAmUmUmCmAmGmUmUmUm | 387 |
| | | Antisense strand | AmsAfsAmCmUmGfAmAmUmAmGmCmAmAfAmCfAmCmCmsUmsUm | 388 |
| Preparation Example 14 | Cy5-siP KKf1M1S | Sense strand | GmsGmsUmCmUmGmUfGfCfUmGmGmCmUmAmUmAmAmAm | 389 |
| | | Antisense strand | UmsUfsUmAmUmAfGmCmCmAmGmCmAmCfAmGfAmCmCmsAmsUm | 390 |
| Preparation Example 15 | Cy5-L10 -siPKKa 1M1SP | Sense strand | GmsGmsUmGmUmUmUfGfCfUmAmUmUmCmAmGmUmUmUm | 391 |
| | | Antisense strand | P-AmsAfsAmCmUmGfAmAmUmAmGmCmAmAfAmCfAmCmCmsUmsUm | 392 |
| Preparation Example 16 | Cy5-L10-siPKKf 1M1S | Sense strand | GmsGmsUmCmUmGmUfGfCfUmGmGmCmUmAmUmAmAmAm | 393 |
| | | Antisense strand | UmsUfsUmAmUmAfGmCmCmAmGmCmAmCfAmGfAmCmCmsAmsUm | 394 |

Wherein, capital letters C, G, U, and A indicated the base composition of the nucleotides; the lowercase m indicated that the nucleotide adjacent to the left side of the letter m was a methoxy modified nucleotide; the lowercase f indicated that the nucleotide adjacent to the left side of the letter f was a fluoro modified nucleotide; and the lowercase letter s indicated that the two nucleotides adjacent to the left and right of the letter s are linked by phosphorothioate group.

After preparation, the molecular weights of the prepared siRNA conjugates or siRNAs were respectively detected according to the method of the Preparation Example 1, and the measured values were consistent with the theoretical values, indicating that the synthesized siRNA conjugate was a target designed double-stranded nucleic acid sequence with the L-9 conjugating molecule, and the synthesized siRNA had the double-stranded nucleic acid sequence corresponding to Table 5.

After the siRNA or the siRNA conjugate of the present disclosure was prepared, the siRNA or siRNA conjugate was freeze-dried into solid powder for later use. When in use, for example, water for injection, normal saline (NS), phosphate buffer (PB) or phosphate buffer solution (PBS) could be used to redissolve the siRNA or siRNA conjugate into a solution with the required concentration for use.

Experimental Example 1

Inhibitory activity of siRNA in an in vitro psiCHECK system

HEK293A cells (purchased from Nanjing COBIOER Biotechnology Co., Ltd.) were cultured in DMEM complete media (HyClone company) containing 10% fetal bovine serum (FBS, Hyclone company) and 0.2v% Penicillin-Streptomycin (Gibco, Invitrogen company) at 37°C in an incubator containing 5% $CO_2$/95% air.

According to the methods disclosed in Kumico Ui-Tei et.al., Functional dissection of siRNA sequence DNA substitution: modified siRNA with a DNA seed arm is a powerful tool for mammalian gene silencing with significantly

reduced off-target effect. Nucleic Acids Research, 2008.36(7), 2136-2151, on-target detection plasmids were constructed, and co-transfected into the HEK293A cells together with the to-be-evaluated siRNAs, and the inhibitory activity of the siRNA was reflected by the expression level of double luciferase reporter gene.

The specific steps were as follows:

[1] Construction of detection plasmids

Detection plasmids were constructed using psiCHECK™-2(Promega™) plasmid. The plasmid comprised a target sequence, i.e., the target sequence of the siRNA. For the to-be-evaluated siRNAs, the target sequences were respectively as shown below:

The target sequence of the siPKKa1M1S was:
GGTGTTTGCTATTCAGTTT (SEQ ID NO: 395)

The target sequence of the siPKKb1M1S was:
GCTTCTTGAAAGATAGTGT (SEQ ID NO: 396)

The target sequence of the siPKKc1M1S was:
GAAGCAATGTGGTCATCAA (SEQ ID NO: 397)

The target sequence of the siPKKdIMIS was:
CACAAAGAAATGTTTGTCT (SEQ ID NO: 398)

The target sequence of the siPKKe1M1S was:
GAAUUCCAAAAACCAAUAU (SEQ ID NO: 399)

The target sequence of the siPKKf1M1S was:
GGUCUGUGCUGGCUAUAAA (SEQ ID NO: 400)

The target sequence was cloned into the Xho I/Not I site of the psiCHECKTM-2 plasmid.

[2] Transfection

HEK293A cells were seeded in a 96-well plate with 8 $\times$ $10^3$ cells/well. After 16 hours, when the growth density of the cells reached 70-80%, the H-DMEM complete media in the culture wells were sucked up, and 80 μl of Opti-MEM medium (GIBCO company) was added to each well to continue the culture for 1.5 hours.

For each siRNA, the above detection plasmid was diluted into 200 ng/μl detection plasmid working solution with DEPC water. For each siRNA, siRNA and DEPC water were used to prepare siRNA working solutions with concentrations (calculated by siRNA) of 10 nM, 1 nM and 0.1 nM respectively.

1A1 solution was prepared, and each part of the 1A1 solution contained 1 μl of siRNA working solution with a concentration of 10 nM, 0.05 μl of detection plasmid working solution (containing 10 ng of detection plasmids) and 10 μl of Opti-MEM media.

1A2 solution was prepared, and each part of the 1A2 solution contained 1 μl of siRNA working solution with a concentration of 1 nM, 0.05 μl of detection plasmid working solution (containing 10 ng of detection plasmids) and 10 μl of Opti-MEM media.

1A3 solution was prepared, and each part of the 1A3 solution contained 1 μl of siRNA working solution with a concentration of 0.1 nM, 0.05 μl of detection plasmid working solution (containing 10 ng of detection plasmids) and 10 μl of Opti-MEM media.

1B solution was prepared, and each part of the 1B solution contained 0.2 μl of Lipofectamine™ 2000 and 10 μl of Opti-MEM media.

1C solution was prepared, and each part of the 1C solution contained 0.05 μl of detection plasmid working solution (containing 10 ng of detection plasmids) and 10 μl of Opti-MEM media

For each siRNA, one part of the 1B solution was mixed with one part of the 1A1 solution, one part of the 1A2 solution and one part of the 1A3 solution, and incubated for 20 minutes at room temperature to obtain transfection complexes 1X1, 1X2 or 1X3 respectively. One part of the 1B solution was mixed with one part of the 1C solution and incubated for 20 minutes at room temperature to obtain a transfection complex 1X4.

For each siRNA, the transfection complex 1X1 was respectively added into three culture wells, and mixed evenly, with an addition amount of 20 μl/ well, to obtain a co-transfection mixture with the final concentration of the siRNA

about 0.1 nM, which was labeled as test group 1.

For each siRNA, the transfection complex 1X2 was respectively added into another three culture wells, and mixed evenly, with an addition amount of 20 μl/ well, to obtain a co-transfection mixture with the final concentration of the siRNA about 0.01 nM, which was labeled as test group 2.

For each siRNA, the transfection complex 1X3 was respectively added into another three culture wells, and mixed evenly, with an addition amount of 20 μl/ well, to obtain a co-transfection mixture with the final concentration of the siRNA about 0.001 nM, which was labeled as test group 3.

The transfection complex 1X4 was respectively added into another three culture wells, and mixed evenly, with an addition amount of 20 μl/ well, to obtain a co-transfection mixture not containing the siRNA, which was labeled as a control group.

The co-transfection mixture containing the siRNA and the co-transfection mixture not containing the siRNA were co-transfected in culture wells for 4 hours, and then 100 μl of H-DMEM complete media containing 20% FBS was added to each well. The 96-well plate was placed in a $CO_2$ incubator to continuously co-transfect for 24 hours.

[3] Detection

The media in the culture wells were sucked off, and 150 μl of a mixed solution of Dual-Glo® Luciferase and H-DMEM (at a volume ratio 1: 1) was added to each well, thoroughly mixed, and incubated at room temperature for 10 minutes, then 120 μl of the mixed solution was transferred to a 96-well enzyme-labeled plate, and a Firefly chemiluminescence value (Fir) in each culture well was read by using Synergy II multifunctional microplate reader (BioTek company); then, 60 μl of Dual-Glo® Stop & Glo® was added to each well, thoroughly mixed, incubated at room temperature for 10 minutes, then a *Renilla* chemiluminescence value (Ren) in each culture well was read with a microplate reader according to the arrangement of reading the Fir.

The luminous ratio (Ratio = Ren/Fir) of each well was calculated, and the luminous Ratio (test) or Ratio (control) of each test group or control group was the average value of the Ratio of three culture wells; on the basis of the luminous ratio of the control group, the luminous ratio of each test group was normalized to obtain the ratio R of the Ratio (test)/Ratio (control), which was used to express the relative expression level of *Renilla* reporter gene, i.e., the residual activity. Inhibition percentage of the siRNA to the target sequence = (1-R) × 100%.

FIG. 1 showed the residual activity of *Renilla* reporter genes in HEK293A cells after transfection of siPKKa1M1S, siPKKb1M1S, siPKKc1M1S, siPKKdIMIS, siPKKe1M1S and siPKKf1M1S respectively.

Experimental Example 1

In vitro inhibitory activity of reference siRNA NC

According to the method of Experimental Example 1, the inhibitory activity of the reference siRNA NC in the psiCHECK system was also investigated. The only difference was in that the tested siRNA was the reference siRNA NC. The results were as shown in FIG. 1.

The results show that the siPKKa1M1S, siPKKb1M1S, siPKKc1M1S, siPKKdIMIS, siPKKe1M1S and siPKKf1M1S all have good inhibitory activity on the target sequences in vitro at all concentrations, and show a concentration dependence. Especially, the inhibition percentage of the siPKKa1M1S, siPKKdIMIS and siPKKf1M1S is as high as 90% when the concentration of the siRNA is 0.1 nM, showing a good effect of inhibiting the expression of PKK genes.

Experimental Example 2

Construction of hPKK stably transfected cell lines and activity analysis of siRNA

A gene segment as shown by SEQ ID NO: 401 was constructed into a pcDNA3.1 universal carrier to obtain a pcDNA3.1 recombinant plasmid, and the gene segment was a segment of the gene encoding human PKK mRNA. This construction work was entrusted to GENEWIZ Biotechnology Co., Ltd.

ATGATTTTATTCAAGCAAGCAACTTATTTCATTTCCTTGTTTGCTACAGTTTC
CTGTGGATGTCTGACTCAACTCTATGAAAACGCCTTCTTCAGAGGTGGGGA
TGTAGCTTCCATGTACACCCCAAATGCCCAATACTGCCAGATGAGGTGCAC
ATTCCACCCAAGGTGTTTGCTATTCAGTTTTCTTCCAGCAAGTTCAATCAAT
GACATGGAGAAAAGGTTTGGTTGCTTCTTGAAAGATAGTGTTACAGGAACC
CTGCCAAAAGTACATCGAACAGGTGCAGTTTCTGGACATTCCTTGAAGCAA
TGTGGTCATCAAATAAGTGCTTGCCATCGAGACATTTATAAAGGAGTTGATA
TGAGAGGAGTCAATTTTAATGTGTCTAAGGTTAGCAGTGTTGAAGAATGCC
AAAAAAGGTGCACCAGTAACATTCGCTGCCAGTTTTTTTCATATGCCACGC
AAACATTTCACAAGGCAGAGTACCGGAACAATTGCCTATTAAAGTACAGTC
CCGGAGGAACACCTACCGCTATAAAGGTGCTGAGTAACGTGGAATCTGGAT
TCTCACTGAAGCCCTGTGCCCTTTCAGAAATTGGTTGCCACATGAACATCT
TCCAGCATCTTGCGTTCTCAGATGTGGATGTTGCCAGGGTTCTCACTCCAGA
TGCTTTTGTGTGTCGGACCATCTGCACCTATCACCCCAACTGCCTCTTCTTT
ACATTCTATACAAATGTATGGAAAATCGAGTCACAAAGAAATGTTTGTCTTC
TTAAAACATCTGAAAGTGGCACACCAAGTTCCTCTACTCCTCAAGAAAACA
CCATATCTGGATATAGCCTTTTAACCTGCAAAAGAACTTTACCTGAACCCTG
CCATTCTAAAATTTACCCGGGAGTTGACTTTGGAGGAGAAGAATTGAATGT
GACTTTTGTTAAAGGAGTGAATGTTTGCCAAGAGACTTGCACAAAGATGAT
TCGCTGTCAGTTTTTCACTTATTCTTTACTCCCAGAAGACTGTAAGGAAGAG

AAGTGTAAGTGTTTCTTAAGATTATCTATGGATGGTTCTCCAACTAGGATTG
CGTATGGGACACAAGGGAGCTCTGGTTACTCTTTGAGATTGTGTAACACTG
GGGACAACTCTGTCTGCACAACAAAAACAAGCACACGCATTGTTGGAGGA
ACAAACTCTTCTTGGGGAGAGTGGCCCTGGCAGGTGAGCCTGCAGGTGAA
GCTGACAGCTCAGAGGCACCTGTGTGGAGGGTCACTCATAGGACACCAGT
GGGTCCTCACTGCTGCCCACTGCTTTGATGGGCTTCCCCTGCAGGATGTTT
GGCGCATCTATAGTGGCATTTTAAATCTGTCAGACATTACAAAAGATACACC
TTTCTCACAAATAAAAGAGATTATTATTCACCAAAACTATAAAGTCTCAGAA
GGGAATCATGATATCGCCTTGATAAAACTCCAGGCTCCTTTGAATTACACTG
AATTCCAAAAACCAATATGCCTACCTTCCAAAGGTGACACAAGCACAATTT
ATACCAACTGTTGGGTAACCGGATGGGGCTTCTCGAAGGAGAAAGGTGAA
ATCCAAAATATTCTACAAAAGGTAAATATTCCTTTGGTAACAAATGAAGAAT
GCCAGAAAAGATATCAAGATTATAAAATAACCCAACGGATGGTCTGTGCTG
GCTATAAAGAAGGGGGAAAAGATGCTTGTAAGGGAGATTCAGGTGGTCCC
TTAGTTTGCAAACACAATGGAATGTGGCGTTTGGTGGGCATCACCAGCTGG
GGTGAAGGCTGTGCCCGCAGGGAGCAACCTGGTGTCTACACCAAAGTCGC
TGAGTACATGGACTGGATTTTAGAGAAAACACAGAGCAGTGATGGAAAAG
CTCAGATGCAGTCACCAGCATGAGAAGCAGTCCAGAGTCTAGGCAATTTTT
ACAACCTGAGTTCAAGTCAAATTCTGAGCCTGGGGGGTCCTCATCTGCAAA
GCATGGAGAGTGGCATCTTCTTTGCATCCTAAGGACGAAAAACACAGTGCA
CTCAGAGCTGCTGAGGACAATGTCTGGCTGAAGCCCGCTTTCAGCACGCC
GTAACCAGGGGCTGACAATGCGAGGTCGCAACTGAGATCTCCATGACTGT
GTGTTGTGAAATAAAATGGTGAAAGATCA (SEQ ID NO: 401)

HEK293A cells (purchased from Nanjing COBIOER Biotechnology Co., Ltd.) were cultured in DMEM complete media (HyClone company) containing 10% fetal bovine serum (FBS, Hyclone company) and 0.2v% Penicillin-Streptomycin (Gibco, Invitrogen company) at 37°C in an incubator containing 5% $CO_2$/95% air. Before use, the HEK293A cells were prepared into a cell suspension with a concentration of $1 \times 10^5$ cells /mL in DMEM complete media.

The HEK293A cell suspension was seeded into 25 $cm^3$ plastic culture flasks, and each flask was seeded with 5 mL. When the cells were cultured to a confluence rate of about 70%, the DMEM complete media in the culture flasks were sucked up, and 5 mL OPTI-MEM media (GIBCO company) was added into the culture flasks to continue culturing for 1.5 hours.

1A solution was prepared, and each part of the 1A solution contained 3 μg of pcDNA3.1 recombinant plasmid and 500 μl of Opti-MEM media, and then the solution was blended.

1B solution was prepared, and each part of the 1B solution contained 9 μl of Lipofectamine™ 2000 and 500 μl of Opti-MEM media, and then the solution was gently blended.

One part of 1B solution and one part of 1A solution were incubated for 20min minutes at room temperature to obtain one part of transfection complex IX.

One part of the transfection complex IX was added into each culture flask aforementioned for culturing the HEK293A cells and mixed evenly, replaced with fresh media after 4 hours, and cultured for 48 hours to obtain to-be-screened HEK293A cells.

Antibiotic G418 (Solarbio company, article number G8160-1g) was dissolved into 70 mg/ml solution with 1 × PBS, and then diluted with H-DMEM complete media till concentrations of the G418 were 700 μg/ml and 350 μg/ml respectively, thus obtaining G418 media and G418 media with semi-screening concentration.

For the to-be-screened HEK293A cells mentioned above, the culture media in the culture flasks was sucked out, and the G418 culture media was added for screening. The new G418 culture media were replaced every two days,

and the cell death was observed every day. When the cell number did not decrease significantly during the two observations (normal cells were considered to have died completely), the cells were cultured with the new H-DMEM complete media.

When the cells reached a confluence rate of 60%, the culture media in the culture flasks were sucked out, and the new G418 culture media were added for culture. After 4 days, the new G418 media were replaced again. On the $15^{th}$ day after transfection, the cultured cells were collected to obtain the stably transfected cell line hPKK-HEK293A. The stably transfected cell line hPKK-HEK293A was continuously cultured in the G418 media with semi-screening concentration.

It could be known through the analysis of the obtained stably transfected cell line hPKK-HEK293A by real-time fluorescence quantitative PCR that the obtained stably transfected cell line hPKK-HEK293A had successfully integrated hPKK gene with high copy. A full-length gene length was amplified by ordinary PCR and the size of the amplified segment was detected. The results showed that a transcript with the full-length sequence as shown in SEQ ID NO: 401 had been integrated into the stably transfected cell line hPKK-HEK293A.

In the following, a relative inhibition level of the siRNA compounds of the present disclosure on PKK mRNA in the stably transformed cell line hPKK-HEK293A was measured by Quantitative Real-Time PCR (hereinafter referred to as qPCR).

The hPKK-HEK293A cells were diluted to $1.5 \times 10^5$ cells /ml with H-DMEM complete media to obtain a hPKK-HEK293A cell suspension, which was seeded in a 12-well plate in an amount of 1000 μl/ well and cultured for 14.5 hours. The H-DMEM complete media in the culture wells were sucked out, and 1000 μl of Opti-MEM media was added to each well to continue the culture for 1.5 hours.

DEPC water was used to respectively dissolve each siRNA in the following siRNAs into 20 μM siRNA working solution, and the siRNA used was respectively siPKKa1M1S, siPKKb1M1S, siPKKc1M1S, siPKKdIMIS, siPKKe1M1S or siPKKf1M1S.

1A solution was prepared. For each siRNA, the 1A solution was prepared respectively, and each part of the 1A solution contained 3 μl of the siRNA working solution above and 100 μl of Opti-MEM media in turn.

1B solution was prepared, and each part of the 1B solution contained 2 μl of Lipofectamine™ 2000 and 100 μl of Opti-MEM media.

One part of the 1B solution was respectively mixed with one part of the obtained 1A solution of each siRNA, and respectively incubated at room temperature for 20 minutes to obtain the transfection complex IX of each siRNA.

One part of the 1B solution was mixed with 100 μl of Opti-MEM media and incubated at room temperature for 20 minutes to obtain a control complex IX'.

The transfection complex IX of each siRNA was added to different culture wells seeded with the hPKK-HEK293A cells above, with an addition amount of 200 μl/ well, and mixed evenly. A transfection mixture with a final concentration of each siRNA about 50 nM was obtained, and the transfection complex IX of each siRNA was transfected into two culture wells to obtain transfection mixtures containing siRNA, which was labeled as test groups.

The transfection complex 1X' was respectively added into another two culture wells seeded with the hPKK-HEK293A cells with an addition amount of 200 μl/ well, to obtain a transfection mixture not containing the siRNA, which was labeled as blank control groups.

The transfection mixture containing siRNA and the transfection mixture not containing siRNA were transfected in culture wells for 4 hours, then 1000 μl of H-DMEM complete media containing 20% FBS (purchased from RMBIO company, article number FBS-P500) was added to each well, and then the 12-well plate was continuously cultured in an incubator containing 5% $CO_2$/95% air at 37°C for 24 hours.

Then, a UNIQ column total RNA extraction kit (Sangon Biotech, B511361-0100) was used to extract the total RNA from cells in each well according to the method described in the kit manual, wherein $1.2 \times 10^5$ cells were taken for extraction from cells in each well.

For the total RNA of each cell sample, reagents provided by a reverse transcription kit Goldenstar™ RT6 cDNA Synthesis Kit (purchased from Beijing Tsingke Biotechnology Co., Ltd., article number TSK301M) was used, wherein Goldenstar™ Oligo $(dT)_{17}$ was selected as the primer, and 20 μl of reverse transcription reaction system was configured according to the reverse transcription operation steps in the kit manual. The conditions for reverse transcription were as follows: for each reverse transcription reaction system, the reverse transcription reaction system was incubated at 50°C for 50 minutes, then incubated at 85°C for 5 minutes, and finally incubated at 4°C for 30 seconds; after the reaction, 80 μl of DEPC water was added to each reverse transcription reaction system to obtain a solution containing cDNA.

For each reverse transcription reaction system, 5 μl of the above solution containing cDNA was taken as the template, and 20 μl of qPCR reaction system was prepared by using the reagents provided by NovoStart® SYBR qPCR SuperMix Plus (purchased from Novoprotein Science and Technology Co., Ltd., article No. E096-01B), wherein the PCR primer sequences for amplifying the target gene PKK and internal reference gene GAPDH were shown in Table 6, and the final concentration of each primer was 0.25 μM. Each qPCR reaction system was placed on ABI StepOnePlus Real-Time PCR instrument, and amplified by three-step method. The amplification procedure was pre-denatured at 95°C for

10 minutes, then denatured at 95°C for 30 seconds, annealed at 60°C for 25 seconds, and extended at 72°C for 30 seconds. After repeating the above denaturation, annealing and extension processes for 35 times, the product W2 containing the amplified target gene PKK and the internal reference gene GAPDH was obtained. The product W2 was incubated at 95°C for 15 seconds, 60°C for 1 minute and 95°C for 15 seconds in turn. The dissolution curves of the target gene PKK and the internal reference gene GAPDH in the product W2 were collected by real-time fluorescence quantitative PCR, and the Ct values of the target gene PKK and the internal reference gene GAPDH were obtained.

Table 6 Sequences of Detection Primers

| Gene | Upstream primer (sequence direction 5'-3') | Downstream primer (sequence direction 5'-3') |
|---|---|---|
| Human PKK | AACTAGGATTGCGTATGGG (SEQ ID NO: 402) | AGTGGGCAGCAGTGAGGA (SEQ ID NO: 403) |
| Human GAPDH | GGTCGGAGTCAACGGATTT (SEQ ID NO: 404) | CCAGCATCGCCCCACTTGA (SEQ ID NO: 405) |

Comparative Ct (ΔΔCt) method was used to calculate relative quantitative expression of the target gene PKK in each of the test group and the control group. The calculation method was as follows:

$$\Delta Ct(\text{test group}) = Ct(\text{target gene of test group}) - Ct(\text{internal reference gene of test group})$$

$$\Delta Ct(\text{control group}) = Ct(\text{target gene of control group}) - Ct(\text{internal reference gene of control group})$$

$$\Delta\Delta Ct(\text{test group}) = \Delta Ct(\text{test group}) - \Delta Ct(\text{mean value of control group})$$

$$\Delta\Delta Ct(\text{control group}) = \Delta Ct(\text{control group}) - \Delta Ct(\text{mean value of control group})$$

Each of the test group was respectively hPKK-HEK293A cells treated with each siRNA, and the control groups were the hPKK-HEK293A cells not treated with siRNA. ΔCt(mean value of control group) was the arithmetic mean value of ΔCt(control group) of each of two culture wells of the control group. Therefore, each culture well of the test group and the control group corresponded to one ΔΔCt value.

On the basis of the control group, the expression level of PKK mRNA in the test group was normalized, and the expression level of PKK mRNA in the control group was defined as 100%.

$$\text{The relative expression level of PKK mRNA in the test group} = 2^{-\Delta\Delta Ct(\text{test group})} \times 100\%.$$

For the siRNAs of the same test group, the mean value of the relative expression level of the PKK mRNA of the test group at each concentration was the arithmetic mean value of the relative expression level of two culture wells at the concentration.

The inhibition percentage of the siRNA on PKK mRNA expression was calculated according to the following equation: inhibition percentage = (1-relative expression level of PKK mRNA in the test group) × 100%.

The relative expression level of PKK mRNA was as shown in FIG. 2.

It can be seen from the results in FIG. 2 that the siRNA provided by the present disclosure has a good inhibitory effect on PKK mRNA in hPKK-HEK293A cells, and in particular, the inhibition percentages of siPKKa1M1S and siPKKf1M1S on the PKK mRNA are both as high as about 80%.

Experimental Example 3

Inhibitory effect of siRNA conjugate on PKK mRNA in hPKK-HEK293A cells

According to the method of Experimental Example 2, the inhibition efficiency of the siRNA conjugate on PKK mRNA in hPKK-HEK293A cells was tested, and the only difference was in that one of the following siRNA conjugates was used instead of the siRNA used in Experimental Example 2, and the concentration of the siRNA conjugate was calculated based on the siRNA. The siRNA conjugates were respectively L10-siPKKa1M1SP, L10-siPKKb1M1SP, L10-siPKKc1M1SP, L10-siPKKd1M1SP, L10-siPKKe1M1S and L10-siPKKf1M1S. The results were as shown in FIG. 3.

It can be seen from the results in FIG. 3 that the siRNA conjugate of the present disclosure has a good inhibitory effect on PKK mRNA in hPKK-HEK293A cells, and the inhibition percentage is at least 53.2%; and even as high as 83.1%. It can be seen that the siRNA conjugate of the present disclosure has good application potential for inhibiting PKK-related diseases.

Experimental Example 4

Distribution of siRNA conjugates in various organs of C57 mice

1 × PBS solution was used to respectively dissolve Cy5-siPKKa1M1S, Cy5-L10-siPKKa1M1SP, siPKKf1M1S or Cy5-L10-siPKKf1M1S into 0.6 mg/ml solution (based on siRNA).

Ten C57BL/6J female mice of 6-7 weeks (purchased from Beijing Vital River Laboratory Animal Technology Co., Ltd) were randomly divided into groups with two mice in each group, and injected with 1 × PBS and the solution of Cy5-siPKKa1M1S, the Cy5-L10-siPKKa1M1SP, the Cy5-siPKKf1M1S or the Cy5-L10-siPKKf1M1S prepared above subcutaneously. The administration doses of all animals were calculated according to their body weights, and the administration volume was 5 ml/kg body weight. Based on the amount of siRNA, the administration dose of each animal was 3 mg/kg body weight of mouse (based on the siRNA).

After the administration for 2 hours, 6 hours, 24 hours and 48 hours, the mice of each group were placed in a small animal in vivo optical imaging system IVIS Lumina Series III. The mice were anesthetized with isoflurane gas, and the anesthetized mice were placed with the abdomens facing up for living imaging in the small animal in vivo optical imaging system to dynamically detect Cy5 fluorescence signals, and track the distribution of Cy5-labeled siRNAs or Cy5-labeled siRNA conjugates in living animals. After 48 hours, all the mice were sacrificed, and five organs of each mouse, comprising heart, lung, liver, spleen and kidney, were taken out for fluorescence imaging in the IVIS Lumina Series III. One animal was selected from each group of mice, and the above-mentioned five organs thereof were arranged longitudinally in turn. The organs of the mice administered with 1 × PBS, the Cy5-siPKKa1M1S or Cy5-L10-siPKKa1M1SP were photographed under the same visual field, and the results were shown in FIG. 4A. The organs of the mice administered with 1 × PBS, the Cy5-siPKKf1M1S or Cy5-L10-siPKKf1M1S were photographed under the same visual field, and the results were shown in FIG. 4B.

It can be seen from FIG. 4A and FIG. 4B that only the Cy5-L10-siPKKa1M1SP and the Cy5-L10-siPKKf1M1S can be aggregated in a large amount in the liver; the Cy5-siPKKa1M1S and the Cy5-siPKKf1M1S are aggregated in a small amount in the kidney, but are not aggregated in other organs, indicating that the siRNA conjugate of the present disclosure can effectively deliver siRNA specifically to the liver. Therefore, FIG. 4A and FIG. 4B indicate that the siRNA conjugate provided by the present disclosure can specifically target the liver, and can be expected to specifically inhibit the expression of PKK gene in the liver. Further, combined with the results of Experimental Example 3, it indicates that the siRNA conjugate provided by the present disclosure can specifically inhibit the expression of the PKK gene in the liver.

Some embodiments of the present disclosure are described in detail above, but the present disclosure is not limited to the specific details of the above-described embodiments. Various simple variations of the technical solution of the present disclosure can be made within the scope of the technical concept of the present disclosure, and these simple variations are within the protection scope of the present disclosure.

In addition, it is to be noted that each of the specific technical features described in the above embodiments can be combined in any suitable manner as long as no contradiction is caused. In order to avoid unnecessary repetition, the various possible combination manners are no longer described in the present disclosure.

In addition, the various different embodiments of the present disclosure may also be carried out in any combination as long as it does not contravene the idea of the present disclosure, which should also be regarded as the disclosure of the present disclosure.

## Claims

**1.** An siRNA, wherein the siRNA comprises a sense strand and an antisense strand, and each nucleotide in the siRNA is independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region, wherein:

the nucleotide sequence I has the same length as and no more than three nucleotide differences from the nucleotide sequence shown in SEQ ID NO:1; and the nucleotide sequence II has the same length as and no more than three nucleotide differences from the nucleotide sequence shown in SEQ ID NO:2:

5'-GGUGUUUGCUAUUCAGUUZi-3' (SEQ ID NO: 1);
5'-$Z_2$AACUGAAUAGCAAACACC-3' (SEQ ID NO: 2),
wherein, $Z_1$ is U, and $Z_2$ is A; and
the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand; or,

the nucleotide sequence I has the same length as and no more than three nucleotide differences from the nucleotide sequence shown in SEQ ID NO: 61; and the nucleotide sequence II has the same length as and no more than three nucleotide differences from the nucleotide sequence shown in SEQ ID NO: 62:

5'-GCUUCUUGAAAGAUAGUG$Z_5$-3' (SEQ ID NO: 61);
5'-$Z_6$CACUAUCUUUCAAGAAGC-3' (SEQ ID NO: 62),
wherein, $Z_5$ is U, and $Z_6$ is A; and
the nucleotide sequence I comprises a nucleotide $Z_7$ at a corresponding site to $Z_5$, the nucleotide sequence II comprises a nucleotide $Z_8$ at a corresponding site to $Z_6$, and $Z_8$ is the first nucleotide from the 5' terminal of the antisense strand; or,

the nucleotide sequence I has the same length as and no more than three nucleotide differences from the nucleotide sequence shown in SEQ ID NO: 121; and the nucleotide sequence II has the same length as and no more than three nucleotide differences from the nucleotide sequence shown in SEQ ID NO: 122:

5'-GAAGCAAUGUGGUCAUCA$Z_9$-3' (SEQ ID NO: 121);
5'-$Z_{10}$UGAUGACCACAUUGCUUC-3' (SEQ ID NO: 122),
wherein, $Z_9$ is A, and $Z_{10}$ is U; and
the nucleotide sequence I comprises a nucleotide $Z_{11}$ at a corresponding site to $Z_9$, the nucleotide sequence II comprises a nucleotide $Z_{12}$ at a corresponding site to $Z_{10}$, and $Z_{12}$ is the first nucleotide from the 5' terminal of the antisense strand; or,

the nucleotide sequence I has the same length as and no more than three nucleotide differences from the nucleotide sequence shown in SEQ ID NO: 181; and the nucleotide sequence II has the same length as and no more than three nucleotide differences from the nucleotide sequence shown in SEQ ID NO: 182:

5'-CACAAAGAAAUGUUUGUC$Z_{13}$-3' (SEQ ID NO: 181);
5'-$Z_{14}$GACAAACAUUUCUUUGUG-3' (SEQ ID NO: 182),
wherein, $Z_{13}$ is U, and $Z_{14}$ is A; and
the nucleotide sequence I comprises a nucleotide $Z_{15}$ at a corresponding site to $Z_{13}$, the nucleotide sequence II comprises a nucleotide $Z_{16}$ at a corresponding site to $Z_{14}$, and $Z_{16}$ is the first nucleotide from the 5' terminal of the antisense strand; or,

the nucleotide sequence I has the same length as and no more than three nucleotide differences from the nucleotide sequence shown in SEQ ID NO: 241; and the nucleotide sequence II has the same length as and no more than three nucleotide differences from the nucleotide sequence shown in SEQ ID NO: 242:

5'-GAAUUCCAAAAACCAAUA$Z_{17}$-3' (SEQ ID NO: 241);
5'-$Z_{18}$UAUUGGUUUUUGGAAUUC-3' (SEQ ID NO: 242),
wherein, $Z_{17}$ is U, and $Z_{18}$ is A; and
the nucleotide sequence I comprises a nucleotide $Z_{19}$ at a corresponding site to $Z_{17}$, the nucleotide sequence II comprises a nucleotide $Z_{20}$ at a corresponding site to $Z_{18}$, and $Z_{20}$ is the first nucleotide from the 5' terminal of the antisense strand; or,

the nucleotide sequence I has the same length as and no more than three nucleotide differences from the nucleotide sequence shown in SEQ ID NO: 301; and the nucleotide sequence II has the same length as and no more than three nucleotide differences from the nucleotide sequence shown in SEQ ID NO: 302:

5'-GGUCUGUGCUGGCUAUAA$Z_{21}$-3' (SEQ ID NO: 301);
5'-$Z_{22}$UUAUAGCCAGCACAGACC-3' (SEQ ID NO: 302),
wherein, $Z_{21}$ is A, and $Z_{22}$ is U; and
the nucleotide sequence I comprises a nucleotide $Z_{23}$ at a corresponding site to $Z_{21}$, the nucleotide sequence II comprises a nucleotide $Z_{24}$ at a corresponding site to $Z_{22}$, and $Z_{24}$ is the first nucleotide from the 5' terminal of the antisense strand.

**2.** The siRNA according to claim 1, wherein the nucleotide sequence I has no more than one nucleotide difference from the nucleotide sequence shown in SEQ ID NO: 1, and/or the nucleotide sequence II has no more than one nucleotide difference from the nucleotide sequence shown in SEQ ID NO: 2;

or, the nucleotide sequence I has no more than one nucleotide difference from the nucleotide sequence shown in SEQ ID NO: 61, and/or the nucleotide sequence II has no more than one nucleotide difference from the nucleotide sequence shown in SEQ ID NO: 62;
or, the nucleotide sequence I has no more than one nucleotide difference from the nucleotide sequence shown in SEQ ID NO: 121, and/or the nucleotide sequence II has no more than one nucleotide difference from the nucleotide sequence shown in SEQ ID NO: 122;
or, the nucleotide sequence I has no more than one nucleotide difference from the nucleotide sequence shown in SEQ ID NO: 181, and/or the nucleotide sequence II has no more than one nucleotide difference from the nucleotide sequence shown in SEQ ID NO: 182;
or, the nucleotide sequence I has no more than one nucleotide difference from the nucleotide sequence shown in SEQ ID NO: 241, and/or the nucleotide sequence II has no more than one nucleotide difference from the nucleotide sequence shown in SEQ ID NO: 242;
or, the nucleotide sequence I has no more than one nucleotide difference from the nucleotide sequence shown in SEQ ID NO: 301, and/or the nucleotide sequence II has no more than one nucleotide difference from the nucleotide sequence shown in SEQ ID NO: 302.

**3.** The siRNA according to claim 1 or 2, wherein the nucleotide difference between the nucleotide sequence II and the nucleotide sequence shown in SEQ ID NO: 2 comprises a difference at the position of $Z_4$, and $Z_4$ is selected from U, C or G;

or, the nucleotide difference between the nucleotide sequence II and the nucleotide sequence shown in SEQ ID NO: 62 comprises a difference at the site of $Z_8$, and $Z_8$ is selected from U, C or G;
or, the nucleotide difference between the nucleotide sequence II and the nucleotide sequence shown in SEQ ID NO: 122 comprises a difference at the site of $Z_{12}$, and $Z_{12}$ is selected from A, C or G;
or, the nucleotide difference between the nucleotide sequence II and the nucleotide sequence shown in SEQ ID NO: 182 comprises a difference at the site of $Z_{16}$, and $Z_{16}$ is selected from U, C or G;
or, the nucleotide difference between the nucleotide sequence II and the nucleotide sequence shown in SEQ ID NO: 242 comprises a difference at the site of $Z_{20}$, and $Z_{20}$ is selected from U, C or G;
or, the nucleotide difference between the nucleotide sequence II and the nucleotide sequence shown in SEQ ID NO: 302 comprises a difference at the site of $Z_{24}$, and $Z_{24}$ is selected from A, C or G.

**4.** The siRNA according to any one of claims 1-3, wherein $Z_3$ is a nucleotide complementary to $Z_4$; or $Z_7$ is a nucleotide complementary to $Z_8$; or $Z_{11}$ is a nucleotide complementary to $Z_{12}$; or $Z_{15}$ is a nucleotide complementary to $Z_{16}$; or $Z_{19}$ is a nucleotide complementary to $Z_{20}$; or $Z_{23}$ is a nucleotide complementary to $Z_{24}$.

**5.** The siRNA according to any one of claims 1-4, wherein lengths of the sense strand and the antisense strand are the same or different, the length of the sense strand is 19-23 nucleotides, and the length of the antisense strand is 19-26 nucleotides; and,

the nucleotide sequence I is the nucleotide sequence shown in SEQ ID NO: 3, and the nucleotide sequence II is the nucleotide sequence shown in SEQ ID NO: 4:

5'-GGUGUUUGCUAUUCAGUU$Z_3$-3' (SEQ ID NO: 3);
5'-$Z_4$AACUGAAUAGCAAACACC-3' (SEQ ID NO: 4),
wherein, $Z_4$ is selected from A, U, G or C; and $Z_3$ is a nucleotide complementary to $Z_4$;

or, the nucleotide sequence I is the nucleotide sequence shown in SEQ ID NO: 63, and the nucleotide sequence II is the nucleotide sequence shown in SEQ ID NO: 64:

5'-GCUUCUUGAAAGAUAGUG$Z_7$-3' (SEQ ID NO: 63);
5'-$Z_8$CACUAUCUUUCAAGAAGC-3' (SEQ ID NO: 64),
wherein, $Z_8$ is selected from A, U, G or C; and $Z_7$ is a nucleotide complementary to $Z_8$;

or, the nucleotide sequence I is the nucleotide sequence shown in SEQ ID NO: 123, and the nucleotide sequence II is the nucleotide sequence shown in SEQ ID NO: 124:

5'-GAAGCAAUGUGGUCAUCA$Z_{11}$-3' (SEQ ID NO: 123);
5'-$Z_{12}$UGAUGACCACAUUGCUUC-3' (SEQ ID NO: 124),

wherein, $Z_{12}$ is selected from A, U, G or C; and $Z_{11}$ is a nucleotide complementary to $Z_{12}$;

or, the nucleotide sequence I is the nucleotide sequence shown in SEQ ID NO: 183, and the nucleotide sequence II is the nucleotide sequence shown in SEQ ID NO: 184:

5'-CACAAAGAAAUGUUUGUC$Z_{15}$-3' (SEQ ID NO: 183);
5'-$Z_{16}$GACAAACAUUUCUUUGUG-3' (SEQ ID NO: 184),
wherein, $Z_{16}$ is selected from A, U, G or C; and $Z_{15}$ is a nucleotide complementary to $Z_{16}$;

or, the nucleotide sequence I is the nucleotide sequence shown in SEQ ID NO: 243, and the nucleotide sequence II is the nucleotide sequence shown in SEQ ID NO: 244:

5'-GAAUUCCAAAAACCAAUA$Z_{19}$-3' (SEQ ID NO: 243);
5'-$Z_{20}$UAUUGGUUUUUGGAAUUC-3' (SEQ ID NO: 244),
wherein, $Z_{20}$ is selected from A, U, G or C; and $Z_{19}$ is a nucleotide complementary to $Z_{20}$;

or, the nucleotide sequence I is the nucleotide sequence shown in SEQ ID NO: 303, and the nucleotide sequence II is the nucleotide sequence shown in SEQ ID NO: 304:

5'-GGUCUGUGCUGGCUAUAA$Z_{23}$-3' (SEQ ID NO: 303);
5'-$Z_{24}$UUAUAGCCAGCACAGACC-3' (SEQ ID NO: 304),
wherein, $Z_{24}$ is selected from A, U, G or C; and $Z_{23}$ is a nucleotide complementary to $Z_{24}$.

**6.** The siRNA according to claim 5, wherein $Z_3$ is U, and $Z_4$ is A; or, $Z_7$ is U, and $Z_8$ is A; or, $Z_{11}$ is A, and $Z_{12}$ is U; or, $Z_{15}$ is U, and $Z_{16}$ is A; or, $Z_{19}$ is U, and $Z_{20}$ is A; or, $Z_{23}$ is A, and $Z_{24}$ is U.

**7.** The siRNA according to any one of claims 1-6, wherein the sense strand further comprises a nucleotide sequence III, the antisense strand further comprises a nucleotide sequence IV, the nucleotide sequence III and the nucleotide sequence IV each independently have a length of 1-4 nucleotides, the nucleotide sequence III is linked to the 5' terminal of the nucleotide sequence I, the nucleotide sequence IV is linked to the 3' terminal of the nucleotide sequence II, and the nucleotide sequence III has the same length and is substantially reverse complementary or completely reverse complementary to the nucleotide sequence IV; the substantially reverse complementary refers to no more than one base mispairing between two nucleotide sequences; and the completely reverse complementary refers to no mispairing between two nucleotide sequences.

**8.** The siRNA according to claim 7, wherein the nucleotide sequence I has the same length as and no more than three nucleotide differences from the nucleotide sequence shown in SEQ ID NO: 1; and, the nucleotide sequences III and IV both have a length of one nucleotide, and the base of the nucleotide sequence III is A; or, the nucleotide sequences III and IV both have a length of two nucleotides, and in the direction from the 5' terminal to the 3' terminal, the base composition of the nucleotide sequence III is AA; or, the nucleotide sequences III and IV both have a length of three nucleotides, and in the direction from the 5' terminal to the 3' terminal, the base composition of the nucleotide sequence III is CAA; or, the nucleotide sequences III and IV both have a length of four nucleotides, and in the direction from the 5' terminal to the 3' terminal, the base composition of the nucleotide sequence III is CCAA;

or, the nucleotide sequence I has the same length as and no more than three nucleotide differences from the nucleotide sequence shown in SEQ ID NO: 61; and, the nucleotide sequences III and IV both have a length of one nucleotide, and the base of the nucleotide sequence III is U; or, the nucleotide sequences III and IV both have a length of two nucleotides, and in the direction from the 5' terminal to the 3' terminal, the base composition of the nucleotide sequence III is UU; or, the nucleotide sequences III and IV both have a length of three nucleotides, and in the direction from the 5' terminal to the 3' terminal, the base composition of the nucleotide sequence III is GUU; or, the nucleotide sequences III and IV both have a length of four nucleotides, and in the direction from the 5' terminal to the 3' terminal, the base composition of the nucleotide sequence III is GGUU;
or, the nucleotide sequence I has the same length as and no more than three nucleotide differences from the nucleotide sequence shown in SEQ ID NO: 121; and, the nucleotide sequences III and IV both have a length of one nucleotide, and the base of the nucleotide sequence III is U; or, the nucleotide sequences III and IV both have a length of two nucleotides, and in the direction from the 5' terminal to the 3' terminal, the base composition of the nucleotide sequence III is UU; or, the nucleotide sequences III and IV both have a length of three nucleotides, and in the direction from the 5' terminal to the 3' terminal, the base composition of the nucleotide sequence

III is CUU; or, the nucleotide sequences III and IV both have a length of four nucleotides, and in the direction from the 5' terminal to the 3' terminal, the base composition of the nucleotide sequence III is CCUU;
or, the nucleotide sequence I has the same length as and no more than three nucleotide differences from the nucleotide sequence shown in SEQ ID NO: 181; and, the nucleotide sequences III and IV both have a length of one nucleotide, and the base of the nucleotide sequence III is U; or, the nucleotide sequences III and IV both have a length of two nucleotides, and in the direction from the 5' terminal to the 3' terminal, the base composition of the nucleotide sequence III is GU; or, the nucleotide sequences III and IV both have a length of three nucleotides, and in the direction from the 5' terminal to the 3' terminal, the base composition of the nucleotide sequence III is AGU; or, the nucleotide sequences III and IV both have a length of four nucleotides, and in the direction from the 5' terminal to the 3' terminal, the base composition of the nucleotide sequence III is GAGU;
or, the nucleotide sequence I has the same length as and no more than three nucleotide differences from the nucleotide sequence shown in SEQ ID NO: 241; and, the nucleotide sequences III and IV both have a length of one nucleotide, and the base of the nucleotide sequence III is U; or, the nucleotide sequences III and IV both have a length of two nucleotides, and in the direction from the 5' terminal to the 3' terminal, the base composition of the nucleotide sequence III is CU; or, the nucleotide sequences III and IV both have a length of three nucleotides, and in the direction from the 5' terminal to the 3' terminal, the base composition of the nucleotide sequence III is ACU; or, the nucleotide sequences III and IV both have a length of four nucleotides, and in the direction from the 5' terminal to the 3' terminal, the base composition of the nucleotide sequence III is CACU;
or, the nucleotide sequence I has the same length as and no more than three nucleotides differences from the nucleotide sequence shown in SEQ ID NO: 301; and, the nucleotide sequences III and IV both have a length of one nucleotide, and the base of the nucleotide sequence III is U; or, the nucleotide sequences III and IV both have a length of two nucleotides, and in the direction from the 5' terminal to the 3' terminal, the base composition of the nucleotide sequence III is AU; or, the nucleotide sequences III and IV both have a length of three nucleotides, and in the direction from the 5' terminal to the 3' terminal, the base composition of the nucleotide sequence III is GAU; or, the nucleotide sequences III and IV both have a length of four nucleotides, and in the direction from the 5' terminal to the 3' terminal, the base composition of the nucleotide sequence III is GGAU.

**9.** The siRNA according to any one of claims 1-8, wherein the antisense strand further comprises a nucleotide sequence V, which has a length of 1-3 nucleotides and is linked to 3' terminal of the antisense strand, thereby constituting a 3' overhang of the antisense strand.

**10.** The siRNA according to claim 9, wherein the nucleotide sequence V has a length of two nucleotides.

**11.** The siRNA according to claim 9 or 10, wherein the nucleotide sequence V is two continuous thymidine deoxyribonucleotides or two continuous uridine ribonucleotides; or the nucleotide sequence V is complementary to a nucleotide at a corresponding site of a target mRNA.

**12.** The siRNA according to any one of claims 1-11, wherein the sense strand of the siRNA comprises the nucleotide sequence shown in SEQ ID NO: 5, and the antisense strand comprises the nucleotide sequence shown in SEQ ID NO: 6:

5'-GGUGUUUGCUAUUCAGUU$Z_3$-3' (SEQ ID NO: 5);
5'-$Z_4$AACUGAAUAGCAAACACCUU-3' (SEQ ID NO: 6),
or, the sense strand of the siRNA comprises the nucleotide sequence shown in SEQ ID NO: 7, and the antisense strand of the siRNA comprises the nucleotide sequence shown in SEQ ID NO: 8;
5'-AAGGUGUUUGCUAUUCAGUU$Z_3$-3' (SEQ ID NO: 7);
5'-$Z_4$AACUGAAUAGCAAACACCUUGG-3' (SEQ ID NO: 8),
wherein, $Z_4$ is the first nucleotide from 5' terminal of the antisense strand; $Z_3$ is selected from A, U, G or C; and $Z_4$ is a nucleotide complementary to $Z_3$;
or, the sense strand of the siRNA comprises the nucleotide sequence shown in SEQ ID NO: 65, and the antisense strand of the siRNA comprises the nucleotide sequence shown in SEQ ID NO: 66:

5'-GCUUCUUGAAAGAUAGUG$Z_7$-3' (SEQ ID NO: 65);
5'-$Z_8$CACUAUCUUUCAAGAAGCAA-3' (SEQ ID NO: 66),

or, the sense strand of the siRNA comprises the nucleotide sequence shown in SEQ ID NO: 67, and the antisense strand of the siRNA comprises the nucleotide sequence shown in SEQ ID NO: 68:

5'-UUGCUUCUUGAAAGAUAGUG$Z_7$-3' (SEQ ID NO: 67);
5'-$Z_8$CACUAUCUUUCAAGAAGCAACC-3' (SEQ ID NO: 68),
wherein, $Z_8$ is the first nucleotide from 5' terminal of the antisense strand; $Z_7$ is selected from A, U, G or C; and $Z_8$ is a nucleotide complementary to $Z_7$;

or, the sense strand of the siRNA comprises the nucleotide sequence shown in SEQ ID NO: 125, and the antisense strand of the siRNA comprises the nucleotide sequence shown in SEQ ID NO: 126:

5'-GAAGCAAUGUGGUCAUCA$Z_{11}$-3' (SEQ ID NO: 125);
5'-$Z_{12}$UGAUGACCACAUUGCUUCAA-3' (SEQ ID NO: 126),

or, the sense strand of the siRNA comprises the nucleotide sequence shown in SEQ ID NO: 127, and the antisense strand of the siRNA comprises the nucleotide sequence shown in SEQ ID NO: 128:

5'-UUGAAGCAAUGUGGUCAUCA$_{11}$-3' (SEQ ID NO: 127);
5'-$Z_{12}$UGAUGACCACAUUGCUUCAAGG-3' (SEQ ID NO: 128),
wherein, $Z_{12}$ is the first nucleotide from 5' terminal of the antisense strand; $Z_{11}$ is selected from A, U, G or C; and $Z_{12}$ is a nucleotide complementary to $Z_{11}$;

or, the sense strand of the siRNA comprises the nucleotide sequence shown in SEQ ID NO: 185, and the antisense strand of the siRNA comprises the nucleotide sequence shown in SEQ ID NO: 186:

5'-CACAAAGAAAUGUUUGUC$Z_{15}$-3' (SEQ ID NO: 185);
5'-$Z_{16}$GACAAACAUUUCUUUGUGAC-3' (SEQ ID NO: 186),

or, the sense strand of the siRNA comprises the nucleotide sequence shown in SEQ ID NO: 187, and the antisense strand of the siRNA comprises the nucleotide sequence shown in SEQ ID NO: 188:

5'-GUCACAAAGAAAUGUUUGUC$Z_{15}$-3' (SEQ ID NO: 187);
5'-$Z_{16}$GACAAACAUUUCUUUGUGACUC-3' (SEQ ID NO: 188),
wherein, $Z_{16}$ is the first nucleotide from 5' terminal of the antisense strand; $Z_{15}$ is selected from A, U, G or C; and $Z_{16}$ is a nucleotide complementary to $Z_{15}$;

or, the sense strand of the siRNA comprises the nucleotide sequence shown in SEQ ID NO: 245, and the antisense strand of the siRNA comprises the nucleotide sequence shown in SEQ ID NO: 246:

5'-GAAUUCCAAAAACCAAUA$Z_{19}$-3' (SEQ ID NO: 245);
5'-$Z_{20}$UAUUGGUUUUUGGAAUUCAG-3' (SEQ ID NO: 246),

or, the sense strand of the siRNA comprises the nucleotide sequence shown in SEQ ID NO: 247, and the antisense strand of the siRNA comprises the nucleotide sequence shown in SEQ ID NO: 248:

5'-CUGAAUUCCAAAAACCAAUA$Z_{19}$-3' (SEQ ID NO: 247);
5'-$Z_{20}$UAUUGGUUUUUGGAAUUCAGUG-3' (SEQ ID NO: 248),
wherein, $Z_{20}$ is the first nucleotide from 5' terminal of the antisense strand; $Z_{19}$ is selected from A, U, G or C; and $Z_{20}$ is a nucleotide complementary to $Z_{19}$;

or, the sense strand of the siRNA comprises the nucleotide sequence shown in SEQ ID NO: 305, and the antisense strand of the siRNA comprises the nucleotide sequence shown in SEQ ID NO: 306:

5'-GGUCUGUGCUGGCUAUAA$Z_{23}$-3' (SEQ ID NO: 305);
5'-$Z_{24}$UUAUAGCCAGCACAGACCAU-3' (SEQ ID NO: 306),

or, the sense strand of the siRNA comprises the nucleotide sequence shown in SEQ ID NO: 307, and the antisense strand of the siRNA comprises the nucleotide sequence shown in SEQ ID NO: 308:

5'-AUGGUCUGUGCUGGCUAUAA$Z_{23}$-3' (SEQ ID NO: 307);
5'-$Z_{24}$UUAUAGCCAGCACAGACCAUCC-3' (SEQ ID NO: 308),

wherein, $Z_{24}$ is the first nucleotide from 5' terminal of the antisense strand; $Z_{23}$ is selected from A, U, G or C; and $Z_{24}$ is a nucleotide complementary to $Z_{23}$.

**13.** The siRNA according to any one of claims 1-12, wherein the siRNA is siPKKa1, siPKKa2, siPKKb1, siPKKb2, siPKKc1, siPKKc2, siPKKd1, siPKKd2, siPKKe1, siPKKe2, siPKKf1 and siPKKf2.

**14.** The siRNA according to any one of claims 1-13, wherein at least one nucleotide in the sense strand or the antisense strand is a modified nucleotide, and/or at least one phosphate ester group is a phosphate ester group with modified group.

**15.** The siRNA according to any one of claims 1-14, wherein each nucleotide in the sense strand and the antisense strand is independently a fluoro modified nucleotide or a non-fluoro modified nucleotide.

**16.** The siRNA according to claim 15, wherein the fluoro modified nucleotides are located in the nucleotide sequence I and the nucleotide sequence II; and in the direction from 5' terminal to 3' terminal, at least the nucleotides at positions 7, 8, 9 of the nucleotide sequence I are fluoro modified nucleotides; and in the direction from 5' terminal to 3' terminal, at least the nucleotides at positions 2, 6, 14, 16 of the nucleotide sequence II are fluoro modified nucleotides.

**17.** The siRNA according to claim 16, wherein in the direction from 5' terminal to 3' terminal, the nucleotides at positions 7, 8, 9 or at positions 5, 7, 8, 9 of the nucleotide sequence I in the sense strand are fluoro modified nucleotides, and the nucleotides at the rest of positions in the sense strand are non-fluoro modified nucleotides; and in the direction from 5' terminal to 3' terminal, the nucleotides at positions 2, 6, 14, 16 or at positions 2, 6, 8, 9, 14, 16 of the nucleotide sequence II in the antisense strand are fluoro modified nucleotides, and the nucleotides at the rest of positions in the antisense strand are non-fluoro modified nucleotides.

**18.** The siRNA according to any one of claims 15-17, wherein each non-fluoro modified nucleotide is independently selected from one of a nucleotide formed by substituting the hydroxy at the 2'-position of the ribose group of a nucleotide with a non-fluoro group, or a nucleotide analogue.

**19.** The siRNA according to claim 18, wherein the nucleotide formed by substituting the hydroxy at the 2'-position of the ribose group of a nucleotide with the non-fluoro group is selected from one of a 2'-alkoxy modified nucleotide, a 2'-substituted alkoxy modified nucleotide, a 2'-alkyl modified nucleotide, a 2'-substituted alkyl modified nucleotide, a 2'-amino modified nucleotide, a 2'-substituted amino modified nucleotide, a 2'-deoxy nucleotide; and the nucleotide analogue is selected from one of an isonucleotide, an LNA, an ENA, a cET, a UNA and a GNA.

**20.** The siRNA according to any one of claims 15-19, wherein each non-fluoro modified nucleotide is a methoxy modified nucleotide, and the methoxy modified nucleotide refers to a nucleotide formed by substituting the 2'-hydroxy of the ribose group of a nucleotide with a methoxy.

**21.** The siRNA according to claim 17, wherein in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides;
or, in the direction from 5' terminal to 3' terminal, the nucleotides at positions 7, 8 and 9 of the nucleotide sequence I in the sense strand of the siRNA are fluoro modified nucleotides, and the nucleotides at the rest of positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides.

**22.** The siRNA according to any one of claims 1-21, wherein the siRNA is any one of siPKKa1-M1, siPKKa1-M2, siPKKa1-M3, siPKKa2-M1, siPKKa2-M2, siPKKa2-M3, siPKKb1-M1, siPKKb1-M2, siPKKb1-M3, siPKKb2-M1, siPKKb2-M2, siPKKb2-M3, siPKKc1-M1, siPKKc1-M2, siPKKc1-M3, siPKKc2-M1, siPKKc2-M2, siPKKc2-M3, siPKKd1-M1, siPKKd1-M2, siPKKd1-M3, siPKKd2-M1, siPKKd2-M2, siPKKd2-M3, siPKKe1-M1, siPKKe1-M2, siPKKe1-M3, siPKKe2-M1, siPKKe2-M2, siPKKe2-M3, siPKKf1-M1, siPKKf1-M2, siPKKf1-M3, siPKKf2-M1, siPKKf2-M2 and siPKKf2-M3.

**23.** The siRNA according to claim 14, wherein the phosphate ester group with modified group is a phosphorothioate group formed by substituting at least one oxygen atom in a phosphodiester bond of the phosphate ester group with a sulfur atom.

**24.** The siRNA according to claim 14 or 23, wherein the phosphate ester group with modified group is a phosphorothioate group having a structure as shown by Formula (1):

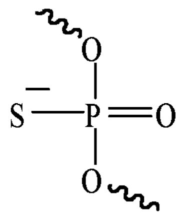
Formula (1).

**25.** The siRNA according to claim 23 or 24, wherein, in the siRNA, the linkage of phosphorothioate group exists in at least one of group consisting of the following positions:

the position between the first nucleotide and the second nucleotide at 5' terminal of the sense strand;
the position between the second nucleotide and the third nucleotide at 5' terminal of the sense strand;
the position between the first nucleotide and the second nucleotide at 3' terminal of the sense strand;
the position between the second nucleotide and the third nucleotide at 3' terminal of the sense strand;
the position between the first nucleotide and the second nucleotide at 5' terminal of the antisense strand;
the position between the second nucleotide and the third nucleotide at 5' terminal of the antisense strand;
the position between the first nucleotide and the second nucleotide at 3' terminal of the antisense strand; and
the position between the second nucleotide and the third nucleotide at 3' terminal of the antisense strand.

**26.** The siRNA according to any one of claims 1-25, wherein the siRNA is any one of siPKKa1-M1S, siPKKa1-M2S, siPKKa1-M3S, siPKKa2-M1S, siPKKa2-M2S, siPKKa2-M3S, siPKKb1-M1S, siPKKb1-M2S, siPKKb1-M3S, siPKKb2-M1S, siPKKb2-M2S, siPKKb2-M3S, siPKKc1-M1S, siPKKc1-M2S, siPKKc1-M3S, siPKKc2-M1S, siPKKc2-M2S, siPKKc2-M3S, siPKKd1-M1S, siPKKd1-M2S, siPKKd1-M3S, siPKKd2-M1S, siPKKd2-M2S, siPKKd2-M3S, siPKKe1-M1S, siPKKe1-M2S, siPKKe1-M3S, siPKKe2-M1S, siPKKe2-M2S, siPKKe2-M3S, siPKKf1-M1S, siPKKf1-M2S, siPKKf1-M3S, siPKKf2-M1S, siPKKf2-M2S and siPKKf2-M3S.

**27.** The siRNA according to any one of claims 1-26, wherein the siRNA is any one of siPKKa1-M1P1, siPKKa1-M2P1, siPKKa1-M3P1, siPKKa2-M1P1, siPKKa2-M2P1, siPKKa2-M3P1, siPKKa1-M1SP1, siPKKa1-M2SP1, siPKKa1-M3SP1, siPKKa2-M1SP1, siPKKa2-M2SP1, siPKKa2-M3SP1, siPKKb1-M1P1, siPKKb1-M2P1, siPKKb1-M3P1, siPKKb2-M1P1, siPKKb2-M2P1, siPKKb2-M3P1, siPKKb1-M1SP1, siPKKb1-M2SP1, siPKKb1-M3SP1, siPKKb2-M1SP1, siPKKb2-M2SP1, siPKKb2-M3SP1, siPKKc1-M1P1, siPKKc1-M2P1, siPKKc1-M3P1, siPKKc2-M1P1, siPKKc2-M2P1, siPKKc2-M3P1, siPKKc1-M1SP1, siPKKc1-M2SP1, siPKKc1-M3SP1, siPKKc2-M1SP1, siPKKc2-M2SP1, siPKKc2-M3 SP1, siPKKd1-M1P1, siPKKd1-M2P1, siPKKd1-M3P1, siPKKd2-M1P1, siPKKd2-M2P1, siPKKd2-M3P1, siPKKd1-M1SP1, siPKKd1-M2SP1, siPKKd1-M3SP1, siPKKd2-M1SP1, siPKKd2-M2SP1, siPKKd2-M3SP1, siPKKe1-M1P1, siPKKe1-M2P1, siPKKe1-M3P1, siPKKe2-M1P1, siPKKe2-M2P1, siPKKe2-M3P1, siPKKe1-M1SP1, siPKKe1-M2SP1, siPKKe1-M3SP1, siPKKe2-M1SP1, siPKKe2-M2SP1, siPKKe2-M3SP1, siPKKf1-M1P1, siPKKf1-M2P1, siPKKf1-M3P1, siPKKf2-M1P1, siPKKf2-M2P1, siPKKf2-M3P1, siPKKf1-M1SP1, siPKKf1-M2SP1, siPKKf1-M3SP1, siPKKf2-M1SP1, siPKKf2-M2SP1 and siPKKf2-M3SP1.

**28.** A pharmaceutical composition, wherein the pharmaceutical composition comprises the siRNA according to any one of claims 1-27 and a pharmaceutically acceptable carrier.

**29.** The pharmaceutical composition according to claim 28, wherein a weight ratio of the siRNA to the pharmaceutically acceptable carrier is 1:(1-500).

**30.** The pharmaceutical composition according to claim 29, wherein a weight ratio of the siRNA to the pharmaceutically acceptable carrier is 1:(1-50).

**31.** The pharmaceutical composition according to any one of claims 28-30, wherein the pharmaceutically acceptable carrier comprises an organic amine, a helper lipid and a pegylated lipid; wherein the organic amine is the compound as shown by Formula (201) and/or a pharmaceutically acceptable salt thereof:

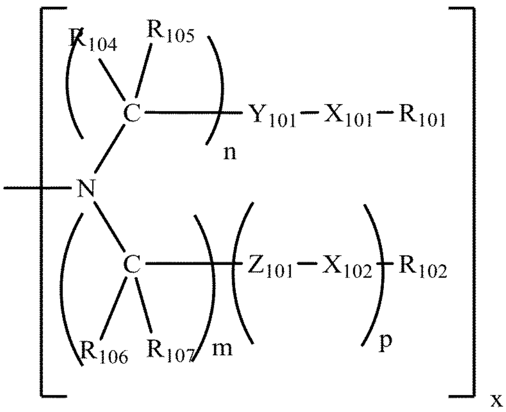

Formula (201),

wherein:

each of $X_{101}$ or $X_{102}$ is independently O, S, N-A or C-A, wherein A is hydrogen or a C1-C20 hydrocarbon chain;
each of $Y_{101}$ or $Z_{101}$ is independently C=O, C=S, S=O, CH-OH or $SO_2$;
each of $R_{101}$, $R_{102}$, $R_{103}$, $R_{104}$, $R_{105}$, $R_{106}$ or $R_{107}$ is independently hydrogen; a cyclic or acyclic, substituted or unsubstituted, branched or linear aliphatic group; a cyclic or acyclic, substituted or unsubstituted, branched or linear heteroaliphatic group; a substituted or unsubstituted, branched or linear acyl group; a substituted or unsubstituted, branched or linear aryl, or a substituted or unsubstituted, branched or linear heteroaryl;
x is an integer of 1-10;
n is an integer of 1-3, m is an integer of 0-20, and p is 0 or 1, wherein if m=p=0, then $R_{102}$ is hydrogen, and if at least one of n or m has is 2, then $R_{103}$ and the nitrogen in Formula (201) form a structure as shown by Formula (202) or (203):

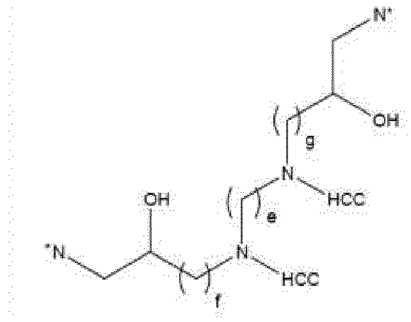

Formula (202),

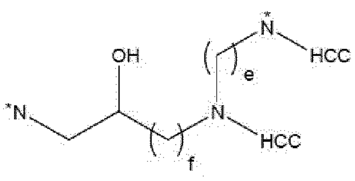

Formula (203);

wherein g, e and f are each independently an integer of 1-6, "HCC" represents a hydrocarbon chain, and each *N represents a nitrogen atom shown in Formula (201).

**32.** The pharmaceutical composition according to claim 31, wherein the organic amine is an organic amine as shown by Formula (214) and/or an organic amine as shown by Formula (215):

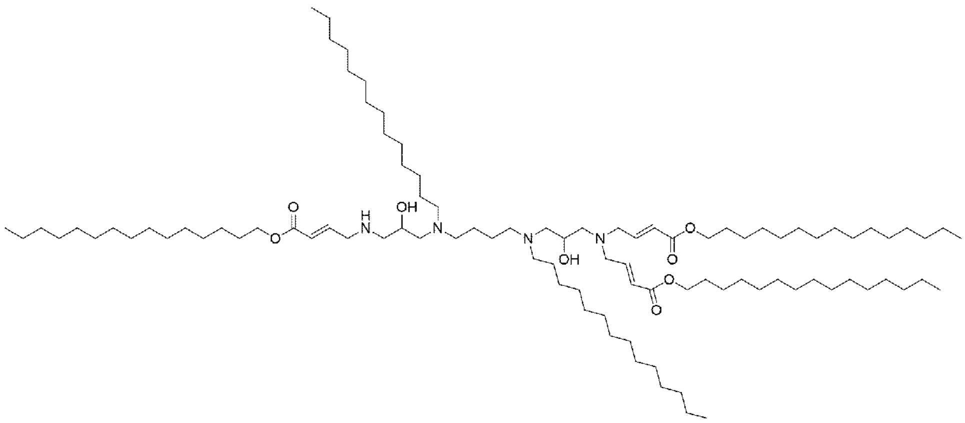

Formula (214);

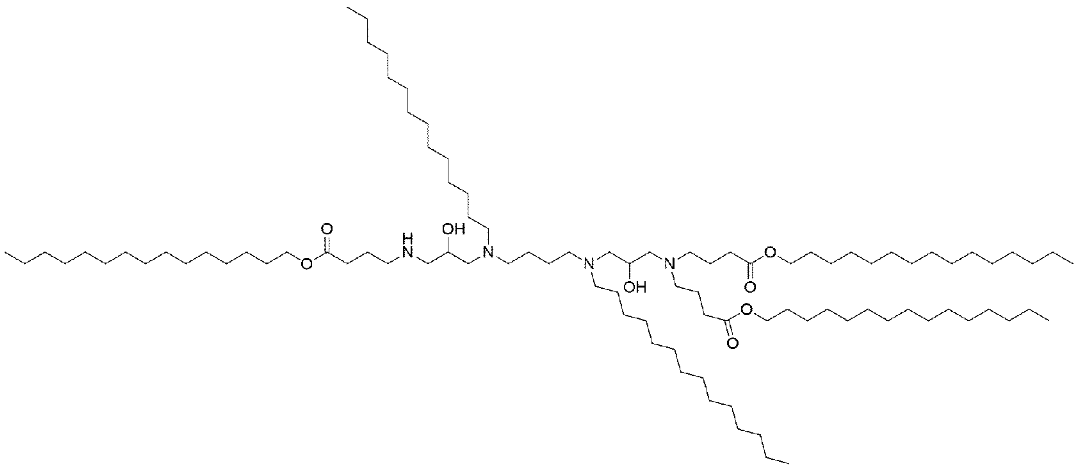

Formula (215);

the helper lipid is a cholesterol, a cholesterol analogue and/or a cholesterol derivative; and
the pegylated lipid is 1,2-dipalmitoyl-sn-glycero-3-phosphatidylethanolamine-N-[methoxy(polyethylene glycol)]-2000.

**33.** The pharmaceutical composition according to claim 31 or 32, wherein the molar ratio among the organic amine, the helper lipid, and the pegylated lipid in the pharmaceutical composition is (19.7-80):(19.7-80):(0.3-50).

**34.** The pharmaceutical composition according to claim 33, wherein the molar ratio among the organic amine, the helper lipid, and the pegylated lipid in the pharmaceutical composition is (50-70):(20-40):(3-20).

**35.** An siRNA conjugate, wherein the siRNA conjugate comprises the siRNA according to any one of claims 1-27 and a conjugating group conjugated to the siRNA.

**36.** The siRNA conjugate according to claim 35, wherein the conjugating group comprises a pharmaceutically acceptable targeting group and a linker, and the siRNA, the linker and the targeting group are covalently or non-covalently linked in sequence.

**37.** The siRNA conjugate according to claim 36, wherein the linker has a structure as shown by Formula (301):

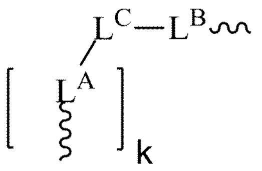

Formula (301),

wherein, k is an integer of 1-3;

$L^A$ is an amide bond-comprising chain moiety that has a structure as shown by Formula (302), each $L^A$ being respectively linked to the targeting group and the $L^C$ moiety through ether bond at two terminals thereof:

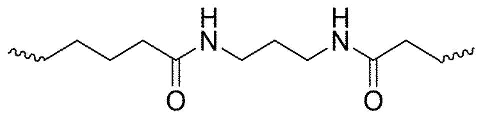

Formula (302);

$L^B$ is an N-acylpyrrolidine-comprising chain moiety that has a structure as shown by Formula (303), the chain moiety having a carbonyl at one terminal thereof and being linked to the $L^C$ moiety through an amide bond, and having an oxygen atom at the other terminal thereof and being linked to the siRNA via a phosphoester bond:

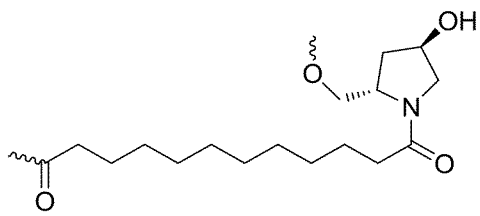

Formula (303);

$L^C$ is a bivalent to tetravalent linking group based on hydroxymethyl aminomethane, dihydroxymethyl aminomethane or trihydroxymethyl aminomethane, the $L^C$ being linked to each of the $L^A$ moieties through ether bond via an oxygen atom, and being linked to the $L^B$ moiety through amide bond via a nitrogen atom.

**38.** The siRNA conjugate according to any one of claims 35-37, wherein the siRNA conjugate has a structure as shown by Formula (305):

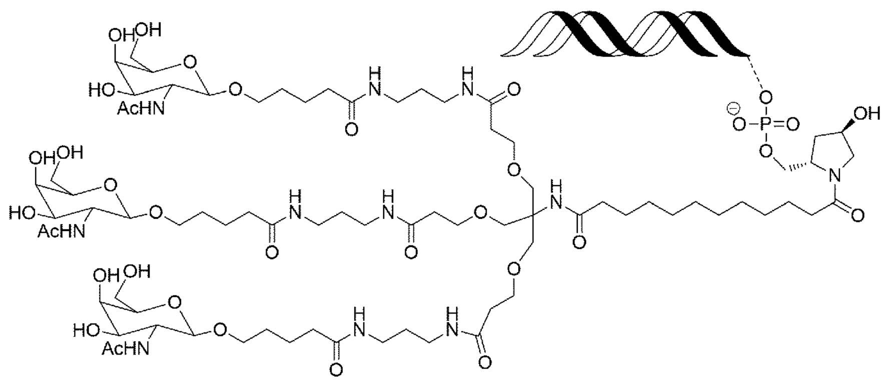

Formula (305),

wherein the double helix structure represents the siRNA.

**39.** The siRNA conjugate according to claim 38, wherein the linker has a structure as shown by Formula (306):

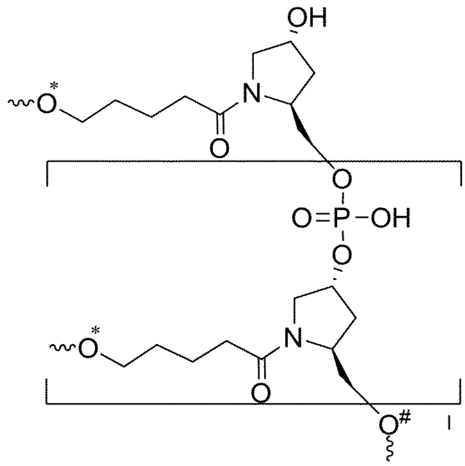

Formula (306),

wherein, 1 is an integer of 0-3;

* represents a site linked to the targeting group via an ether bond on the linker; and
# represents a site linked to the siRNA via a phosphoester bond on the linker.

**40.** The siRNA conjugate according to any one of claims 35, 36 and 39, wherein the siRNA conjugate has a structure as shown by Formula (307):

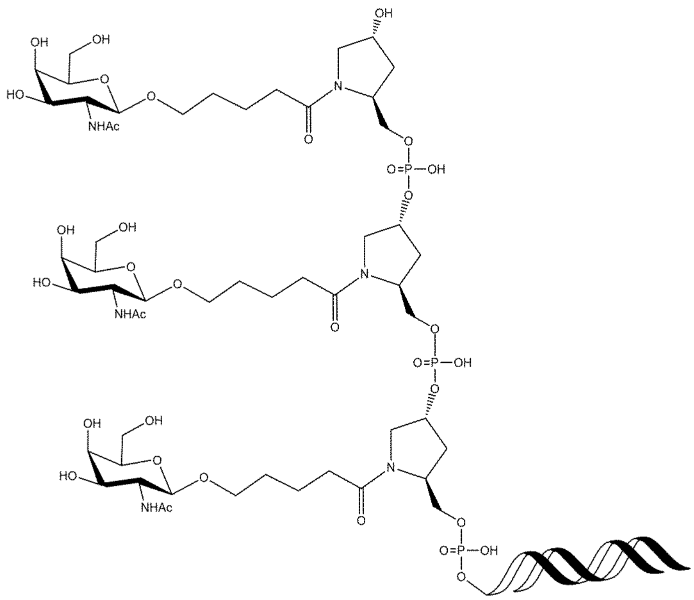

Formula (307),

wherein the double helix structure represents the siRNA.

**41.** The siRNA conjugate according to any one of claims 36-40, wherein the linker is linked to the 3' terminal of the sense strand of the siRNA.

**42.** The siRNA conjugate according to claim 35, wherein the siRNA conjugate has a structure as shown by Formula (308):

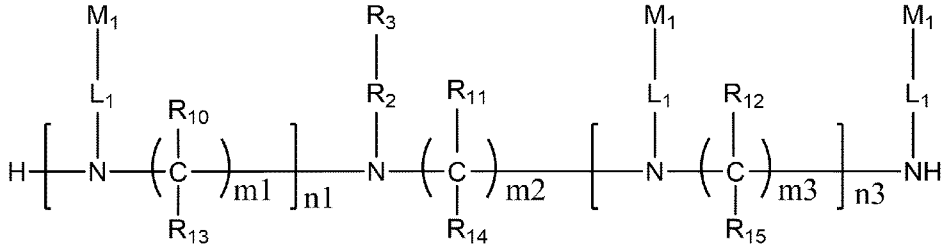

Formula (308),

wherein:

n1 is an integer selected from 1-3, and n3 is an integer selected from 0-4;
each of ml, m2, and m3 is independently an integer selected from 2-10;
each of $R_{10}$, $R_{11}$, $R_{12}$, $R_{13}$, $R_{14}$ and $R_{15}$ is independently H or selected from the group consisting of $C_1$-$C_{10}$ alkyl, $C_1$-$C_{10}$ haloalkyl and $C_1$-$C_{10}$ alkoxy; and
$R_3$ is a group having a structure as shown by Formula A59:

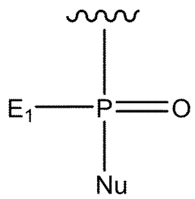

(A59)

wherein, $E_1$ is OH, SH or $BH_2$, and Nu is the siRNA according to any one of claims 1-27;
$R_2$ is a linear alkylene of 1-20 carbon atoms in length, wherein one or more carbon atoms are optionally replaced with any one or more of the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ can optionally have any one or more substituents in the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -CO2H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl);
each $L_1$ is independently a linear alkylene of 1-70 carbon atoms in length, wherein one or more carbon atoms are optionally replaced with any one or more of the group consisting of the following groups: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ can optionally have any one or more substituents in the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl);
represents the site where a group is covalently linked; and
$M_1$ represents a targeting group.

**43.** The siRNA conjugate according to claim 42, wherein each $L_1$ is independently selected from the group consisting of groups A1-A26 and any combinations thereof:

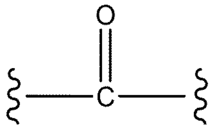, 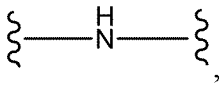, 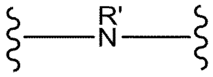, 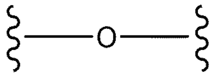,

(A1) (A2) (A3) (A4)

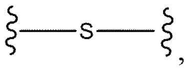, 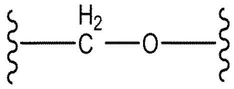, 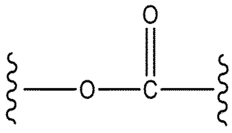, 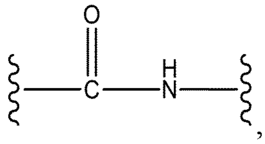,

(A5) (A6) (A7) (A8)

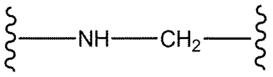, 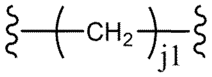, 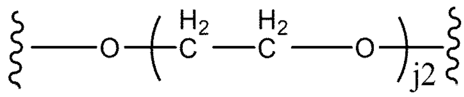,

(A9) (A10) (A11)

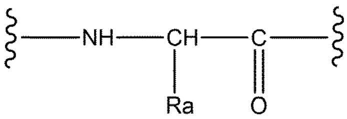, 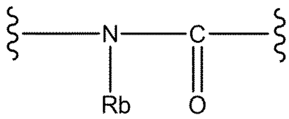, 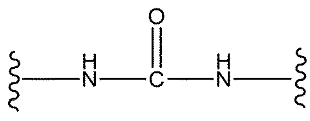,

(A12) (A13) (A14)

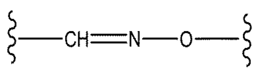, 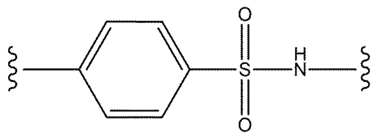, 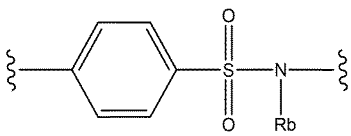,

(A15) (A16) (A17)

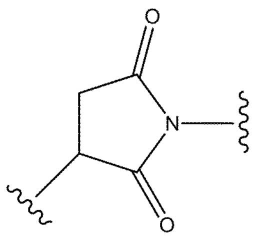, 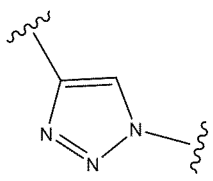, 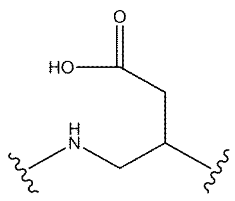, 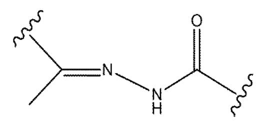,

(A18) (A19) (A20) (A21)

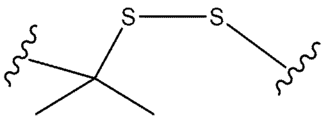,
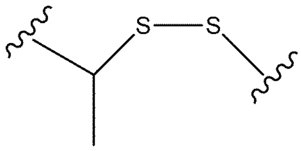,
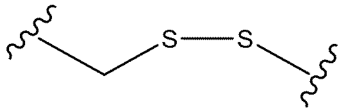,

(A22) (A23) (A24)

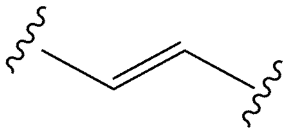,
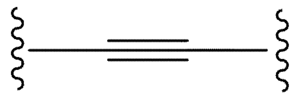;

(A25) (A26)

wherein, each j 1 is independently an integer of 1-20;

each j2 is independently an integer of 1-20;
each R' is independently a $C_1$-$C_{10}$ alkyl; and
each Ra is selected from the group consisting of groups A27-A45 and any combinations thereof:

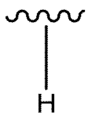,
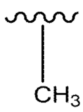,
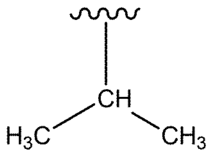,
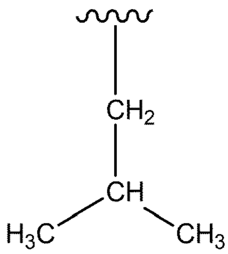,
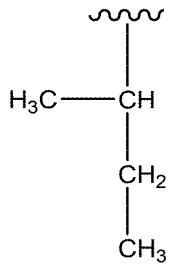,
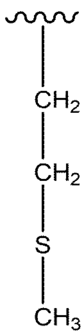,

(A27) (A28) (A29) (A30) (A31) (A32)

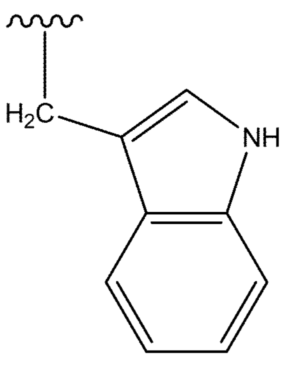,
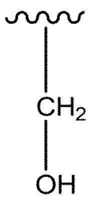,
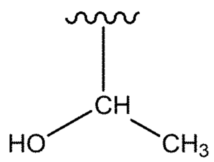,
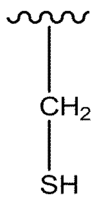,
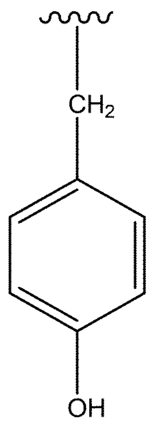,

(A33) (A34) (A35) (A36) (A37)

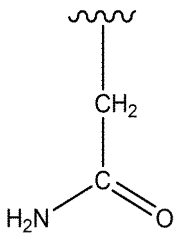, 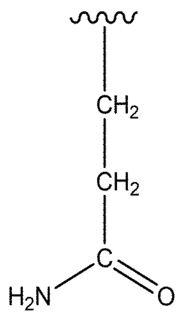, 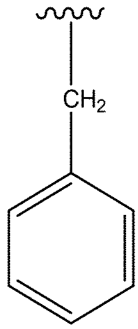, 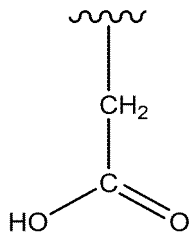, 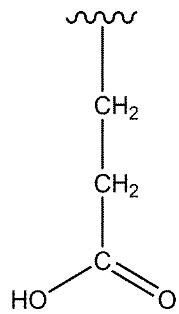,

(A38) (A39) (A40) (A41) (A42)

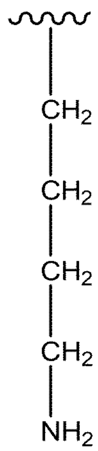, 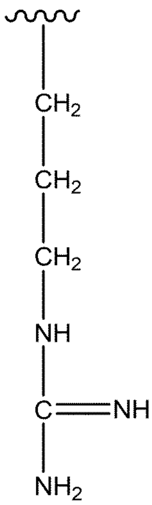 or 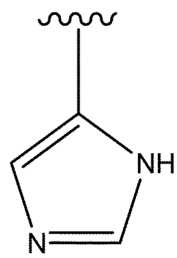;

(A43) (A44) (A45)

each Rb is independently a $C_1$-$C_{10}$ alkyl; and

represents a site where the group is covalently linked.

**44.** The siRNA conjugate according to claim 43, wherein $L_1$ is selected from the group consisting of groups A1, A4, A5, A6, A8, A10, A11, A13, and connection combinations thereof.

**45.** The siRNA conjugate according to claim 44, wherein $L_1$ is a connection combination of at least two of groups A1, A4, A8, A10, and A11.

**46.** The siRNA conjugate according to claim 45, wherein $L_1$ is a connection combination of at least two of groups A1, A8, and A10.

**47.** The siRNA conjugate according to any one of claims 42-46, wherein the length of $L_1$ is 3-25 atoms.

**48.** The siRNA conjugate according to claim 47, the length of $L_1$ is 4-15 atoms.

**49.** The siRNA conjugate according to any one of claims 43-48, wherein j 1 is an integer of 2-10, j2 is an integer of 2-10, R' is a $C_1$-$C_4$ alkyl, Ra is selected from one of A27, A28, A29, A30, and A31, and Rb is a $C_1$-$C_5$ alkyl.

**50.** The siRNA conjugate according to claim 49, wherein j 1 is an integer of 3-5, j2 is an integer of 3-5, R' is one of methyl, ethyl and isopropyl, Ra is A27 or A28, and Rb is one of a methyl, ethyl, isopropyl and butyl.

**51.** The siRNA conjugate according to any one of claims 42-50, wherein n1 is an integer of 1-2, n3 is an integer of 0-1,

and n1+n3=2-3.

**52.** The siRNA conjugate according to any one of claims 42-51, wherein each of ml, m2 and m3 is independently an integer of 2-5.

**53.** The siRNA conjugate according to any one of claims 42-52, wherein m1=m2=m3.

**54.** The siRNA conjugate according to any one of claims 35-53, wherein each targeting group is independently a ligand that has affinity with an asialoglycoprotein receptor on a surface of a mammalian hepatocyte.

**55.** The siRNA conjugate according to claim 54, wherein each targeting group is independently an asialoglycoprotein or saccharide.

**56.** The siRNA conjugate according to claim 55, wherein each targeting group is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-a-neuraminic acid, 5-thio-β-D-glucofuranose, methyl 2,3,4-tris-O-acetyl-1-thio-6-0-trityl-a-D-glucofuranose, 4-thio-β-D-galactopyranose, ethyl 3,4,6,7-tetra-O-acetyl-2-deoxy-1,5-dithio-a-D-glucoheptopyranoside, 2,5-anhydro-D-allononitrile, ribose, D-ribose, D-4-thioribose, L-ribose, and L-4-thioribose.

**57.** The siRNA conjugate according to claim 56, wherein at least one or each of the targeting group is galactose or N-acetylgalactosamine.

**58.** The siRNA conjugate according to any one of claims 42-57, wherein each of Rio, $R_{11}$, $R_{12}$, $R_{13}$, $R_{14}$ and $R_{15}$ is independently H, methyl or ethyl.

**59.** The siRNA conjugate according to any one of claims 43-58, wherein $R_2$ has both a site linking to the N on a nitrogenous backbone and a site linking to a P atom in $R_3$.

**60.** The siRNA conjugate according to claim 42-49, wherein, on $R_2$, the site linking to the N on the nitrogenous backbone forms an amide bond with the N, and the site linking to the P atom in $R_3$ forms a phosphoester bond with the P atom.

**61.** The siRNA conjugate according to any one of claims 42-60, wherein $R_2$ is selected from B5, B6, B5' or B6':

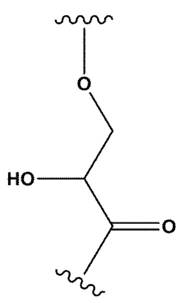
,
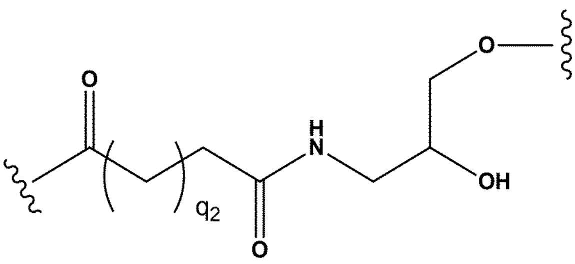
;

(B5) (B6),

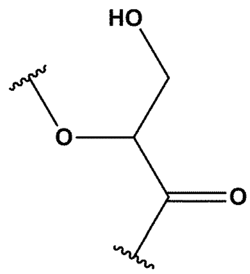

,

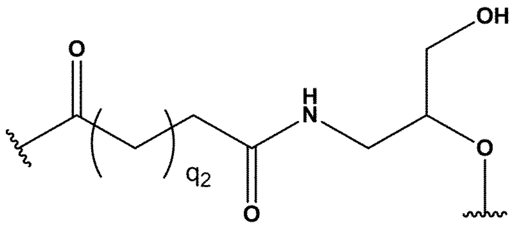

;

(B5') (B6'),

wherein, ∿∿∿ represents the site where the group is covalently linked, and $q_2$ is an integer of 1-10.

**62.** The siRNA conjugate according to claim 61, wherein $q_2$ is an integer of 1-5.

**63.** The siRNA conjugate according to any one of claims 35, and 42-62, wherein the siRNA conjugate has a structure as shown by Formula (403), (404), (405), (406), (407), (408), (409), (410), (411), (412), (413), (414), (415), (416), (417), (418), (419), (420), (421) or (422):

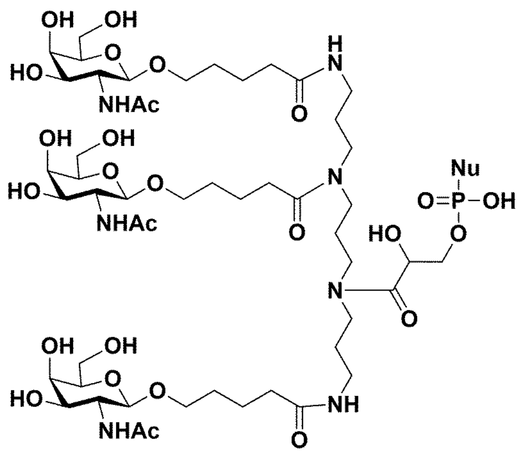

Formula (403)

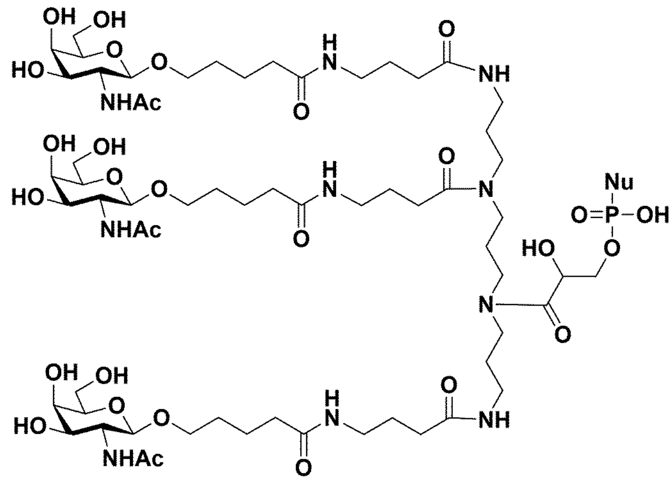

Formula (404)

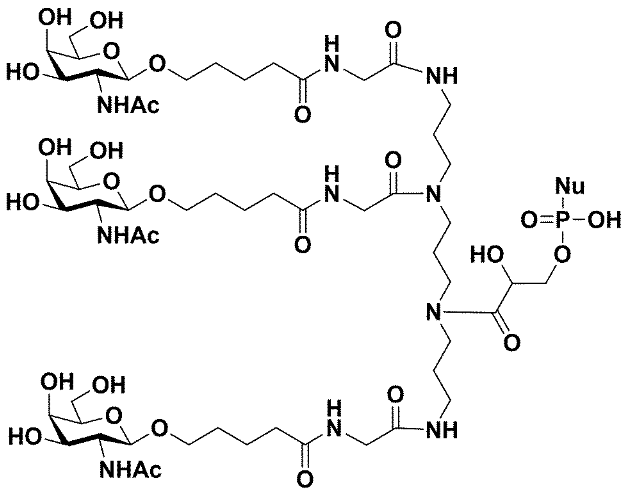

Formula (405)

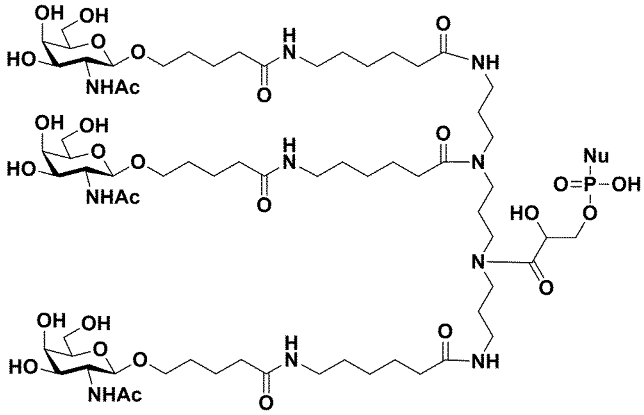

Formula (406)

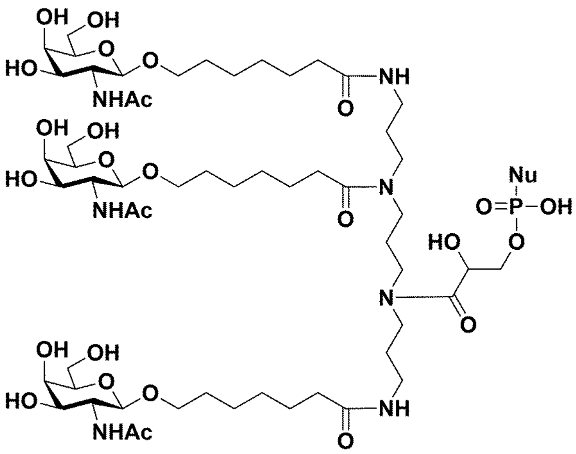

Formula (407)

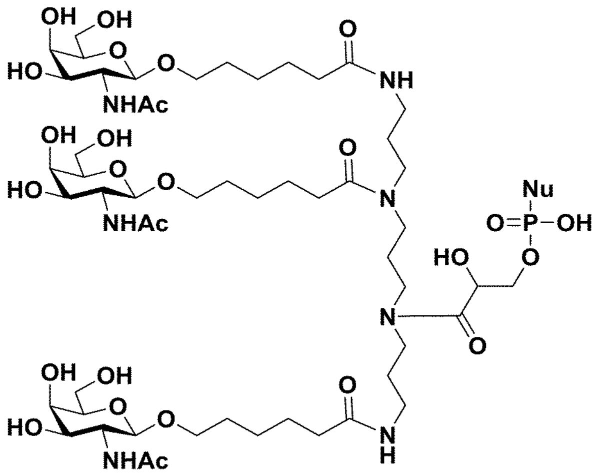

Formula (408)

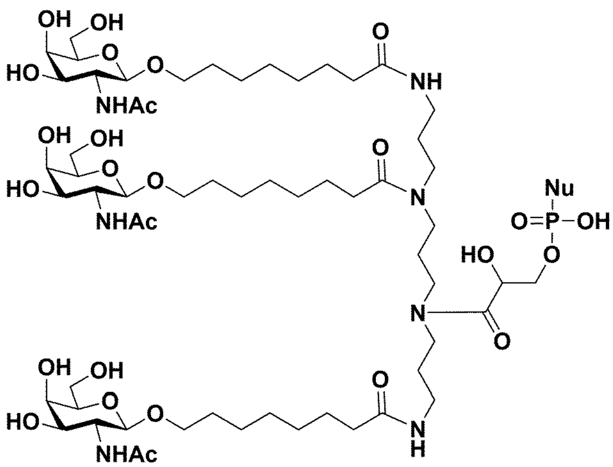

Formula (409)

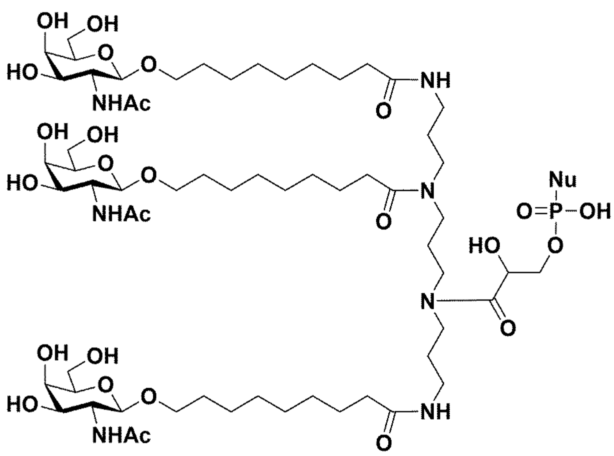

Formula (410)

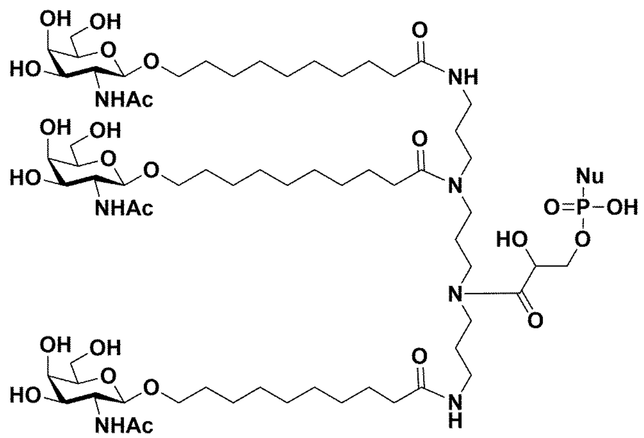

Formula (411)

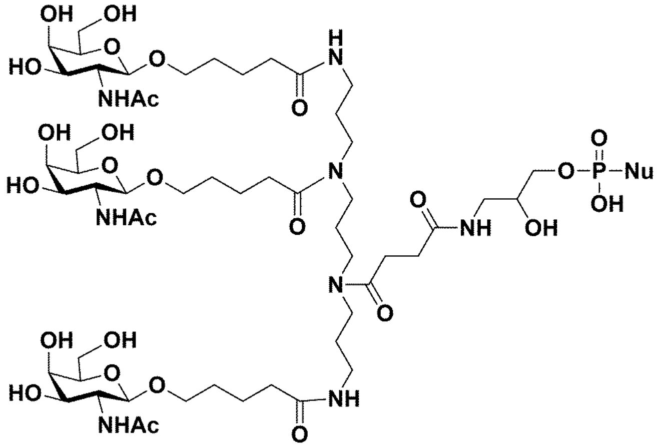

Formula (412)

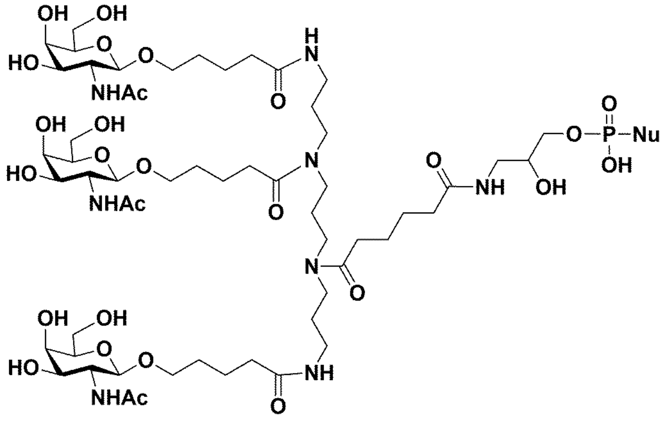

Formula (413)

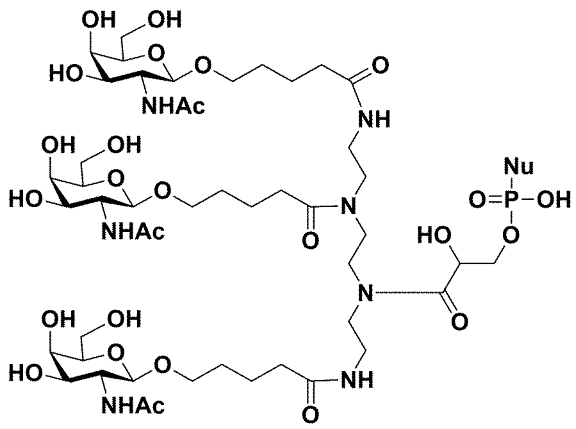

Formula (414)

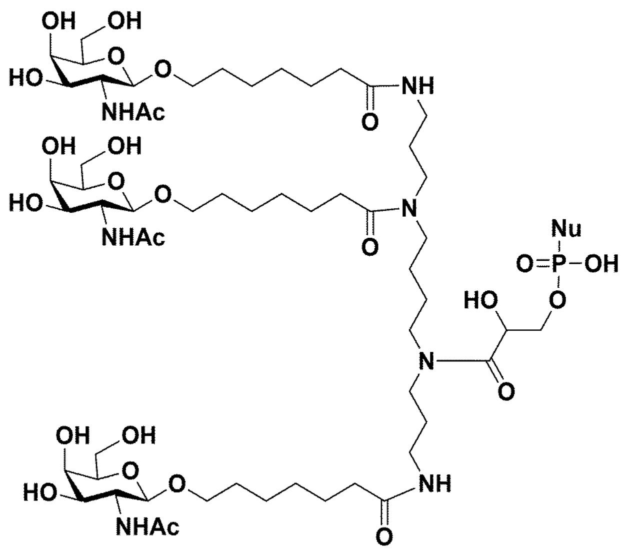

Formula (415)

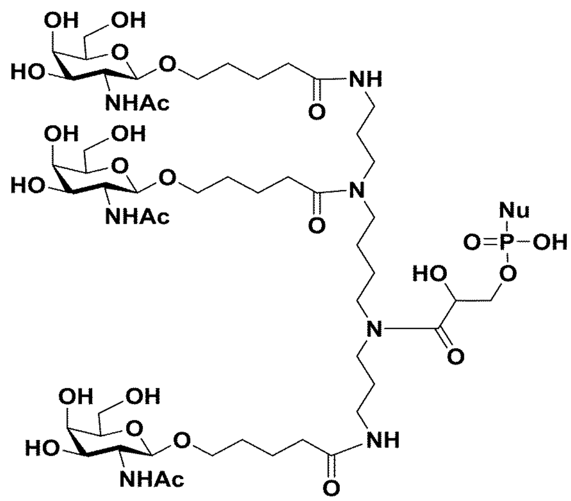

Formula (416)

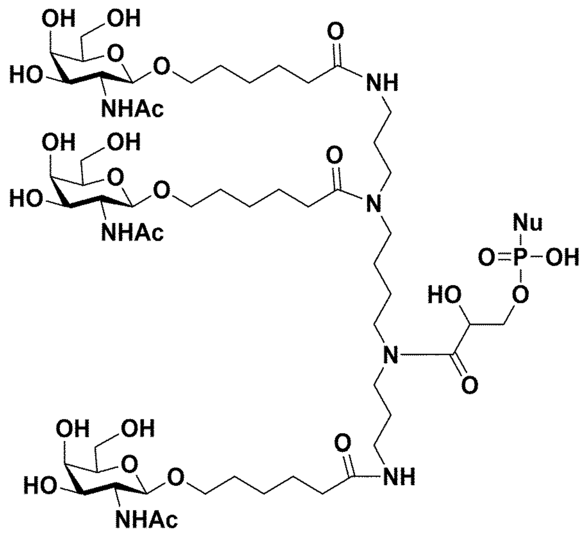

Formula (417)

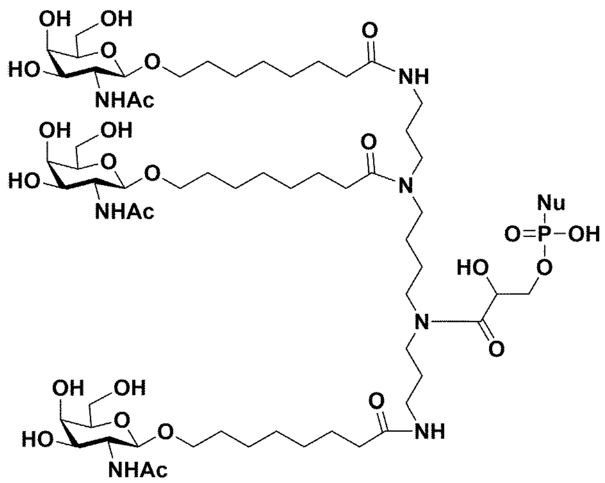

Formula (418)

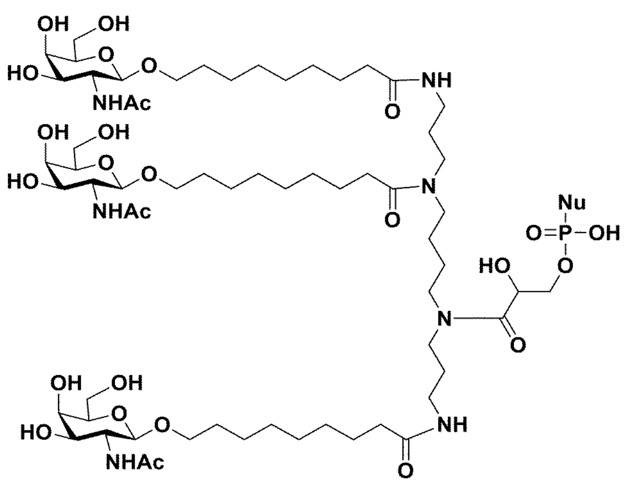

Formula (419)

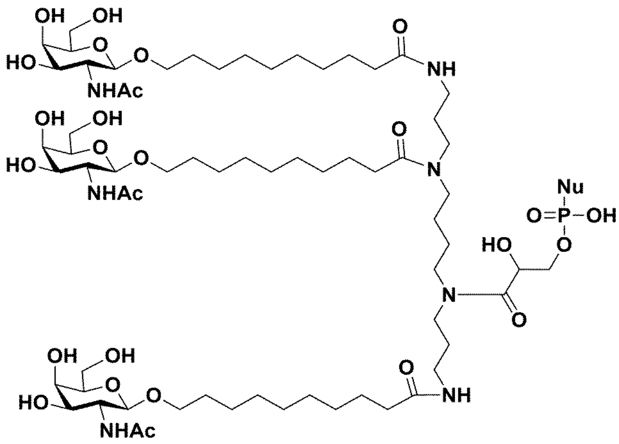

Formula (420)

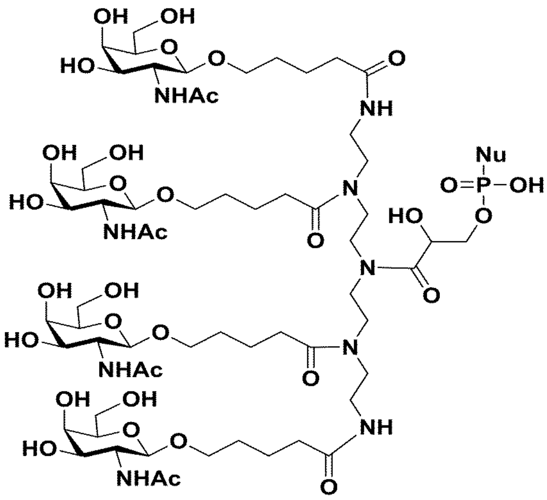

Formula (421)

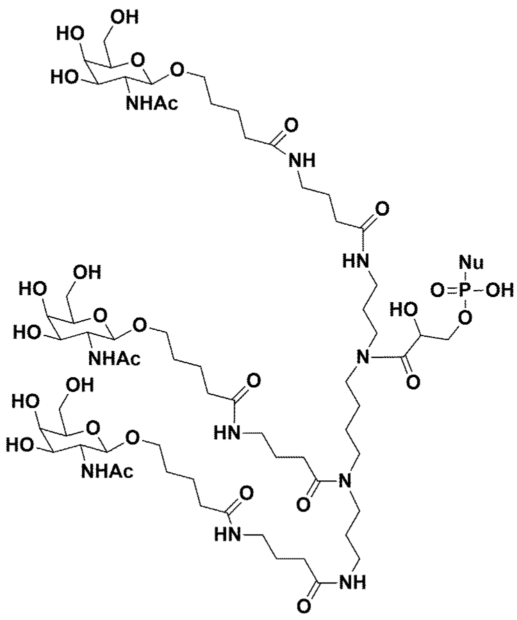

Formula (422).

**64.** The siRNA conjugate according to any one of claims 42-63, wherein the P atom in Formula A59 is linked to an end of the sense strand or antisense strand of the siRNA, and the end refers to the first four nucleotides closest to either terminal of the sense strand or antisense strand.

**65.** The siRNA conjugate according to claim 64, wherein the P atom in Formula A59 is linked to the terminal of the sense strand or antisense strand of the siRNA.

**66.** The siRNA conjugate according to claim 65, wherein the P atom in Formula A59 is linked to the 3' terminal of the sense strand of the siRNA.

**67.** The siRNA conjugate according to any one of claims 42-66, wherein the P atom in Formula A59 is linked to position 2', 3', or 5' of the nucleotide in the siRNA by a phosphodiester bond.

**68.** Use of the siRNA according to any one of claims 1-27, the pharmaceutical composition according to any one of claims 28-34, and/or the siRNA conjugate according to any one of claims 35-67 in the manufacture of a medicament for treating and/or preventing a PKK related disease.

**69.** The use according to claim 68, wherein the PKK related disease is an inflammatory disease or a thrombembolia disease.

**70.** The use according to claim 69, wherein the PKK related disease is hereditary angioedema HAE, edema, angioedema, swelling, angioedema of eyelid, eye edema, macular edema, cerebral edema, thrombus, thrombembolia, deep vein thrombosis, pulmonary embolism, myocardial infarction, apoplexy or infarction.

**71.** A method for treating, preventing or alleviating an inflammatory disease or a thrombembolia disease, wherein the method comprises administering an effective amount of the siRNA according to any one of claims 1-27, the pharmaceutical composition according to any one of claims 28-34, and/or the siRNA conjugate according to any one of claims 35-67 to a subject in need.

**72.** A method for inhibiting expression of a PKK gene in a cell, comprising contacting an effective amount of the siRNA according to any one of claims 1-27, the pharmaceutical composition according to any one of claims 28-34, and/or the siRNA conjugate according to any one of claims 35-67 to the cell.

**73.** A kit, wherein the kit comprises the siRNA according to any one of claims 1-27, the pharmaceutical composition according to any one of claims 28-34, and/or the siRNA conjugate according to any one of claims 35-67.

Figure 1

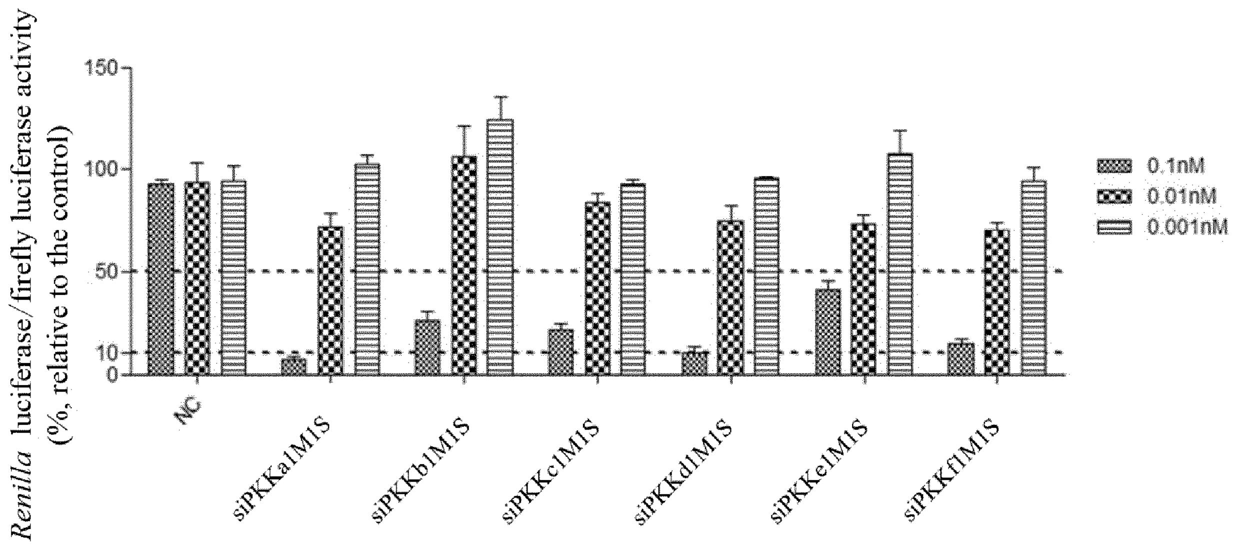


Figure 2

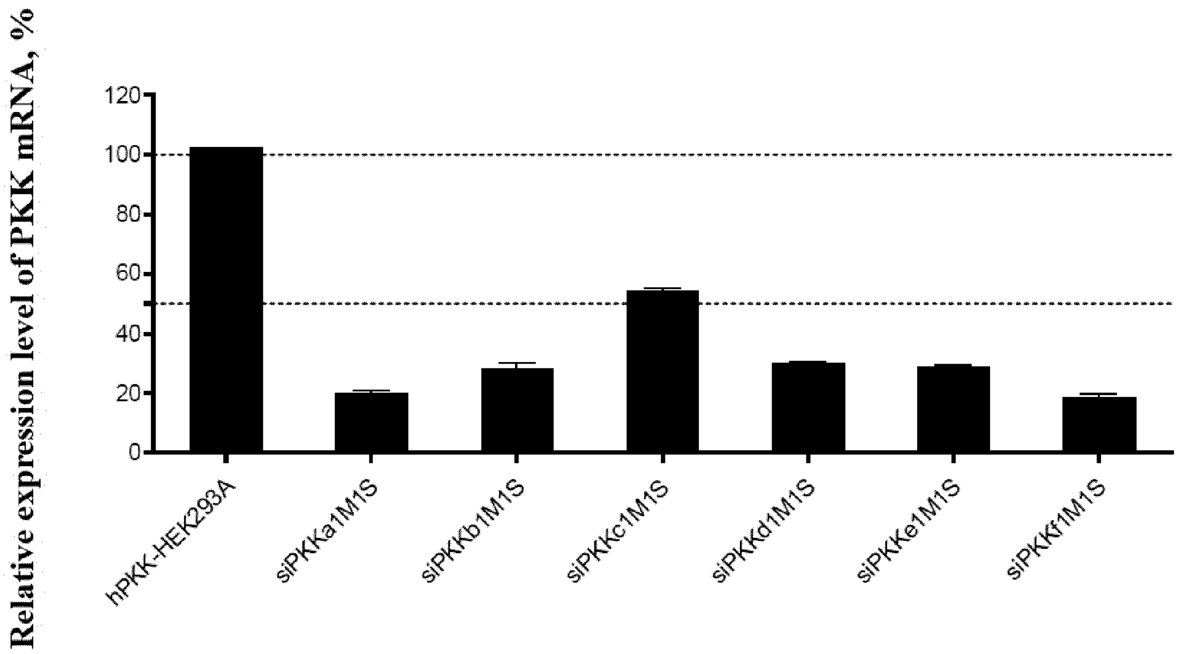

Figure 3

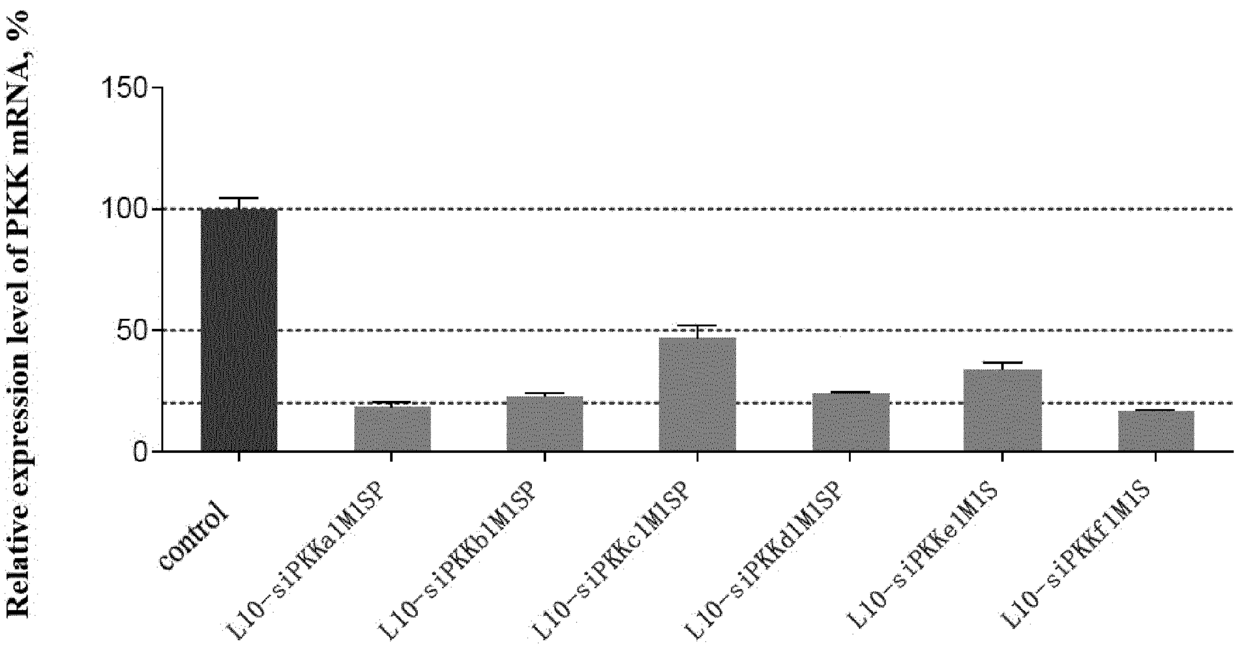
Relative expression level of PKK mRNA, %
150
100
50
0
control
L10-siPKKa1M1SP
L10-siPKKb1M1SP
L10-siPKKc1M1SP
L10-siPKKd1M1SP
L10-siPKKe1M1S
L10-siPKKf1M1S

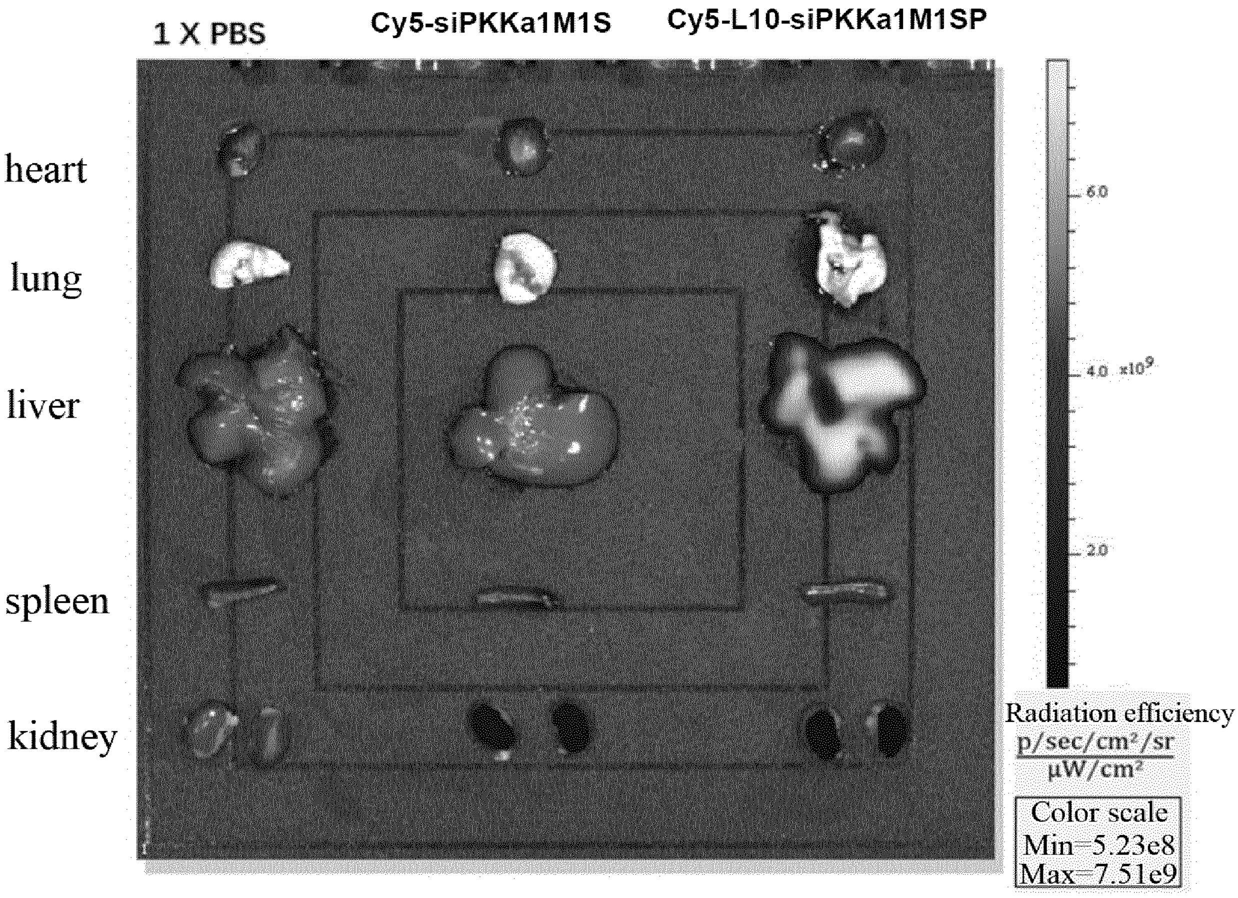
1 X PBS
Cy5-siPKKa1M1S
Cy5-L10-siPKKa1M1SP
heart
lung
liver
spleen
kidney
6.0
4.0 x10^9
2.0
Radiation efficiency
p/sec/cm²/sr
μW/cm²
Color scale
Min=5.23e8
Max=7.51e9

Figure 4A

Figure 4B

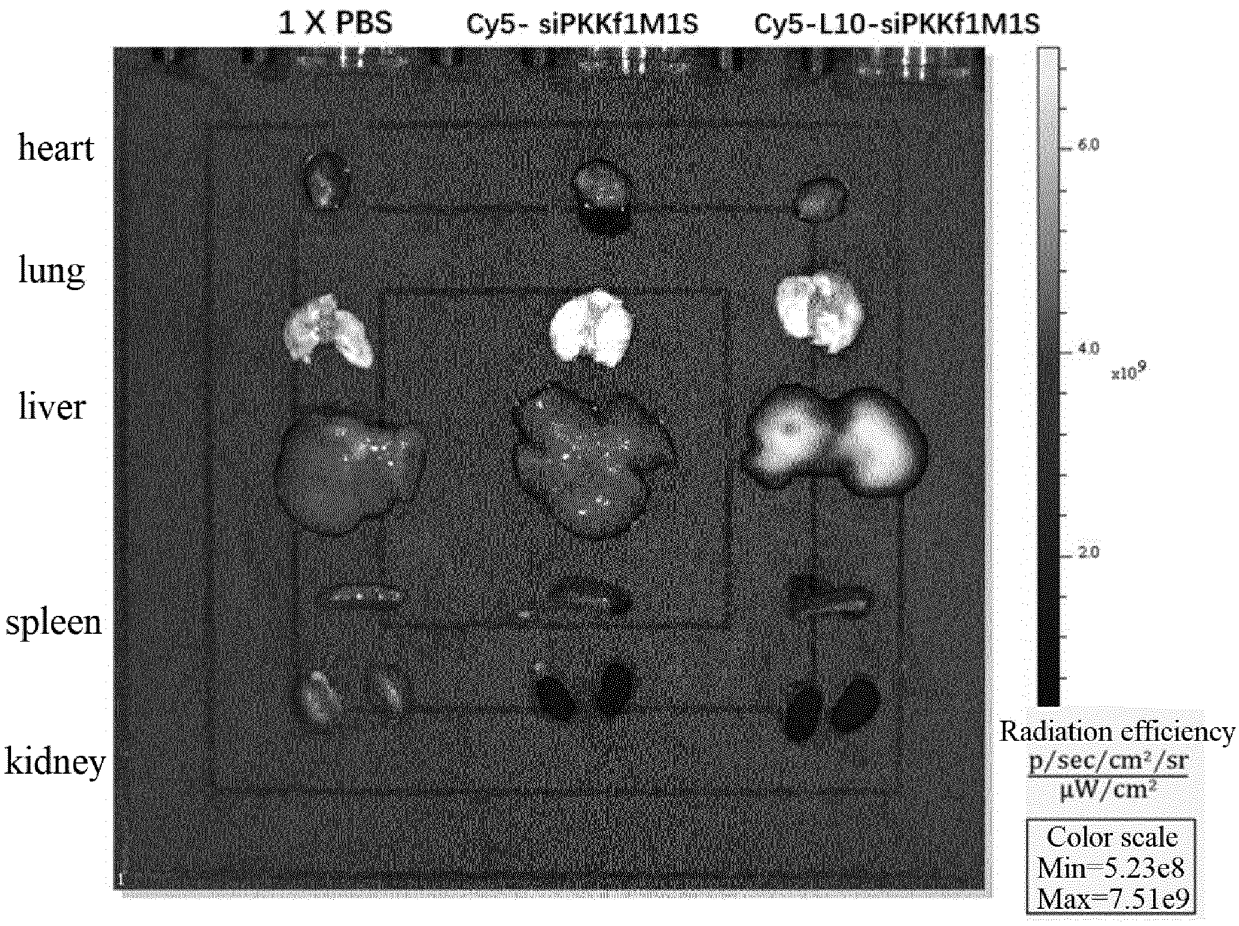
1 X PBS
Cy5- siPKKf1M1S
Cy5-L10-siPKKf1M1S
heart
lung
liver
spleen
kidney
6.0
4.0
x10$^9$
2.0
Radiation efficiency
p/sec/cm²/sr
μW/cm²
Color scale
Min=5.23e8
Max=7.51e9

**INTERNATIONAL SEARCH REPORT**

International application No.
**PCT/CN2020/091606**

**A. CLASSIFICATION OF SUBJECT MATTER**

C12N 15/113(2010.01)i; A61K 31/713(2006.01)i; A61K 47/28(2006.01)i; A61P 9/10(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, VEN(DWPI+SIPOABS), CNTXT, EPTXT, USTXT, WOTXT, CNKI, 百度学术搜索, ISI-WEB OF SCIENCE, PUBMED: 前激肽释放酶, 抑制, 小干扰, PKK, INHIBIT, SIRNA; Genbank, EMBL, 中国专利生物序列检索系统: 对SEQ ID NOs: 1-8, 61-68, 181-188, 241-248 Sequence search

**C. DOCUMENTS CONSIDERED TO BE RELEVANT**

| Category* | Citation of document, with indication, where appropriate, of the relevant passages | Relevant to claim No. |
|---|---|---|
| A | CN 105517556 A (IONIS PHARMACEUTICALS, INC.) 20 April 2016 (2016-04-20) see abstract, and claims | 1-70 and 73 (all in part) |
| A | CN 106132442 A (DYAX CORP.) 16 November 2016 (2016-11-16) see entire document | 1-70 and 73 (all in part) |
| A | CN 105713092 A (DYAX CORP.) 29 June 2016 (2016-06-29) see entire document | 1-70 and 73 (all in part) |
| A | US 10294477 B2 (IONIS PHARMACEUTICALS INC) 21 May 2019 (2019-05-21) see embodiment 137 | 1-70 and 73 (all in part) |
| A | EP 2213738 B1 (DHARMACON INC.) 10 October 2012 (2012-10-10) See sequence list | 1-70 and 73 (all in part) |
| A | WO 2016179342 A2 (ALNYLAM PHARMACEUTICALS INC) 10 November 2016 (2016-11-10) see entire document | 1-70 and 73 (all in part) |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 August 2020** | **02 September 2020** |

Name and mailing address of the ISA/CN
**China National Intellectual Property Administration (ISA/CN)**
**No. 6, Xitucheng Road, Jimenqiao Haidian District, Beijing 100088**
**China**
Facsimile No. **(86-10)62019451**

Authorized officer

Telephone No.

**INTERNATIONAL SEARCH REPORT**

International application No.
**PCT/CN2020/091606**

**C. DOCUMENTS CONSIDERED TO BE RELEVANT**

| Category* | Citation of document, with indication, where appropriate, of the relevant passages | Relevant to claim No. |
|---|---|---|
| A | 刘兆 等 (LIU, Zhao et al.). "人血浆前激肽释放酶的测定 (DETERMINATION OF HUMAN PLASMA PRE-KALLIKREIN)"<br>*中国医科大学学报 (Journal of China Medical University),*<br>Vol. 17, No. 6, 31 December 1988 (1988-12-31),<br>ISSN: 0238-4646,<br>pp. 432-436, see entire document | 1-70 and 73 (all in part) |

Form PCT/ISA/210 (second sheet) (January 2015)

**INTERNATIONAL SEARCH REPORT**

International application No.
**PCT/CN2020/091606**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/091606**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **71和72**
because they relate to subject matter not required to be searched by this Authority, namely:

[1] Disease treatment or prevention methods for the human or animal body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] Invention 1: the subject matter in claims 1-70 and 73 relating to SEQ ID NOs: 1-8.

[2] Invention 2: the subject matter in claims 1-70 and 73 relating to SEQ ID NOs: 61-68.

[3] Invention 3: the subject matter in claims 1-70 and 73 relating to SEQ ID NOs: 121-128.

[4] Invention 4: the subject matter in claims 1-70 and 73 relating to SEQ ID NOs: 181-188.

[5] Invention 5: the subject matter in claims 1-70 and 73 relating to SEQ ID NOs: 241-248.

[6] Invention 6: the subject matter in claims 1-70 and 73 relating to SEQ ID NOs: 301-308.

[7] The technical feature shared by inventions 1-6 are the siRNA structures. However, the general siRNA structures are well-known siRNA frameworks in the present field, and do not constitute a special technical feature. Therefore, inventions 1-6 do not share a same or corresponding special technical feature, and do not satisfy the criteria of PCT Rule 13.1, 13.2 and 13.3.

Form PCT/ISA/210 (continuation of first sheet) (January 2015)

**INTERNATIONAL SEARCH REPORT**

International application No.
**PCT/CN2020/091606**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **The subject matter in claims 1-70 and 73 relating to SEQ ID NOs: 1-8, 61-68, 181-188 and 241-248.**

4. ☐ No required additional search fees were timely paid by the applicant. Consequently, this international search report is restricted to the invention first mentioned in the claims; it is covered by claims Nos.:

**Remark on Protest**

☑ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2020/091606**

| Patent document cited in search report | Publication date (day/month/year) | Patent family member(s) | Publication date (day/month/year) |
|---|---|---|---|
| CN 105517556 A | 20 April 2016 | JP 2020072732 A | 14 May 2020 |
| | | RU 2016110848 A3 | 27 November 2018 |
| | | EP 3038627 A4 | 17 May 2017 |
| | | US 9670492 B2 | 06 June 2017 |
| | | RU 2016110848 A | 27 November 2018 |
| | | JP 6652922 B2 | 26 February 2020 |
| | | AU 2014312196 B2 | 18 April 2019 |
| | | US 2017314026 A1 | 02 November 2017 |
| | | IL 244047 D0 | 21 April 2016 |
| | | EP 3038627 B1 | 04 March 2020 |
| | | RU 2712559 C2 | 29 January 2020 |
| | | AU 2019204977 A1 | 01 August 2019 |
| | | US 10100310 B2 | 16 October 2018 |
| | | CA 2921839 A1 | 05 March 2015 |
| | | AU 2014312196 A1 | 03 March 2016 |
| | | HK 1225639 A1 | 15 September 2017 |
| | | US 2017002359 A1 | 05 January 2017 |
| | | US 2019233827 A1 | 01 August 2019 |
| | | AU 2014312196 C1 | 19 December 2019 |
| | | BR 112016004093 A2 | 17 October 2017 |
| | | JP 2016533751 A | 04 November 2016 |
| | | KR 20160048119 A | 03 May 2016 |
| | | WO 2015031679 A3 | 14 May 2015 |
| | | WO 2015031679 A2 | 05 March 2015 |
| | | EP 3038627 A2 | 06 July 2016 |
| | | MX 2016002587 A | 20 February 2017 |
| CN 106132442 A | 16 November 2016 | EP 3096798 A4 | 23 August 2017 |
| | | KR 20160122169 A | 21 October 2016 |
| | | WO 2015112578 A1 | 30 July 2015 |
| | | EA 201691470 A1 | 30 December 2016 |
| | | US 2017002094 A1 | 05 January 2017 |
| | | EP 3096798 A1 | 30 November 2016 |
| | | AU 2015209481 A1 | 25 August 2016 |
| | | BR 112016016916 A2 | 23 January 2018 |
| | | IL 246864 A | 31 December 2018 |
| | | IL 263669 D0 | 31 January 2019 |
| | | JP 2017503820 A | 02 February 2017 |
| | | JP 2020002171 A | 09 January 2020 |
| | | MX 2016009428 A | 13 April 2017 |
| | | CA 2937329 A1 | 30 July 2015 |
| | | BR 112016016916 A8 | 02 May 2018 |
| CN 105713092 A | 29 June 2016 | CN 103635489 B | 13 April 2016 |
| | | KR 20200047783 A | 07 May 2020 |
| | | US 8816055 B2 | 26 August 2014 |
| | | AU 2012204202 A1 | 11 July 2013 |
| | | KR 102011156 B1 | 16 August 2019 |
| | | KR 20140020248 A | 18 February 2014 |
| | | CN 105713092 B | 10 September 2019 |
| | | IL 227305 D0 | 30 September 2013 |
| | | EP 2661450 A4 | 23 April 2014 |

Form PCT/ISA/210 (patent family annex) (January 2015)

**INTERNATIONAL SEARCH REPORT**
**Information on patent family members**

International application No.
**PCT/CN2020/091606**

| Patent document cited in search report | | | Publication date (day/month/year) | Patent family member(s) | | | Publication date (day/month/year) |
|---|---|---|---|---|---|---|---|
| | | | | CA | 2823776 | A1 | 12 July 2012 |
| | | | | US | 2012201756 | A1 | 09 August 2012 |
| | | | | IL | 269565 | D0 | 28 November 2019 |
| | | | | EP | 2661450 | A1 | 13 November 2013 |
| | | | | KR | 102107695 | B1 | 07 May 2020 |
| | | | | JP | 2014506257 | A | 13 March 2014 |
| | | | | US | 2020017602 | A1 | 16 January 2020 |
| | | | | US | 9266964 | B2 | 23 February 2016 |
| | | | | WO | 2012094587 | A1 | 12 July 2012 |
| | | | | US | 10370453 | B2 | 06 August 2019 |
| | | | | US | 2016102150 | A1 | 14 April 2016 |
| | | | | US | 2014302048 | A1 | 09 October 2014 |
| | | | | KR | 20190095965 | A | 16 August 2019 |
| | | | | CN | 103635489 | A | 12 March 2014 |
| US | 10294477 | B2 | 21 May 2019 | RU | 2016146818 | A | 04 June 2018 |
| | | | | JP | 2017518045 | A | 06 July 2017 |
| | | | | EP | 3137091 | A4 | 27 December 2017 |
| | | | | AU | 2015252917 | A1 | 29 September 2016 |
| | | | | AU | 2019284028 | A1 | 23 January 2020 |
| | | | | WO | 2015168532 | A3 | 14 April 2016 |
| | | | | RU | 2016146818 | A3 | 26 October 2018 |
| | | | | WO | 2015168532 | A2 | 05 November 2015 |
| | | | | BR | 112016022855 | A2 | 17 October 2017 |
| | | | | JP | 6665111 | B2 | 13 March 2020 |
| | | | | RU | 2703411 | C2 | 16 October 2019 |
| | | | | EP | 3137091 | A2 | 08 March 2017 |
| | | | | IL | 247862 | D0 | 30 November 2016 |
| | | | | US | 2020056185 | A1 | 20 February 2020 |
| | | | | CA | 2943705 | A1 | 05 November 2015 |
| | | | | IL | 273058 | D0 | 30 April 2020 |
| | | | | KR | 20160147891 | A | 23 December 2016 |
| | | | | CN | 106232125 | A | 14 December 2016 |
| | | | | AU | 2015252917 | B2 | 26 September 2019 |
| | | | | MX | 2016014140 | A | 15 September 2017 |
| | | | | US | 2017183661 | A1 | 29 June 2017 |
| EP | 2213738 | B1 | 10 October 2012 | US | 2009298176 | A1 | 03 December 2009 |
| | | | | US | 2007207974 | A1 | 06 September 2007 |
| | | | | US | 2010075869 | A1 | 25 March 2010 |
| | | | | US | 2008300395 | A1 | 04 December 2008 |
| | | | | US | 7579457 | B2 | 25 August 2009 |
| | | | | US | 2010291681 | A1 | 18 November 2010 |
| | | | | US | 2007088155 | A1 | 19 April 2007 |
| | | | | US | 7642349 | B2 | 05 January 2010 |
| | | | | EP | 1560931 | A2 | 10 August 2005 |
| | | | | US | 7745611 | B2 | 29 June 2010 |
| | | | | US | 2008139798 | A1 | 12 June 2008 |
| | | | | US | 2007088154 | A1 | 19 April 2007 |
| | | | | ES | 2440284 | T3 | 28 January 2014 |
| | | | | US | 8008474 | B2 | 30 August 2011 |
| | | | | US | 2010004142 | A1 | 07 January 2010 |

Form PCT/ISA/210 (patent family annex) (January 2015)

**INTERNATIONAL SEARCH REPORT**
**Information on patent family members**

International application No.
**PCT/CN2020/091606**

| Patent document cited in search report | Publication date (day/month/year) | Patent family member(s) | Publication date (day/month/year) |
|---|---|---|---|
| | | EP 2284266 B1 | 06 November 2013 |
| | | EP 2278005 A2 | 26 January 2011 |
| | | DK 2284266 T3 | 13 January 2014 |
| | | US 2007088153 A1 | 19 April 2007 |
| | | US 7592444 B2 | 22 September 2009 |
| | | EP 2278005 A3 | 23 May 2012 |
| | | US 2010087335 A1 | 08 April 2010 |
| | | US 2007141611 A1 | 21 June 2007 |
| | | US 2007088152 A1 | 19 April 2007 |
| | | US 7674896 B2 | 09 March 2010 |
| | | US 2007031844 A1 | 08 February 2007 |
| | | US 2009082556 A1 | 26 March 2009 |
| | | US 7696344 B2 | 13 April 2010 |
| | | US 7893247 B2 | 22 February 2011 |
| | | AU 2003295600 A8 | 15 June 2004 |
| | | US 2008221317 A1 | 11 September 2008 |
| | | US 2008091004 A1 | 17 April 2008 |
| | | US 2009163702 A1 | 25 June 2009 |
| | | US 2005245475 A1 | 03 November 2005 |
| | | US 7820809 B2 | 26 October 2010 |
| | | US 8093370 B2 | 10 January 2012 |
| | | US 2007093653 A1 | 26 April 2007 |
| | | US 7507811 B2 | 24 March 2009 |
| | | US 2019345573 A1 | 14 November 2019 |
| | | US 2008091003 A1 | 17 April 2008 |
| | | US 2009163701 A1 | 25 June 2009 |
| | | US 2008114162 A1 | 15 May 2008 |
| | | US 2008306015 A1 | 11 December 2008 |
| | | US 2005246794 A1 | 03 November 2005 |
| | | US 2009088563 A1 | 02 April 2009 |
| | | PT 2284266 E | 17 December 2013 |
| | | US 7691997 B2 | 06 April 2010 |
| | | US 7985854 B2 | 26 July 2011 |
| | | US 8030474 B2 | 04 October 2011 |
| WO 2016179342 A2 | 10 November 2016 | CA 2984636 A1 | 10 November 2016 |
| | | US 2020131513 A1 | 30 April 2020 |
| | | BR 112017021967 A2 | 31 July 2018 |
| | | EP 3292201 A2 | 14 March 2018 |
| | | EA 201792444 A1 | 29 June 2018 |
| | | WO 2016179342 A9 | 08 December 2016 |
| | | AU 2016257996 A1 | 26 October 2017 |
| | | JP 6695902 B2 | 20 May 2020 |
| | | UY 36671 A | 30 December 2016 |
| | | TW 201704472 A | 01 February 2017 |
| | | JP 2018519797 A | 26 July 2018 |
| | | US 2018100150 A1 | 12 April 2018 |
| | | CN 108271386 A | 10 July 2018 |
| | | WO 2016179342 A3 | 12 January 2017 |
| | | KR 20180002688 A | 08 January 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A
- WO 2009082607 A2
- CN 105378082 A
- WO 2015006740 A2
- WO 2014025805 A1
- US 8106022 B2


### Non-patent literature cited in the description


- **BEAUCAGE et al.** Tetrahedron. 1992, vol. 48, 2223-2311
- **GREENE ; WUTS.** Protective Groups in Organic Synthesis. John Wiley & Sons, 1991
- **J.K. WATTS ; G. F. DELEAVEY ; M. J.DAMHA.** Chemically Modified siRNA: tools and applications. *Drug Discov Today,* 2008, (19-20), 842-55
- **ANASTASIA KHVOROVA ; JONATHAN K. WATTS.** The chemical evolution of oligonucleotide therapies of clinical utility. *Nature Biotechnology,* 2017, vol. 35 (3), 238-48
- **MUTHIAH MANOHARAN.** siRNA conjugates carrying sequentially assembled trivalent N-acetylgalactosamine linked through nucleosides elicit robust gene silencing in vivo in hepatocytes. *ACS Chemical biology,* 2015, vol. 10 (5), 1181-7
- *ChemBioChem,* 2015, vol. 16, 903-908
- *J. Am. Chem. Soc.,* 2014, vol. 136, 16958-16961
- Molecular Cloning. Cold Spring Harbor Laboratory Press, 1989
- *Nucleic Acids Research,* 2008, vol. 36 (7), 2136-2151